# EUROPEAN PATENT SPECIFICATION

(11) **EP 2 344 531 B1**
(45) Date of publication and mention of the grant of the patent: **10.12.2014**
(21) Application number: 08876414.7
(22) Date of filing: 15.10.2008
(51) Int. Cl.: A61K 8/64, C12N 1/21, A61K 38/55, A61P 17/00, A61P 17/06, A61P 17/14, A61Q 19/00, A61Q 7/00, C07K 14/81, C12N 15/62

(54) **MODIFIED VARIANT BOWMAN BIRK PROTEASE INHIBITORS**
MODIFIZIERTE VARIANTEN VON BOWMAN-BIRK-PROTEASE-INHIBITOREN
VARIANTS MODIFIÉS D'INHIBITEURS DE PROTÉASES DE TYPE BOWMAN-BIRK

(43) Date of publication of application: 20.07.2011
(73) Proprietor: Danisco US Inc., Palo Alto, CA 94304 (US)
(72) Inventor: AMIN, Neelam, S., Palo Alto, CA 94304 (US); COLLIER, Katherine, D., Palo Alto, CA 94304 (US); ESTABROOK, Melodie, Palo Alto, CA 94304 (US); ESTELL, David, A., Palo Alto, CA 94304 (US); FOX, Bryan, Palo Alto, CA 94304 (US); POWER, Scott, D., Palo Alto, CA 94304 (US); SCHMIDT, Brian, F., Palo Alto, CA 94304 (US); VOGTENTANZ, Gudrun, Palo Alto, CA 94304 (US)
(74) Representative: Forrest, Graham Robert
(86) International application number: PCT/US2008/079951
(87) International publication number: WO 2010/044786

(56) References cited:
- WO-A-00/05406
- WO-A-2005/046709
- WO-A-2005/046710
- WO-A-2005/047302
- WO-A-2005/047314
- WO-A-2005/047511
- WO-A-2006/121610
- WO-A-2007/085846
- DATABASE UniProt [Online] 21 July 1986 (1986-07-21), "RecName: Full=Bowman-Birk type proteinase inhibitor;" XP002533200 retrieved from EBI accession no. UNIPROT:P01056 Database accession no. P01056

## Description

### 1. FIELD OF THE INVENTION

The present invention relates to modified variant Bowman Birk Protease Inhibitor proteins (BBPIs) that comprise peptides that bind target proteins, and that are further modified to have greater protease inhibitory activity and/or be produced at greater yields than the unmodified BBPIs. The invention encompasses polynucleotide constructs and expression vectors containing polynucleotide sequences that encode the modified variant BBPIs, the transformed host cells that express and produce the modified variant BBPIs, the modified variant BBPI proteins, the compositions comprising the modified variant BBPIs, and the methods for making and using the modified variant BBPIs in personal care.

### 2. BACKGROUND OF THE INVENTION

Proteases are involved in a wide variety of biological processes. Disruption of the balance between proteases and protease inhibitors is often associated with pathologic tissue destruction.

Various studies have focused on the role of proteases in tissue injury, and it is thought that the balance between proteinases and proteinase inhibitors is a major determinant in maintaining tissue integrity. Serine proteinases from inflammatory cells, including neutrophils, are implicated in various inflammatory disorders, such as pulmonary emphysema, arthritis, atopic dermatitis and psoriasis. These and other inflammatory conditions are often associated with dysregulated levels of cytokines.

Proteases also degrade the vascular basement membrane and participate in the remodeling of the extracellular matrix to facilitate cell migration and invasion to promote tumor angiogenesis. Proteases release angiogenic growth factors bound to the extracellular matrix and to generate chemotactically active fragments derived from extracellular matrix components, which in turn exert chemotactic and mitogenic, modulatory activates on endothelial cells, smooth muscle cells and fibroblasts to participate in angiogenic processes. The list of protein factors angiogenically active in vivo includes fibroblast growth factors, Angiogenin, Angiopoietin-1, EGF, HGF, NPY, VEGF, TNF-alpha, TGF-beta, PD-ECGF, PDGF, IGF, IL8, and Growth hormone. Risks associated with current cytokine blocking agents include serious infections, anaphylaxis, and lupus-like syndrome. In addition, there is observed loss of clinical benefit after the drugs are stopped, and a small proportion of patients develop antibodies to the biological agents, which is likely to limit their efficacy with repeated use.

Synthetic and natural protease inhibitors have been shown to inhibit tumor promotion *in vivo* and *in vitro.* Previous research investigations have indicated that certain protease inhibitors belonging to a family of structurally-related proteins classified as serine protease inhibitors or SERPINS, are known to inhibit several proteases including trypsin, cathepsin G, thrombin, tissue kallikrein, as well as neutrophil elastase. The SERPINS are extremely effective at preventing/suppressing carcinogen-induced transformation *in vitro* and carcinogenesis in animal model systems. Systemic delivery of purified protease inhibitors reduces joint inflammation and cartilage and bone destruction as well.

Topical administration of protease inhibitors finds use in such conditions as atopic dermatitis, a common form of inflammation of the skin, which may be localized to a few patches or involve large portions of the body. The depigmenting activity of protease inhibitors and their capability to prevent ultraviolet-induced pigmentation have been demonstrated both *in vitro* and *in vivo.* Paine et al., Journal of Investigative Dermatology 116:587-595 [2001]. Also, protease inhibitors have been found to help wound healing (http://www.sciencedaily.com/releases/2000/10/001002071718.htm). Secretory leukocyte protease inhibitor was demonstrated to reverse the tissue destruction and speed the wound healing process when applied topically. In addition, serine protease inhibitors can also help to reduce pain in lupus erythematosus patients (See US Patent No. 6537968).

Naturally occurring protease inhibitors can be found in a variety of foods such as cereal grains (oats, barley, and maize), Brussels sprouts, onion, beetroot, wheat, finger millet, and peanuts. One source of interest is the soybean. The average level in soybeans is around 1.4 percent and 0.6 percent for Kunitz and Bowman-Birk respectively, two of the most important protease inhibitors. These low levels make it impractical to isolate the natural protease inhibitor for clinical applications.

WO2006/121610, WO2005/046709, WO2005/047511, WO2005/047314, WO2005/046710, WO2005/047302 and WO2007/085046 describe compositions containing pepides having affinity for a target molecule (such as VEGF, FGF-5 and TGF-β) supported in a scaffold protein such as a BBPI.

There is a need for a method to produce large quantities of protease inhibitors that have desired characteristics of protein therapeutics, and for compositions that effectively deliver the protease inhibitor in a usable form.

The compositions and methods according to the invention fulfill some of the above needs and in particular offer an advantage in providing protease inhibitors that specifically target the activity of cytokines involved in pathologic and non-pathologic processes.

### 3. SUMMARY OF THE INVENTION

The present invention relates to modified variant Bowman Birk Protease Inhibitor proteins (BBPIs) that comprise peptides that bind target proteins, and that are further modified to have greater protease inhibitory activity and/or be produced at greater yields than the unmodified BBPIs. The invention encompasses polynucleotide constructs and expression vectors containing polynucleotide sequences that encode the modified variant BBPIs, the transformed host cells that express and produce the modified variant BBPIs, the modified variant BBPI proteins, the compositions comprising the modified variant BBPIs, and the methods for making and using the modified variant BBPIs in personal care.

The invention provides an isolated modified Bowman Birk Protease Inhibitor (BBPI) comprising the sequence: 
DDESSKPCCDQCACTKSNPPQCRCSDMRLNSCHSACKSCACYNLYGWTCFCVDI
TDFCYEPCKPSEDDKEN (SEQ ID NO: 187)
 wherein residues 1 to 41 and 49 to 66 of SEQ ID NO: 187 represent a backbone and residues 42 to 48 of SEQ ID NO: 187 represent a binding peptide;
wherein said modified BBPI further comprises at least one substitution at a position chosen from positions equivalent to 1, 4, 5, 11, 13, 18, 25, 27, 29, 31, 38, 40, 50, 52, 55, and 65 of SEQ ID NO:187;
wherein, if a substitution is present at position 40, that substitution is chosen from 40H, 40K, 40Q, 40R, and 40Y;
wherein said modified BBPI has increased trypsin inhibitory activity and/or production yield compared to the unmodified BBPI lacking said at least one substitution;
and wherein the binding peptide is optionally replaced by an alternative binding peptide which is a VEGF-binding peptide, FGF-5-binding peptide, TGFβ-binding peptide or TNFα-binding peptide.

The binding peptide may be:
a VEGF binding peptide chosen from KYYLYWW (SEQ ID NO:458), TLWKSYW (SEQ ID NO:459), DLYWW (SEQ ID NO:460),SKHSQIT (SEQ ID NO:468) KTNPSGS (SEQ ID NO:469) RPTGHSL (SEQ ID NO:470), KHSAKAE (SEQ ID NO:471) KPSSASS (SEQ ID NO:472), PVTKRVH (SEQ ID NO:473), TLHWWVT (SEQ ID NO:492), PYKASFY (SEQ ID NO:493), PLRTSHT (SEQ ID NO:494), EATPROT (SEQ ID NO:495), NPLHTLS (SEQ ID NO:496), KHERIWS (SEQ ID NO:497), ATNPPPM (SEQ ID NO:498), STTSPNM (SEQ ID NO:499), ADRSFRY (SEQ ID NO:500), PKADSKQ (SEQ ID NO:501), PNQSHLH (SEQ ID NO:502), SGSETWM (SEQ ID NO:503), ALSAPYS (SEQ ID NO:504), KMPTSKV (SEQ ID NO:505), ITPKRPY (SEQ ID NO:506), KWIVSET (SEQ ID NO:507), PNANAPS (SEQ ID NO:508), NVQSLPL (SEQ ID NO:509), TLWPTFW (SEQ ID NO:510), NLWPHFW (SEQ ID NO:511), SLWPAFW (SEQ ID NO:512), SLWPHFW (SEQ ID NO:513), APWNSHI (SEQ ID NO:514), APWNLHI (SEQ ID NO:515), LPSWHLR (SEQ ID NO:516), PTILEWY (SEQ ID NO:517), TLYPQFW (SEQ ID NO:518), HLAPSAV (SEQ ID NO:519), KYYLSWW (SEQ ID NO:520), WYTLYKW (SEQ ID NO:521), TYRLYWW (SEQ ID NO:522), RYSLYYW (SEQ ID NO:523), YYLYYWK (SEQ ID NO:524), NYQLYGW (SEQ ID NO:525), TKWPSYW (SEQ ID NO:226), TLWKSYW (SEQ ID NO:527), PLWPSYW (SEQ ID NO:528), RLWPSYW (SEQ ID NO:529), TLWPKYW (SEQ ID NO:530), KYDLYWW (SEQ ID NO;531), RYDLYWW (SEQ ID NO:532), DYRLYWW (SEQ ID NO:533), DYKLYWW (SEQ ID NO:534), EYKLYWW (SEQ ID NO:535), and RYPLYWW (SEQ ID NO:536);
an FGF-5 binding peptide chosen from TNIDSTP(SEQ ID NO:544), HLQTTET (SEQ ID NO:545), SLNNLTV (SEQ ID NO:546), TNIDSTP (SEQ ID NO:547), TNIDSTP (SEQ ID NO:548), LRILANK (SEQ ID NO:549), LLTPTLN (SEQ ID NO:550), ALPTHSN (SEQ ID NO:551), TNIDSTP (SEQ ID NO:552), LCRRFEN (SEQ ID NO:553), TNIDSTP (SEQ ID NO:554), TNIDSTP (SEQ ID NO:555), HLQTTET (SEQ ID NO:556), PLGLCPP (SEQ ID NO:557), GYFIPSI (SEQ ID NO:558), TKIDSTP (SEQ ID NO:559), HLQTTET (SEQ ID NO:560), WNIDSTP (SEQ ID NO:561), TWIDWTP (SEQ ID NO:562), RTQPYPL (SEQ ID NO:670) and TWIDSTP (SEQ ID NO:671);
a TGFβ binding peptide chosen from QSACIVYYVGRKPKVECASSD (SEQ ID NO:566), OSACILYYIGKTPKIECASSD (SEQ ID NO:567), OSACILYYVGRTPKVECASSD (SEQ ID NO:568), KHNVRLL (SEQ ID NO:570), NDTPSYF (SEQ ID NO:571), AKLYAGS (SEQ ID NO:572), RGPAHSL (SEQ ID NO:573), NSLAERR (SEQ ID NO:574), HPLASPH (SEQ ID NO:575), QPWNKLK (SEQ ID NO:576), PTKPAQQ (SEQ ID NO:578), PSLNRPQ (SEQ ID NO:579), HHARQEW (SEQ ID NO:580), RHHTPGP (SEQ ID NO:581), ASAINPH (SEQ ID NO:582), PENINVLP (SEQ ID NO;672) and KHNVDWL (SEQ ID NO:673); or
a TNFα binding peptide chosen from RYWQDIP (T1; SEQ ID NO:474), APEPILA (T2; SEQ ID NO:475), DMIMVSI (T3; SEQ ID NO:476), WTPKPTQ (SEQ ID NO:583), ATFPNQS (SEQ ID NO:584), ASTVGGL (SEQ ID NO:585), TMLPYRP (SEQ ID NO:586), AWHSPSV (SEQ ID NO:587), TQSFSS (SEQ ID NO:588), THKNTLR (SEQ ID NO:589), GQTHFHV (SEQ ID NO:590), LPILTQT (SEQ ID NO:591), SILPVSH (SEQ ID NO:592), SQPIPI (SEQ ID NO:593), and QPLRKLP (SEQ ID NO:594).

In some embodiments, said substituted amino acid at position 1 is chosen from A and C;
said at least one substitution at position 4 is V;
said at least one substitution at position 5 is chosen from P and A;
said at least one substitution at position 11 is 11G;
said at least one substitution at position 13 is chosen from 13Y, 13I, 13F, 13M, 13L, 13V, 13K, and 13R;
said at least one substitution at position 18 is chosen from 18I and 18V and 18L;
said at least one substitution at position 25 is chosen from 25K, 25N, 25W, 25I, 25A and 25R;
said at least one substitution at position 27 is chosen from 27H, 27K, 27V, 27A, and 27Q;
said at least one substitution at position 29 is chosen from 29R, 29K, and 29P;
said at least one substitution at position 31 is chosen from 31Q, 31 H, 31E, 31 A, 31 R, 31W,31Kand31T;
said at least one substitution at position 38 is chosen from 38N, 38K, and 38R;
said at least one substitution at position 50 is chosen from 50R, 50Q, 50K, 50T, 50V, 50M, and 50S;
said at least one substitution at position 52 is chosen from 52K, 52T, 52R, 52Q, 52L, 52H, 52A, 52M, 52S and 52E;
said at least one substitution at position 55 is 55M; or
said at least one substitution at position 65 is chosen from 65E, 65Q, and 65D.

The isolated modified BBPI may further comprise an insert of SEQ ID NO: 389.

The isolated modified variant BBPI may be expressed as a fusion protein comprising a catalytic domain from a cellulase, cutinase or disulfide isomerase. For example, said modified variant BBPI may be expressed as the fusion protein of SEQ ID NO:195.

The modified variant BBPI may comprise a combination of two amino acid substitutions at amino acid positions equivalent to positions 50 and 52 of SEQ ID NO:187. For example, the modified variant BBPI may have the amino acid sequence of SEQ ID NO:595.

The modified variant BBPI may comprise a combination of three amino acid substitutions chosen from amino acid substitutions at positions equivalent to positions 25, 29, 40, 50 and 52 of SEQ ID NO:187. For example, the combination of three amino acids may be chosen from combinations 25L-50T-52A, 29P-50T-52A, 40K-50T-52A. For example, the modified variant BBPI may have an amino acid sequence chosen from SEQ ID NOS:603, 607 and 609.

The modified variant BBPI may comprise a combination of four amino acid substitutions chosen from amino acid substitutions at positions equivalent to positions 13, 29, 50 and 52 of SEQ ID NO:187. For example, the combination of four amino acids may be chosen from combinations 13I-25L-50T-52A, 13I-29P-50T-52A, 13I-A0K-50T-52A, 25L-29P-50T-52A, 25L-40K-50T-52A, and 29P-40K-50T-52A. For example, the modified variant BBPI may have an amino acid sequence chosen from SEQ ID NOS:596, 600, 602, 604, 606 and 608.

The modified variant BBPI may comprise a combination of five amino acid substitutions chosen from amino acid substitutions at positions equivalent to positions 13, 25, 29, 40, 50, and 52 of SEQ ID NO:187. For example, the combination of five amino acids may be chosen from combinations 13I-25L-29P-50T-52A, 13I-25L-40K-50T-52A, A131-29P-40K-50T-52A, 25L-29P-40K-50T-52A, 131-29P-40K-50K-52A, and 13I-29P-40K-50T-52T. For example, the modified variant BBPI may have an amino acid sequence chosen from SEQ ID NOS:432, 434, 443, 445, 446, 597, 599. 601, 605, 615, 620, 624, and 625.

The modified variant BBPI may comprise a combination of six amino acid substitutions chosen from amino acid substitutions at positions equivalent to positions 1, 4, 5, 11, 13, 25, 29, 40, 50 and 52 of SEQ ID NO:187. For example, the combination of six amino acids is chosen from combinations 13I-25L-29P-40K-50T-52A, 1C-13I-29P-40K-50T-52A, 4V-13I-29P-40K-50T-52A, 5P-13I-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A, and 13I-29P-40K-50T-52A-65E. For example, the modified variant BBPI may have an amino acid sequence chosen from SEQ ID NOS:598, 611, 612, 613, 614, 616, 619, 621, 622, 623, and 626.

The modified variant BBPI may comprise a combination of seven amino acid substitutions chosen from amino acid substitutions at positions equivalent to positions 13, 25, 29, 31, 40, 50 and 52 of SEQ ID NO:187. For example, the combination of seven amino acids may be chosen from combinations 13L-25R-29P-31A-40K-50T-52A, 13L-25R-29P-31R-40K-50T-52A and 131-25R-27A-29P-31A-50K-52T. For example, the modified variant BBPI may have an amino acid sequence chosen from SEQ ID NOS:491, 617, 618, and 632-639.

The modified variant BBPI may comprise a combination of eight amino acid substitutions chosen from amino acid substitutions at positions equivalent to positions 13, 25, 27, 29, 31, 40, 50 and 52 of SEQ ID NO:187. The combination of eight amino acids may be chosen from combinations 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L and 13I-25K-27Q-29P-31E-40H-50R-52Q. For example, the modified variant BBPI may have an amino acid sequence chosen from SEQ ID NOS:627-631.

The invention further provides an isolated polynucleotide encoding a modified BBPI or a fusion protein as described above.

The invention further provides an expression vector comprising a polynucleotide of the invention, and a host cell comprising such an expression vector. The host cell may, for example, be a *Bacillus* species host cell.

The invention further provides a method of producing a modified BBPI or fusion protein of the invention, comprising culturing a host cell of the invention under suitable culture conditions to allow expression of said modified BBPI or fusion protein, optionally recovering said modified variant BBPI, and further optionally activating said modified variant BBPI.

The invention further provides a personal care composition comprising a modified BBPI of the invention. The personal care composition may, for example, be:
a skin care composition selected from the group consisting of skin creams, lotions, sprays, emulsions, colloidal suspensions, foams, aerosols, liquids, gels, sera, and solids;
a skin care composition selected from moisturizing body washes, body washes, antimicrobial cleansers, skin protective creams, body lotions, facial creams, moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, sunscreens, dipilatories, hair growth inhibitors and radioprotectives;
a cosmetic composition chosen from pressed powder formulations, and foundations; a skin care composition for treating an angiogenic skin disorder;
a skin care composition for improving the appearance and/or condition of skin in a subject suffering from a skin disorder chosen from psoriasis, scleroderma, venous ulcers, acne, rosacea, warts, eczema, hemangiomas and lymphangiogenesis; or a hair care composition.

Where the binding peptide is a VEGF-binding peptide or a TGFβ-binding peptide, the modified BBPI of the invention may find use in a method of improving the appearance and/or condition of skin in a subject suffering from a skin disorder.

Where the binding peptide is a TNFα-binding peptide, the modified BBPI of the invention may find use in a method of improving the condition of skin in a subject suffering from a dermatological inflammatory disorder.

The invention also provides a method of inhibiting hair growth in a subject comprising applying a composition comprising a modified BBPI of the invention wherein the binding peptide is a VEGF-binding peptide to an area where inhibition of hair growth is desired.

Where the binding peptide is a FGF5-binding peptide or a TGFβ-binding peptide, the BBPI of the invention may find use in a method of promoting hair growth in a subject suffering from a condition that involves hair loss, e.g. a condition selected from inflammatory alopecia, pseudopelade, scleroderma, tick bites, lichen planus, psoriasis, lupus, seborrheic dermatitis, loose hair syndrome, hemochromatosis, androgenic alopecia, alopecia areata and cancer.

The invention also provides a method of promoting hair growth in a subject comprising applying a composition comprising a modified BBPI of the invention wherein the binding peptide is a FGF-5-binding peptide, a TGFβ-binding peptide, or a TNFα-binding peptide to the subject in an area where hair growth is desired.

### 4. BRIEF DESCRIPTION OF THE DRAWINGS

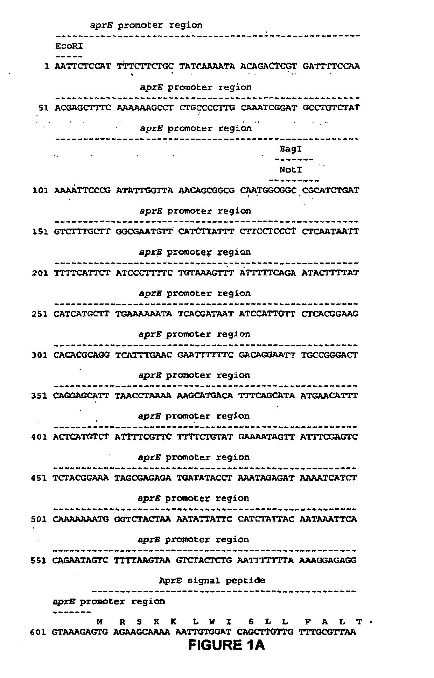

Figure 1 A-D provides the DNA and amino acid sequences of the *aprE-*BCE103-BBI-Histag expression cassette (*Eco*RI-*Hind*III) cloned into the pJM103 integration vector (SEQ ID NOS:1 and 2).

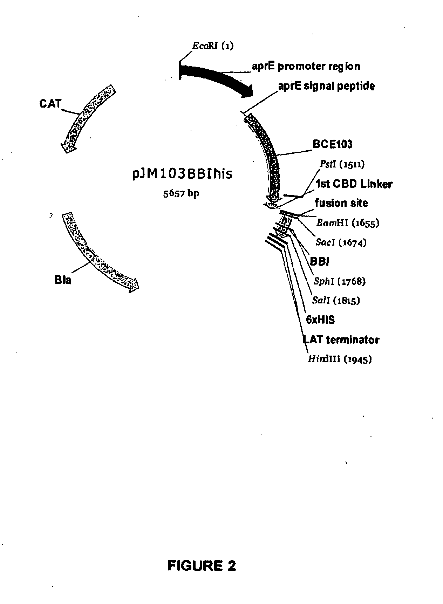

Figure 2 provides a schematic map of the pJM103BBIhis expression vector.

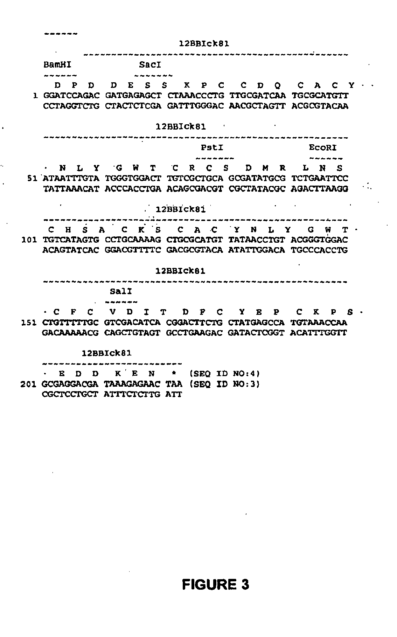

Figure 3 provides the DNA and amino acid sequences of 12BBIck81 from the BCE103 fusion site (at the *Bam*HI) to the end of the gene (SEQ ID NOS:3 and 4). The CK37281 peptide sequence (ACYNLYGWTC (SEQ ID NO:9) is inserted into both the trypsin and chymotrypsin inhibitory loops.

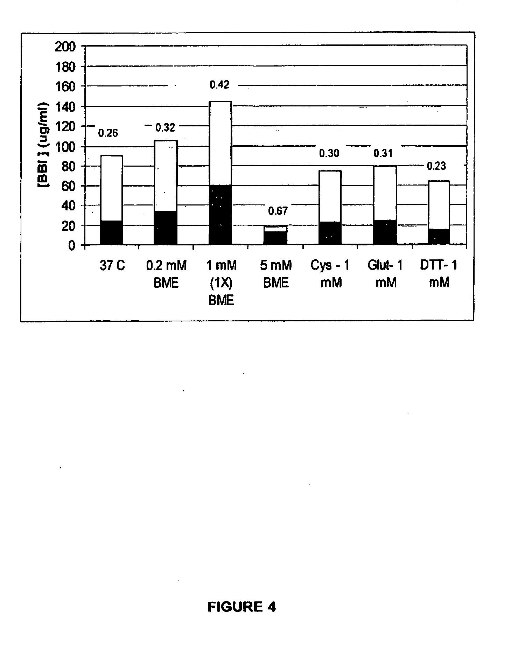

Figure 4 provides a graph showing titers of active versus inactive 2BBIck81 with various thiol reducing agents added at the given concentrations during the growth of the culture. The white portion of the bars represents the amount of active 2BBIck81 (determined by trypsin inhibition) and the black portion of the bars represents the amount of inactive 2BBIck81 (determined from BCE103 activity and assumes BCE103 and 2BBIck 81 are produced in 1:1 molar ratio). The fraction of active 2BBIck81 is indicated at the top of the bars. In this Figure, 37 C = no additions, BME = 2-mercaptoethanol, Cys = cysteine, Glut = reduced glutathione, DTT = dithiothreitol.

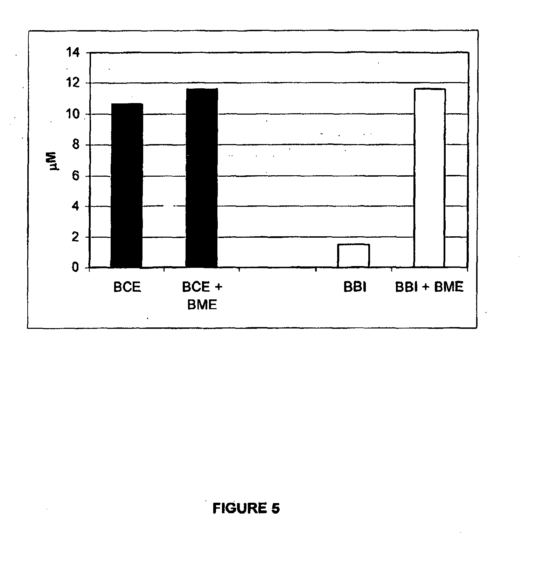

Figure 5 provides a graph showing the activation of 2BBIck81 with 2-mercaptoethanol (BME) after partial purification of the BCE-Ink2-2BBck81 fusion protein by ion exchange chromatography. The concentrations (µM) of BCE (black bars) and 2BBIck81 (white bars) measured by activity assays are shown before and after treatment with 3 mM 2-mercaptoethanol (BME).

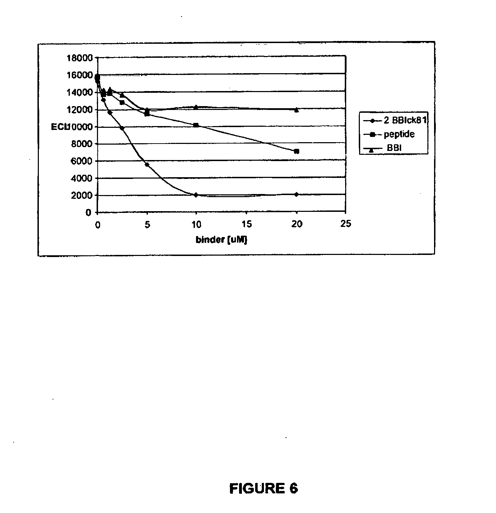

Figure 6 provides a graph showing results from a competition analysis of 2BBIck81 versus anti-VegF antibody for binding to VegF in a BioVeris assay. The binding competition with the CK37281 peptide is shown for comparison and the binding with the wild-type BBI is included as a negative control.

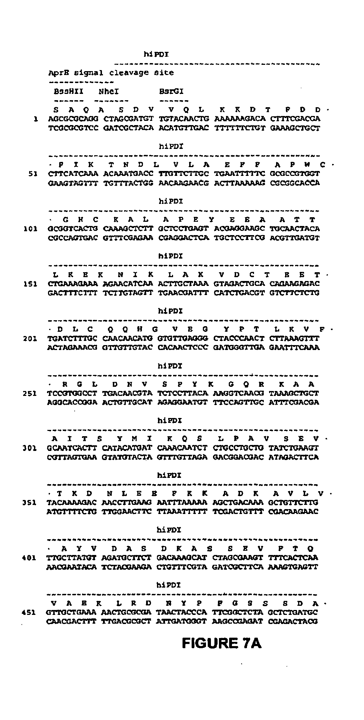

Figure 7A-C provides the sequence of the synthetic DNA fragment carrying the *H. insolens* PDI-(hiPDI) that was inserted into the *B. subtilis* BBI expression vector (using the BssHII and Sacl sites), as well as the amino acid sequence (SEQ ID NOS:5 and 6)

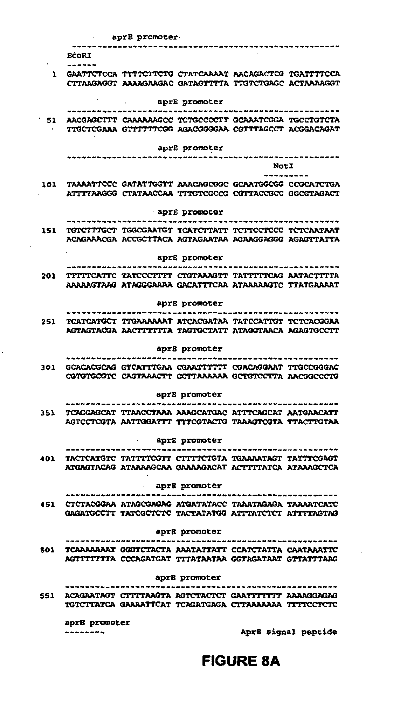

Figure 8 A-C provides the DNA and amino acid sequences of the *aprE-*cutinase expression cassette that was ligated into the *Eco*RI*-Bam*HI sites of p2JM103-Ink2-2BBIck81 (SEQ ID NOS:7 and 8).

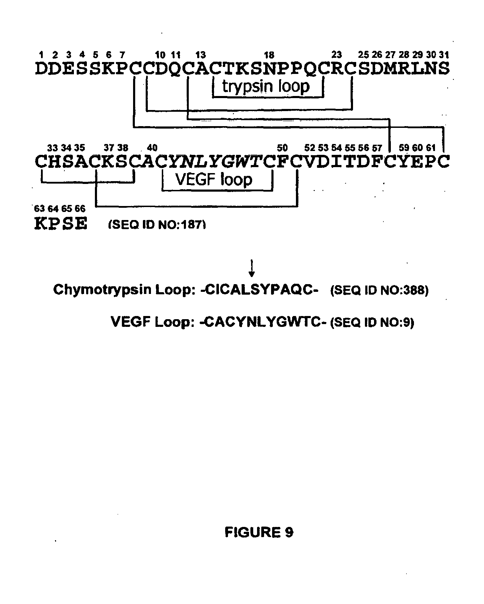

Figure 9 provides the amino acid sequence of the unmodified BBIt-AV protein (SEQ ID NO:187). Site-saturation libraries were constructed at all the numbered amino acids. The trypsin inhibitory loop and the VEGF binding peptide loop (in italics) are indicated and disulfide bonds are shown by the connecting lines. At the bottom, the sequence of the VEGF binding peptide is compared to the chymotrypsin inhibitory loop in sBBI that it replaces. The downward arrow indicates the potential protease cleavage site between the P₁ and P₁' residues of the chymotrypsin reactive site.

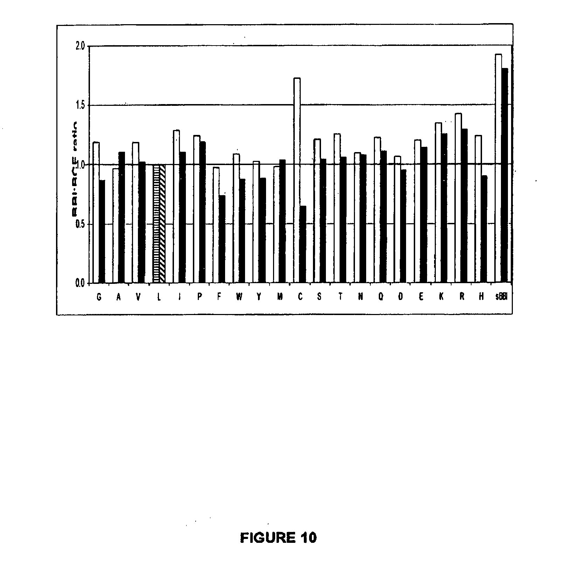

Figure 10 provides the relative BBI:BCE activity ratios for each amino acid substitution made at position 29. The ratios were averaged from quadruplicate cultures grown in microtiter plates in the absence (white bars) or presence (black bars) of 2-mercaptoethanol. The activity ratios for sBBI are included for comparison as a positive control. The ratios were normalized to the value determined for the wild-type amino acid in BBIt-AV (SEQ ID NO:187) (indicated by a horizontal line), leucine, that is shown in the absence (horizontally lined bar) or in the presence (diagonally lined bar) of 2-mercaptoethanol.

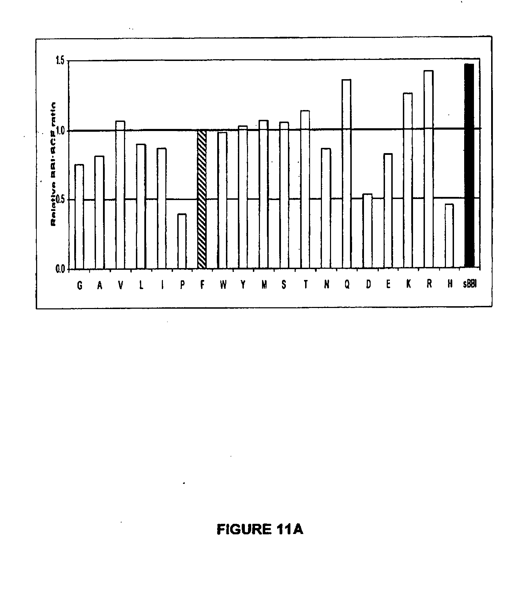

Figures 11 A - B respectively provide the relative BBI:BCE activity ratios for the amino acid substitutions made at position 50 (A) and position 37 (B) (white bars). The ratios were averaged from quadruplicate cultures in the presence of 2-mercaptoethanol. The activity ratios for sBBI are included as a positive control for comparison (black bars). The ratios are normalized to the value (indicated by a horizontal line) determined for the wild-type amino acids in BBIt-AV (SEQ ID NO:187) and is shown with diagonally lined bars.

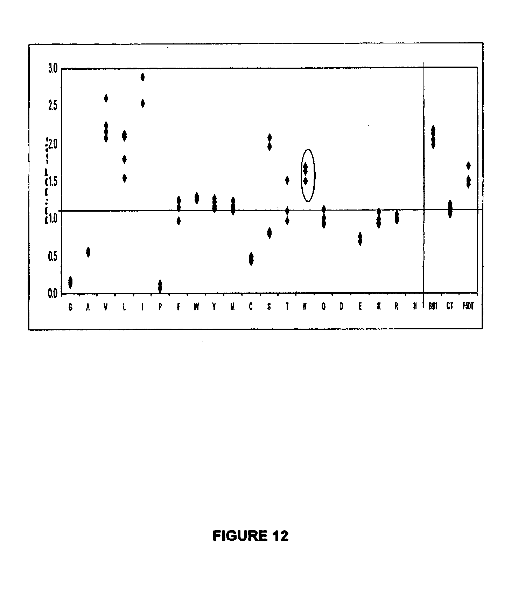

Figure 12 provides the relative BBI:BCE activity ratios for the amino acid substitutions at the P₂' position (residue 18 in Figure 9, SEQ ID NO:187) in the trypsin inhibitory loop. The ratios were determined in quadruplicate in the presence of 2-mercaptoethanol (individual data points shown). The activity ratios for sBBI (BBI; SEQ ID NO:13), BBIt-AV (CT; SEQ ID NO:187) and BBIt-AV-F50T (F50T) are shown on the right side of the horizontal line and were added for comparison. The ratios are normalized to the value (shown by a horizontal line) determined for BBIt-AV (CT).

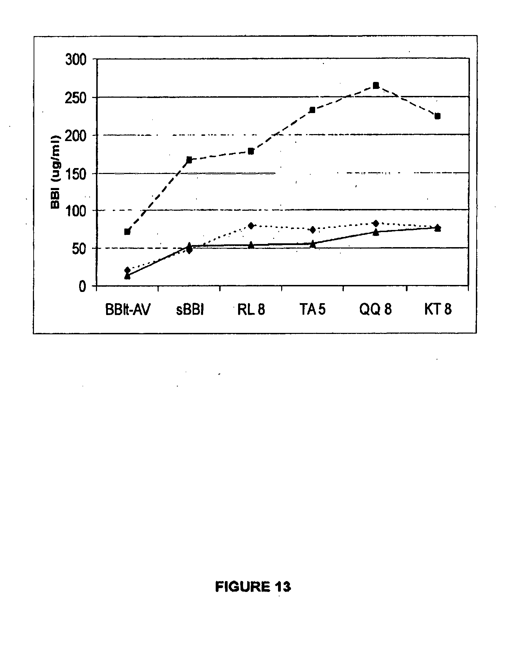

Figure 13 provides a comparison of the production of three BBIt-AVs containing combinations of 8 amino acid substitutions: octuple variant BBIt-AV-A13I-S25K-M27A-L29R-S31A-A40H-F50R-V52L (RL8; SEQ ID NO:630), BBIt-AV-A13I-S25K-M27A-L29R-S31E-A40K-F50Q-V52Q (QQ8; SEQ ID NO:628), and BBIt-AV-A13I-S25R-M27A-L29P-S31A-A40H-F50K-V52T (KT8; SEQ ID NO:627). The amount of active BBI species was determined by trypsin inhibition after growth (◆), after activation with 2-mercaptoethanol (■) and after acid/heat treatment (▲). BBIt-AV, the quintuple variant BBIt-AV-A13I-L29P-A40K-F50T-V52A (TA5; SEQ ID NO:601) and sBBI are included for comparison.

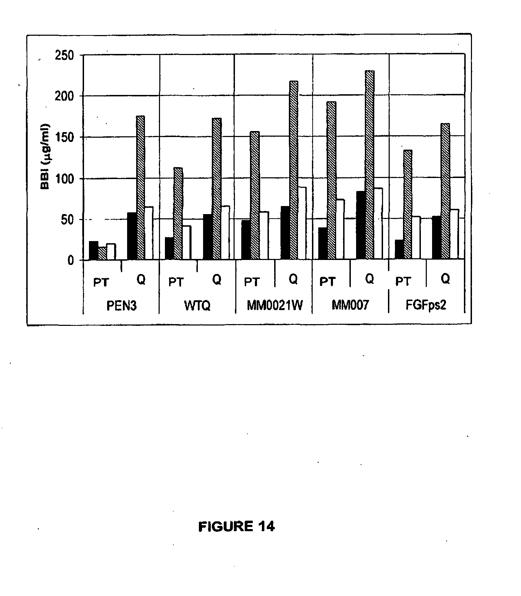

Figure 14 provides a comparison of the active BBI species (by trypsin inhibition) after growth (black bars), after activation with 2-mercaptoethanol (diagonally lined bars) and after acid/heat treatment (white bars) of different binding peptides (three TGFβ and two FGF5 binders) in the precursor variant BBPI scaffold (designated parent (PT)) versus the modified variant BBIt-AV-A13I-L29P-F50T-V52A scaffold (Q; SEQ ID NO:600). In all scaffolds, the binding peptides replaced the chymotrypsin inhibitory loop. PEN3 (SEQ ID NO:436), WTQ (SEQ ID NO:438) and MM021W (SEQ ID NO:437) are TGFβ binding peptides, and MM007 (SEQ ID NO:430) and FGFps2 (SEQ ID NO:431) are FGF5 binding peptides.

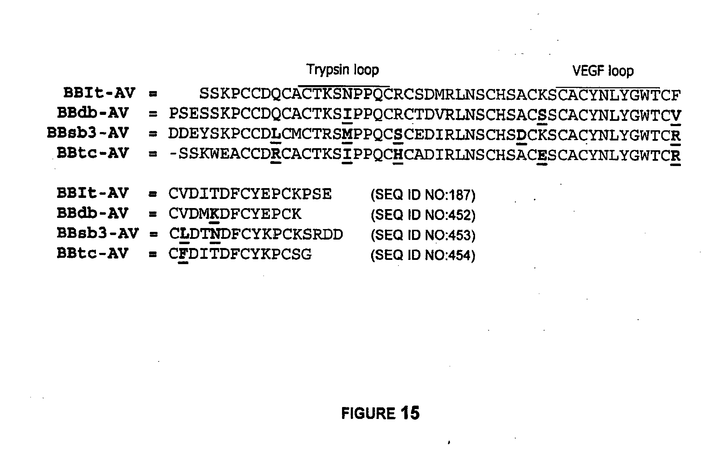

Figure 15 provides the amino acid sequences of the variant Bowman Birk Inhibitor scaffolds from *Dolichos biflorus* (BBdb-AV, SEQ ID NO:452), the varianr protease inhibitor IV or D-II (BBsb3-AV, SEQ ID NO:453) from *Glycine max* (soybean), and from *Torresea (Amburana) cearensis* (BBtc-AV, SEQ ID NO:454) in which the chymotrypsin loop is replaced with a VEGF-binding peptide, that are aligned to the sequence of the variant BBI, BBIt-AV (SEQ ID NO:187). The differences from the BBIt-AV sequence between C9 and C58 are shown underlined in bold font. In all scaffolds, the CK3781 binding peptide (ACYNLYGWT; SEQ ID NO:9) replaces the second protease inhibitory loop.

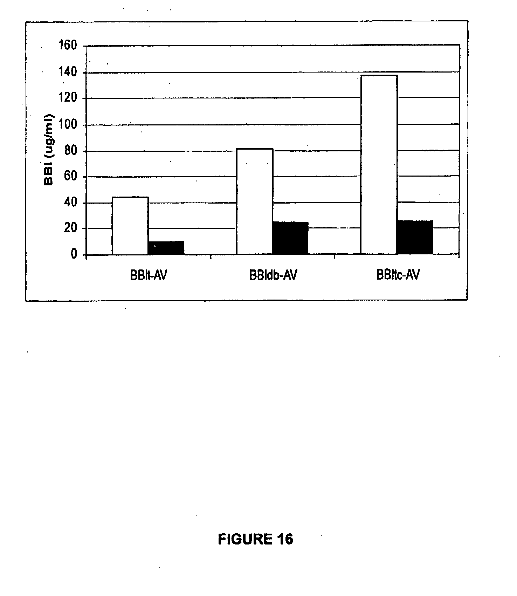

Figure 16 provides a comparison of the active BBI concentration (by trypsin inhibition) of BBIt-AV, BBdb-AV and BBtc-AV after activation with 2-mercaptoethanol (white bars) and after acid/heat treatment (black bars).

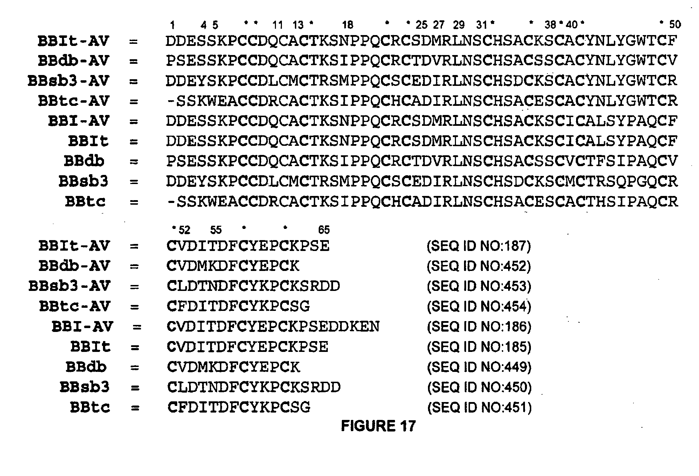

Figure 17 provides an alignment of unmodified variant BBIt-AV, (SEQ ID NO:187), BBdb-AV,(SEQ ID NO:452), BBsb3-AV (SEQ ID NO:453) and BBtc-AV (SEQ ID NO:454) and the corresponding unmodified precursor BBIt (SEQ ID NO:187), BBsb3 (SEQ ID NO:449), BBtc (SEQ ID NO:450) and BBdb (SEQ ID NO:451) BBPIs. The star symbol (*) identifies the invariant conserved C residues. The numbered residues identify the amino acids that are substituted to generate the modified variant BBPIs of the invention.

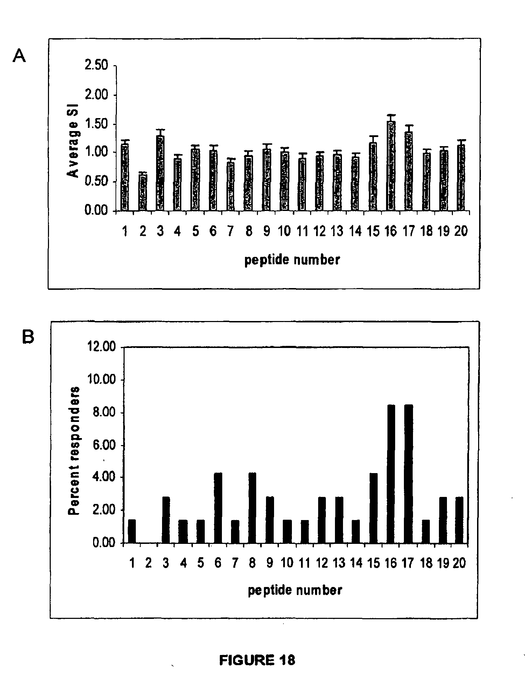

Figure 18 Identification of T-cell epitopes in wild type BBI. (A) The stimulation indices (SI) values for all 71 donors were averaged and are shown, with standard deviations in error bars. (B) The percent responders to each peptide in the 20 wt BBI peptide set are shown.

### 5. DETAILED DESCRIPTION OF THE INVENTION

The present invention relates to modified variant Bowman Birk Protease Inhibitor proteins (BBPIs) that comprise peptides that bind target proteins, and that are modified to have greater protease inhibitory activity and/or be produced at greater yields than the unmodified BBPIs. The invention encompasses polynucleotide constructs and expression vectors containing polynucleotide sequences that encode the modified variant BBPIs, the transformed host cells that express and produce the modified variant BBPIs, the modified variant BBPI proteins, the compositions comprising the modified variant BBPIs, and the methods for making and using the modified variant BBPIs in personal care.

Unless otherwise indicated, the practice of the present invention involves conventional techniques commonly used in molecular biology, microbiology, protein purification, protein engineering, protein and DNA sequencing, and recombinant DNA fields, which are within the skill of the art. Such techniques are known to those of skill in the art and are described in numerous standard texts and reference works.

Unless defined otherwise herein, all technical and scientific terms used herein have the same meaning as commonly understood by one of ordinary skill in the art to which this invention belongs. Various scientific dictionaries that include the terms included herein are well known and available to those in the art. Although any methods and materials similar or equivalent to those described herein find use in the practice or testing of the present invention, some preferred methods and materials are described. Accordingly, the terms defined immediately below are more fully described by reference to the Specification as a whole. It is to be understood that this invention is not limited to the particular methodology, protocols, and reagents described, as these may vary, depending upon the context they are used by those of skill in the art.

As used herein, the singular terms "a", "an," and "the" include the plural reference unless the context clearly indicates otherwise. Unless otherwise indicated, nucleic acids are written left to right in 5' to 3' orientation and amino acid sequences are written left to right in amino to carboxy orientation, respectively.

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The headings provided herein are not limitations of the various aspects or embodiments of the invention which can be had by reference to the Specification as a whole. Accordingly, as indicated above, the terms defined immediately below are more fully defined by reference to the specification as a whole.

### 5.1 Definitions

As used herein, the terms "isolated" and "purified" refer to a nucleic acid or amino acid (or other component) that is removed from at least one component with which it is naturally associated.

As used herein, the term "modified" when referring to a protease inhibitor (PI) *e.g.* Bowman Birk Protease Inhibitor (BBPI), refers to a BBPI having an amino acid sequence that is derived from the amino acid sequence of an unmodified "precursor" or "parent" BBPI protein. The unmodified precursor BBPI can be a naturally-occurring or wild-type protein, or a variant BBPI. The amino acid sequence of the modified protein is "derived" from the precursor protein, amino acid sequence by the substitution, deletion or insertion of one or more amino acids of the region of the precursor amino acid sequence The parent protease inhibitor is a variant BBPI, which contains a chymotrypsin loop that has been replaced with a variant sequence. Substitution of at least one amino acid of a variant precursor BBPI generates a modified variant BBPI protease inhibitor. The modified variant PI comprises an amino acid substitution at least at one position equivalent to positions 1, 4, 5, 11, 13, 18, 25, 27, 29, 31, 38, 40, 50, 52, and 65 of SEQ ID NO:187 (DDESSKPCCDQCACTKSNPPQCRCSDMRLNSCHSACKSCACYNLYGWTCFCVDI TDFCYEPCKPSE: SEQ ID NO:187). In other embodiments, the modified variant PI comprises a combination of substitutions as described herein. In yet other embodiments, the modified variant PI comprises an insertion. Such modifications are of the "precursor DNA sequence" which encodes the amino acid sequence of the precursor protease inhibitor rather than manipulation of the precursor protease inhibitor per se. The modified protease inhibitors herein encompass the substitution of any of the nineteen naturally occurring amino acids at any one of the amino acid residues in regions other than the reactive loops *e.g.* trypsin and/or chymotrypsin loops(s). The polynucleotides that encode the modified sequence are referred to as "modified polynucleotides", and the polynucleotides that encode the precursor protease inhibitor are referred to as "precursor polynucleotides".

As used herein, the term "protease inhibitor" (PI) herein refers to and is used interchangeably with Bowman Birk Protease Inhibitor (BBPI), which is a cysteine-rich protease inhibitor as described, for example, by Prakash et al. [J mol Evol 42:560-569 (1996)].

As used herein, the term "scaffold" refers to a BBPI protein sequence into which a variant sequence is introduced. In some embodiments, the scaffold is a variant scaffold, which has either the first protease inhibitory loop *e.g.* the trypsin loop, and/or the second protease inhibitory loop *e.g.* the chymotrypsin replaced with a binding peptide sequence. In other embodiments, the scaffold is a wild type BBPI scaffold The term "modified variant scaffold" refers to a variant scaffold that comprises modifications *e.g.* amino acid substitutions or insertions in the backbone of the BBPI scaffold.

As used herein, the term "backbone" when used in reference to a variant BBPI scaffold refers to the portion of the variant BBPI that is outside of the binding peptide sequence that has been introduced to replace the second protease inhibitory loop *i.e.* the chymotrypsin loop. For example, the backbone of the variant BBPI of SEQ ID NO:187 refers to amino acids 1-41 and 49-66 *i.e*. the amino acids N-terminal and C-terminal to the VEGF binding sequence that was introduced to replace the chymotrypsin loop found in the wild type BBPI *i.e*. BBI of SEQ ID NO:13 (DDESSKPCCDQCACTKSNPPQCRCSDMRLNSCHSACKSCICALSYPAQCFCVDIT DFCYEPCKPSEDDKEN; SEQ ID NO:13) including the invariant cysteine residues at amino acid positions 41 and 49. In some embodiments, the scaffold has at least 80%, at least 85%, at least 90%, at least 95%, at least 98%, or at least 99% identity to the scaffold of the BBIt of SEQ ID NO:187.

As used herein, the terms "binding peptide", "binding sequence", "variant sequence" and "variant peptide" are used interchangeably, and refer to the short polypeptide sequence(s) that replace the second protease inhibitory loop of protease inhibitor *e.g.* Bowman Birk Inhibitor The binding peptide does not need to be of the same length as the protease inhibitory loop sequence it is replacing in the scaffold, and it differs from the protease inhibitory loop sequence of the wild-type BBPI by at least two amino acids. Replacing the second protease inhibitory loop (chymotrypsin loop) of a precursor protease inhibitor alters the sequence that is equivalent to that spanning amino acids 42 to 48 of wild-type SEQ ID NO:13 or variant SEQ ID NO:187 BBPI. Replacing the first protease inhibitory loop (trypsin loop) of a precursor protease inhibitor alters the sequence that is equivalent to that spanning amino acids 15 to 21 of wild-type SEQ ID NO:13 or variant SEQ ID NO:187 BBPI. The binding peptide sequence is heterologous to that of the protease inhibitor. Binding peptides bind to target proteins.

A "VEGF binding peptide", an "FGF binding peptide", a "TGF binding peptide" and a "TNF binding peptide" herein refer to a peptide sequence that binds VEGF, FGF5, TGFβ and TNFα, respectively.

A "VEGF composition", an "FGF composition", a "TGF composition" and a "TNF composition" herein refer to a composition *e.g.* a personal care composition, which comprises a VEGF-BBPI, an FGF-BBPI, a TGF-BBPI and a TGF-BBPI, respectively.

A "compound" when used in reference to a formulation described herein, refers to a modified variant BBPI *e.g.* a VEGF-BBPI, an FGF-BBPI, a TGF-BBPI or a TNF-BBPI. A formulation may include more than one type of modified variant BBPI *i.e.* the formulation may comprise a VEGF-BBPI and a TNF-BBPI, or any other combination of the four types of BBPIs disclosed herein.

The term "a" when used in reference to a modified variant BBPI comprised in a personal care composition, herein refers to a modified variant BBPI having a particular sequence. "a", in this context does not limit the number of modified variant BBPI molecules that are needed in the personal care composition.

The terms "chymotrypsin loop" and "second protease inhibitory loop" are herein used interchangeably and refer to the sequence of amino acids that spans the amino acid sequence that corresponds to the second reactive site loop of a protease inhibitor of the Bowman Birk family. For example, the second protease inhibitory loop of the wild-type BBPI inhibitor from *Glycine max i.e.* BBI of SEQ ID NO:13 is the peptide sequence that spans the second reactive site loop encompassed by cysteine 10 and cysteine 11 (see Prakash et al. supra). C10 and C11 encopass an amino acid sequence that is equivalent to that which spans from amino acid 42 to amino acid 48 in the variant BBIt-AV of SEQ ID NO:187 (Figure 9). The second protease inhibitory loop or chymotrypsin loop of a variant BBPI e.g. BBIt-AV of SEQ ID NO:187, corresponds to the amino acid sequence that spans amino acids 42-48 encompassed by the cysteines at positions 41 and 49, which are the cysteine residues equivalent to C10 and C11 as described by Prakash *et al..* While the amino acid sequence of second protease inhibitory loop of the wild-type BBI of SEQ ID NO:13 is a chymotrypsin inhibitory peptide, the second protease inhibitory loop of the variant BBIt-AV of SEQ ID NO:187 is a VEGF-binding peptide. Thus, the terms "chymotrypsin loop" or "second protease inhibitory loop" herein refer to the position of the loop and do not intend to imply that the sequence between C10 and C11 imparts protease inhibitory activity to a variant BBPI. Similarly, the terms "trypsin loop " and "first protease inhibitory loop" are herein used interchangeably and refer to the sequence of amino acids that spans the amino acid sequence that corresponds to the first reactive site loop of a protease inhibitor of the Bowman Birk family. For example, the second protease inhibitory loop of the wild-type BBPI inhibitor from *Glycine max i.e.* BBI of SEQ ID NO:13 is the peptide sequence that spans the second reactive site loop encompassed by cysteine 4 and cysteine 5 (see Prakash et al. supra). C4 and C5 encopass an amino acid sequence that is equivalent to that which spans from amino acid 15 to amino acid 21 in the variant BBIt-AV of SEQ ID NO:187 (Figure 9).

As used herein, the term "target protein" refers to protein (*e.g.,* enzyme, hormone, etc.), whose action would be blocked by the binding of the variant peptide. In some embodiments, the variant peptide binds the target protein when the peptide replaces the trypsin and/or chymotrypsin loop of a BBPI.

As used herein, "substituted" and "substitutions" refer to replacement(s) of an amino acid residue or nucleic acid base in a parent sequence. In some embodiments, the substitution involves the replacement of a naturally occurring residue or base. In some embodiments, two or more amino acids are substituted to generate a modified BBPI that comprises a combination of amino acid substitutions. In some embodiments, combinations of substitutions are denoted by the amino acid position at which the substitution is made. For example, a combination denoted by 25-50-52 means that three amino acids at positions 25, 50 and 52 are substituted. In other embodiments, the combination of substitutions is denoted by the amino acid position and the amino acid resulting from the substitution. For example, a modified BBPI that comprises the combination of substitutions 13I-25L-50T-52A is a modified BBPI wherein the amino acid at position 13 has been substituted with an isolucine, the amino acid at position 25 has been substituted with a leucine, the amino acid at position 50 has been substituted with a threonine, and the amino acid at position 52 has been substituted with an alanine. Amino acid positions are positions equivalent to the the numbered poisitions in the BBPI of SEQ ID NO:187. In some embodiments, the combination of substitutions is given in the context of the scaffold in which the substitutions are made. For example, the modified variant BBPI of SEQ ID NO:601 is also referred to as BBIt-AV-13I-29P-40K-50T-52A, indicating that the BBIt-AV scaffold (SEQ ID NO:187) has been modified to contain the resulting substitutions at amino acids 13, 29, 40, 50, and 52. In some embodiments, the original and substituted amino acid are indicated *e.g.* the modified variant BBPI of SEQ ID NO:601 can be referred to as BBIt-AV-A13I-L29P-A40K-F50T-V52A.

As used herein, "modification" and "modify" refer to any change(s) in an amino acid or nucleic acid sequence, including, but not limited to deletions, insertions, interruptions, and substitutions. In some embodiments, the modification involves the replacement of a naturally occurring residue or base. In other embodiments, the modification comprises a combination of at least one amino acid substitution. In yet other embodiments, the modification comprises an insertion with or without the insertion being combined with at least one amino acid substitution.

As used herein, the term "equivalent" when used in reference to an amino acid residue or the position of an amino acid residue in a BBPI refers to the position of an amino acid residue in a modified BBPI that corresponds in position in the primary sequence of the unmodified precursor BBPI. In order to establish the position of equivalent amino acid positions in a BBPI, the amino acid sequence of the BBPI that is modified is directly compared to the BBPI of SEQ ID NO:187, and in particular to the cysteine residues that are known to be invariant in protease inhibitors of the Bowman Birk Inhibitor family (Prakash et al. supra). After aligning the conserved cysteine residues, allowing for insertions and deletions in order to maintain alignment (*i.e*, avoiding the elimination of conserved cysteine residues through arbitrary deletion or insertion), the residues at positions equivalent to particular amino acid positions in the sequence of the BBPI of SEQ ID NO:187 are defined. Alignment of conserved cysteine residues preferably should conserve 100% of such residues. For example, in Figure 17 the amino acid sequences of variant and wild-type BBPIs are aligned to provide the maximum amount of homology between amino acid sequences. A comparison of these sequences shows that the invariant cysteine residues are conserved. While the primary sequence is the preferred structure for determining the position of equivalent amino acids in the BBPIs of the invention, equivalent residues may also be identified by determining homology at the level of tertiary structure for a protein BBPI protein.
Equivalent amino acid positions are defined as those for which the atomic coordinates of two or more of the main chain atoms of a particular amino acid residue of the protein having putative equivalent residues and the protein of interest (N on N, CA on CA, C on C and O on O) are within 0.13 nm and preferably 0.1 nm after alignment. Alignment is achieved after the best model has been oriented and positioned to give the maximum overlap of atomic coordinates of non-hydrogen protein atoms of the proteins analyzed. The preferred model is the crystallographic model giving the lowest R factor for experimental diffraction data at the highest resolution available, determined using methods known to those skilled in the art of crystallography and protein characterization/analysis. The crystal structure of the Bowman Birk inhibitor from soybean has been determined (Hwang et al. J Biol Chem. 10;252(3):1099-101 [1977],Wei et al., J Biol Chem. 10;254(11):4892-4 [1979], Voss et al. Eur J Biochem. 15;242(1):122-31 [1996]) and can be used as outlined above to determine equivalent amino acid positions on the level of tertiary structure.

As used herein, "fusion polypeptides," "fusion proteins," and "fusion analogs" encode from the amino-terminus a signal peptide functional as a secretory sequence functional in a host cell, a secreted polypeptide or portion thereof normally secreted from a host cell, a cleavable linker polypeptide and a desired polypeptide. In some embodiments, the fusion polypeptides include a spacer peptide positioned between the secretory sequence and a secreted polypeptide. In some embodiments, the fusion protein is processed by host cell enzymes (*e.g.,* a protease), to yield the desired protein free from the other protein sequences in the fusion protein. As used herein, the terms "fusion analog," "fusion polypeptide," and "fusion protein" are used interchangeably.

As used herein, the term "activity" refers to any activity associated with a particular protein, such as enzymatic activity associated with a protease. In some embodiments, the activity is biological activity. In further embodiments, activity encompasses binding of proteins to receptors which results in measurable downstream effects (*e.g.,* VEGF binding to its cognate receptor). "Biological activity" refers to any activity that would normally be attributed to that protein by one skilled in the art.

As used herein, "protease inhibitory activity" refers to the activity of a BBPI in inhibiting the proteolytic activity of a protease *i.e.* inhibiting the ability of a protease to hydrolyze peptides or substrates having peptide linkages.

As used herein, the term "expression" refers to the process by which a polypeptide is produced based on the nucleic acid sequence of a gene. The process includes both transcription and translation.

The term "production" with reference to a BBPI, encompasses the two processing steps of a full-length protease including: 1. the removal of the signal peptide, which is known to occur during protein secretion; and 2. the removal of the pro region, which creates the active mature form of the BBPI and which is known to occur during the maturation process (Wang et al., Biochemistry 37:3165-3171 (1998); Power et al., Proc Natl Acad Sci USA 83:3096-3100 (1986)).

As used herein, the term "production yield" refers to the level at which an unmodified and/or modified variant protease inhibitor *e.g.* a variant BBPI is produced. The greater the production yield, the greater the level or amount of protease inhibitor that is produced.

As used herein, the term "efficient production" herein to the production of a protein e.g. a modified variant BBPI, and implies that said protein is produced at a level that is greater than that of an unmodified or precursor variant BBPI.

As used herein, the term "substantially pure" when applied to the proteins or fragments thereof of the present invention means that the proteins are essentially free of other substances to an extent practical and appropriate for their intended use. In particular, the proteins are sufficiently pure and are sufficiently free from other biological constituents of the host cells so as to be useful in, for example, protein sequencing, and/or producing pharmaceutical preparations.

As used herein, the term "substantially free" encompasses preparations of the desired polypeptide having less than about 20% (by dry weight) other proteins (*i.e.,* contaminating protein), less than about 10% other proteins, less than about 5% other proteins, or less than about 1% other proteins.

As used herein, the terms "polynucleotide", "nucleic acid molecule" and "nucleic acid sequence" include sequences of any form of nucleic acid, including, but not limited to RNA, DNA and cDNA molecules. It will be understood that, as a result of the degeneracy of the genetic code, a multitude of nucleotide sequences encoding a given protein may be produced.

As used herein, the terms "DNA construct", "polynucleotide construct" and "transforming DNA" are used interchangeably to refer to DNA used to introduce sequences into a host cell or organism. The DNA may be generated in vitro by PCR or any other suitable technique(s) known to those in the art. In some embodiments, the DNA construct comprises a sequence of interest (e.g., a modified sequence). In some embodiments, the sequence is operably linked to additional elements such as control elements (e.g., promoters, etc.). In some embodiments, the DNA construct comprises sequences homologous to the host cell chromosome. In other embodiments, the DNA construct comprises non-homologous sequences. Once the DNA construct is assembled in vitro it may be used to mutagenize a region of the host cell chromosome (i.e., replace an endogenous sequence with a heterologous sequence).

As used herein, the term "heterologous DNA sequence" refers to a DNA sequence that does not naturally occur in a host cell. In some embodiments, a heterologous DNA sequence is a chimeric DNA sequence that is comprised of parts of different genes, including regulatory elements.

As used herein, the term "heterologous protein" refers to a protein or polypeptide that does not naturally occur in the host cell i.e. it is encoded by a heterologous sequence.

As used herein, "homologous protein" refers to a protein or polypeptide native or naturally occurring in a cell.

As used herein, the term "vector" refers to a polynucleotide construct designed to introduce nucleic acids into one or more cell types. Vectors include cloning vectors, expression vectors, shuttle vectors, and plasmids. In some embodiments, the polynucleotide construct comprises a DNA sequence encoding a modified variant BBPI.

As used herein, the term "expression vector" refers to a vector that has the ability to incorporate and express heterologous DNA fragments in a foreign cell. Many prokaryotic and eukaryotic expression vectors are commercially available. Selection of appropriate expression vectors is within the knowledge of those of skill in the art.

As used herein, the term "plasmid" refers to a circular double-stranded (ds) DNA construct used as a cloning vector, and which forms an extrachromosomal self-replicating genetic element in some eukaryotes or prokaryotes, or integrates into the host chromosome.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, conjugation, and transduction (See e.g., Ferrari et al., "Genetics," in Hardwood et al, (eds.), Bacillus, Plenum Publishing Corp., pages 57-72, [1989]).

As used herein, the terms "transformed" and "stably transformed" refers to a cell that has a non-native (heterologous) polynucleotide sequence integrated into its genome or as an episomal plasmid that is maintained for at least two generations.

As used herein, "percent (%) sequence identity" or "percent homology" when used in reference to a polyncleotide or to a polypeptide sequence is defined as the percentage of nucleotide or amino acid residues in a candidate sequence that are identical with the nucleotide or amino acid residues of a sequence disclosed herein. The percent identity shared by polynucleotide or polypeptide sequences is determined by direct comparison of the sequence information between the molecules by aligning the sequences and determining the identity by methods known in the art. In some embodiments, the alignment includes the introduction of gaps in the sequences to be aligned. In addition, for sequences which contain either more or fewer nucleotides or amino acids than those of the candidate polynucleotide or polypepitde sequences, it is understood that the percentage of homology will be determined based on the number of homologous nucleotides or amino acids in relation to the total number of nucleotides or amino acids. Thus, for example, homology of sequences shorter than those of the sequences identified herein will be determined using the number of nucleosites or amino acids in the shorter sequence. This homology is determined using standard techniques known in the art (*See e.g.,* Smith and Waterman, Adv. Appl. Math., 2:482 [1981]; Needleman and Wunsch, J. Mol. Biol., 48:443 [1970]; Pearson and Lipman, Proc. Natl. Acad. Sci. USA 85:2444 [1988]; programs such as GAP, BESTFIT, FASTA, and TFASTA in the Wisconsin Genetics Software Package (Genetics Computer Group, Madison, WI); and Devereux et al., Nucl. Acid Res., 12:387-395 [1984]).

As used herein, an "analogous sequence" is one wherein the function of the protein is essentially the same as that designated for the Bowman Birk family of protease inhibitors. Additionally, analogous proteins include at least 60%, 65%, 70%, 75%, 80%, 85%, 90%, 95%, 97%, 98%, 99% or 100% sequence identity with the sequence of the variant BBPI of SEQ ID NO:187. Analogous sequences are determined by known methods of sequence alignment. A commonly used alignment method is BLAST, although as indicated above and below, there are other methods that also find use in aligning sequences.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng and Doolittle (Feng and Doolittle, J. Mol. Evol., 35:351-360 [1987]). The method is similar to that described by Higgins and Sharp (Higgins and Sharp, CABIOS 5:151-153 [1989]). Useful PILEUP parameters including a default gap weight of 3.00, a default gap length weight of 0.10, and weighted end gaps.

Another example of a useful algorithm is the BLAST algorithm, described by Altschul *et al.,* (Altschul et al., J. Mol. Biol., 215:403-410, [1990]; and Karlin et al., Proc. Natl. Acad. Sci. USA 90:5873-5787 [1993]). A particularly useful BLAST program is the WU-BLAST-2 program (*See,* Altschul et al., Meth. Enzymol.,, 266:460-480 [1996]). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored). A preferred method utilizes the BLASTN module of WU-BLAST-2 set to the default parameters, with overlap span and overlap fraction set to 1 and 0.125, respectively.

A "host cell" refers to a suitable cell from a cell that serves as a host for an expression vector comprising DNA according to the present invention. A suitable host cell may be a naturally occurring or wild-type host cell, or it may be an altered host cell. In one embodiment, the host cell is a Gram positive microorganism. In some preferred embodiments, the term refers to cells in the genus *Bacillus.*

As used herein, *"Bacillus sp."* includes all species within the genus *"Bacillus,"* as known to those of skill in the art, including but not limited to *B. subtilis, B. licheniformis, B. lentus, B. brevis, B. stearothermophilus, B. alkalophilus, B. amyloliquefaciens, B. clausii, B. halodurans, B. megaterium, B. coagulans, B. circulans, B. lautus,* and *B. thuringiensis.* It is recognized that the genus *Bacillus* continues to undergo taxonomical reorganization. Thus, it is intended that the genus include species that have been reclassified, including but not limited to such organisms as *B. stearothermophilus,* which is now named *"Geobacillus stearothermophilus."* The production of resistant endospores in the presence of oxygen is considered the defining feature of the genus *Bacillus,* although this characteristic also applies to the recently named *Alicyclobacillus, Amphibacillus, Aneurinibacillus, Anoxybacillus, Brevibacillus, Filobacillus, Gracilibacillus, Halobacillus, Paenibacillus, Salibacillus, Thermobacillus, Ureibacillus,* and *Virgibacillus.*

As used herein, a "promoter sequence" refers to a DNA sequence which is recognized by the bacterial host for expression purposes. In preferred embodiments, it is operably linked to a DNA sequence encoding the fusion polypeptide. Such linkage comprises positioning of the promoter with respect to the translation initiation codon of the DNA sequence encoding the fusion DNA sequence. In particularly preferred embodiments, the promoter sequence contains transcription and translation control sequences which mediate the expression of the fusion DNA sequence.

As used herein, a nucleic acid is "operably linked" when it is placed into a functional relationship with another nucleic acid sequence. For example, DNA encoding a secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Operably linked DNA sequences are usually contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

As used herein, "recombinant" includes reference to a cell or vector, that has been modified by the introduction of a heterologous nucleic acid sequence or that the cell is derived from a cell so modified. Thus, for example, recombinant cells express genes that are not found in identical form within the native (non-recombinant) form of the cell or express native genes that are otherwise abnormally expressed, under expressed or not expressed at all as a result of deliberate human intervention.

As used herein, the term "personal care composition" refers to a product for application to the skin, hair, nails, oral cavity and related membranes for the purposes of improving, cleaning, beautifying, treating, and/or caring for these surfaces and membranes. In some embodiments, the personal care composition is in the form of an emulsified vehicle, such as a nutrient cream or lotion, a stabilized gel or dispersioning system, such as skin softener, a nutrient emulsion, a nutrient cream, a massage cream, a treatment serum, a liposomal delivery system, a topical facial pack or mask, a surfactant-based cleansing system such as a shampoo or body wash, an aerosolized or sprayed dispersion or emulsion, a hair or skin conditioner, styling aid, or a pigmented product such as makeup in liquid, cream, solid, anhydrous or pencil form. However, it is not intended that the present invention be limited to any particular form, as various forms find use in the present invention.

Personal care products can be classified/described as cosmetic, over-the-counter ("OTC") compounds that find use in personal care applications (e.g., cosmetics, skin care, oral care, hair care, nail care). In some embodiments, the modified variant BBPI is added to a personal care composition such as a hair care composition, a skin care composition, a nail care composition, a cosmetic composition, or any combinations thereof.

As used herein, "skin care composition" refers to compositions that are applied to skin in order to provide beneficial properties, including but not limited to wrinkle minimizing, wrinkle removal, decoloring, coloring, skin softening, skin smoothing, depilation, cleansing, etc. In some particularly preferred embodiments, the present invention provides skin care compositions that improve skin tone. In these embodiments, the improvement comprises lessening of wrinkles, smoothing skin texture, modifying skin coloration, and other desired cosmetic benefits. In further embodiments, the skin care composition is in a form selected from the group consisting of body washes, moisturizing body washes, deodorant body washes, antimicrobial cleansers, skin protecting treatments, body lotions, facial creams, moisturizing creams, facial cleansing emulsions, surfactant-based facial cleansers, facial exfoliating gels, facial toners, exfoliating creams, facial masks, after shave lotions, balms, and/or radioprotective compositions (*e.g.,* sunscreens).

As used herein, "cosmetic composition" refers to compositions that find use in the cosmetics. The Food Drug and Cosmetic Act (FD&C Act) definition is used herein. Thus, cosmetics are defined by their intended use, as articles intended to be rubbed, poured, sprinkled, or sprayed on, introduced into, or otherwise applied to the human body for cleansing, beautifying, promoting attractiveness, or altering appearance. These compositions provide non-therapeutic benefits and are not regulated as pharmaceuticals. However, in some situations, cosmetic compositions are incorporated into pharmaceutical compositions to provide cosmetic benefits (*e.g.,* products that treat skin or hair diseases, but also contain cosmetic compositions for their coloring or other benefits). Cosmetic compositions include makeup compositions as defined herein. Also, it is intended that the present invention encompass the use of cosmetics on animals other than humans.

As used herein, the terms "pharmaceutical compositions" and "therapeutic compositions" refer to compositions such as drugs that provide medical benefits, rather than solely cosmetic benefits. In the United States, pharmaceutical and therapeutic compositions are approved by the Food and Drug Administration for treatment and/or prevention of particular conditions.

As used herein, the term "drug" is defined as it is in the FD&C Act definition. Thus, drugs are defined as articles intended for use in the diagnosis, cure, mitigation, treatment or prevention of disease, and articles (other than food) intended to affect the structure or any function of the body of man or other animals.

As used herein, "leave-on" refers to a composition that is applied to a subject and not removed (*e.g.,* cleansed by washing, rinsing, etc.) for a period of typically at least several hours (*e.g.,* 4-12 hours) before the area exposed to the composition is cleansed.

As used herein, a "rinse-off" composition is a composition that is applied and cleansed (*e.g.,* by washing, rinsing, etc.) soon after its application (generally within about 30 minutes of application). In some preferred embodiments, rinse-off compositions are formulated so as to deposit an effective amount of active(s) on the area treated.

As used herein, the term "cosmetic benefit" refers to a desired cosmetic change that results from the administration of a personal care composition. Cosmetic benefits include but are not limited to improvements in the condition of skin, hair, nails, and the oral cavity. In preferred embodiments, at least one cosmetic benefit is provided by the skin care, hair care, nail care, and makeup compositions of the present invention.

As used herein, "cosmetically acceptable" refers to materials that are suitable for use in contact with tissues of humans and/or other animals, without undue toxicity, incompatibility, instability, irritation, allergic responses, etc., commensurate with a reasonable benefit/risk ratio.

As used herein, the terms "pigment," "color pigment," and "dye" used in reference to the compositions of the present invention encompasses any compound that provides a color to the composition and/or imparts a color to the surface (*e.g.,* skin and/or hair) to which the composition is applied.

The term "radioprotective" refers to a substance capable of blocking or filtering. UV radiation sunscreens and sunblocks.

As used herein, "improving the appearance and/or condition of skin" refers to any benefit achieved through use of the personal care compositions of the present invention. Examples of benefits include but are not limited to reducing the reducing imperfections and/or blemishes in skin color, including lightening hyperpigmented regions of skin and/or evening skin pigmentation, relieving dryness, eliminating rough, dry spots, improving the skin's ability to retain moisture and/or protect itself from environmental stresses, reducing the appearance of fine lines and wrinkles, improving appearance and skin tone, increasing skin firmness and/or suppleness, decreasing sagging of skin, increasing skin glow and clarity, increasing the skin renewal process, and/or removing vellus hair. Improving the visual appearance of skin also encompasses regulating wrinkles, atrophy, skin lightening, skin darkening, skin smoothness, and/or reducing the visual appearance of pores. In some embodiments, improving the appearance and/or condition of the skin results in skin improvements due to the treatment of a skin disorder with the personal care composition of the invention.

The term "angiogenesis" refers to the biological processes which result in the development of blood vessels and/or increase in the vascularization of tissue in an organism.

The terms "angiogenic disease," "angiogenic disorder," and "angiogenic skin disorder," are used in reference to a disorder, generally a skin disorder or related disorder which occurs as a consequence of or which results in increased vascularization in tissue. Oftentimes, the etiology of the angiogenic disease is unknown. However, whether angiogenesis is an actual cause of a disease state or is simply a condition of the disease state is unimportant, but the inhibition of angiogenesis in treating or reversing the disease state or condition is an important aspect of the present invention. Thus, it is not intended that the present invention be limited to any particular mechanisms of action. Examples of angiogenic skin disorders which are suitable for treatment utilizing compounds of the present invention include, but are not limited to psoriasis, acne, rosacea, warts, eczema, hemangiomas and lymphangiogenesis, Sturge-Weber syndrome, neurofibromatosis, tuberous sclerosis, chronic inflammatory disease, and arthritis. Any skin disorder which has as a primary or secondary characterization, increased vascularization, is considered an angiogenic skin disorder herein. Thus, the personal care compositions comprising VEGF-binding BBPIs (VEGF-BBPIs) provided by the present invention find use in treatment of a wide variety of angiogenic skin disorders and/or conditions.

The term "rosacea" is used to describe acne, rosacea, or erythematosa characterized by vascular and follicular dilation typically involving the nose and contiguous portions of the cheeks. Rosacea may vary from very mild but persistent erythema to extensive hyperplasia of the sebaceous glands with deep-seated papules and pustules and be accompanied by telangiectasia at the affected erythematous sites. This condition is also referred to as "hypertrophic rosacea" or "rhinophyma," depending upon the severity of the condition. It is intended that the term encompass all of the various forms of the condition.

The term "psoriasis" is used to describe a skin condition which is characterized by the eruption of circumscribed, discrete and confluent, reddish, silvery-scaled maculopapules. Although it is not intended that the present invention be limited to any particular body area, psoriatic lesions typically occur on the elbows, knees, scalp and trunk. Microscopically, these lesions demonstrate characteristic parakeratosis and elongation of rete ridges.

The term "acne" is used to describe a condition of the skin characterized by inflammatory follicular, papular and pustular eruptions involving the sebaceous apparatus. Although there are numerous forms of acne, the most common form is known as acne simplex or acne vulgaris which is characterized by eruptions of the face, upper back and chest and is primarily comprised of comedones, cysts, papules and pustules on an inflammatory base. The condition occurs primarily during puberty and adolescence due to an overactive sebaceous apparatus which is believed to be affected by hormonal activity.

The term "eczema" is a generic term used to describe acute or chronic inflammatory conditions of the skin, typically erythematous, edematous, papular, vesicular and/or crusting. These conditions are often followed by lichenification, scaling and occasionally, by duskiness of the erythema and, infrequently, hyperpigmentation. Eczema is often accompanied by the sensation of itching and burning. Eczema vesicles form due to intraepidermal spongiosis. Eczema is sometimes referred to colloquially as "tetter," "dry tetter," and "scaly tetter." There are numerous subcategories of eczema, all of which are treated by one or more of the compounds according to the present invention.

The term "hemangioma" refers to a benign self-involuting tumour of endothelial cells (the cells that line blood vessels). Hemangiomas are connected to the circulatory system and filled with blood. The appearance depends on location. If they are on the surface of the skin they look like a ripe strawberry, if they are just under the skin they present as a bluish swelling. Sometimes they grow in internal organs such as the liver or larynx. Approximately 80% are located on the face and neck, with the next most prevalent location being the liver. The personal care compositons of the invention are intended to treat hemangiomas of the skin *i.e.* hemangiomas that are on the surface of the skin and hemangiomas that are just under the skin.

The term "scleroderma" herein refers to a chronic disease characterized by excessive deposits of collagen in the skin or other organs. Scleroderma affects the skin, and in more serious cases it can affect the blood vessels and internal organs. The most evident symptom is usually the hardening of the skin and associated scarring. The skin may appear tight, reddish or scaly. Blood vessels may also be more visible. A significant player in the process is transforming growth factor (TGFβ). Topical treatment for the skin changes of scleroderma do not alter the disease course, but may improve pain and ulceration.

As used herein, "hair care composition" refers to compositions that are applied to hair to provide beneficial properties such as thickening, thinning, coloring, decoloring, cleansing, conditioning, softening, shaping, etc. In some embodiments, the hair care composition is in a form selected from the group consisting of shampoos, conditioners, anti-dandruff treatments, styling aids, styling conditioners, hair repair or treatment sera, lotions, creams, pomades, and chemical treatments. In other embodiments, the styling aids are selected from the group consisting of sprays, mousses, rinses, gels, foams, and combinations thereof. In further embodiments, the chemical treatments are selected from the group consisting of permanent waves, relaxers, and permanents, semi-permanents, temporary color treatments and combinations thereof.

As used herein, "inhibiting hair growth" and "inhibition of hair growth" refer to an observed lessening of hair length and/or thickness. Thus, in some preferred embodiments, application of a personal care composition of the present invention provides a benefit in lessening hair length and/or thickness as compared to an area in which a personal care composition of the present invention has not been applied. In some embodiments, the observed reduction of hair growth and/or thickness is a range from less than 1% to more than 99%, as compared to untreated areas, while in other embodiments, the observed reduction is from about 100% to about 90%, from about 90% to about 80%, from about 80% to about 70%, from about 70% to about 60%, from about 60% to about 50%, from about 50% to about 40%, from about 40% to about 30%, from about 30% to about 20%, from about 20% to about 10%, from about 10% to about 1%. Indeed, it is not intended that the term be limited to any particular percentage reduction, as long as the reduction is observable by visual (*i.e*., by eye) or other means. It is also intended that the term encompass "preventing hair growth" to any degree, as described above. It is not intended that the term be limited to the complete prevention of hair growth (*i.e.,* there is no observed growth of hair).

The terms "dermatological inflammatory disorder" or "inflammatory skin disorder" refer to skin condition associated with inflammation of the skin. In some embodiments, the inflammatory skin disorder is a disorder associated with elevated levels of inflammatory cytokines *e.g*. TNFα.

As used herein, in some embodiments, the term "compound" refers to the BBPI comprised in the personal care compositions of the invention.

The term "effective amount" is used throughout the specification to describe concentrations or amounts of compounds according to the present invention which may be used to produce a favorable change in the disease or condition treated, *e.g*. whether that change is hair growth, prevention of hair growth or for ameliorating a condition caused or associated with a disorder. As used herein, "safe and effective amount" refers to a sufficient amount of a material that significantly induces a positive modification to the area upon which the material is applied and also does not result in the production of serious side effects (at a reasonable risk/benefit ratio). The safe and effective amount of the material may vary with the particular skin or other body part being treated, the age of the subject being treated, the severity of the condition being treated, the duration of treatment, the nature of concurrent therapy, the specific material used, the particular carrier utilized, etc. Those of skill in the art are capable of adjusting the concentration of the personal care compositions provided herein for the desired application of the compositions.

As used herein, "active" (and "actives") refers to a composition that imparts a benefit to a subject being treated. For example, in some embodiments, the present invention provides personal care compositions comprising a modified variant BBPI, *e.g*. modified variant VEGF-BBPI, a "primary active" which functions to provide benefit to the area to which it is applied. Thus, in some embodiments, the modified variant VEGF-BBPI is present in skin care formulations and serves to treat the skin of subjects suffering from an angiogenic skin disorder. It is not intended that the term be limited to VEGF-BBPI, as there are additional constituents present in the personal care compositions of the present invention which impart benefits. In some embodiments, these additional constituents are encompassed by the designation "secondary actives." Primary and secondary actives are collectively referred to as "actives" herein. Other "primary actives" provided by the invention include FGF-BBPIs, TGF-BBPls and TNF-BBPls.

As used herein, "vitamin B₃ compound" means a compound having the formula: 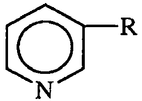 wherein R is - CONH₂ (*i.e.,* niacinamide), - COOH (*i.e*., nicotinic acid) or - CH₂OH (*i.e.,* nicotinyl alcohol); derivatives thereof; and salts of any of the foregoing.

As used herein, "non-vasodilating" means that an ester does not commonly yield a visible flushing response after application to the skin in the subject compositions. It is contemplated that the majority of the general population would not experience a visible flushing response, although such compounds may cause vasodilation not visible to the naked eye.

As used herein, "retinoid" includes all natural and/or synthetic analogs of Vitamin A and/or retinol-like compounds which possess the biological activity of Vitamin A in/on the skin, as well as the geometric isomers and stereoisomers of these compounds. However, it is not intended that the term be limited to these compounds, as the term encompasses vitamin A alcohol (retinol) and its derivatives such as vitamin A aldehyde (retinal), vitamin A acid (retinoic acid) and vitamin A esters (*e.g*., retinyl acetate, retinyl propionate and retinyl palmitate), etc. It is further intended that the term encompass all-trans-retinoic acids and 13-cis-retinoic acids. It is also intended that the term encompass compositions that are encapsulated, as well as provided for use in various forms. The terms "retinol" and "retinal" preferably comprise the all-trans compounds. The retinoid preferably used for the formulation of the present invention is all-trans-retinol, generally referred to as "retinol" herein.

As used herein, "carotenoid" is used in reference to β-carotene, lycopene, lutein, astaxanthin, zeaxanthin, cryptoxanthin, citranaxanthin, canthaxanthin, bixin, β-apo-4-carotenal, β-apo-8-carotenal, β-apo-8-carotenoic esters, alone, as well as in combination. Carotenoids which are preferably used are β-carotene, lycopene, lutein, astaxanthin, zeaxanthin, citranaxanthin and canthaxanthin. In some embodiments, carotenoids are utilized in crystalline form, as well as in formulations, including but not limited to dry powders (*See e.g.,* dry powders, as described in EP 0 065 193). In some embodiments, the preferred use in the case of lycopene, astaxanthin and canthaxanthin is of lycopene-, astaxanthin- and canthaxanthin-containing dry powders, for example LYCOVIT^{®}, LUCANTIN^{®} Pink and LUCANTIN^{®} Red (10% dry powders respectively of lycopene, astaxanthin and canthaxanthin, commercially available from BASF AG, Ludwigshafen, Germany.
As used herein, the term "dispersed phase" is used as by those of skill in the art of emulsion technology as the phase that exists as small particles or droplets suspended in and surrounded by a continuous phase. The dispersed phase is also known as the "internal" or "discontinuous" phase.

As used herein, "penetration enhancers" refer to compositions that facilitate penetration through the upper stratum corneum barrier to the deeper skin layers. Examples of penetration enhancers include, but are not limited to, propylene glycol, azone, ethoxydiglycol, dimethyl isosorbide, urea, ethanol, dimethyl sulfoxide, micoroemulsions, liposomes, and nanoemulsions.

As used herein, the terms "emulsifier" and "surfactant" refer to compounds that disperse and suspend the dispersed phase within the continuous phase of a material. Surfactants find particular use in products intended for skin and/or hair cleansing. In particular embodiments, the term "surfactant(s)" is used in reference to surface-active agents, whether used as emulsifiers or for other surfactant purposes such as skin cleansing.

In various embodiments, the present invention also includes "protectants" such as UV absorbers (*e.g*., octyl methoxycinnamate, benzophenone-3, titanium dioxide, and octyl salicylate); film-forming agents (*e.g*., VP/Eicosene copolymer); cosmeceutical agents (*e.g*., peptides and proteins, alpha hydroxy acids, and retinol and retinoic acid derivatives); antioxidants (*e.g*., tocopherol and derivatives thereof and ascorbic acid and derivatives thereof); vitamins (*e.g*., B, D, K and their derivatives); antiperspirant actives (*e.g*., aluminum hydroxide and zirconium hydroxide); depilating agents (*e.g.*, thioglycolate salts); anti-acne agents (*e.g*., salicylic acid and benzoyl peroxide); abrasives and exfoliants (*e*.*g*., silicates, pumice, and polyethylene); and extracts of plant, fruit, vegetable and/or marine sources.

As used herein, the term "bioactivity" refers to a cause and effect relationship between a composition and a biological system. Thus, the term is used as by those skilled in the art of biotechnology and biological sciences as the phrase that describes a cause and effect relationship between a molecular composition and living biological matter (*e.g.*, tissue, cells, etc.).

As used herein as a noun, the term "bioactive" refers a composition that exhibits bioactivity upon administration to living biological matter (*e.g*., tissue, cells, etc.). The term is used synonymously with "bioactive compound."

As used herein, "silicone gum" means high molecular weight silicones having an average molecular weight in excess of about 200,000 and preferably from about 200,000 to about 4,000,000. It is intended that the definition encompass non-volatile polyalkyl and polyaryl siloxane gums.

As used herein, a "composition comprising a modified variant BBPI" refers broadly to any composition containing the given modified variant BBPI. The composition may be in any form, particularly a form that is suitable for administration.

As used herein, a compound is said to be "in a form suitable for administration" when the compound may be administered to a human or other animal by any desired route (e.g., topical, oral, etc.).

As used herein, "safe and effective amount" refers to a sufficient amount of a material that significantly induces a positive modification to the area upon which the material is applied and also does not result in the production of serious side effects (at a reasonable risk/benefit ratio). The safe and effective amount of the material may vary with the particular skin or other body part being treated, the age of the subject being treated, the severity of the condition being treated, the duration of treatment, the nature of concurrent therapy, the specific material used, the particular carrier utilized, etc. Those of skill in the art are capable of adjusting the concentration of the personal care compositions provided herein for the desired application of the compositions.

### 5.2 Bowman Birk Protease Inhibitor Scaffolds

Bowman Birk Protease Inhibitor proteins (BBPI) are a kinetically and structurally well-characterized family of small proteins (60-90 residues), and have been found only in the seed of monocotyledous and dicotyledonous plants, and have not been identified in any other part of the plant *(See e.g.,* Birk, Int. J. Pept. Protein Res., 25:113-131 [1985]). The sequences of many wild-type BBPI scaffolds have been determined from both monocotyledonous and dicotyledonous seeds, and have been analyzed (Prakash et al., J mol Evol 42:560-569 [1996]). They typically have a symmetrical structure of two tricyclic domains each containing an independent binding loop, although some have one domain and some have more than two domains. The major ∼ 8 kDa Bowman Birk Inhibitor isolated from soybeans (BBI) has two separate reactive site loops, loop I inhibits proteases having trypsin-like specificity and loop II inhibits proteases with chymotrypsin-like specificity (*See e.g.,* Chen et al., J. Biol. Chem., 267:1990-1994 [1992]; Werner and Wemmer, Biochem., 31:999-1010 [1992]; Lin et al., Eur. J. Biochem., 212:549-555 [1993]; Voss et al., Eur. J. Biochem., 242:122-131 [1996]; and Billings et al., Pro. Natl. Acad. Sci., 89:3120-3124 [1992]). These binding regions each contain a "canonical loop" structure, which is a motif found in a variety of serine proteinase inhibitors (Bode and Huber, Eur. J. Biochem., 204:433-451 [1992]). In some embodiments, wild-type BBPI scaffolds serve as wild-type precursor BBPI scaffolds from which variant and modified variant BBPI scaffolds are derived.

The present invention provides for variant BBPI scaffolds in which the chymotrypsin loop (loop II) is replaced by a variant peptide. In some embodiments, the variant BBPI retains trypsin inhibitory activity (TIA). In other embodiments, both the trypsin and the chymotrypsin loops of the BBPI are each replaced with a variant peptide. The modifiied BBPIs of the invention are based on the wild type scaffold of the soybean inhibitor from *Glycine max*
(BBI; SEQ ID NO:13).. An unmodified variant precursor scaffolds in which the chymotrypsin loop has been replaced with a VEGF binding variant sequence is BBIt-AV (SEQ ID NO:187;
DDESSKPCCDQCACTKSNPPQCRCSDMRLNSCHSACKSCACYNLYGWTCFCVDI TDFCYEPCKPSE),

### 5.3 Variant BBPIs

As indicated above, BBPIs have binding loops (*i.e.* trypsin and chymotrypsin loops) that inhibit proteases. The present invention provides modified variant BBPIs, which are derived from unmodified variant BBPI precursor scaffolds in which Loop II (chymotrypsin) has been replaced with a variant peptide that binds a target protein selected from TNF-α, TGF-β, FGF-5 VEGF.

In some embodiments, the chymotrypsin loop of the BBPI precursor scaffold is replaced with variant sequence that binds VEGF to generate variant VEGF-BBPI proteins.

In some embodiments, the VEGF variant sequences include, but are not limited to VEGF-binding peptides disclosed in US 2002/0098524 (09/832,723) and US 7,524,816 (10/984,270), including peptides KYYLYWW (SEQ ID NO:458), TLWKSYW (SEQ ID NO:459), DLYWW (SEQ ID NO:460),SKHSQIT (SEQ ID NO:468) KTNPSGS (SEQ ID NO:469) RPTGHSL (SEQ ID NO:470), KHSAKAE (SEQ ID NO:471) KPSSASS (SEQ ID NO:472), PVTKRVH (SEQ ID NO:473), TLHWWVT (SEQ ID NO:492), PYKASFY (SEQ ID NO:493), PLRTSHT (SEQ ID NO:494), EATPROT (SEQ ID NO:495), NPLHTLS (SEQ ID NO:496), KHERIWS (SEQ ID NO:497), ATNPPPM (SEQ ID NO:498), STTSPNM (SEQ ID NO:499), ADRSFRY (SEQ ID NO:500), PKADSKQ (SEQ ID NO:501), PNQSHLH (SEQ ID NO:502), SGSETWM (SEQ ID NO:503), ALSAPYS (SEQ ID NO:504), KMPTSKV (SEQ ID NO:505), ITPKRPY (SEQ ID NO:506), KWIVSET (SEQ ID NO:507), PNANAPS (SEQ ID NO:508), NVQSLPL (SEQ ID NO:509), TLWPTFW (SEQ ID NO:510), NLWPHFW (SEQ ID NO:511), SLWPAFW (SEQ ID NO:512), SLWPHFW (SEQ ID NO:513), APWNSHI (SEQ ID NO:514), APWNLHI (SEQ ID NO:515), LPSWHLR (SEQ ID NO:516), PTILEWY (SEQ ID NO:517), TLYPQFW (SEQ ID NO:518), and HLAPSAV (SEQ ID NO:519),. In some other embodiments, the VEGF variant sequences include, but are not limited to VEGF-binding peptides disclosed in U.S. 2012/0014885 (11/919,717), including peptides KYYLSWW (SEQ ID NO:520), WYTLYKW (SEQ ID NO:521), TYRLYWW (SEQ ID NO:522), RYSLYYW (SEQ ID NO:523), YYLYYWK (SEQ ID NO:524), NYQLYGW (SEQ ID NO:525), TKWPSYW (SEQ ID NO:226), TLWKSYW (SEQ ID NO:527), PLWPSYW (SEQ ID NO:528), RLWPSYW (SEQ ID NO:529), TLWPKYW (SEQ ID NO:530), KYDLYWW (SEQ ID NO;531), RYDLYWW (SEQ ID NO:532), DYRLYWW (SEQ ID NO:533), DYKLYWW (SEQ ID NO:534), EYKLYWW (SEQ ID NO:535), and RYPLYWW (SEQ ID NO:536).

In some embodiments, the chymotrypsin loop of the BBPI precursor scaffold is replaced with variant sequences that interact with FGF5 to generate variant FGF-BBPI proteins.

In some embodiments, the FGF5 variant sequences include, but are not limited to FGF5-binding peptides disclosed in U.S. 7,485,618 (10/984,410) and US 2008/0269139 (12/033,848), including peptides TNIDSTP(SEQ ID NO:544), HLQTTET (SEQ ID NO:545), SLNNLTV (SEQ ID NO:546), TNIDSTP (SEQ ID NO:547), TNIDSTP (SEQ ID NO:548), LRILANK (SEQ ID NO:549), LLTPTLN (SEQ ID NO:550), ALPTHSN (SEQ ID NO:551), TNIDSTP (SEQ ID NO:552), LCRRFEN (SEQ ID NO:553), TNIDSTP (SEQ ID NO:554), TNIDSTP (SEQ ID NO:555), HLQTTET (SEQ ID NO:556), PLGLCPP (SEQ ID NO:557), GYFIPSI (SEQ ID NO:558), TKIDSTP (SEQ ID NO:559), HLQTTET (SEQ ID NO:560), WNIDSTP (SEQ ID NO:561), TWIDWTP (SEQ ID NO:562), RTQPYPL (SEQ ID NO:670) and TWIDSTP (SEQ ID NO:671).

In some embodiments, the chymotrypsin loop of the BBPI precursor scaffold is replaced with variant sequences that interact with TGFβ to generate variant TGF-BBPIs.
In some embodiments, the TGFβ variant sequences include, but are not limited to TGFβ-binding peptides disclosed in U.S. 2008/0113917 (10/581,142), including peptides QSACIVYYVGRKPKVECASSD (SEQ ID NO:566), QSACILYYIGKTPKIECASSD (SEQ ID NO:567), QSACILYYVGRTPKVECASSD (SEQ ID NO:568), KHNVRLL (SEQ ID NO:570), NDTPSYF (SEQ ID NO:571), AKLYAGS (SEQ ID NO:572), RGPAHSL (SEQ ID NO:573), NSLAERR (SEQ ID NO:574), HPLASPH (SEQ ID NO:575), QPWNKLK (SEQ ID NO:576), PTKPAQQ (SEQ ID NO:578), PSLNRPQ (SEQ ID NO:579), HHARQEW (SEQ ID NO:580), RHHTPGP (SEQ ID NO:581), ASAINPH (SEQ ID NO:582), PENINVLP (SEQ ID NO;672) and KHNVDWL (SEQ ID NO:673).

In some embodiments, the chymotrypsin loop of the BBPI precursor scaffold is replaced with variant sequences that interact with TNFα to generate variant TNF-BBPIs.

In some embodiments, the TNFα binding sequences include, but are not limited to TNF-binding peptides disclosed in U.S. 2005/0112692 (10/968,732), including peptides RYWQDIP (T1; SEQ ID NO:474), APEPILA (T2; SEQ ID NO:475), DMIMVSI (T3; SEQ ID NO:476), WTPKPTQ (SEQ ID NO:583), ATFPNQS (SEQ ID NO:584), ASTVGGL (SEQ ID NO:585), TMLPYRP (SEQ ID NO:586), AWHSPSV (SEQ ID NO:587), TQSFSS (SEQ ID NO:588), THKNTLR (SEQ ID NO:589), GQTHFHV (SEQ ID NO:590), LPILTQT (SEQ ID NO:591), SILPVSH (SEQ ID NO:592), SOPIPI (SEQ ID NO:593), and QPLRKLP (SEQ ID NO:594).

In some embodiments, the variant BBPIs further comprises a peptide insert that is positioned at the N-terminus of the modified variant BBPI. In some embodiments, the peptide insert comprises a sequence of between 1 and 15 amino acids. In other embodiments, the peptide insert comprises a sequence between 5 and 10 amino acids. In some embodiments, the peptide insert comprises the peptide of SEQ ID NO:389 (DDEPSKPCCDPDP; SEQ ID NO:389). Examples of modified variant BBPIs that comprise the peptide insert of SEQ ID NO:389 are the modified variant 4D13BBIt-AV of
(DDEPSKPCCDPDPDDESSKPCCDQCACTKSNPPQCRCSDMRLNSCHSACKSCA CYNLYGWTCFCVDITDFCYEPCKPSE; SEQ ID NO:390), and the modified variant BBIt-AV-4D13-13I-29P-40K-50T-52A of SEQ ID NO: 413

(DPDDEPSKPCCDPDPDDESSKPCCDQCICTKSNPPQCRCSDMRPNSCHSACKSC
KCYNLYGWTCTCADITDFCYEPCKPSE; SEQ ID NO:413).

In most embodiments, the loop is replaced with a variant sequence *i.e.,* peptides often 3 to 14 amino acids in length, with 5 to 10 amino acids being preferred, to generate the variant BBPI. Longer sequences find use in the present invention, as long as they provide the binding and/or inhibition desired. In addition, peptides suitable for use as replacements of the binding loop(s) preferably adopt a functional conformation when contained within a constrained loop (*i.e.,* a loop formed by the presence of a disulfide bond between two cysteine residues). In some specific embodiments, the peptides are between 7 and 9 amino acids in length. In other embodiments, the variant sequences are peptides of 10 amino acids in length.

### 5.4 Modified Variant BBPI proteins

### 5.4.1 Modified Variant BBPIs: single amino acid substitutions

The invention provides modified variant BBPIs, which are variant BBPIs that further comprise at least one amino acid substitution in the backbone of the BBPI. Thus, modified variant BBPIs are variant BBPIs that contain a chymotrypsin loop that has been replaced by a variant sequence that binds to a target protein, and that are further altered by comprising at least one amino acid substitution at at least one position equivalent to a position chosen from positions equivalent to positions 1, 4, 5, 11, 13, 18, 25, 27, 29, 31, 38, 40, 50, 52, 55, and 65 of SEQ ID NO: 187.

An amino acid residue of a modified variant BBPI is at an equivalent to the position of a residue of a precursor BBPI if it is homologous (*i.e.* corresponding in position in primary structure) to a specific residue. In order to establish homology to primary structure, the amino acid sequence of a precursor BBPI is directly compared to the primary amino acid sequence, and particularly to the set of cysteine residues known to be conserved in BBPIs for which the sequence is known. Figure 17 shows the conserved cysteine residues among exemplary wild type and variant precursor BBPIs described herein. Equivalent residues that are substituted in the modified variant BBPIs of the invention are numbered.

The substitution of the at least one amino acid generates a modified variant BBPI that has a greater trypsin inhibitory activity than that of the unmodified variant precursor BBPI and/or increased production yield than that of the unmodified variant precursor BBPI.

In one embodiment, the substituted amino acid at the amino acid position equivalent to position 1 of SEQ ID NO:187 is chosen from A and C. In another embodiment, the substituted amino acid at the amino acid position equivalent to position 4 of SEQ ID NO: 187 is V. In another embodiment, the substituted amino acid at the amino acid position equivalent to position 5 of SEQ ID NO:187 is chosen from P, and A. In another embodiment, , the substituted amino acid at the amino acid position equivalent to position 11 of SEQ ID NO:187 is G. In another embodiment, the substituted amino acid at the amino acid position equivalent to position 13 of SEQ ID NO:187 is chosen from Y, I, F, M, L, V, K, and R. In another embodiment, the substituted amino acid at the amino acid position equivalent to position 18 of SEQ ID NO:187 include I, V and L. In another embodiment, the substituted amino acid at the amino acid position equivalent to position 25 of SEQ ID NO:187 is chosen from K, N, W, I, A and R. In another embodiment, the substituted amino acid at the amino acid position equivalent to position 27 of SEQ ID NO:187 include R, K, V, A, and Q. In another embodiment, the substituted amino acid at the amino acid position equivalent to position 29 of SEQ ID NO:187 is chosen from R, K, and P. In another embodiment, the substituted amino acid at the amino acid position equivalent to position 31 of SEQ ID NO:187 is chosen from Q, H, E, A, R, W, K and T. In another embodiment, the substituted amino acid at the amino acid position equivalent to position 38 of SEQ ID NO:187 is chosen from N, K and R. In another embodiment, the substituted amino acid at the amino acid position equivalent to position 50 of SEQ ID NO:187 is chosen from R, Q, K, T, V, M, and S. In another embodiment, the substituted amino acid at the amino acid position equivalent to position 52 of SEQ ID NO:187 is chosen from K, T, R, Q, L, H, A, M, S and E. In another embodiment, the substituted amino acid at the amino acid position equivalent to position 55 of SEQ ID NO:187 is M. In another embodiment, the substituted amino acid at the amino acid position equivalent to position 65 of SEQ ID NO:187 is chosen from E, Q, and D.

If a substitution is present at position 40, the substituted amino acid at the amino acid position equivalent to position 40 is chosen from H, K, Q, R and Y. Any one of the single amino acid substitutions described above and made in the variant BBIt-AV BBPI of SEQ ID NO:187 generated modified variant BBIT-AV BBPIs that have greater trypsin inhibitory activity than the unmodified precursor variant BBIt-AV (SEQ ID NO:187; see Example 10).

### 5.4.2 Modified Variant BBPIs: combinations of amino acid substitutions

The invention encompasses modified variant BBPIs comprising at least two, at least three, at least four, at least five, at least six, at least seven, at least eight, at least nine, at least ten, at least eleven, at least twelve, at least thirteen, at least fourteen, at least fifteen and at least sixteen amino acid substitutions. In some embodiments, the at least two, at least three, at least four, at least five, at least six, at least seven, at least eight amino, at least nine, at least ten, at least eleven, at least twelve, at least thirteen, at least fourteen, at least fifteen and at least sixteen acid substitutions generate modified variant BBPIs that have greater TIA than the unmodified precursor variant BBPI. In other embodiments, the at least two, at least three, at least four, at least five, at least six, at least seven, at least eight, amino acid substitutions generate modified variant BBPIs that have greater TIA and production yield than the unmodified precursor variant BBPI.

In some embodiments, the modified variant BBPI comprises a combination of two amino acid substitutions at amino acids at positions equivalent to positions 50 and 52 of SEQ ID NO:187. In some embodiments, the combination of two amino acid substitutions is 50T-52A. The invention provides for any one of the variant BBPI scaffolds described in Section 5.3 and further comprising the combination of the two amino acid substitutions 50T-52A, as described in section 5.4. In one embodiment, the chymotrypsin loop of the variant scaffold is a VEGF variant and the variant scaffold is altered further to comprise a combination of three amino acid substitutions at positions equivalent to positions 13, 50 and 52 of SEQ ID NO:187 to generate a modified variant BBPI scaffold. In one embodiment, the modified variant BBPI comprising a combination of two amino acid substitutions is the modified variant BBIt-AV-F50T-V52A of SEQ ID NO: 595

(DPDDESSKPCCDQCACTKSNPPQCRCSDMRLNSCHSACKSCACYNLYGWTCTC
ADITDFCYEPCKPSE ; SEQ ID NO:595).

In some embodiments, the modified variant BBPI comprises a combination of three amino acid substitutions at amino acids at positions equivalent to positions 13, 50 and 52 of SEQ ID NO:187. In some embodiments, the combination of three amino acid substitutions is chosen from a combination of substitutions at positions 25-50-52, 29-50-52, 40-50-52, and 13-50-52. In some embodiments, the combination of three amino acid substitutions is chosen from 25L-50T-52A, 29P-50T-52A, 40K-50T-52A and 13I-50T-52A. The invention provides for any one of the variant BBPI scaffolds described in Section 5.3 and further comprising the combination of the three amino acid substitutions chosen from 25L-50T-52A, 29P-50T-52A, 40K-50T-52A and 13I-50T-52A, as described in section 5.4. In one embodiment, the chymotrypsin loop of the variant scaffold is a VEGF variant peptide and the variant scaffold is altered further to comprise a combination of three amino acid substitutions at positions equivalent to positions 13, 50 and 52 of SEQ ID NO:187 to generate a modified variant BBPI scaffold. In one embodiment, the modified variant BBPI comprising a combination of three amino acid substitutions is chosen from the modified variant BBIt-AV-S25L-F50T-V52A of SEQ ID NO: 603 (DPDDESSKPCCDQCACTKSNPPQCRCLDMRLNSCHSACKSCACYNLYGWTCTC ADITDFCYEPCKPSE ; SEQ ID NO:603), the modified variant BBIt-AV-L29P-F50T-V52A of SEQ ID NO:607
(DPDDESSKPCCDQCACTKSNPPQCRCSDMRPNSCHSACKSCACYNLYGWTCTC ADITDFCYEPCKPSE ; SEQ ID NO:607), and the modified variant BBIt-AV-A40K-F50T-V52A of SEQ ID NO:609
(DPDDESSKPCCDQCACTKSNPPQCRCSDMRLNSCHSACKSCKCYNLYGWTCTC ADITDFCYEPCKPSE; SEQ ID NO:609).

In some embodiments, the modified variant BBPI comprises a combination of four amino acid substitutions at amino acids at positions equivalent to positions 13, 29, 50 and 52 of SEQ ID NO:187. In some embodiments, the combination of four amino acid substitutions is chosen from a combination of substitutions at positions 13-25-50-52, 13-29-50-52, 25-29-50-52, 13-40-50-52, 25-40-50-52, and 29-40-50-52. In some embodiments, the combination of four amino acid substitutions is chosen from 13I-25L-50T-52A, 13I-29P-50T-52A, 25L-29P-50T-52A, 13I-40K-50T-52A, 25L-40K-50T-52A, and 29P-40K-50T-52A. The invention provides for any one of the variant BBPI scaffolds described in Section 5.3 and further comprising the combination of the four amino acid substitutions chosen from 13I-25L-50T-52A, 131-29P-50T-52A, 25L-29P-50T-52A, 13I-40K-50T-52A, 25L-40K-50T-52A, and 29P-40K-50T-52A, as described in section 5.4. In one embodiment, the chymotrypsin loop of the variant scaffold is a VEGF variant peptide such as SEQ ID NO:460, and the variant scaffold is altered further to comprise a combination of four amino acid substitutions at positions equivalent to positions 13, 29, 50 and 52 of SEQ ID NO:187 to generate a modified variant BBPI scaffold. In one embodiment, the modified variant BBPI comprising a combination of four amino acid substitutions is chosen from the modified variant BBIt-AV-A13I-S25L-F50T-V52A of SEQ ID NO:596 (DPDDESSKPCCDQCICTKSNPPQCRCLDMRLNSCHSACKSCACYNLYGWTCTCA DITDFCYEPCKPSE ; SEQ ID NO:596), the modified variant BBIt-AV-A13I-L29P-F50T-V52A of SEQ ID NO:600
(DPDDESSKPCCDQCICTKSNPPQCRCSDMRPNSCHSACKSCACYNLYGWTCTCA DITDFCYEPCKPSE ; SEQ ID NO:600), the modified variant BBIt-AV-A13I-A40K-F50T-V52A of SEQ ID NO:602
(DPDDESSKPCCDQCICTKSNPPQCRCSDMRLNSCHSACKSCKCYNLYGWTCTCA DITDFCYEPCKPSE ; SEQ ID NO:602), the modified variant BBIt-AV-S25L-L29P-F50T-V52A of SEQ ID NO:604
(DPDDESSKPCCDQCACTKSNPPQCRCLDMRPNSCHSACKSCACYNLYGWTCTC ADITDFCYEPCKPSE ; SEQ ID NO:604), the modified variant BBIt-AV-S25L-A40K-F50T-V52A of SEQ ID NO:606
(DPDDESSKPCCDQCACTKSNPPQCRCLDMRLNSCHSACKSCKCYNLYGWTCTC ADITDFCYEPCKPSE ; SEQ ID NO:606), and the modified variant BBIt-AV-L29P-A40K-F50T-V52A of SEQ ID NO:608
(DPDDESSKPCCDQCACTKSNPPQCRCSDMRPNSCHSACKSCKCYNLYGWTCTC ADITDFCYEPCKPSE ; SEQ ID NO:608). In one embodiment, the modified variant BBPI is chosen from the modified variant BBIt-MM007-Q-A13I-L29P-F50T-V52A of SEQ ID NO:432, and the modified variant BBIt-FGFps2-Q-A13I-L29P-F50T-V52A of SEQ ID NO:434. In another embodiment, the chymotrypsin loop of the variant scaffold is a TGFβ variant peptide chosen from SEQ ID NOS: 672, 673, and 674 and the variant scaffold is altered further to comprise a combination of four amino acid substitutions at positions equivalent to positions 13, 29, 50 and 52 of SEQ ID NO:187 to generate a modified variant BBPI scaffold. In one embodiment, the modified variant BBPI comprising a combination of four amino acid substitutions is chosen from the modified variant BBIt-PEN3-Q-A13I-L29P-F50T-V52A of SEQ ID NO:443, the modified variant BBIt-MM021W-Q-A13I-L29P-F50T-V52A of SEQ ID NO:445, and the modified variant BBIt-WTQ-Q-A13I-L29P-F50T-V52A of SEQ ID NO:447.

In some embodiments, the modified variant BBPI comprises a combination of five amino acid substitutions at amino acids at positions equivalent to positions 13, 29, 40, 50 and 52 of SEQ ID NO:187. In some embodiments, the combination of five amino acid substitutions is chosen from a combination of substitutions at positions 13-25-29-50-52, 13-29-40-50-52, 13-25-40-50-52, 25-29-40-50-52, and 13-29-40-50-52. In some embodiments, the combination of five amino acid substitutions is chosen from 13I-25L-29P-50T-52A, 13I-29P-40K-50T-52A, 13I-25L-40K-50T-52A, 25L-29P-40K-50T-52A, 13L-29P-40K-50T-52A, 13I-29K-40K-50T-52A, 13I-29P-40K-50K-52A and 13I-29P-40K-50T-52T. The invention provides for any one of the variant BBPI scaffolds described in Section 5.3 and further comprising the combination of the five amino acid substitutions chosen from 13I-25L-29P-50T-52A, 131-29P-40K-50T-52A, 13I-25L-40K-50T-52A, 25L-29P-40K-50T-52A, 13L-29P-40K-50T-52A, 13I-29K-40K-50T-52A, 13I-29P-40K-50K-52A and 13I-29P-40K-50T-52T, as described in section 5.4. In one embodiment, the modified variant BBPI comprising a combination of five amino acid substitutions is chosen from the modified variant BBIt-AV-A13I-S25L-L29P-F50T-V52A of SEQ ID NO:597
(DPDDESSKPCCDQCICTKSNPPQCRCLDMRPNSCHSACKSCACYNLYGWTCTCA DITDFCYEPCKPSE ; SEQ ID NO:597), the modified variant BBIt-AV-A13I-L29PA40K-F50T-V52A of SEQ ID NO:599
(DPDDESSKPCCDQCICTKSNPPQCRCLDMRLNSCHSACKSCKCYNLYGWTCTCA DITDFCYEPCKPSE ; SEQ ID NO:599), the modified variant BBIt-AV-A13I-S25L-A40K-F50T-V52A of SEQ ID NO:601
(DPDDESSKPCCDQCICTKSNPPQCRCSDMRPNSCHSACKSCKCYNLYGWTCTCA DITDFCYEPCKPSE ; SEQ ID NO:601), the modified variant BBIt-AV-S25L-L29P-A40K-F50T-V52A of SEQ ID NO:605
(DPDDESSKPCCDQCACTKSNPPQCRCLDMRPNSCHSACKSCKCYNLYGWTCTC ADITDFCYEPCKPSE ; SEQ ID NO:605), the modified variant BBIt-AV-A13L-L29PA40K-F50T-V52A of SEQ ID NO:615
(DPDDESSKPCCDQCLCTKSNPPQCRCSDMRPNSCHSACKSCKCYNLYGWTCTC ADITDFCYEPCKPSE ; SEQ ID NO:615), the modified variant BBIt-AV-A13I-L29K-A40K-F50T-V52A of SEQ ID NO:620
(DPDDESSKPCCDQCICTKSNPPQCRCSDMRKNSCHSACKSCKCYNLYGWTCTCA DITDFCYEPCKPSE ; SEQ ID NO:620), the modified variant BBIt-AV-A13I-L29P-A40K-F50K-V52A of SEQ ID NO:624
(DPDDESSKPCCDQCICTKSNPPQCRCSDMRPNSCHSACKSCKCYNLYGWTCKC ADITDFCYEPCKPSE ; SEQ ID NO:624), and the modified variant BBIt-AV-A13I-L29P-A40K-F50T-V52T of SEQ ID NO:625
(DPDDESSKPCCDQCICTKSNPPQCRCSDMRPNSCHSACKSCKCYNLYGWTCTCT DITDFCYEPCKPSE ; SEQ ID NO:625).

In some embodiments, the modified variant BBPI comprises a combination of six amino acid substitutions at amino acids at positions equivalent to positions 13, 25, 29, 40, 50 and 52 of SEQ ID NO:187. In some embodiments, the combination of six amino acid substitutions is chosen from a combination of substitutions at positions 13-25-29-40-50-52, 1-13-29-40-50-52, 4-13-29-40-50-52, 5-13-29-40-50-52, 11-13-29-40-50-52, 13-25-29-40-50-52, 13-27-29-40-50-52, 13-29-31-40-50-52, 13-29-31-40-50-52, 13-29-38-40-50-52, and 13-29-38-40-50-52. In some embodiments, the combination of six amino acid substitutions is chosen from 13I-25L-29P-40K-50T-52A, 1C-13I-29P-40K-50T-52A, 4V-13I-29P-40K-50T-52A, 5P-13I-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A, and 13I-29P-38N-40K-50T-52A. The invention provides for any one of the variant BBPI scaffolds described in Section 5.3 and further comprising the combination of the six amino acid substitutions chosen from 13I-25L-29P-40K-50T-52A, 1C-13I-29P-40K-50T-52A, 4V-13I-29P-40K-50T-52A, 5P-13I-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A, and 13I-29P-38N-40K-50T-52A, as described in section 5.4. In one embodiment, the modified variant BBPI comprising a combination of six amino acid substitutions is chosen from the modified variant BBIt-AV-A13I-S25L-L29P-A40K-F50T-V52A of SEQ ID NO:598
(DPDDESSKPCCDQCICTKSNPPQCRCLDMRPNSCHSACKSCKCYNLYGWTCTCA DITDFCYEPCKPSE ; SEQ ID NO:598), the modified variant BBIt-AV-D1C-A13I-L29P-A40K-F50T-V52A of SEQ ID NO:611
(DPDDEVSKPCCDQCICTKSNPPQCRCSDMRPNSCHSACKSCKCYNLYGWTCTCA DITDFCYEPCKPSE ; SEQ ID NO:611), the modified variant BBIt-AV-S4V-A13I-L29P-A40K-F50T-V52A of SEQ ID NO:612
(DPDDEVSKPCCDQCICTKSNPPQCRCSDMRPNSCHSACKSCKCYNLYGWTCTCA DITDFCYEPCKPSE ; SEQ ID NO:612), the modified variant BBIt-AV-S5P-A13I-L29P-A40K-F50T-V52A of SEQ ID NO:613
(DPDDESPKPCCDQCICTKSNPPQCRCSDMRPNSCHSACKSCKCYNLYGWTCTCA DITDFCYEPCKPSE ; SEQ ID NO:613), the modified variant BBIt-AV-Q11G-A13I-L29P-A40K-F50T-V52A of SEQ ID NO:614
(DPDDESSKPCCDGCICTKSNPPQCRCSDMRPNSCHSACKSCKCYNLYGWTCTCA DITDFCYEPCKPSE ; SEQ ID NO:614), the modified variant BBIt-AV-A13I-S25R-L29P-A40K-F50T-V52A - of SEQ I D NO:616
(DPDDESSKPCCDQCICTKSNPPQCRCRDMRPNSCHSACKSCKCYNLYGWTCTC ADITDFCYEPCKPSE ; SEQ ID NO:616), the modified variant BBIt-AV-A13I-M27R-L29P-A40K-F50T-V52A of SEQ ID NO:619
(DPDDESSKPCCDQCICTKSNPPQCRCSDRRPNSCHSACKSCKCYNLYGWTCTCA DITDFCYEPCKPSE ; SEQ ID NO:619), the modified variant BBIt-AV-A13I-L29P-S31A-A40K-F50T-V52A of SEQ ID NO:621
(DPDDESSKPCCDQCICTKSNPPQCRCSDMRPNACHSACKSCKCYNLYGWTCTCA DITDFCYEPCKPSE ; SEQ ID NO:621), the modified variant BBIt-AV-A13I-L29P-S31R-A40K-F50T-V52A of SEQ ID NO:622
(DPDDESSKPCCDQCICTKSNPPQCRCSDMRPNRCHSACKSCKCYNLYGWTCTC ADITDFCYEPCKPSE ; SEQ ID NO:622), the modified variant BBIt-AV-A13I-L29P-S38N-A40K-F50T-V52A of SEQ ID NO:623
(DPDDESSKPCCDQCICTKSNPPQCRCSDMRPNSCHSACKNCKCYNLYGWTCTC ADITDFCYEPCKPSE ; SEQ ID NO:623), and the modified variant BBIt-AV-A13I-L29P-S38N-A40K-F50T-V52A of SEQ ID NO:626
(DPDDESSKPCCDQCICTKSNPPQCRCSDMRPNSCHSACKSCKCYNLYGWTCTCA DITDFCYEPCKPEE ; SEQ ID NO:626).

In some embodiments, the modified variant BBPI comprises a combination of seven amino acid substitutions at amino acids at positions equivalent to positions 13, 25, 29, 31, 40, 50 and 52 of SEQ ID NO:187. In some embodiments, the combination of seven amino acid substitutions is chosen from a combination of substitutions at positions 13-25-29-31-40-50-52, 13-25-29-31-40-50-52, 13-25-27-29-31-50-52, 13-25-27-29-31-50-52, 13-25-27-29-31-50-52, 13-25-27-29-31-50-52, 13-25-27-29-31-50-52, 13-25-27-29-31-50-52, 13-25-27-29-31-50-52, 13-25-27-29-31-50-52, and 13-25-27-29-31-50-52. In some embodiments, the combination of seven amino acid substitutions is chosen from 13L-25R-29P-31A-40K-50T-52A, 13L-25R-29P-31R-40K-50T-52A, and 131-25R-27A-29P-31A-50K-52T. The invention provides for any one of the variant BBPI scaffolds described in Section 5.3 and further comprising the combination of the six amino acid substitutions chosen from 13L-25R-29P-31A-40K-50T-52A, 13L-25R-29P-31R-40K-50T-52A, 13I-25R-27A-29P-31A-50K-52T, 13I-25R-27A-29P-31A-50K-52T, 13I-25R-27A-29P-31A-50K-52T, 13I-25R-27A-29P-31A-50K-52T, 13I-25R-27A-29P-31A-50K-52T, 13I-25R-27A-29P-31A-50K-52T, 13I-25R-27A-29P-31A-50K-52T, 13I-25R-27A-29P-31A-50K-52T, and 13I-25R-27A-29P-31A-50K-52T, as described in section 5.4. In one embodiment, the modified variant BBPI comprising a combination of seven amino acid substitutions is chosen from the modified variant BBIt-AV-A13I-S25R-L29P-S31A-A40K-F50T-V52A of SEQ ID NO:617
(DPDDESSKPCCDQCICTKSNPPQCRCRDMRPNACHSACKSCKCYNLYGWTCTC ADITDFCYEPCKPSE ; SEQ ID NO:617), the modified variant BBIt-AV-A13I-S25R-L29P-S31R-A40K-F50T-V52A of SEQ ID NO:618
(DPDDESSKPCCDQCICTKSNPPQCRCRDMRPNRCHSACKSCKCYNLYGWTCTC ADITDFCYEPCKPSE; SEQ ID NO:618), the modified variant of BBIt-VEGF-V1-A13IS25R-M27A-L29P-S31A-F50K-V52T SEQ ID NO:491 (D P D D E S S K P C C D Q
C I C T K S N P P Q C R C R D A R P N A C H S A C K S C A C S K H S Q I T C K C T D I T D F C Y E P C K P S E; SEQ ID NO:491), the modified variant BBIt-VEGF-V2-A13I-S25R-M27A-L29P-S31A-F50K-V52T of SEQ ID NO:632 (DPDDESSK P C C D Q C I C T K S N P P Q C R C R D A R P N A C H S A C K S C A C K T N P S G S C K C T D I T D F C Y E P C K P S E: SEQ ID NO:632), the modified variant BBIt-VEGF-V3-A13I-S25R-M27A-L29P-S31A-F50K-V52T of SEQ ID NO:633 (D P D D E S S K P C C D Q C I C T K S N P P Q C R C R D A R P N A C H S A C K S C A C R P T G H S L C K C T D I T D F C Y E P C K P S E; SEQ ID NO:633), the modified variant BBIt-VEGF-V4- A13I-S25R-M27A-L29P-S31A-F50K-V52T of SEQ ID NO:634 (D P D D E S S K P C C D Q C I C T K S N P P Q C R C R D A R P N A C H S A C K S C A C K H S A K A E C K C T D I T D F C Y E P C K P S E; SEQ ID NO:634), the modified variant BBIt-VEGF-V5-A13I-S25R-M27A-L29P-S31A-F50K-V52T of SEQ ID NO:635 (D P D D E S S K P C C D Q C I C T K S N P P Q C R C R D A R P N A C H S A C K S C A C K P S S A S S C K C T D I T D F C Y E P C K P S E;SEQ ID NO:635), the modified variant BBIt-VEGF-V6- A13I-S25R-M27A-L29P-S31A-F50K-V52T of SEQ ID NO:636 (D P D D E S S K P C C D Q C I C T K S N P P Q C R C R D A R P N A C H S A C K S C A C P V T K R V H C K C T D I T D F C Y E P C K P S E; SEQ ID NO:636), the modified variant BBIt -TNFα-T1-A13I-S25R-M27A-L29P-S31A-F50K-V52T of SEQ ID NO:637 (D P D D E S S K P C C D Q C I C T K S N P P Q C R C R D A R P N A C H S A C K S C A C R Y W Q D I P C K C T D I T D F C Y E P C K P S E; SEQ ID NO:637), the modified variant BBIT -TNF α-T2-A13I-S25R-M27A-L29P-S31A-F50K-V52T of SEQ ID NO:638 (D P D D E S S K P C C D Q C I C T K S N P P Q C R C R D A R P N A C H S A C K S C A C A P E P I L A C K C T D I T D F C Y E P C K P S E; SEQ ID NO:638), and the modified variant BBIt - TNF α-T3-A13I-S25R-M27A-L29P-S31A-F50K-V52T of SEQ ID NO:639 (D P D D E S S K P C C D Q C I C T K S N P P Q C R C K D Q R P N E C H S A C K S C H C Y N L Y G W T C R C Q D I T D F C Y E P C K P S E; SEQ ID NO:639).
In some embodiments, the modified variant BBPI comprises a combination of eight amino acid substitutions at amino acids at positions equivalent to positions 13, 25, 27, 29, 31, 40, 50 and 52 of SEQ ID NO:187. In some embodiments, the combination of eight amino acid substitutions is chosen from a combination of substitutions at positions 13-25-27-29-31-40-50-52, 13-25-27-29-31-40-50-52, 13-25-27-29-31-40-50-52, 13-25-27-29-31-40-50-52, and 13-25-27-29-31-40-50-52. In some embodiments, the combination of eight amino acid substitutions is chosen from combinations 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L, and 13I-25K-27Q-29P-31E-40H-50R-52Q. The invention provides for any one of the variant BBPI scaffolds described in Section 5.3 and further comprising the combination of the eight amino acid substitutions chosen from combinations 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L, and 13I-25K-27Q-29P-31E-40H-50R-52Q, as described in section 5.4. In one embodiment, the modified variant BBPI comprising a combination of eight amino acid substitutions is chosen from the modified variant BBIt-AV-A13I-S25R-M27A-L29P-S31A-A40H-F50K-V52T of SEQ ID NO:627 (DPDDESSKPCCDQCICTKSNPPQC RCRDARPNACHSACKSCHCYNLYGWTCKCTDITDFCYEP C K P S E; SEQ ID NO:627; KT8), the modified variant of BBIt-AV-A13I-S25K-M27A-L29R-S31E-A40K-F50Q-V52Q of SEQ ID NO:628 (DPDDESSKPCCDQC) I CTKSNPPQCRCKDARRNECHSACKSCKCYNLYGWTCQC QDITDFCYEPCKPSE; SEQ ID NO:628; QQ8), the modified variant BBIt-AV-A13I-S25K-M27R-L29E-S31A-A40H-F50R-V52K of SEQ ID NO:629 (D P D D E S S K P C C D Q C I C T K S N P P Q C R C K D R R E N A C H S A C K S C H C Y N L Y G W T C R C K D I T D F C Y E P C K P S E; SEQ ID NO:629; RK8), the modified variant BBIt-AV-A13I-S25K-M27A-L29R-S31A-A40H-F50R-V52L of SEQ ID NO:630 (D P D D E S S K P C C D Q C I C T K S N P P Q C R C K D A R R N A C H S A C K S C H C Y N L Y G W T C R C L D I T D F C Y E P C K P S E;SEQ ID NO: 630; RL8) and the modified variant BBIt-AV- A13I-S25K-M27Q-L29P-S31E-A40H-F50R-V52Q of SEQ ID NO:631 (D P D D E S S K P C C D Q C I C T K S N P P Q C R C K D Q R P N E C H S A C K S C H C Y N L Y G W T C R C Q D I T D F C Y E P C K P S E ; SEQ ID NO:631; RQ8).

The invention further provides for modified variant BBPIs that comprise any one combination of the amino acid substitutions described above and that have greater protease inhibitory activity than the unmodified precursor variant BBPI. In some embodiments, modified variant BBPIs which contain a variant peptide in place of the chymotrypsin loop of the corresponding precursor unmodified BBPI have greater trypsin inhibitory activity (TIA) than that of the precursor unmodified BBPI scaffold.

As shown in the Examples, substitutions of at least one amino acid in the backbone of the variant BBPI generates a modified variant BBPI that has a greater production yield that the unmodified variant BBPI. In some embodiments, BBPIs that comprise a combination of two, three, four, five, six, seven or eight amino acid substitutions have a greater production yield than the unmodified precursor BBPI. Thus, the invention provides for modified variant BBPIs that comprise any one combination of the amino acid substitutions described above and that have greater production yield (PY) than the unmodified precursor variant BBPIs. In yet other embodiments, the invention provides for modified variant BBPIs that comprise any one combination of the amino acid substitutions described above and that have greater trypsin inhibitory activity and greater production yield than the TIA and PY of the unmodified precursor variant BBPIs.

In some embodiments, the modified variant BBPIs further comprise a peptide insert that is positioned at the N-terminus of the modified variant BBPI. In some embodiments, the peptide insert comprises a sequence of between 1 and 15 amino acids. In other embodiments, the peptide insert comprises a sequence between 5 and 10 amino acids. In some embodiments, the peptide insert comprises the peptide of SEQ ID NO:389 (DDEPSKPCCDPDP; SEQ ID NO:389). Examples of modified variant BBPIs that the peptide insert of SEQ ID NO:389 are the modified variant 4D13BBIt-AV of
(DDEPSKPCCDPDPDDESSKPCCDQCACTKSNPPQCRCSDMRLNSCHSACKSCA CYNLYGWTCFCVDITDFCYEPCKPSE; SEQ ID NO:390), and the modified variant BBIt-AV-4D13-13I-29P-40K-50T-52A of SEQ ID NO: 413

(DPDDEPSKPCCDPDPDDESSKPCCDQCICTKSNPPQCRCSDMRPNSCHSACKSC
KCYNLYGWTCTCADITDFCYEPCKPSE; SEQ ID NO:413).

### 5.4.3 BBPI Fusion Proteins

In some embodiments, each modified variant BBPI is expressed as fusion protein comprising a catalytic domain, a cleavage site and the BBPI scaffold. The catalytic domain is chosen from cellulase, cutinase, and disulfide isomerase. In some embodiments, the catalytic domain comprised in the BBPI fusion protein is the cellulase catalytic domain of SEQ ID NO:669

DDYSVVEEHGQLSISNGELVNERGEQVQLKGMSSHGLQWYGQFVNYESMKWLRD
DWGITVFRAAMYTSSGGYIDDPSVKEKVKETVEAAIDLGIYVIIDWHILSDNDPNIYKE
EAKDFFDEMSELYGDYPNVIYEIANEPNGSDVTWDNQIKPYAEEVIPVIRDNDPNNIV
IVGTGTWSQDVHHAADNQLADPNVMYAFHFYAGTHGQNLRDQVDYALDQGAAIFV
SEWGTSAATGDGGVFLDEAQVWIDFMDERNLSWANWSLTHKDESSAALMPGANP
TGGWTEAELSPSGTFVREKIRESAS (SEQ ID NO:669)

The fusion protein is processed by a protease or acid/heat treatment to liberate the modified variant BBPI. In some embodiments, the fusion protein further comprises at least one linker sequence. In some embodiments, the linker sequence is selected from the group consisting of SEQ ID NOS:141-143. Although cleavage of the fusion polypeptide to release the modified variant BBPI will often be useful, it is not necessary. Modified variant BBPIs expressed and secreted as fusion proteins surprisingly retain their function.

The modified variant BBPI fusion proteins are each expressed by the host bacterial cell from a fusion polynucleotide sequence. Such fusion polynucleotide sequences are assembled in proper reading frame from the 5' terminus to 3' terminus in the order of first, second, third and fourth polynucleotide sequences. As so assembled, the polynucleotide sequence encodes a "fusion polypeptide" encoding from its amino-terminus 1. a signal peptide functional as a secretory sequence in a bacterial species, 2. a secreted polypeptide or portion thereof normally secreted from a bacterial species *e.g*. cellulase or portion thereof, 3. a cleavable linker peptide and a 4. desired polypeptide (*e.g*., a modified variant BBPI). In some embodiments, the above-defined fusion polynucleotide sequence further comprises a polynucleotide encoding a portion of a propeptide that functions as a spacer between the first and second polynucleotide sequences of a fusion protein. The function of the spacer is intended to increase the distance between the first and second encoded polypeptides. In some embodiments, the spacer sequence is 1 - 10 amino acids long. In other embodiments, the above-defined fusion polynucleotide sequence further comprises a polynucleotide encoding a peptide insert between the linker peptide and the modified variant BBPI. In some embodiments, the peptide insert comprises a sequence of between 1 and 15 amino acids. In other embodiments, the peptide insert comprises a sequence between 5 and 10 amino acids. In some embodiments, the peptide insert comprises the peptide of SEQ ID NO:389.

Various methods are known to those in the art for the production of fusion proteins *(See e.g.,* US Patents 5,411,873, 5,429,950, and 5,679,543). Thus, it is intended that any suitable method will find use in the present invention.

### 5.5 Bowman Birk Protease Inhibitor Polynucleotides

To the extent that the present invention depends on the production of fusion proteins, it relies on routine techniques in the field of recombinant genetics. Basic texts disclosing the general methods of use in this invention include Sambrook et al., Molecular Cloning, A Laboratory Manual ((2nd ed.) [1989]); Kriegler, Gene Transfer and Expression: A Laboratory Manual (1990); and Ausubel et al., (eds.), Current Protocols in Molecular Biology (1994).

The invention provides for compositions including polynucleotide constructs, vectors and host cells that enable the expression of modified variant BBPIs. The polynucleotide constructs of the invention comprise a promoter sequence and a fusion polynucleotide sequence that encodes a fusion protein comprising a modified variant BBPI. As described above, the fusion polynucleotide sequence comprises a catalytic domain, a cleavage site and the BBPI scaffold. Natural or synthetic polynucleotide fragments encoding a BBPI may be incorporated into the polynucleotide constructs. The at least one amino acid substitution introduced into a variant BBPI may be generated by means of site saturation mutagenesis in at least one codon. In alternative embodiments, the at least one amino acid substitution is encoded by DNA oligonucleotides that contain the encoding sequence, and that are annealed and ligated into the protease inhibitor DNA sequence. The desired DNA sequence is then isolated and used in the methods provided herein.

In some embodiments, the polynucleotide constructs of the invention comprise polynucleotide sequences that encode a modified variant BBPI that shares at least about 65% amino acid sequence identity, at least about 70% amino acid sequence identity, at least about 75% amino acid sequence identity, at least about 80% amino acid sequence identity, at least about 85% amino acid sequence identity, at least about 90% amino acid sequence identity, at least about 92% amino acid sequence identity, at least about 95% amino acid sequence identity, at least about 97% amino acid sequence identity, at least about 98% amino acid sequence identity, and at least about 99% amino acid sequence identity with the amino acid sequence of the unmodified precursor variant BBPI and has greater protease inhibitory activity than the unmodified precursor variant BBPI. The invention further provides for polynucleotides encoding modified variant BBPIs that comprise any one combination of the amino acid substitutions described above and that have greater protease inhibitory activity e.g. trypsin inhibitory activity, than the unmodified precursor variant BBPI.

In some embodiments, the polynucleotide constructs of the invention comprise a polynucleotide sequence that may be codon optimized for expression of a modified variant BBPI in the host cell used. Since codon usage tables listing the usage of each codon in many cells are known in the art (See, *e.g.,* Nakamura et al., Nucl. Acids Res., 28:292 [2000]) or readily derivable, such nucleic acids can be readily designed giving the amino acid sequence of a protein to be expressed.

The invention also encompasses polynucleotide constructs that comprise coding sequences encoding modified variant BBPI proteins that are related by being structurally and/or functionally similar. In some embodiments, a modified variant BBPI is derived from a naturally-occurring BBPI belonging to a different genus and/or species. In some embodiments, related proteins are provided from the same species. Indeed, it is not intended that the present invention be limited to related proteins from any particular source(s). In addition, the term "related proteins" encompasses tertiary structural homologs and primary sequence homologs. For example, the present invention encompasses such homologues including but not limited to such BBPI proteins such as those described by Prakash et al. (J mol Evol 42:560-569 [1996]).

In some embodiments, the promoter sequence comprised in the polynucleotide constructs of the invention is operably linked to the BBPI-encoding polynucleotide. Exemplary promoters include both constitutive promoters and inducible promoters. Such promoters are well known to those of skill in the art. Those skilled in the art are also aware that a natural promoter can be modified by replacement, substitution, addition or elimination of one or more nucleotides without changing its function. The practice of the present invention encompasses and is not constrained by such alterations to the promoter. The choice of promoter used in the genetic construct is within the knowledge of one skilled in the art.

In some embodiments, the promoter sequence may be obtained from a bacterial source. In some embodiments, the promoter sequence may be obtained from a Gram- positive bacterium such as as a *Bacillus* strain (*e.g., Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* or *Bacillus thuringiensis);* or a *Streptomyces* strain (*e.g., Streptomyces lividans* or *Streptomyces murinus);* or from a gram negative bacterium (*e.g., E. coli* or *Pseudomonas sp.).*

The promoter can be any DNA sequence having transcription activity in the host organism of choice and can be derived from genes that are homologous or heterologous to the host organism. Examples of suitable promoters that can be used to express a modified variant BBPI in a bacterial host include, but are not limited to the promoter of the *lac* operon of *E. coli,* the *Streptomyces coelicolor* agarase gene *dagA* promoters, the promoters of the *Bacillus licheniformis* α-amylase gene (*amyL*)*,* the aprE promoter of *Bacillus subtilis,* the promoters of the *Bacillus stearothermophilus* maltogenic amylase gene (*amyM*)*,* the promoters of the *Bacillus amyloliquefaciens* α-amylase gene (*amyQ*), the promoters of the *Bacillus subtilis xylA* and *xylB* genes and a promoter derived from a *Lactococcus* sp.-derived promoter including the PI70 promoter. When the gene encoding the compound is expressed in a bacterial species such as *E. coli,* a suitable promoter can be selected, for example, from a bacteriophage promoter including a T7 promoter and a phage lambda promoter. For transcription in a fungal species, examples of useful promoters are those derived from the genes encoding the *Aspergillus oryzae* TAKA amylase, *Rhizomucor miehei* aspartic proteinase, *A. niger* neutral α-amylase, A. *niger* acid stable α-amylase, *A. niger* glucoamylase, *Rhizomucor miehei* lipase, A. *oryzae* alkaline protease, *A. oryzae* triose phosphate isomerase, and A. *nidulans* acetamidase. Examples of suitable promoters for the expression in a yeast species include but are not limited to the Gal 1 and Gal 10 promoters of *Saccharomyces cerevisiae* and the *Pichia pastoris AOX1 or AOX2* promoters.

The invention also encompasses promoter sequences that have been mutated to increase the activity of the promoter when compared to the activity of the corresponding wild-type promoter resulting in the expression of the modified variant BBPI protein. Thus, it is understood that variants of the sequences that define the B. *subtilis* AprE promoter find use in the constructs of the invention. Methods for creating promoter variants in *Bacillus* sp. are well known in the art (*See e.g.,* Helmann et al., 2002. RNA polymerase and sigma factors, pp289-312 In A. L. Sonenshein, J. A. Hoch and R. Losick (ed), Bacillus subtilis and its closest relatives: from genes to cells. American Society for Microbiology, Washington, D.C.) It is not intended that the present invention be limited to any particular promoter, as any suitable promoter known to those skilled in the art finds use with the present invention.

In embodiments, in addition to a promoter sequence, the polynucleotide construct also contains a transcription termination region downstream of the structural gene to provide for efficient termination. In some embodiments, the termination region is obtained from the same gene as the promoter sequence, while in other embodiments it is obtained from another gene. The selection of suitable transcription termination signals is well-known to those of skill in the art.

### 5.6 Bowman Birk Protease Inhibitor Vectors

The invention provides vectors comprising the polynucleotide constructs of the invention. The vectors are introduced into a host cell to express the modified variant BBPI proteins of the invention. Any vector may be used as long as it is replicable and viable in the cells into which it is introduced. Large numbers of suitable vectors and promoters are known to those of skill in the art, and are commercially available. Appropriate cloning and expression vectors are also described in various references known to those in the art (*See e.g.,* Sambrook *et al., supra* and Ausubel *et al., supra*). The appropriate BBPI-encoding DNA sequence is inserted into a plasmid or vector (collectively referred to herein as "vectors") by any suitable method. In general, the DNA sequence is inserted into an appropriate restriction endonuclease site(s) by standard procedures known to those in the art.

Appropriate vectors are typically equipped with a selectable marker-encoding nucleic acid sequence, insertion sites, and suitable control elements, such as termination sequences. In some embodiments, the vectors comprise regulatory sequences, including, for example, control elements (*i.e*., promoter and terminator elements or 5' and/or 3' untranslated regions), effective for expression of the coding sequence in host cells (and/or in a vector or host cell environment in which a modified soluble protein coding sequence is not normally expressed), operably linked to the coding sequence. Large numbers of suitable vectors and promoters are known to those of skill in the art, many of which are commercially available and known to those in the art. The choice of the proper selectable marker will depend on the host cell. Appropriate markers for different bacterial hosts are well known in the art. Typical selectable marker genes encode proteins that (a) confer resistance to antibiotics or other toxins (*e.g*., , ampicillin, methotrexate, tetracycline, neomycin mycophenolic acid, puromycin, zeomycin, or hygromycin; or (b) complement an auxotrophic mutation or a naturally occurring nutritional deficiency in the host strain.

In some embodiments, expression of a fusion BBPI polypeptide results from the expression of one or more copies of the corresponding fusion polypeptide-encoding polynucleotide that is present on a multicopy/replicating plasmid that has been introduced into a host cell. In some embodiments, the vector is a multicopy/replicating plasmid vector which forms an extrachromosomal self-replicating genetic element that expresses a fusion BBPI protein in the host cell. Typically, the vector is a plasmid vector, which carries a selectable marker gene that allows for ease of selecting the host cells that contain the plasmid. Vectors that replicate autonomously in a host cell include vectors that comprise an origin of replication, which enables the vector to replicate autonomously in the *Bacillus* cell. Examples of bacterial origins of replication are the origins of replication of plasmids pBR322, pUC19, pACYC177, and pACYC184 permitting replication in *E. coli,* and pUB110, pC194, pE194, pTA1060, and pAMβ1 permitting replication in *Bacillus.* The origin of replication may be one having a mutation to make its function temperature-sensitive in the *Bacillus* cell (See, *e.g.,* Ehrlich, Proceedings of the National Academy of Sciences USA 75:1433 [1978]). Additional bacterial expression vectors that find use in the present invention include bacteriophages A and M13, and fusion expression systems such as MBP, GST, and LaZ. In further embodiments, epitope tags are added to recombinant proteins, in order to provide convenient methods of isolation (*e.g*., c-myc).

In some embodiments, the expression of a BBPI fusion polypeptide results from the expression of at least one copy of a BBPI fusion-encoding polynucleotide that is integrated into the genome of the host cell. Thus, in some embodiments, the invention provides a BBPI encoding polynucleotide construct that is incorporated into an integrating vector. Thus, when the vector is introduced into the host cell, it is integrated into the genome and replicated together with the genome into which it has integrated. Multiple copies of the BBPI gene can be integrated at several positions in the genome of the host cell. Alternatively, an amplifiable expression cassette carrying a sequence encoding a BBPI fusion protein and a selectable marker (*e.g.,* an antimicrobial resistance marker, such as a gene coding chloramphenicol acetyl transferase) can be integrated in the genome via a single cross-over event and then amplified by challenging the transformed host cell with increasing concentrations of the appropriate antimicrobial (*e.g.,* chloramphenicol).

### 5.7 Bowman Birk Protease Inhibitor Host Cells

In one embodiment, the invention provides for a host cell transformed with an expression vector comprising a polynucleotide sequence encoding a modified variant BBPI. After the expression vector is introduced into the host cells, the transformed host cells are cultured under conditions favoring expression of gene encoding the desired BBPI fusion protein. Large batches of transformed cells can be cultured as described above. Finally, product is recovered from the culture using techniques known in the art.

Methods for introducing DNA into *Bacillus* cells involving plasmid constructs and transformation of plasmids into bacterial host cells are well known. In some embodiments, the plasmids are subsequently isolated from *E. coli* and transformed into *Bacillus.* However, it is not essential to use intervening microorganisms such as *E. coli,* and in some embodiments, a DNA construct or vector is directly introduced into a *Bacillus* host. Those of skill in the art are well aware of suitable methods for introducing polynucleotide sequences into *Bacillus* cells (*See e.g.,* Ferrari et al., "Genetics," in Harwood et al. (ed.), Bacillus, Plenum Publishing Corp. [1989], pages 57-72; Saunders et al., J. Bacteriol., 157:718-726 [1984]; Hoch et al., J. Bacteriol., 93:1925 -1937 [1967]; Mann et al., Current Microbiol., 13:131-135 [1986]; and Holubova, Folia Microbiol., 30:97 [1985]; Chang et al., Mol. Gen. Genet., 168:11-115 [1979]; Vorobjeva et al., FEMS Microbiol. Lett., 7:261-263 [1980]; Smith et al., Appl. Env. Microbiol., 51:634 [1986]; Fisher et al., Arch. Microbiol., 139:213-217 [1981]; and McDonald, J. Gen. Microbiol.,130:203 [1984]). Indeed, such methods as transformation, including protoplast transformation and congression, transduction, and protoplast fusion are known and suited for use in the present invention. Methods of transformation are particularly preferred to introduce a DNA construct provided by the present invention into a host cell.

In addition to commonly used methods, in some embodiments, host cells are directly transformed (*i.e*., an intermediate cell is not used to amplify, or otherwise process, the DNA construct prior to introduction into the host cell). Introduction of the DNA construct into the host cell includes those physical and chemical methods known in the art to introduce DNA into a host cell without insertion into a plasmid or vector. Such methods include, but are not limited to electroporation, insertion of naked DNA or liposomes and the like. In additional embodiments, DNA constructs are co-transformed with a plasmid, without being inserted into the plasmid. In further embodiments, a selective marker is deleted from the altered *Bacillus* strain by methods known in the art (*See*, Stahl et al., J. Bacteriol., 158:411-418 [1984]; and Palmeros et al., Gene 247:255 -264 [2000]).

Methods known in the art to transform *Bacillus,* include such methods as plasmid marker rescue transformation, which involves the uptake of a donor plasmid by competent cells carrying a partially homologous resident plasmid (Contente et al., Plasmid 2:555-571 [1979]; Haima et al., Mol. Gen. Genet., 223:185-191 [1990]; Weinrauch et al., J. Bacteriol., 154:1077-1087 [1983]; and Weinrauch et al., J. Bacteriol., 169:1205-1211 [1987]). In this method, the incoming donor plasmid recombines with the homologous region of the resident "helper" plasmid in a process that mimics chromosomal transformation.

Other methods involving transformation by protoplast transformation are well known in the art (*See e.g.,* Chang and Cohen, Mol. Gen. Genet., 168:111-115 [1979]; Vorobjeva et al., FEMS Microbiol. Lett., 7:261-263 [1980]; Smith et al., Appl. Env. Microbiol.,51:634 [1986]; Fisher et al., Arch. Microbiol., 139:213-217 [1981]; McDonald [1984] J. Gen. Microbiol., 130:203 [1984]; and Bakhiet et al., 49:577 [1985]). In addition, Mann et *al.,* (Mann et al., Curr. Microbiol., 13:131-135 [1986]) describe transformation of *Bacillus* protoplasts, and Holubova (Holubova, Microbiol., 30:97 [1985]) describe methods for introducing DNA into protoplasts using DNA-containing liposomes. In some embodiments, marker genes are used in order to indicate whether or not the gene of interest is present in the host cell. In some embodiments, the BBPI fusion polynucleotide sequence contained in the vector of the invention encodes for a BBPI fusion protein having SEQ ID NO:195. In addition to these methods, in other embodiments, host cells are directly transformed. In "direct transformation," an intermediate cell is not used to amplify, or otherwise process, the modified polynucleotide prior to introduction into the host (*i.e., Bacillus*) cell. Introduction of the modified polynucleotide into the host cell includes those physical and chemical methods known in the art to introduce modified polynucleotide into a host cell without insertion into a plasmid or vector. Such methods include but are not limited to the use of competent cells, as well as the use of "artificial means" such as calcium chloride precipitation, electroporation, etc. to introduce DNA into cells. Thus, the present invention finds use with naked DNA, liposomes and the like.

Examples of suitable bacterial host organisms are Gram positive species, including, but not limited to members of the *Bacillus* species, which *Bacillaceae, (e.g., B. subtilis, B. licheniformis, B. lentus, B. brevis, B. stearothermophilus, B. alkalophilus, B. amyloliquefaciens, B. clausii, B. halodurans, B. coagulans, B. circulans, B. lautus, B. megaterium* and *B. thuringiensis*), *Streptomyces* species (*e.g.*, *S. murinus* and *S*. *lividans*) lactic acid bacteria (*e.g., Lactococcus* spp. such as *Lactococcus lactis; Lactobacillus* spp. including *Lactobacillus reuteri; Leuconostoc spp.; Pediococcus* spp.; and *Streptococcus spp.* Alternatively, strains of Gram-negative species belonging to *Enterobacteriaceae* (*e.g., E. coli*) or members of the *Pseudomonadaceae* find use in the present invention.

In some embodiments, a suitable yeast host organism is selected from various biotechnologically useful yeasts species, including but not limited to *Pichia sp., Hansenula* sp or *Kluyveromyces, Yarrowinia , Saccharomyces* (*e.g., Saccharomyces cerevisiae*), *Schizosaccharomyce* (*e.g., S. pombe*). In some embodiments, strains of the methylotrophic yeast species *Pichia pastoris* are used as the host organism, while in other embodiments, the host organism is a *Hansenula* species. Suitable host organisms among filamentous fungi include species of *Aspergillus (e.g., A. niger, A. oryzae, A. tubigensis, A. awamori and Aspergillus nidulans*). Alternatively, strains of *Fusarium* species (*e.g. F. oxysporum*) and *Rhizomucor* (*e.g., Rhizomucor miehei*) find used as the host organism. Additional suitable strains include, but are not limited to *Thermomyces* and *Mucor* species. Accessory proteins such as thiol-disulfide oxidoreductases or chaperones find use in some embodiments, as they may be beneficial to help fold the secretory protein into its active conformation. Thiol-disulsfide oxidoreductases and protein disulfide isomerases catalyze the formation of the correct disulfide bonds in the protein. Overexpression of the *bdbDC* operon in *B. subtilis* has been shown to be beneficial for the production of a protein with disulfide bonds (See *e.g.,* Meima et al., J. Biol. Chem., 277:6994-7001, [2002]). Chaperones help the secretory protein to fold by binding to exposed hydrophobic regions in the unfolded states and preventing unfavourable interactions and prolyl-peptidyl cis-trans isomerases assist in formation of the proper conformation of the peptide chain adjacent to proline residues.

In some embodiments of the present invention, the host cells are transformed with an expression vector encoding at least one thiol-disulfide oxidoreductase or chaperone. It is not intended that the present invention be limited to any particular thiol-disulfide oxidoreductase or chaperone, as any suitable thiol-disulfide oxidoreductase or chaperone known to those skilled in the art will find use in the present invention.

In some embodiments of the present invention, and as described further below, the fraction of properly folded secretory protein is increased by the addition of chemicals to the growth medium that reduce/oxidize disulfide bonds, and/or alter the general redox potential, and/or chemicals that alter solvent properties thus affecting protein conformation and aggregation. In particularly preferred embodiments, a reagent that reduces disulfide bonds, such as 2-mercaptoethanol (βME), is preferred to increase the fraction of correctly folded protein. However, in other embodiments and depending on the medium used, other disulfide reducing or oxidizing agents (*e.g*., DTT, TCEP, reduced and oxidized glutathione, cysteine, cystine, cysteamine, thioglycolate, S₂O₃²⁻, S₂O₄²⁻, S₂O₅²⁻, SO₃²⁻, S₂O₇²⁻, Cu+, etc.), either used alone or in combination, find use in the present invention. It is contemplated that other adjuvants that alter solvent properties, (*e.g*., urea, DMSO, TWEEN®-80, etc.), either added to the growth medium alone or preferably in combination with disulfide reducing/oxidizing agents, such as βME, will also increase the fraction of correctly folded secretory protein and find use in various embodiments of the present invention. In some preferred embodiments, the BME is used at concentrations ranging from 0.5 to 4 mM, while in other embodiments, the concentrations range from 0.1 mM to 10 mM. Indeed, those of skill in the art know how to select the best growth medium and growth conditions to optimize the effects of the added thiol reducing/oxidizing agents and/or other adjuvants, as well as the concentration of thio reducing/oxidizing agents and/or other adjuvants to use. It is not intended that the present invention be limited to any particular disulfide reducing/oxidizing agent or adjuvant, as any suitable reagents known to those skilled in the art find use in the present invention.

### 5.7.1 Fermentation Parameters

The present invention relies on fermentation procedures for culturing bacterial species. Fermentation procedures for production of heterologous proteins by bacterial species are well known in the art. Culturing is accomplished in a growth medium comprising an aqueous mineral salts medium, organic growth factors, the carbon and energy source material, molecular oxygen (for aerobic and facultative bacteria), and, of course, a starting inoculum of one or more particular microorganism species to be employed.

In addition to the carbon and energy source, oxygen, assimilable nitrogen, and an inoculum of the microorganism, it is necessary to supply suitable amounts in proper proportions of mineral nutrients to assure proper microorganism growth, maximize the assimilation of the carbon and energy source by the cells in the microbial conversion process, and achieve maximum cellular yields with maximum cell density in the fermentation medium.

Various culture media find use in the present invention, as known to those of skill in the art. However, standard bacterial culture media find use in the present invention. In some preferred media formulations, the media include, in addition to nitrogen, suitable amounts of phosphorus, magnesium, calcium, potassium, sulfur, and sodium, in suitable soluble assimilable ionic and combined forms, and also present preferably should be certain trace elements such as copper, manganese, molybdenum, zinc, iron, boron, and iodine, and others, again in suitable soluble assimilable form, all as known in the art.

In some embodiments, the fermentation reaction involves an aerobic process in which the molecular oxygen needed is supplied by a molecular oxygen-containing gas such as air, oxygen-enriched air, or even substantially pure molecular oxygen, provided to maintain the contents of the fermentation vessel with a suitable oxygen partial pressure effective in assisting the microorganism species to grow in a thriving fashion. In effect, by using an oxygenated hydrocarbon substrate, the oxygen requirement for growth of the microorganism is reduced. Nevertheless, molecular oxygen must be supplied for growth of aerobic and to a lesser extent, facultative organisms.

Although the aeration rate can vary over a considerable range, aeration generally is conducted at a rate which is in the range of about 0.5 to 10, preferably about 0.5 to 7, volumes (at the pressure employed and at 25°C.) of oxygen-containing gas per liquid volume in the fermentor per minute. This amount is based on air of normal oxygen content being supplied to the reactor, and in terms of pure oxygen the respective ranges would be about 0.1 to 1.7, or preferably about 0.1 to 1.3, volumes (at the pressure employed and at 25°C.) of oxygen per liquid volume in the fermentor per minute.

The pressure employed for the microbial conversion process can range widely. Pressures generally are within the range of about 0 to 50 psig, presently preferably about 0 to 30 psig, more preferably at least slightly over atmospheric pressure, as a balance of equipment and operating cost versus oxygen solubility achieved. Greater than atmospheric pressures are advantageous in that such pressures do tend to increase a dissolved oxygen concentration in the aqueous ferment, which in turn can help increase cellular growth rates. At the same time, this is balanced by the fact that high atmospheric pressures do increase equipment and operating costs.

The fermentation temperature can vary somewhat, but for most bacterial species used in the present invention, the temperature generally will be within the range of about 20°C to 40°C, generally preferably in the range of about 28°C to 37°C, depending on the strain of microorganism chosen, as known to those skilled in the art.

The microorganisms also require a source of assimilable nitrogen. The source of assimilable nitrogen can be any nitrogen-containing compound or compounds capable of releasing nitrogen in a form suitable for metabolic utilization by the microorganism. While a variety of organic nitrogen source compounds, such as protein hydrolysates, can be employed, usually cheap nitrogen-containing compounds such as ammonia, ammonium hydroxide, urea, and various ammonium salts such as ammonium phosphate, ammonium sulfate, ammonium pyrophosphate, ammonium chloride, or various other ammonium compounds can be utilized. Ammonia gas itself is convenient for large scale operations, and can be employed by bubbling through the aqueous ferment (fermentation medium) in suitable amounts. At the same time, such ammonia can also be employed to assist in pH control.

The pH range in the aqueous microbial ferment (fermentation admixture) should be in the exemplary range of about 2.0 to 8.0. However, pH range optima for certain microorganisms are dependent on the media employed to some extent, as well as the particular microorganism, and thus change somewhat with change in media as known to those skilled in the art.

While the average retention time of the fermentation admixture in the fermentor can vary considerably, depending in part on the fermentation temperature and culture employed, as known in the art.

In some embodiments, the fermentation is preferably conducted in such a manner that the carbon-containing substrate can be controlled as a limiting factor, thereby providing good conversion of the carbon-containing substrate to cells and avoiding contamination of the cells with a substantial amount of unconverted substrate. The latter is not a problem with water-soluble substrates, since any remaining traces are readily removed. It may be a problem, however, in the case of non-water-soluble substrates, and require added product-treatment steps such as suitable washing steps. The time needed to reach this limiting substrate level is not critical and may vary with the particular microorganism and fermentation process being conducted. However, it is well known in the art how to determine the carbon source concentration in the fermentation medium and whether or not the desired level of carbon source has been achieved.

Although in some embodiments, the fermentation is conducted as a batch or continuous operation, fed batch operation is generally preferred for ease of control, production of uniform quantities of products, and most economical uses of all equipment.

If desired, part or all of the carbon and energy source material and/or part of the assimilable nitrogen source such as ammonia can be added to the aqueous mineral medium prior to feeding the aqueous mineral medium into the fermentor. Indeed, each of the streams introduced into the reactor preferably is controlled at a predetermined rate, or in response to a need determinable by monitoring such as concentration of the carbon and energy substrate, pH, dissolved oxygen, oxygen or carbon dioxide in the off-gases from the fermentor, cell density measurable by light transmittancy, or the like. The feed rates of the various materials can be varied so as to obtain as rapid a cell growth rate as possible, consistent with efficient utilization of the carbon and energy source, to obtain as high a yield of microorganism cells relative to substrate charge as possible, but more importantly to obtain the highest production of the desired protein per unit volume.

In either a batch, or the preferred fed batch operation, all equipment, reactor, or fermentation means, vessel or container, piping, attendant circulating or cooling devices, and the like, are initially sterilized, usually by employing steam such as at about 121°C for at least about 15 minutes. The sterilized reactor then is inoculated with a culture of the selected microorganism in the presence of all the required nutrients, including oxygen, and the carbon-containing substrate. The type of fermentor employed is not critical, though in some embodiments, the 15L Biolafitte (Saint-Germain-en-Laye, France) is preferred.

### 5.8 Protein Separation

In some embodiments, host cells transformed with polynucleotide sequences encoding modified proteases are cultured under conditions suitable for the expression and recovery of the encoded protein from cell culture. The protein produced by a recombinant host cell comprising a fusion BBPI of the present invention is secreted into the culture media. In some embodiments, the secreted fusion BBPI is recovered.
In some embodiments, the present invention provides methods of separating a desired protein from its fusion analog. It is contemplated that the methods described herein will find use in the separation of the modified variant BBPI from the fusion analog.

The collection and purification of the desired fusion BBPI protein from the fermentation broth can also be achieved using procedures known to those of skill in the art. The fermentation broth will generally contain cellular debris, including cells, various suspended solids and other biomass contaminants, as well as the desired protein product, which are preferably removed from the fermentation broth by means known in the art. Suitable processes for such removal include conventional solid-liquid separation techniques (*e.g*., centrifugation, filtration, dialysis, microfiltration, rotary vacuum filtration, or other known processes), to produce a cell-free filtrate. In some embodiments, it is preferable to further concentrate the fermentation broth or the cell-free filtrate prior to the purification and/or crystallization process using techniques such as ultrafiltration, evaporation and/or precipitation.

Precipitating the proteinaceous components of the supernatant or filtrate may be accomplished by means of a salt (*e.g.*, ammonium sulfate) or low pH (typically less than 3), followed by purification by a variety of chromatographic procedures (*e.g.,* ion exchange chromatography, affinity chromatography, hydrophobic interaction chromatography, hydrophobic charge induction chromatography etc.) or similar art recognized procedures. It is not intended that the present invention be limited to any particular separation method, as it is contemplated that any method will find use in the present invention.

In certain preferred embodiments, when the expressed desired polypeptide is secreted from the bacterial cells, the polypeptide is purified from the growth media. In preferred embodiments, the expression host cells are removed from the media before purification of the polypeptide (*e.g*. by centrifugation).

When the expressed recombinant desired polypeptide is not secreted from the host cell, the host cell is preferably disrupted and the polypeptide released into an aqueous "extract" which is the first stage of purification. Preferably, the expression host cells are collected from the media before the cell disruption (*e.g.* by centrifugation). The cell disruption may be performed by using any suitable means known in the art, such as by lysozyme or beta-glucanase digestion or by forcing the cells through high pressure (*See e.g.,* Scobes, Protein Purification, Second edition, Springer-Verlag)

In some embodiments, other recombinant constructions include the addition of purification facilitating domains to the nucleotide sequence encoding a modified variant BBPI polypeptide domain which facilitates purification of the soluble proteins (Kroll DJ et al (1993) DNA Cell Biol 12:441-53). In some embodiments, the addition of six histidine residues (*i.e.,* a "His Tag") to the C-terminus of the modified variant BBPI is used as an aid in the purification of the desired protein and its fusion analog. Use of the His tags as a purification aid is well known in the art (*See e.g.,* Hengen, TIBS 20:285-286 [1995]). The 6x his-tagged proteins are easily purified using Immobilized Metal ion Affinity Chromatography (IMAC), as known to those skilled in the art. Other purification facilitating domains include, but are not limited to, metal chelating peptides such as histidine-tryptophan modules that allow purification on immobilized metals (Porath J (1992) Protein Expr Purif 3:263-281), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego CA) between the purification domain and the heterologous protein also find use to facilitate purification.

Thus, any method suitable for recovering the fusion BBPIs of the present invention finds use in the present invention. Indeed, it is not intended that the present invention be limited to any particular purification method.

For some applications, it is of great importance that the protease inhibitors produced using the present invention be highly pure (*e.g.*, having a purity of more than 99%). This is particularly true whenever the desired protein is to be used as a therapeutic, but is also necessary for other applications. The methods described herein provide a way of producing substantially pure desired proteins. The desired proteins described herein are useful in pharmaceutical and personal care compositions. However, it is contemplated that proteins of varying purity levels will be produced using the methods of the present invention and it is not intended that the proteins produced using the present invention be limited to any particular level of purity.

### 5.8.1 Activation of BBPI during purification: Protease Inhibitory Activity

In some embodiments of the present invention, after growth during the purification process, the activity of the protein *i.e*. the protease inhibitory activity, is increased by the addition of chemicals that reduce/oxidize disulfide bonds and/or alter the general redox potential, and/or chemicals that alter solvent properties thus affecting protein conformation and aggregation. In some particularly preferred embodiments, addition of a reagent that reduces disulfide bonds, such as 2-mercaptoethanol, is used to increase activity of the protein. However, as those skilled in the art appreciate, depending purity and buffer composition, other disulfide reducing or oxidizing agents (*e.g*., DTT, TCEP, reduced and oxidized glutathione, cysteine, cystine, cysteamine, thioglycolate, S₂O₃²⁻, S₂O₄²⁻, S₂O₅²⁻, SO₃²⁻, S₂O₇²⁻, Cu+, protein disulfide isomerases, protein thiol-disulfide oxidoreductases, etc.), either used alone or in combination, find use in the present invention. Other adjuvants, which alter solvent properties, (*e.g.* ethanolamine, DMSO, TWEEN®-80, arginine, urea, *etc*.), are either added alone or preferably in combination with disulfide reducing/oxidizing agents, such as βME, during the purification process also find use in the present invention by increasing the activity of the protein. In certain preferred embodiments, partially purified protein is diluted in buffer (in some particularly preferred embodiments, a zwitterionic buffer with TWEEN®-80 at basic pH) and activated with βME and a disulfide oxidizing agent (in alternative preferred embodiments, oxidized glutathione or sodium sulfite).

In addition, it is contemplated that conditions will be screened in order to determine the optimal activation of the BBPI protein, if desired. For example, various βME concentrations (0.1 - 10 mM), oxidizing agent concentrations (0 to 1/20 to 20 times the βME concentration) pH (7.5 - 9.5), temperatures (15 - 40°C), dilutions (1 - 20 fold), incubation times (12 - 72 h), aeration (incubations under inert gas to vigorous mixing under oxygen containing gases), buffer types (Tris, CHES, CAPS, Tricine, TAPS, other zwitterionic buffers, etc.), buffer concentrations (0.1 - 1 M), and the addition of various adjuvants known to alter solvent properties thereby affecting protein conformation and aggregation (*e.g*., ethanolamine, DMSO, TWEEN®-80, arginine, urea, etc.) are tested in order to determine the optimum conditions for the expression system used. It is not intended that the present invention be limited to any particular disulfide reducing/oxidizing agent, dilution, temperature, pH, buffer type or composition, or adjuvant, as any suitable reagents known to those skilled in the art find use in the present invention.

Optimal activation of BBPIs by thiol reducing agents and/or oxidizing agents is monitored by measuring an increase in the protease inhibitory activity of the unmodified variant BBPI. Trypsin inhibitory activity is the protease inhibitory that is assayed in variant BBPIs in which the chymotrypsin loop has been replaced by a variant peptide. In some embodiments, the effect of at least one amino acid substitution on the trypsin inhibitory activity of a modified BBPI is assayed and compared to the trypsin inhibitory activity of the unmodified precursor BBPI.

One measure of enhancement in trypsin inhibitory activity can be determined as the increase in an enzymatic activity ratio *i.e.* BCE:BBPI, in the modified variant BBPI when compared to that of the unmodified variant BBPI. The BCE:BBPI enzymatic activity ratio is the ratio of BBPI protease inhibitory activity to the BCE *i.e.* cellulase enzymatic activity. The ratio of the unmodified variant BBPI is assigned a value of 1. A ratio equal or greater than 1 for a modified variant BBPI indicates that the modified variant BBPI has a greater protease inhibitory activity than that of the unmodified precursor BBPI. In some embodiments, the activity ratio of the modified variant BBPI is at least 1, at least about 1.05, about at least about 1.1, at least about 1.2, at least about 1.3 , at least about 1.4, at least about 1.5, at least about 1.6, at least about 1.7, at least about 1.8. at least about 1.9, and at least about 2. In other embodiments, the activity ratio is at least about 2.1, at least about 2.2, at least about 2.3, at least about 2.4, at least about 2.5, at least about 2.6, at least about 2.7, at least about 2.8, at least about 2.9 and at least about 3. In yet other embodiments, the activity ratio is at least about 3.5, at least about 4.0, and at least about 5. Thus, in some embodiments, the protease inhibitory activity of the modified variant BBPI greater than that of the corresponding unmodified precursor BBPI by at least about 0.5%, about 1.0%, about 1.5%, about 2.0%, about 2.5%, about 3.0%, about 4.0%, about 5.0%, about 8.0%, about 10%, about 15%, about 20%, about 25%, about 30%, about 40%, about 50%, at least about 60%, at least about 70%, at least about 80%, at least about 90%, at least about 100% or more. In other embodiments, the protease inhibitory activity e.g. protease inhibitory activity, of the modified variant BBPI greater than that of the corresponding unmodified precursor BBPI by at least about 110%, 120%, 130%, 140%, 150%, 160%, 170%, 180%, 190%, and up to at least about 200%.

In other embodiments, modified variant BBPIs comprising a combination of amino acid substitutions have greater trypsin inhibitory activity than that of the unmodified precursor BBPI. In some embodiments, the at least two, at least three, at least four, at least five, at least six, at least seven, and at least eight amino acid substitutions generate modified variant BBPIs that have greater TIA than the unmodified precursor variant BBPI. All modified variant BBPIs comprising a combination of amino acid substitutions as described in section 5.4 have greater trypsin inhibitory activity than the corresponding unmodified precursor BBPIs.

### 5.8.2 BBPI Production yield

In some embodiments, the amino acid substitutions made in the precursor variant BBPI are assessed for the ability of the resulting modified variant BBPI to have a greater production yield that is greater than that at which the unmodified precursor BBPI is produced *i.e.* the modified variant BBPI is produced at a level that is greater than that at which the unmodified precursor BBPI is produced. In some embodiments, the invention provides for modified variant BBPIs comprising a combination of amino acid substitutions as described in section 5.4 that have greater protease inhibitory activity *e.g*. trypsin inhibitory activity, and greater production yield than the corresponding unmodified precursor BBPIs.

Modified variant BBPIs that have greater TIA and PY than the corresponding precursor unmodified BBPIs each comprise at least two, at least three, at least four, at least five, at least six, at least seven, and at least eight amino acid substitutions (see Examples 12, 13, 14 and 15).

One measure of enhancement in production yield can be determined as the increase in the level of free BBPI following cleavage of the BBPI from the C-terminus of the BCE core. As described above, in some embodiments, the BBPI fusion proteins can be cleaved using proteases or by chemical means. In other embodiments, the BBPI can be cleaved from the BBPI fusion protein by acid/heat treatment, which cleaves the A-P bonds present in the CBD linker that joins the BCE to the BBPI. In yet other embodiments, the BBPI fusion proteins can be cleaved using glutamyl endopeptidase I from *B. licheniformis.* In some embodiments, the enhancement in production yield of a modified variant BBPI is measured as the increase in the level of free modified variant BBPI following activation of the fusion BBPI and following acid heat treatment of the BBPI fusion protein, when compared to the level of free unmodified precursor BBPI that was subjected to the same treatment. In other embodiments, the enhancement in production yield of a modified variant BBPI is measured as the increase in the level of free modified variant BBPI without activation of the fusion BBPI and following acid heat treatment of the BBPI fusion protein, when compared to the level of the corresponding free unmodified precursor BBPI that was subjected to the same treatment. In some embodiments, the free modified variant BBPI has a greater production yield and a greater TIA than the corresponding precursor BBPI.

In some embodiments, the production yield of the modified variant BBPI is greater than that of the corresponding unmodified precursor BBPI by at least about 0.5%, about 1.0%, about 1.5%, about 2.0%, about 2.5%, about 3.0%, about 4.0%, about 5.0%, about 8.0%, about 10%, about 15%, about 20%, about 25%, about 30%, about 40%, about 50%, at least about 60%, at least about 70%, at least about 80%, at least about 90%, at least about 100% or more. In other embodiments, the production yield of the modified variant BBPI is greater than that of the corresponding unmodified precursor BBPI by at least about 110%, 120%, 130%, 140%, 150%, 160%, 170%, 180%, 190%, and up to at least about 200%.

### 6. Personal Care Compositions

### 6.0.1 Personal Care Compositions Comprising Modified Variant BBPIs

The present invention provides personal care compositions comprising at least one modified variant BBPI for medical and nonmedical conditions. In some embodiments, the present invention provides personal care compositions and the methods of their use. In some embodiments, the invention provides personal care compositions for use in the skin care. In other embodiments, the personal care compositions are for use in hair care. In some embodiments, the personal care compositions for use in skin care include cosmetic compositions. In other embodiments, the personal care compositions of the invention are for use in the treatment of various disorders.

As described in greater detail herein, the present invention provides compositions for use in numerous aspects of personal care, including but not limited to hair and skin care, as well as cosmetics (*e.g.*, make-up). For example, the present invention provides compositions that find use in daily personal care, skin care, sun care (*e.g*., sunscreens, as well as tanners), hair care (*e.g.*, shampoos, leave-on and/or rinse off conditioners, hair tonics, hair sprays, gels, foams, mousses, setting products, hair colorants, permanent formulations, other styling and cleaning products, etc.), after-sun care for skin, hair and lips, oral care (*e.g*., toothpastes and gels, mouthwashes, rinses, etc.), bathing (*e.g.*, washes, shower soaps, bath soaps, salts, pearls, etc.), skin lighteners, cleansing treatments for skin conditions (*e.g*., pimples, acne, skin toners, etc.), depilatories, wet wipes, deodorants, anti-perspirants, facial masks, shaving (*e.g*., shaving creams, gels, etc.), after-shave, skin peeling (*e.g.*, exfoliants), intimate care products (*e.g*., feminine hygiene products), personal fresheners, and foot care. The present invention also provides compositions that find use in cosmetics (*e.g*., foundations, mascara, eye shadows, eye liners, lipsticks, lip glosses, blushers, etc.). In addition, the present invention provides personal care compositions for use in ameliorating a hair condition associated with a disorder. In other embodiments, the personal care composition are for use in ameliorating a skin condition associated with a disorder. It is contemplated that the compositions of the present invention will find use various forms, including but not limited to solids, liquids, colloidal suspensions, emulsions, oils, gels, aerosols, foams, powders, pump sprays, etc., as well as being used in conjunction with items such as wet wipes, etc. Indeed, it is contemplated that the present invention will find use in any suitable form for the intended use(s).

In some embodiments, the personal care composition comprises a modified variant BBPI comprising a variant peptide and at least one amino acid substitution, as described in section 5.4.1 above. In other embodiments, the personal care composition comprises a modified variant BBPI comprising a variant peptide and a combination of amino acid substitutions, as described in section 5.4.2 above. In some embodiments, the personal care composition comprises a modified variant BBPI in which the equivalent chymotrypsin loop is a VEGF -binding peptide. In other embodiments, the personal care composition comprises a modified variant BBPI in which the equivalent chymotrypsin loop is an FGF5 binding peptide. In other embodiments, the personal care composition comprises a modified variant BBPI in which the equivalent chymotrypsin loop is a TGFβ binding peptide. In yet other embodiments, the personal care composition comprises a modified variant BBPI in which the equivalent chymotrypsin loop is a TNFα binding peptide. In some embodiments, the modified variant BBPI comprises from about 0.0001 weight percent to about 5 weight percent of the personal care composition, while in alternative embodiments, the modified variant BBPI comprises from about 0.001 weight percent to about 0.5 weight percent of the personal care composition, and in yet additional embodiments, the modified variant BBPI comprises from about 0.01 weight percent to about 1 weight percent of the personal care composition.

### 6.0.2 Personal Care Compositions Comprising Modified Variant VEGF-BBPIs

VEGF plays a central role in promoting angiogenesis as well as influencing diverse cell functions including cell survival, proliferation and the generation of nitric oxide and prostacyclin (Zachary et al., Cardiovasc Res, 49: 568-81 [2001]). The recognition of VEGF as a primary stimulus of angiogenesis in pathological conditions has led to various attempts to block VEGF activity. Inhibitory anti-VEGF receptor antibodies, soluble receptor constructs, antisense strategies, RNA aptamers against VEGF and low molecular weight VEGF receptor tyrosine kinase (RTK) inhibitors have all been proposed for use in interfering with VEGF signaling (*See*, Siemeister *et al.,* [1998]). In fact, monoclonal antibodies against VEGF have been shown to inhibit human tumor xenograft growth and ascites formation in mice (*See,* Kim *et al.,* [1993]; Asano *et al.,* [1998]; Mesiano *et al.,* [1998]; Luo *et al.,* [1998a] and [1998b]; and Borgstrom *et al*., [1996] and [1998]).

Angiogenesis, involving VEGF and RTKs is not only involved in cancer development, as many other diseases or conditions affecting different physiological systems are angiogenesis-dependent, such as arthritis and atherosclerotic plaques (bone and ligaments), diabetic retinopathy, neovascular glaucoma, macular degeneration, ocular herpes, trachoma and corneal graft neovascularization (eye) and angiofibroma. VEGF expression is upregulated in the hyperplastic epidermis of psoriasis patients (Detmar and Yeo *et al.* [1995]), and in other skin diseases characterized by enhanced angiogenesis including, scleroderma, rosacea, hemangioma, contact dermatitis, and hypertrophic scarring of the skin. Targeted overexpression of VEGF in the epidermis of transgenic mice was reported to result in enhanced skin vascularization with equal numbers of tortuous and leaky blood vessels (*See e.g.,* Brown *et al.,* [1998]). Also, chronic synthesis of VEGF in mouse skin leads to the first histologically equivalent murine model of human psoriasis (Xia *et al.,* [2003]) that is reversible by binding agents specific for VEGF. In addition, ultraviolet radiation induces VEGF production in keratinocytes and enhances cutaneous angiogenesis (Kim et al., Soc. Investigative Dermatol. 126:2697 [2006]; Blaudshun et al., FEBS Let. 474:195-200 [2000]; Kosmadaki et al., FASEB J. 17:446-8 [2003]).

In addition, the expression of VEGF in the outer root sheath of murine hair follicles was found to be temporally and spatially associated with capillary proliferation during anagen. Transgenic overexpression of VEGF in the outer root sheath increased perifollicular vascularization and led to accelerated hair growth following depilation and the growth of larger hairs (Yano et al. J Clin Invest 107: 409-17 [2001]).

Thus, VEGF is involved in the vascularization associated with pathologic and non-pathologic conditions.

The invention provides for personal skin care and/or hair care compositions that comprise a modified variant VEGF-BBPI. The VEGF-BBPIs comprised in the personal care compositions of the invention are modified variant BBPIs in which the equivalent chymotrypsin loop of the precursor scaffold of the VEGF-BBPI has been replaced by a VEGF variant peptide, and which further comprise at least one amino acid substitution as described in sections 5.4.1 and 5.4.2. In some embodiments of the present invention, binding of the modified variant VEGF-BBPI to VEGF prevents VEGF from increasing perifollicular vascularization and inhibits the VEGF from promoting hair growth. In other embodiments, binding of the modified variant VEGF-BBPI to VEGF prevents VEGF from increasing vascularization of the skin in a subject suffering from an angiogenic skin disorder *e.g*. hemangioma and lichen planus. In yet other embodiments, binding of the modified variant VEGF-BBPI to VEGF prevents VEGF from promoting disregulated angiogenesis associated with inflammatory skin disorders including psoriasis, scleroderma, venous ulcers, acne, rosacea, warts, eczema, and lymphangiogenesis. However, it is not intended that the present invention be limited to any particular mechanism.

In some embodiments, the invention provides a personal care composition comprising a VEGF-BBPI that binds to VEGF. In alternative embodiments, the binding of the VEGF-BBPI to VEGF blocks the downstream activity of VEGF. In some embodiments, the composition is capable of modulating angiogenesis.

In some embodiments, the personal care composition comprises a modified variant BBPI-VEGF for use in skin care. In some embodiments, the skin care compositions are for cosmetic use in improving the appearance of skin. In other embodiments, the skin care compositions are for therapeutic use in improving the appearance of skin in a subject suffering from a skin disorder. In some embodiments, the skin disorder is an angiogenic skin disorder. In additional embodiments, the skin disorder is at least one chosen from psoriasis, scleroderma, venous ulcers, acne, rosacea, warts, eczema, hemangiomas and lymphangiogenesis. In some embodiments, the skin disorder is rosacea. In other embodiments, the skin disorder is psoriasis.

In one embodiment, the personal skin care composition comprising a VEGF-BBPI is chosen from skin creams, lotions, sprays, emulsions, colloidal suspensions, foams, aerosols, liquids, gels, sera, and solids. In another embodiment, the personal care composition is a skin care composition selected from moisturizing body washes, body washes, antimicrobial cleansers, skin protective creams, body lotions, facial creams, moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, skin lightening compositions, skin redness reduction compositions, sunscreens, depilatories, hair growth inhibitors, and radioprotectives. Radioprotectives are chosen from non-water-resistant sunscreens, very water-resistant sunscreens, and water-in-silicone sunscreens.

In one embodiment, the personal care skin care composition comprising a VEGF-BBPI is comprises topically applied over-the-counter compositions, anti-fungal treatments, anti-acne treatments, skin protectants, sunscreens, deodorants, and antiperspirants. In other embodiments, the skin care composition is capable of lightening skin tone, reducing redness, preventing skin tone darkening or preventing color development.

The present invention also provides personal care compositions that are cosmetic compositions. In some preferred embodiments, the cosmetic compositions comprising a VEGF-BBPI are chosen from pressed powder formulations and foundations. In some preferred embodiments, the cosmetic compositions comprise at least one pigment.

In yet additional embodiments, the makeup compositions are pressed powder formulations selected from loose powders, blushes, and bronzing powders. In still further embodiments, the makeup compositions are foundations selected from water-in-oil foundations, water-in-silicone foundations, oil-in-water foundations, anhydrous makeup sticks, and cream-to-powder foundations.

In some embodiments, the personal skin care compositions comprise a modified variant VEGF-BBPI, as set forth herein, and a physiologically acceptable carrier or excipient. Preferably, the VEGF-BBPI is present in an amount of about 0.0001% to about 5% by weight based on the total weight of the composition. Also preferably, the VEGF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In some other embodiments, the invention provides a personal care composition that comprises a modified variant VEGF-BBPI for use in hair care.

In some embodiments, the hair care compositions find use in inhibiting hair growth. In other embodiments, inhibition of hair growth comprises hair removal for treatment of at least one disease or condition for which decreased hair growth is desirable. In some embodiments, inhibition and/or removal comprises depilation. In some embodiments, the hair is selected from the group consisting of facial hair, leg hair, arm hair, and torso hair.

In one embodiment, the hair care composition is selected from the group consisting of shampoos, conditioners, hair styling compositions, hair colorants, permanent wave formulations, creams, gels, mousses, sprays, emulsions, colloidal suspensions, liquids, foams, and solids. In some embodiments, the hair care composition further comprises a radioprotective. As described for the personal skin care compositions the radioprotective is a sunscreen chosen from non-water-resistant sunscreens, very water-resistant sunscreens, and water-in-silicone sunscreens. In other embodiments, the radioprotective is a sunscreen chosen from non-water-resistant sunscreens, very water-resistant sunscreens, and water-in-silicone sunscreens.

In some embodiments, the personal hair care compositions comprise a modified variant VEGF-BBPI, as set forth herein, and a physiologically acceptable carrier or excipient. Preferably, the VEGF-BBPI is present in an amount of about 0.0001% to about 5% by weight based on the total weight of the composition. Also preferably, the VEGF-BBPI is present in an amount of about 0.001% to about 1% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the personal care compositions of the invention are for use in the treatment of various diseases associated with elevated levels of VEGF.

The present invention also provides methods for inhibiting hair growth of a subject, comprising the steps of providing the personal care VEGF hair care composition of the present invention; providing a subject to be treated; and applying the composition to the subject in an area in which inhibition of hair growth is desired. In some embodiments, the inhibition of hair growth comprises inhibiting the growth of the subject's hair, wherein the hair to be inhibited is selected from the group consisting of facial air, underarm hair, leg hair, torso hair, and arm hair, and head hair. In additional embodiments, the method for inhibiting hair growth using a VEGF hair care composition of the invention comprises a personal care composition comprising a VEGF-BBPI having an amino acid sequence chosen from SEQ ID NOS SEQ ID NOS:601, 602, 627, 628, 629, 630, 631, 491, 632, 633, 634, 635 and 636.

The compositions of the invention may be used in a method for improving the appearance and/or condition of skin in a subject suffering from a skin disorder, comprisiing providing the personal care composition of the present invention; providing a subject to be treated; and applying the composition to the affected skin of the subject. In some embodiments, the skin disorder is chosen from psoriasis, venous ulcers, acne, rosacea, warts, eczema, hemangiomas, cutaneous lichen planus, and lymphangiogenesis, etc. In some particularly preferred embodiments, the skin disorder is rosacea. In other embodiments, the skin disorder is psoriasis. In additional embodiments, the method for improving the appearance and/or condition of skin in a subject suffering from a skin disorder using a VEGF skin care composition of the invention comprises a personal care composition comprising a VEGF-BBPI having an amino acid sequence chosen from SEQ ID NOS:601, 602, 627, 628, 629, 630, 631, 491, 632, 633, 634, 635 and 636.

### 6.0.3 Personal Care Compositions Comprising Modified Variant FGF-5-BBPIs

The Fibroblast Growth Factor (FGF) family is a superfamily of growth factors containing at least 23 members, many of which are potent regulators of cell proliferation, differentiation and cell function. All of the FGFs have a conserved 120 amino acid core. Members of the family share conserved cysteine residues and 30-50% sequence homology at the amino acid level. The molecular weight of the FGFs ranges from 7 kDa for FGF-1 to 38 kDa for FGF-5. The length of the proteins is from 60 amino acids in the case of an FGF-1 splice variant to 288 amino acids for FGF-2. Binding to heparin is an essential step required for an FGF factor to interact with cell surface receptors. FGF5 is a secreted signaling protein consisting of 268 amino acids with a 17 amino acid signal sequence and a 251 amino acid mature peptide. The human gene also gives rise to a glycosylated alternate splice form that is 18 kDa in size and 123 amino acid in length. The murine homologue of FGF-5 was cloned and found to be 84% homologous to the human protein at the amino acid sequence level. Human FGF-5 consists of three exons and maps to chromosome 4q21 and cross-reacts with murine FGF-5.

Formation of hair follicles involves a complex series of steps: growth (anagen), regression (catagen), rest (telogen) and shedding (exogen). FGF-5 has been implicated as one of the major drivers of the transition from anagen to catagen in the hair cycle. Expression of FGF-5 is detected in hair follicles from wild-type mice and is localized to the outer root sheath during the anagen phase. Mice homozygous for a predicted null allele of FGF-5, fgf5neo, have abnormally long hair (See, Hebert et al., Cell 78: 1017-25 [1994]). The phenotype appears identical to that of mice homozygous for the spontaneous mutation angora (go). Recently, partial FGF-5 sequences, FGF5S, thought to compete with FGF-5 in binding to the receptor have been identified, (See, Ito et al., J. Cell Physiol., 197:272-83 [2003]).

The invention provides personal hair care compositions that comprises at least one modified variant FGF-BBPI for use in promoting hair growth and/or preventing hair loss. In one embodiment, the invention provides FGF-BBPI compositions for personal skin care. In other embodiments, the invention provides FGF-BBPI compositions for hair care. The FGF-BBPIs comprised in the personal care compositions of the invention are modified variant BBPIs in which the equivalent chymotrypsin loop of the precursor scaffold of the FGF-5-BBPI has been replaced by an FGF-5 variant peptide, and which further comprise at least one amino acid substitution as described in sections 5.4.1 and 5.4.2. In the present invention, binding of the modified variant FGF-BBPI to FGF-5 prevents FGF-5 from interacting with its cognate receptor and inhibits transition from the anagen to the catagen promoting hair growth and preventing hair loss. However, it is not intended that the present invention be limited to any particular mechanism.

In some embodiments, the invention provides a personal care composition comprising a FGF-BBPI that binds to FGF. In alternative embodiments, the binding of the FGF-BBPI to FGF blocks the downstream activity of FGF. In some embodiments, the composition is capable of promoting hair growth.

In some embodiments, the personal care composition comprising a modified variant BBPI-FGF is for use in skin care. In some embodiments, the skin care compositions are cosmetic compositions for use in promoting hair growth. In some embodiments, the skin care compositions are for use in promoting hair growth in a subject suffering from a disease or condition that involves hair loss. In some of these embodiments, the disease or condition is at least one selected from the group consisting of inflammatory alopecias, pseudopelade, scleroderma, tick bites, lichen planus, psoriasis, lupus, seborrheic dermatitis, loose hair syndrome, hemochromatosis, androgenic alopecia, alopecia areata, cancer, conditions that affect defective hair fiber production, and environmental factors that affect hair production. In a preferred embodiment, the disease is androgenic alopecia or alopecia areata.

In one embodiment, the personal care composition comprising an FGF-BBPI is a skin care composition is chosen from skin creams, lotions, sprays, emulsions, colloidal suspensions, foams, aerosols, liquids, gels, sera, and solids. In another embodiment, the personal care composition is a skin care composition selected from moisturizing body washes, body washes, antimicrobial cleansers, skin protective creams, body lotions, facial creams, moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, skin lightening compositions, skin redness reduction compositions, sunscreens, depilatories, hair growth inhibitors, .and radioprotectives. Radioprotectives are chosen from non-water-resistant sunscreens, very water-resistant sunscreens, and water-in-silicone sunscreens.

In one embodiment, the personal skin care composition comprising an FGF-BBPI is a skin care composition comprising topically applied over-the-counter compositions, anti-fungal treatments, anti-acne treatments, skin protectants, sunscreens, deodorants, and antiperspirants.

In some embodiments, the personal skin care compositions comprise a modified variant FGF-BBPI, as set forth herein, and a physiologically acceptable carrier or excipient. Preferably, the FGF-BBPI is present in an amount of about 0.0001% to about 5% by weight based on the total weight of the composition. Also preferably, the FGF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the invention provides a personal care composition comprising a modified variant FGF-BBPI for use in hair care.

In some embodiments, the hair care compositions find use in promoting hair growth. In other embodiments, modulation comprises promoting hair growth in a subject suffering from a disease or condition that involves hair loss. In some of these embodiments, the disease or condition is at least one selected from the group consisting of inflammatory alopecias, pseudopelade, scleroderma, tick bites, lichen planus, psoriasis, lupus, seborrheic dermatitis, loose hair syndrome, hemochromatosis, androgenic alopecia, alopecia areata, cancer, conditions that affect defective hair fiber production, and environmental factors that affect hair production. In a preferred embodiment, the disease is androgenic alopecia or alopecia areata.

In one embodiment, the hair care composition is selected from the group consisting of shampoos, conditioners, hair styling compositions, hair colorants, permanent wave formulations, creams, gels, mousses, sprays, emulsions, colloidal suspensions, liquids, foams, and solids. In some embodiments, the hair care composition further comprises a radioprotective. As described for the personal skin care compositions the radioprotective is a sunscreen chosen from non-water-resistant sunscreens, very water-resistant sunscreens, and water-in-silicone sunscreens. In other embodiments, the radioprotective is a sunscreen chosen from non-water-resistant sunscreens, very water-resistant sunscreens, and water-in-silicone sunscreens.

In some embodiments, the personal hair care compositions comprise a modified variant FGF-BBPI, as set forth herein, and a physiologically acceptable carrier or excipient. Preferably, the FGF-BBPI is present in an amount of about 0.0001% to about 5% by weight based on the total weight of the composition. Also preferably, the FGF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the personal care FGF-BBPI compositions of the invention are for use in the treatment of various diseases associated with elevated levels of FGF.

The present invention also provides methods for promoting hair growth of a subject, comprising the steps of providing the personal care FGF composition of the present invention; providing a subject to be treated; and applying the composition to the subject in an area in which promotion of hair growth is desired. In some embodiments, the FGF composition is a skin care composition. In other embodiments, the FGF composition is a hair composition. In some embodiments, promotion of hair growth comprises promoting the growth of the subject's hair, wherein the hair growth to be promoted is chosen from facial air, underarm hair, leg hair, torso hair, and arm hair, and head hair. In additional embodiments, the method for promoting hair growth using an FGF hair care composition of the invention comprises applying a personal care composition comprising an FGF-BBPI having an amino acid sequence chosen from SEQ ID NOS:439 and 441.

### 6.0.4 Personal Care Compositions Comprising Modified Variant TGFβ-BBPIs

Proteins of the Transforming Growth Factor-β (TGFβ) family are synthesized by almost all cells. The TGFβs are a group of stable, multifunctional polypeptide growth factors whose activities include, among other things, context-specific inhibition and stimulation of cell proliferation, control of the extracellular matrix, degradation and control of mesenchymal-epithelial interactions during embryogenesis, mediation of cell and tissue responses to injury, control of carcinogenesis and modulation of immune responses. TGF-1 is synthesized by virtually all cells (with only a few exceptions). TGFβ-1 has been found in the highest concentration in human platelets and mammalian bone. TGFβ-1 has many functions including suppression of cell proliferation, enhancement of extracellular matrix deposition and physiological immunosuppression. TGFβ-1 has also been determined to be biologically active in hair follicle development. Human TGFβ-1 is a 25.0 kDa protein with subunits that contain approximately 112 amino acids per subunit. Two different receptor proteins are involved in TGFβ-1 binding and signaling, namely TGF-RβII and TGF-RβI. TGFβ-2 is expressed in a variety of cells, including keratinocytes, fibroblasts, osteoclasts, thymic epithelium, skeletal muscle cells, prostatic epithelium, bronchial epithelium, neurons and astrocytes, visceral smooth muscle, macrophages and various other cells. TGFβ-2 has many fundamental activities, including function as a growth inhibitor for most cells, an enhancer for deposition of the extracellular matrix, and immunosuppression. The mature region is 71% identical to TGFβ-1, 80% identical to TGFβ-3, and 97% identical to the mouse homologue of the same protein at the amino acid level. TGFβ-2 dimerizes with formation of disulfide bonds between the 'pro' regions and disulfide bonds between the mature regions. TGFβ-2 is synthesized as a pre-procytokine with a 19 amino acid signal sequence, a 283 pro-region and a 112 mature amino acid segment. The receptor for TGFβ-2 forms a heterotetrameric complex of two type I signal-transduction receptors and two type II ligand-binding receptors.

Formation of hair follicles involves a complex series of steps: growth (anagen), regression (catagen), rest (telogen) and shedding (exogen) (See, Stenn and Paus, Physiol. Rev, Exp. Dermatol., 8:229-233 [1999]). TGFβs have been implicated as one of the major drivers of the transition from anagen to catagen in the hair cycle (*See e.g.,* Foitzik et al, FASEB J., 5:752-760 [2000]; and Soma et al. J. Infect. Dis., J. Invest. Dermatol., 118:993-9997 [2002]), and TGFβ2 is both a required and sufficient inducer of murine hair follicle morphogenesis *(See,* Foitzik et al., Develop. Biol., 212:278-289 [1999]). Conditional TGFβ-1 expression in transgenic mice demonstrates that one can induce alopecia reversibly (*See*, Liu et al., Proc. Natl. Acad. Sci. USA 98:9139-9144 [2001]). In addition, TGFβ-1 mutants have been associated with the delay of catagen onset in mice (*See,* Foitzik *et al,* [2000], *supra*). Recently, it has been shown that catagen can be delayed through the use of TGFβ-2 antibodies (*See*, Soma *et al.,* [2002], *supra*). Finally, androgens that induce TGFβ-1 production in balding dermal papilla cells can inhibit epithelial cell growth (Inui et al., FASEB J., 14:1967-1969 [2002]).

TGFβ is also a potent stimulus of connective tissue accumulation, and is implicated in the pathogenesis of scleroderma and other fibrotic disorders (Blobe et al., N Engl J Med 342:1350-1358 [2000]). Scleroderma is a chronic autoimmune disease characterized by early inflammation and vascular injury, followed by progressive fibrosis of the skin and other organs (Kissin and Korn, Rheum Dis Clin North Am 29:351-369 [2003]). The most evident symptom is usually the hardening of the skin and associated scarring.

TGFβ has also been implicated in the formation of skin tumors (Li et al., Molecular Carcinogenesis 45:389-396 [2006]). The TGFβ signaling pathway is one of the most important mechanisms in the maintenance of epithelial homeostasis. Alterations leading to either the repression or enhancement of this pathway have been shown to affect cancer development. Although TGFβ inhibits growth of normal epithelial cells, it is paradoxically overexpressed in many epithelial cancers. It has been postulated that TGFβ acts as a tumor suppressor at the early stages of carcinogenesis, but overexpression of TGFβ at late stages of carcinogenesis may be a critical factor for tumor invasion and metastasis.

The invention provides personal care compositions that comprise a modified variant TGF-BBPI. The TGFβ-BBPI comprised in the personal care compositions of the invention is a modified variant TGFβ -BBPI in which the chymotrypsin loop of the precursor scaffold of the TGF-BBPI has been replaced by a TGFβ variant peptide, and which further comprise at least one amino acid substitution as described in sections 5.4.1 and 5.4.2. In some embodiments, binding of the modified variant TGF-BBPI to TGFβ1 and/or TGFβ2 prevents TGFβ1 and/or TGFβ2 from interacting with its cognate receptor and inhibits transition from the anagen to the catagen to promote hair growth and preventing hair loss. In other embodiments, binding of the modified variant TGF-BBPI to TGFβ1 and/or TGFβ2 prevents TGFβ1 and/or TGFβ2 from interacting with its cognate receptor to prevent fibrosis of the skin that is characteristic of scleroderma. In other embodiments, binding of the modified variant TGF-BBPI to TGFβ1 and/or TGFβ2 prevents TGFβ1 and/or TGFβ2 from interacting with its cognate receptor to prevent progression of benign to malignant skin lesions. However, it is not intended that the present invention be limited to any particular mechanism.

In some embodiments, the invention provides a personal care composition comprising a TGFβ -BBPI that binds to TGFβ1 and/or TGFβ2. In alternative embodiments, the binding of the TGFβ -BBPI to TGFβ blocks the downstream activity of TGFβ. In some embodiments, the composition is capable of promoting hair growth.

In some embodiments, the personal care composition comprising a modified variant TGF-BBPI is for use in skin care. In some embodiments, the skin care compositions are cosmetic compositions for use in promoting hair growth.

In some embodiments, the skin care compositions are for use in promoting hair growth in a subject suffering from a disease or condition that involves hair loss. In some of these embodiments, the disease or condition is at least one selected from the group consisting of inflammatory alopecias, pseudopelade, scleroderma, tick bites, lichen planus, psoriasis, lupus, seborrheic dermatitis, loose hair syndrome, hemochromatosis, androgenic alopecia, alopecia areata, cancer, conditions that affect defective hair fiber production, and environmental factors that affect hair production. In a preferred embodiment, the disease is androgenic alopecia or alopecia areata.

In other embodiments, the personal care composition comprising a modified variant TGF-BBPI is for use in improving the appearance and/or condition of the skin of a subject suffering from scleroderma. In yet other embodiments, the personal care composition comprising a modified variant TGF-BBPI is for use in improving the appearance and/or condition of the skin of a subject suffering from skin cancer.

In one embodiment, the personal care composition comprising a TGFβ-BBPI is a skin care composition is chosen from skin creams, lotions, sprays, emulsions, colloidal suspensions, foams, aerosols, liquids, gels, sera, and solids. In another embodiment, the personal care composition is a skin care composition selected from moisturizing body washes, body washes, antimicrobial cleansers, skin protective creams, body lotions, facial creams, moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, skin lightening compositions, skin redness reduction compositions, sunscreens, depilatories, hair growth inhibitors, and radioprotectives. Radioprotectives are chosen from non-water-resistant sunscreens, very water-resistant sunscreens, and water-in-silicone sunscreens.

In one embodiment, the personal care composition comprising a TGFβ-BBPI is a skin care composition comprising topically applied over-the-counter compositions, anti-fungal treatments, anti-acne treatments, skin protectants, sunscreens, deodorants, and antiperspirants.

The present invention also provides personal care compositions that are cosmetic compositions. In some preferred embodiments, the cosmetic compositions are selected from mascaras, pressed powder formulations, and foundations. In some preferred embodiments, the makeup compositions comprise at least one pigment.

In some preferred embodiments, the makeup composition comprising at least one pigment is a mascara selected from non-waterproof mascaras, waterproof mascaras, volumizing mascaras, lengthening mascaras, curling mascaras, anhydrous waterproof mascaras, water-based mascaras, and eyelash or eyebrow treatments.

In yet additional embodiments, the makeup compositions are pressed powder formulations selected from loose powders, blushes, eye shadows, and bronzing powders. In still further embodiments, the makeup compositions are foundations selected from water-in-oil foundations, water-in-silicone foundations, oil-in-water foundations, anhydrous makeup sticks, and cream-to-powder foundations.

In some embodiments, the personal skin care compositions comprise a modified variant TGF-BBPI, as set forth herein, and a physiologically acceptable carrier or excipient. Preferably, the TGF-BBPI is present in an amount of about 0.0001% to about 5% by weight based on the total weight of the composition. Also preferably, the TGF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the invention provides a personal care composition that comprises a modified variant TGF-BBPI for use in hair care. In some embodiments, the hair care composition is for use in promoting hair growth.

In some embodiments, the hair care compositions find use in promoting hair growth. In other embodiments, modulation comprises promoting hair growth in a subject suffering from a disease or condition that involves hair loss. In some of these embodiments, the disease or condition is at least one selected from the group consisting of inflammatory alopecias, pseudopelade, scleroderma, tick bites, lichen planus, psoriasis, lupus, seborrheic dermatitis, loose hair syndrome, hemochromatosis, androgenic alopecia, alopecia areata, cancer, conditions that affect defective hair fiber production, and environmental factors that affect hair production. In a preferred embodiment, the disease is androgenic alopecia or alopecia areata.

In one embodiment, the hair care composition is selected from the group consisting of shampoos, conditioners, hair styling compositions, hair colorants, permanent wave formulations, creams, gels, mousses, sprays, emulsions, colloidal suspensions, liquids, foams, and solids. In some embodiments, the hair care composition further comprises a radioprotective. As described for the personal skin care compositions the radioprotective is a sunscreen chosen from non-water-resistant sunscreens, very water-resistant sunscreens, and water-in-silicone sunscreens. In other embodiments, the radioprotective is a sunscreen chosen from non-water-resistant sunscreens, very water-resistant sunscreens, and water-in-silicone sunscreens.

In some embodiments, the personal hair care compositions comprise a modified variant TGFβ -BBPI, as set forth herein, and a physiologically acceptable carrier or excipient. Preferably, the TGFβ-BBPI is present in an amount of about 0.0001% to about 5% by weight based on the total weight of the composition. Also preferably, the TGFβ-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the personal care TGF-BBPI compositions of the invention are for use in the treatment of various diseases associated with elevated levels of TGFβ1 and/or TGFβ2.

The present invention also provides methods for promoting hair growth of a subject, comprising the steps of providing the personal care TGF composition of the present invention; providing a subject to be treated; and applying the composition to the subject in an area in which promotion of hair growth is desired. In some embodiments, the TGF composition is a skin care composition. In other embodiments, the FGF composition is a hair composition. In some embodiments, promotion of hair growth comprises promoting the growth of the subject's hair, wherein the hair growth to be promoted is chosen from facial air, underarm hair, leg hair, torso hair, and arm hair, and head hair. In additional embodiments, the method for promoting hair growth using an TGF hair care composition of the invention comprises applying a personal care composition comprising a TGF-BBPI having an amino acid sequence chosen from SEQ ID NOS:443, 445 and 447.

The compositions of the invention may be used in a method for improving the appearance and/or condition of skin in a subject suffering from a skin disorder, comprising providing the personal skin care composition of the present invention; providing a subject to be treated; and applying the composition to the affected skin of the subject. In some embodiments, the skin disorder is chosen from psoriasis, scleroderma and skin cancer. In additional embodiments, the method for improving the appearance and/or condition of skin in a subject suffering from a skin disorder using a TGF skin care composition of the invention comprises a personal care composition comprising a TGF-BBPI having an amino acid sequence chosen from SEQ ID NOS: 443, 445 and 447.

### 6.0.5 Personal Care Compositions Comprising Modified Variant TNFα-BBPIs

Proteins of the Tissue Necrosis Factor (TNF) family are members of a large cytokine family. Tissue Necrosis Factor alpha (TNFα) is a prototypic member of the TNF family of ligands. It is produced by various immune cells, and plays a key role in immune-mediated inflammatory diseases.

TNFα is synthesized by T-lymphocytes, B cells, synoviocytes, fibroblasts, and macrophages initially as a 26kD protein. Later it is cleaved by TNFα converting enzyme (the metalloproteinase, ADAMS 17) into a monomeric 17-kD molecule. Three of these molecules form, under physiologic conditions, a non-covalently bound, cone-shaped homotrimer that cross-links membrane-bound receptors that exist in 2 isoforms: TNF receptor I (TNF-RI) and TNF receptor II (TNF-RII). Its natural function is to stimulate the recruitment of neutrophils and monocytes to sites of infection and to activate these cells to eradicate microbes, exerting its function by binding to its corresponding TNF receptor on the surface of numerous cell types. It activates the vascular endothelium locally, causing vasodilation and increased permeability. Vascular endothelium adhesion molecules (ICAM-1, VCAM-1, E-selectin) and MHC class II are upregulated leading to recruitment of proinflammatory cells, and induction of immunoglobulins, and complement. Platelets become activated and "stickier," causing small vessel occlusion, and containment of infections. In addition, it induces macrophages and endothelial cells to secrete chemokines and promotes apoptosis of target cells. TNFα has a potent paracrine function, inducing (via a NFkB-mediated mechanism) the secretion of pro-inflammatory cytokines such as IL-1, IL-6, and GM-CSF, and also stimulates the production of various chemokines, including RANTES, IL-8, MCP-1, and MIP-1α. Finally, TNFα plays a role in angiogenesis, which is critical to the growth and propagation of the rheumatoid synovium.

Immune-mediated inflammatory diseases (IMIDs) are triggered by an abnormal production of pro-inflammatory cytokines, including TNFα. These diseases include: rheumatoid arthritis (RA), inflammatory bowel disease (IBD) such as Crohn's disease, chronic plaque psoriasis, psoriatic arthritis, vasculitis, and ankylosing spondylitis.
New developments in the treatment of immune-mediated inflammatory diseases have arisen from basic research in cytokine expression and signaling that identified two key players in the pathophysiology of several diseases in this category: TNFα and interleukin interleukin-1 (IL-1). For example, both cytokines have been found to be elevated in the serum, synovium, and synovial fluid of patients with RA. Moreover, TNFα and IL-1 are capable of inducing and augmenting joint damage in experimental models of arthritis. Such findings led to the development of strategies to block/antagonize their effects, and specific targeting by biological agents has become possible in the recent years. This first generation of products includes monoclonal antibodies (mAb) and soluble receptor fusion proteins, all acting to compete with receptor for binding of the cytokine.

To date, the US FDA has approved three TNFα inhibitors/blocking agents for clinical use in the treatment of several IMIDs, including psoriasis and psoriatic arthritis. They are: Etanercept (Enbrel®, Amgen-Wyeth), a fully human chimeric protein of TNF receptor II fused to the Fc component of human IgG1, Infliximab (Remicade®, Centocor), a chimeric monoclonal antibody, and Adalimumab (Humira®, Abbott) a fully human IgG1 anti-TNFα monoclonal antibody. The proven initial efficacy and safety profile of these agents has to be weighed against the concerns for declining efficacy over time, the high cost of these biopharmaceutical agents and the limitations imposed by the current needle delivery for these agents.

An animal model developed by Boyman and colleagues (Boyman et al., J. Exp. Med. 199:731-736 [2004]) demonstrated the essential role for resident T-cells and TNFα in the spontaneous development of psoriasis. In this model, symptomless pre-psoriatic human skin lesions are grafted onto transgenic AGR129 mice. The group showed that blocking of T cells lead to inhibition of psoriasis development. It also showed that application of neutralizing anti-human TNFα monoclonal antibody (Infliximab, i.v.) or TNF receptor fusion protein (Etanercept, s.c.) led to dramatic inhibition of psoriatic phenotype development. These results have served to support the notion that TNFα antagonists have a direct effect in IMIDs disease development.

Unfortunately, more than one third of patients suffering from any of the approved IMIDs indications do not benefit clinically from anti-TNF treatment (Wong et al., Clin. Immunol. 126:121-136 [2008]). Efforts continue in the development of novel therapeutics to address this medical area. There are a number of "smart" TNFα antagonists currently in clinical trials, including: various anti-TNF monoclonal antibodies, pegylated antibody fragments or truncated TNF receptor molecules. In addition, a small-molecule inhibitor that promotes TNFα subunit disassembly of the trimeric cytokine has shown promising *in vitro* results (He et al., Science 310:1022-1025 [2005]) but no further clinical development has been reported.

Psoriasis is a common skin disorder that affects approximately 2.8 percent of the population (Linden and Weinstein Am. J. Med. 107:595-605 [1999]). An estimated 4.5 million people in the US suffer from psoriais and 1.5 million have moderate to severe plaque psoriasis. The disease is characterized by chronic inflammation of the skin. This inflammation helps drive the formation of red, itchy skin plaques that are often painful and disfiguring. TNF-α plays a critical role in their formation and continued existence because it induces synthesis if IL-1 and IL-8, the triggers of inflammation. TNF-α concentrations are higher in psoriatic lesions than in unaffected skin of psoriatic patients and tend to decline with clearing of the lesions after effective therapy (Mussi et al., J. Biol. Regul. Homeost. Agents 11:115-118 [1997]). TNF-α also promotes keratinocyte proliferation and angiogenesis (Asadullah et al., Drugs today 35:913-924 [1999]), and thus inhibiting this cytokine should halt the disease at multiple stages.

Traditionally, first-line treatment of moderate psoriasis has consisted of topical agents because they are often less invasive than systemic therapy, with a low incidence of the most serious side effects, such as renal or hepatic failure (Kincaid (2005) Drug discovery today 10:884). Some agents such as: antithyroid thioureylenes, propylthiouracil and methimazole, are effective in the treatment of patients with psoriasis with a significant number of patients showing clearing or near clearing of their lesions after a several weeks of treatment. Systemic treatment with the new anti- TNFα biologics: Enbrel, Remicade, and Humira, has been approved for moderate to severe chronic plaque psoriasis. Patients treated with such agents very often show marked improvement in their disease with major clearing in several instances (Chaudhari et al., Lancet 357:1842-1847 [2001]; Leonardi et al., N. Engl. J. Med. 349:2014-2022 [2003]). But present day therapy of the disease is not particularly satisfactory and the many therapies currently in use are associated with significant cumulative toxicity (Gottlieb et al., J Am Acad Dermatol. 48:829-835 [2003]). Risks associated with TNFα blocking agents include serious infections, malignancies, anaphylaxis, hepatitis B reactivation, demyelinating disease, cytopenias, heart failure, and lupus-like syndrome. In addition, there is observed loss of clinical benefit after the drugs are stopped, and a small proportion of patients develop antibodies to the biological agents which is likely to limit their efficacy with repeated use.

Proteins such as the engineered modified variant BBPIs which are much smaller in size and thus presumed to be much less immunogenic should significantly reduce concerns about development of neutralizing antibodies in patients. BBPI molecules, because of their reduced molecular weight in comparison to antibodies or proteins fusions, will likely be more amenable to delivery via a topical route, or via less invasive systemic administrations like subcutaneous injections or needleless delivery methods.

The invention provides personal care compositions comprising a modified variant TNF-BBPI for use in skin and/or hair care. In some embodiments, the personal care composition comprising a TNF-BBPI is used for promoting hair growth and/or improving the condition of the skin of a subject suffering from a dermatological inflammatory disorder. In some embodiments, the inflammatory skin disorder is chosen from dermatitis, eczema, psoriasis, acne, rosacea and hives. In some embodiments, the dermatological inflammatory disorder is psoriasis. Thus, the invention provides for personal skin care and/or hair care compositions. The TNF-BBPIs comprised in the personal care compositions of the invention are modified variant BBPIs in which the equivalent chymotrypsin loop of the precursor scaffold of the TNF-5-BBPI has been replaced by a TNF variant peptide, and which further comprise at least one amino acid substitution as described in sections 5.4.1 and 5.4.2. In the present invention, binding of the modified variant TNF-BBPI to TNFα diverts TNFα from interacting with its cognate receptor and inhibits the TNFα-induced inflammation of the scalp and preventing hair loss. In some embodiments, binding of the modified variant TNF-BBPI to TNFα diminishes the inflammation of the skin and improves the condition of the skin in a subject suffering from a dermatological inflammatory disorder recited herein *e.g*. psoriasis. However, it is not intended that the present invention be limited to any particular mechanism.

In some embodiments, the invention provides a personal care composition comprising a TNF-BBPI that binds to TNF. In alternative embodiments, the binding of the TNF-BBPI to TNF blocks the downstream activity of TNF. In some embodiments, the composition is capable of modulating inflammation.

In some embodiments, the personal care composition comprising a modified variant BBPI-TNF is for use in skin care. In some embodiments, the skin care compositions are for use in improving the appearance and/or condition of skin in a subject suffering from a skin disorder, Thus, in some embodiments, the personal care compositions for use in skin care include cosmetic compositions. In some embodiments, the skin disorder is an inflammatory skin disorder. In some embodiments, the inflammatory skin disorder is psoriasis.

In one embodiment, the personal care composition comprising a TNF-BBPI is a skin care composition is chosen from skin creams, lotions, sprays, emulsions, colloidal suspensions, foams, aerosols, liquids, gels, sera, and solids. In another embodiment, the personal care composition is a skin care composition selected from moisturizing body washes, body washes, antimicrobial cleansers, skin protective creams, body lotions, facial creams, moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, skin lightening compositions, skin redness reduction compositions, sunscreens, depilatories, hair growth inhibitors, and radioprotectives. Radioprotectives are chosen from non-water-resistant sunscreens, very water-resistant sunscreens, and water-in-silicone sunscreens.

In one embodiment, the personal care composition comprising a TNF-BBPI is a skin care composition comprising topically applied over-the-counter compositions, anti-fungal treatments, anti-acne treatments, skin protectants, sunscreens, deodorants, and antiperspirants. In other embodiments, the skin care composition is capable of lightening skin tone, reducing redness, preventing skin tone darkening or preventing color development.

The present invention also provides personal care compositions that are cosmetic compositions. In some preferred embodiments, the cosmetic compositions are selected from mascaras, pressed powder formulations, and foundations. In some preferred embodiments, the makeup compositions comprise at least one pigment.

In some preferred embodiments, the makeup composition comprising at least one pigment is a mascara selected from non-waterproof mascaras, waterproof mascaras, volumizing mascaras, lengthening mascaras, curling mascaras, anhydrous waterproof mascaras, water-based mascaras, and eyelash or eyebrow treatments.

In yet additional embodiments, the makeup compositions are pressed powder formulations selected from loose powders, blushes, eye shadows, and bronzing powders. In still further embodiments, the makeup compositions are foundations selected from water-in-oil foundations, water-in-silicone foundations, oil-in-water foundations, anhydrous makeup sticks, and cream-to-powder.foundations.

In some embodiments, the personal skin care compositions comprise a modified variant TNF-BBPI, as set forth herein, and a physiologically acceptable carrier or excipient. Preferably, the TNF-BBPI is present in an amount of about 0.0001% to about 5% by weight based on the total weight of the composition. Also preferably, the TNF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

In other embodiments, the invention provides a personal care composition comprising a modified variant BBPIs that comprises a TNF variant peptide (TNF-BBPI) for use in hair care.

In some embodiments, the hair care compositions find use in improving the appearance and/or condition of scalp skin in patients suffering from psoriasis.

In one embodiment, the hair care composition is selected from the group consisting of shampoos, conditioners, hair styling compositions, hair colorants, permanent wave formulations, creams, gels, mousses, sprays, emulsions, colloidal suspensions, liquids, foams, and solids.

In some embodiments, the hair care composition further comprises a radioprotective. As described for the personal skin care compositions the radioprotective is a sunscreen chosen from non-water-resistant sunscreens, very water-resistant sunscreens, and water-in-silicone sunscreens. In other embodiments, the radioprotective is a sunscreen chosen from non-water-resistant sunscreens, very water-resistant sunscreens, and water-in-silicone sunscreens.

In some embodiments, the personal hair care compositions comprise a modified variant VEGF-BBPI, as set forth herein, and a physiologically acceptable carrier or excipient. Preferably, the VEGF-BBPI is present in an amount of about 0.0001% to about 5% by weight based on the total weight of the composition. Also preferably, the VEGF-BBPI is present in an amount of about 0.001% to about 0.5% by weight based on the total weight of the composition. The composition may be in the form of an emulsified vehicle, such as a nutrient cream or lotion, a stabilized gel or dispersion system, a treatment serum, a liposomal delivery system, a topical pack or mask, a surfactant-based cleansing system such as a shampoo or body wash, an aerosolized or sprayed dispersion or emulsion, a hair or skin conditioner, styling aid, or a pigmented product such as makeup, as well as other suitable make-up and cosmetic preparations. In some embodiments, the carrier is preferably at least one selected from the group consisting of water, propylene glycol, ethanol, propanol, glycerol, butylene glycol and polyethylene glycol.

### 6.1 Experimental

Certain of the following Examples illustrate the invention; others provide useful background.

In the experimental disclosure which follows, the following abbreviations apply PI (proteinase inhibitor), BBI (Bowman-Birk Inhibitor from *Glycine max* Acc. No. P01055), BBI-AV (Bowman-Birk Inhibitor Anti-VegF), STI (Soybean Trypsin inhibitor from *Glycine max*); VEGF and VegF (vascular endothelial growth factor); BBdb (Bowman Birk Inhibitor from *Dolichos biflorus* Acc. No. AAK97765), BBsb3 (Bowman Birk Inhibitor from *Glycine max* (soybean) protease inhibitor IV or D-II), and BBtc (Bowman Birk Inhibitor from *Torresea cearensis*), FGF-5 (fibroblast growth factor 5), TGFβ (Transforming growth factor β), TNFα (Tumor necrosis factor α), ppm (parts per million); M (molar); mM (millimolar); µM (micromolar); nM (nanomolar); mol (moles); mmol (millimoles); µmol (micromoles); nmol (nanomoles); gm (grams); mg (milligrams); µg (micrograms); pg (picograms); L (liters); ml and mL (milliliters); µl and µL (microliters); cm (centimeters); mm (millimeters); µm (micrometers); nm (nanometers); U (units); V (volts); MW (molecular weight); sec (seconds); min(s) (minute/minutes); h(s) and hr(s) (hour/hours); °C (degrees Centigrade); QS (quantity sufficient); ND (not done); NA (not applicable); rpm (revolutions per minute); H₂O (water); dH₂O (deionized water); (HCl (hydrochloric acid); aa (amino acid); bp (base pair); kb (kilobase pair); kD (kilodaltons); cDNA (copy or complimentary DNA); DNA (deoxyribonucleic acid); ssDNA (single stranded DNA); dsDNA (double stranded DNA); dNTP (deoxyribonucleotide triphosphate); RNA (ribonucleic acid); MgCl₂ (magnesium chloride); NaCl (sodium chloride); w/v (weight to volume); v/v (volume to volume); *g* (gravity); OD (optical density); Dulbecco's phosphate buffered solution (DPBS); SOC (2% Bacto-Tryptone, 0.5% Bacto Yeast Extract, 10 mM NaCl, 2.5 mM KCl); Terrific Broth (TB; 12 g/l Bacto Tryptone, 24 g/l glycerol, 2.31 g/l KH₂PO₄, and 12.54 g/l K₂HPO₄); OD₂₈₀ (optical density at 280 nm); OD₆₀₀ (optical density at 600 nm); A₄₀₅ (absorbance at 405 nm); Vmax (the maximum initial velocity of an enzyme catalyzed reaction); PAGE .(polyacrylamide gel electrophoresis); PBS (phosphate buffered saline [150 mM NaCl, 10 mM sodium phosphate buffer, pH 7.2]); PBST (PBS+0.25% TWEEN® 20); PEG (polyethylene glycol); PCR (polymerase chain reaction); RT-PCR (reverse transcription PCR); SDS (sodium dodecyl sulfate); Tris (tris(hydroxymethyl)aminomethane); HEPES (N-[2-Hydroxyethyl]piperazine-N-[2-ethanesulfonic acid]); HBS (HEPES buffered saline); SDS (sodium dodecylsulfate); bME, BME and βME (beta-mercaptoethanol or 2-mercaptoethanol); Tris-HCl (tris[Hydroxymethyl]aminomethane-hydrochloride); Tricine (N-[tris-(hydroxymethyl)-methyl]-glycine); CHES (2-(N-cyclo-hexylamino) ethane-sulfonic acid); TAPS (3-{[tris-(hydroxymethyl)-methyl]-amino}-propanesulfonic acid); CAPS (3-(cyclo-hexylamino)-propane-sulfonic acid; DMSO (dimethyl sulfoxide); DTT (1,4-dithio-DL-threitol); Glut and GSH (reduced glutathione); GSSG (oxidized glutathione); TCEP (Tris[2-carboxyethyl] phosphine); Ci (Curies) mCi (milliCuries); µCi (microCuries); TLC (thin layer achromatography); Ts (tosyl); Bn (benzyl); Ph (phenyl); Ms (mesyl); Et (ethyl), Me (methyl); *Taq* (Thermus aquaticus DNA polymerase); Klenow (DNA polymerase I large (Klenow) fragment); rpm (revolutions per minute); EGTA (ethylene glycol-bis(ß-aminoethyl ether) N, N, N', N'-tetraacetic acid); EDTA (ethylenediaminetetracetic acid); bla (ß-lactamase or ampicillin-resistance gene); GE Healthcare (GE Healthcare, Chalfont St. Giles, United Kingdom); DNA2.0 (DNA2.0, Menlo Park, CA); OXOID (Oxoid, Basingstoke, Hampshire, UK); Megazyme (Megazyme International Ireland Ltd., Bray Business Park, Bray, Co., Wicklow, Ireland); Corning (Corning Life Sciences, Corning, NY); (NEN (NEN Life Science Products, Boston, MA); Pharma AS (Pharma AS, Oslo, Norway); Dynal (Dynal, Oslo, Norway); Bio-Synthesis (Bio-Synthesis, Lewisville, TX); ATCC (American Type Culture Collection, Rockville, MD); Gibco/BRL (Gibco/BRL, Grand Island , NY); Sigma (Sigma Chemical Co., St. Louis, MO); Pharmacia (Pharmacia Biotech, Pisacataway, NJ); NCBI (National Center for Biotechnology Information); Applied Biosystems (Applied Biosystems, Foster City, CA); Clontech (CLONTECH Laboratories, Palo Alto, CA); Operon Technologies (Operon Technologies, Inc., Alameda, CA); MWG Biotech (MWG Biotech, High Point, NC); Oligos Etc (Oligos Etc. Inc, Wilsonville, OR); Bachem (Bachem Bioscience, Inc., King of Prussia, PA); Difco (Difco Laboratories, Detroit, MI); Mediatech (Mediatech, Herndon, VA; Santa Cruz (Santa Cruz Biotechnology, Inc., Santa Cruz, CA); BioVeris (BioVeris Corp., Gaithersburg, MD); Oxoid (Oxoid Inc., Ogdensburg, NY); Worthington (Worthington Biochemical Corp., Freehold, NJ); GIBCO BRL or Gibco BRL (Life Technologies, Inc., Gaithersburg, MD); Millipore (Millipore, Billerica, MA); Bio-Rad (Bio-Rad, Hercules, CA); Invitrogen (Invitrogen Corp., San Diego, CA); NEB (New England Biolabs, Beverly, MA); Sigma (Sigma Chemical Co., St. Louis, MO); Pierce (Pierce Biotechnology, Rockford, IL); Takara (Takara Bio Inc. Otsu, Japan); Roche (Hoffmann-La Roche, Basel, Switzerland); EM Science (EM Science, Gibbstown, NJ); Qiagen (Qiagen, Inc., Valencia, CA); Biodesign (Biodesign Intl., Saco, Maine); Aptagen (Aptagen, Inc., Herndon, VA); Molecular Devices (Molecular Devices, Corp., Sunnyvale, CA); R&D Systems (R&D Systems, Minneapolis, MN); Stratagene (Stratagene Cloning Systems, La Jolla, CA); Marsh (Marsh Biosciences, Rochester, NY); Bio-Tek (Bio-Tek Instruments, Winooski, VT); (Biacore (Biacore, Inc., Piscataway, NJ); PeproTech (PeproTech, Rocky Hill, NJ); SynPep (SynPep, Dublin, CA); and Microsoft (Microsoft, Inc., Redmond, WA).

### EXAMPLE 1

### Production of BCE103-BBI Fusion Proteins in B. subtilis

In this Example, experiments conducted to produce BCE103-BBI fusion proteins in *B. subtilis* are described. The DNA sequence of the synthetic gene (Operon Technologies) coding for the pro-BBI protein with a C-terminal hexa-histidine tag used in these experiments is:

AACCTGCGTCTGTCTAAGCTTGGCCTGCTTATGAAATCAGACCATCAGCACAGC
AATGACGATGAGAGCTCTAAACCCTGTTGCGATCAATGCGCATGTACGAAATCA
AATCCTCCACAGTGTCGGTGTTCCGATATGCGTCTGAATAGCTGTCATAGTGCA
TGCAAAAGCTGTATCTGCGCCCTGAGTTATCCAGCTCAATGTTTTTGCGTCGAC
ATCACGGACTTCTGCTATGAGCCATGTAAACCAAGCGAGGACGATAAAGAGAAC
CATCATCACCATCACCAT (SEQ ID NO:10)

The protein sequence of pro-BBI with a C-terminal hexa-histidine tagged coded for by the above synthetic gene is:

NLRLSKLGLLMKSDHQHSNDDESSKPCCDQCACTKSNPPQCRCSDMRLNSCHSA
CKSCICALSYPAQCFCVDITDFCYEPCKPSEDDKENHHHHHH (SEQ ID NO:11)

The portion of the DNA sequence of the synthetic gene that codes for the major mature form of BBI is:

GACGATGAGAGCTCTAAACCCTGTTGCGATCAATGCGCATGTACGAAATCAAAT
CCTCCACAGTGTCGGTGTTCCGATATGCGTCTGAATAGCTGTCATAGTGCATGC
AAAAGCTGTATCTGCGCCCTGAGTTATCCAGCTCAATGTTTTTGCGTCGACATCA
CGGACTTCTGCTATGAGCCATGTAAACCAAGCGAGGACGATAAAGAGAAC
(SEQ ID NO:12)

The protein sequence of the major mature form of BBI coded by the above synthetic gene is:

DDESSKPCCDQCACTKSNPPQCRCSDMRLNSCHSACKSCICALSYPAQCFCVDIT
DFCYEPCKPSEDDKEN (SEQ ID NO:13)

The PCR primers used to amplify the BBI gene for fusion to the BCE103 cellulase expression cassette in the pJ103 vector were:
BBIfusion_FW: 5' CAGCACGGATCCAGACGATGAGAGCTCTAAACCC 3' (SEQ ID NO:14)

BBIHindIII_RV: 5' CTGCAGAAGCTTAAAATAAAAAACGGATTTCCTTCA
GGAAATCCGTCCTCTGTTAACTTTTAGTTCTCTTTATCGTCCTCGC 3' (SEQ ID
NO:15)

BBIHIS-HindIII_RV: 5' CTGCAGAAGCTTAAAAATAAAAAAACGGATT
TCCTTCAGGAAATCCGTCCTCTGTTAACTTTTAATGGTGATGGTGATGAT
GGTTCTC 3' (SEQ ID NO:16)

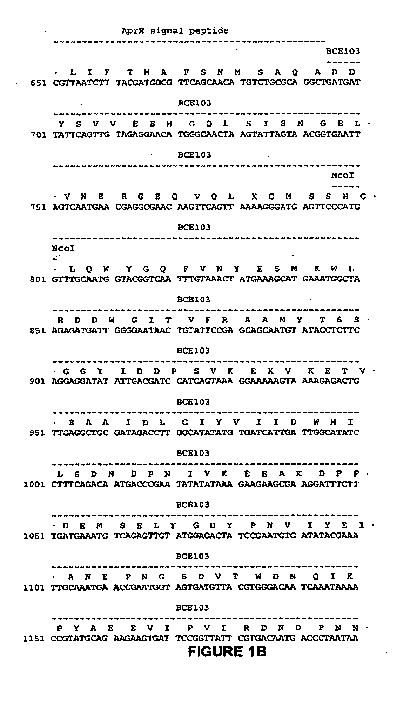

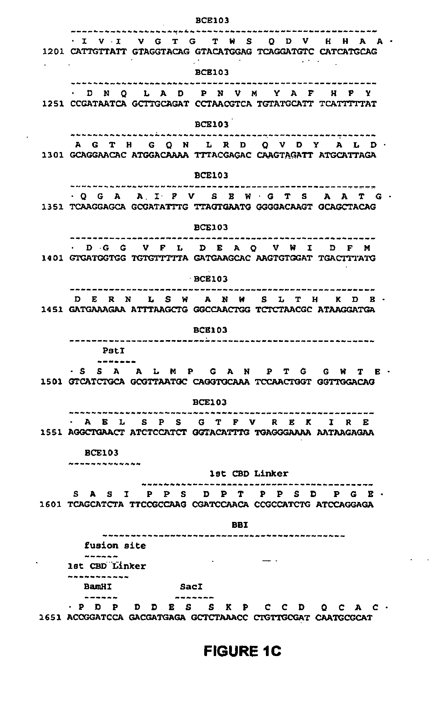

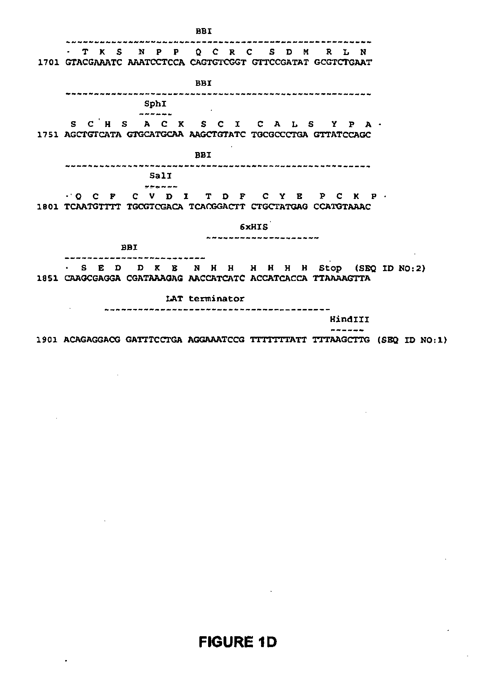

The sequence of the *aprE-*BCE103-BBI-HisTag expression cassette (*Eco*RI-*Hin*dIII) that was cloned into the pJM103 integration vector is provided in Figure 1. A schematic plasmid map of the pJM103BBIHis expression vector is provided in Figure 2.

The alkaline cellulase (BCE103) gene *(See,* van Soligen, U.S .Patent No. 6,063,611) fused to the *B. subtilis aprE* promoter and signal sequence, was cloned from pUCAPR103 (Shaw et al., J. Mol. Biol., 320:303-309 [2002]) as an *Eco*RI-*Bam*HI fragment (*i.e*., a fragment that carries the coding sequence of the BCE103 catalytic domain and first cellulose binding domain linker only) into pJM103 (Perego, "Integrational vectors for genetic manipulation in Bacillus subtilis" In, Bacillus subtilis and Other Gram-positive Bacteria: Biochemistry, Physiology, and Molecular Genetics, Sonenshein, Hoch, and Losick (eds), American Society for Microbiology, Washington D.C., pp. 615-624 [1993]). A gene encoding the soybean Bowman-Birk protease inhibitor (BBI) (Swiss-Prot Accession # P01055; *See,* Odani and Ikenaka, J. Biochem., 71: 839-848 [1972]) with a C-terminal hexa-histidine tag (His-Tag) was synthesized by Operon Technologies (*See*, DNA sequence above). The BBI gene was amplified by PCR with primers (all primers were synthesized by MWG Biotech, Oligos Etc., or Operon Technologies) that generated a 5' *Bam*HI site in the correct reading frame with the BCE103 gene, and at the 3' end introduced a strong transcriptional terminator (LAT, from the *Bacillus licheniformis* α-amylase gene) after the end of the BBI gene with a 3' *Hind*III site for cloning into the pJM103 vector.

PCR fragments with or without a C-terminal His-Tag were generated with the primers BBIfusion_FW (SEQ ID NO:14) and BBIHISHindIII_RV (SEQ ID NO:16), or BBIfusion_FW (SEQ ID NO:14) and BBI-HindIII_RV(SEQ ID NO:15), respectively, using the synthetic BBI gene as a template. Unless indicated otherwise, PCR reactions were typically performed on a thermocycler for 30 cycles with High Fidelity Platinum *Taq* polymerase (Invitrogen) according to the instructions of the supplier (with an annealing temperature of 55°C). The PCR fragments were cloned as *Bam*HI*-Hin*dIII fragments into pJM103 carrying the *aprE*-BCE103 expression cassette. The correct gene sequence was verified by DNA sequencing.

Thus, as shown in Figure 1, the N-terminus of the mature coding region of the BBI gene (with or without the His-Tag) was fused in frame to the C-terminal coding region of the first CBD (cellulose binding domain) linker sequence coded by the BCE103 cellulase gene. Thereby, the two CBD's of BCE103 (Shaw *et al., supra*) are replaced by BBI in the final expression vectors, pJM103BBI or pJM103BBIhis (*See,* Figure 2). The *aprE* promoter controls the expression of the BCE103-BBI gene fusions (*See*, Ferrari et al., J. Bact., 170:289-295 [1988]; and Henner et al., J. Bact., 170: 296-300 [1988]).

Competent *Bacillus subtilis* cells, BG3934comK (*degU^{Hy}32*, *oppA*, Δ*spoIIE3501*, Δ*aprE*, Δ*nprE*, Δ*eper,* Δ*ispA*, Δ*bpr*, *amyE::xylRPxylAcomK-phleo*), were transformed with the expression plasmids, pJM103BBI or pJM103BBIhis. The bacteria were made competent by the induction of the *comK* gene under control of a xylose inducible promoter (Hahn et al., Mol. Microbiol., 21:763-775 [1996]). The transformants were selected on Luria Broth agar (LA) plates containing 5 µg/ml chloramphenicol. To increase the expression by gene amplification, colonies were streaked and grown several times on LA plates with 25 µg/ml chloramphenicol until the growth rate with the antibiotic was similar to growth rate in the absence of chloramphenicol. The BCE103-BBI fusion protein was produced by growth in shake flasks at 37°C in TSB medium (Tryptone Soya Broth from OXOID, 30 g/L) or in MBD medium, a MOPS based defined medium. MBD medium was made essentially as described (Neidhardt et al., J. Bacteriol., 119: 736-747 [1974]), except NH₄Cl₂, FeSO₄, and CaCl₂ were left out of the base medium, 3 mM K₂HPO₄ was used, and the base medium was supplemented with 60 mM urea, 75 g/L glucose, and 1 % soytone. Also, the micronutrients were made up as a 100 X stock containing in one liter, 400 mg FeSO₄ 7H₂O, 100 mg MnSO₄ H₂O, 100 mg ZnSO₄·7H₂O, 50 mg CuCl₂·2H₂O, 100 mg CoCl₂·6H₂O, 100 mg NaMoO₄·2H₂O, 100 mg Na₂B₄O₇·10H₂O, 10 ml of 1 M CaCl₂, and 10 ml of 0.5 M sodium citrate.

BCE103-BBI fusion protein could be easily visualized in samples from cell free supernatants (after 24 h of growth in TSB medium or 48 h in MBD medium) as the major protein band on SDS-PAGE gels (10 % NuPAGE in MES buffer, run as described by the manufacturer, Invitrogen) running at - 44 kDa by using standard protein stains (*e.g.* GelCode Blue Stain Reagent; Pierce). The identity of the BCE103-BBI fusion protein was verified by immunoblots of SDS-PAGE gels using the protocols supplied by the manufacturer (BM Chromogenic Western Blotting Kit; Roche Applied Science using an anti-HisTag antibody or an anti-BCE103 cellulase polyclonal antibody for detection).

To determine the BCE103 activity, cellulase degradation was assessed qualitatively on LA cellulase indicator plates (with 1% carboxymethylcellulose stained with 0.2 % Congo Red, or with 0.5 % azo-CM-cellulose, Megazyme), or quantitatively by a direct assay in Assay Buffer (100 mM Tris pH 8.6, 0.005 % Tween-80) on the culture broth using a the synthetic substrate, 4-nitrophenyl -D-cellobioside (Sigma), using methods known in the art (*See e.g.,* van Tilbeurgh et a/., Meth. Enzymol., 160:45-59 [1988]).

Trypsin inhibitory assays were performed in Assay Buffer to determine the BBI activity. Specifically, a standard curve was generated by making eleven 1:1 serial dilutions (100 µL BBI + 100 µL Assay Buffer) of a 2 µg/mL standard BBI solution. The BBI standard was purified from a 1 mg/ml Trypsin-Chymotrypsin Inhibitor (Sigma Cat. #T-9777) solution in 20 mM MES pH 6.0 using a hydrophobic interaction column (POROS HP2, Phenyl column, Applied Biosystems). The column was equilibrated with 20mM MES pH 6.0, loaded with 5 mg of the inhibitor, washed with the equilibration buffer, and then the BBI was eluted with water. Unknown BBI samples to be tested in the inhibitory assay were diluted as necessary, so that two or more data points would fall within the standard curve (usually 1:10, 1:100, 1:200, 1:1000, 1:2000 sample dilutions were tested and then the dilutions fine tuned if necessary). Each diluted BBI standard or sample, 20 µL, was added to 80 µL of 50 ng/ml bovine pancreatic trypsin (Worthington) (made by diluting a stock 1 mg/mL trypsin solution into Assay Buffer). For convenience, the standards and samples were arrayed in 96 well microtiter plates. The reactions were mixed and incubated 15 min at 25 °C. After the incubation, 100 µL of the 0.5 mg/ml trypsin substrate (diluted in Assay Buffer from a 100 mg/ml solution in DMSO), Suc-AAPR-pNA (succinyl-Ala-Ala-Pro-Arg-para-nitroanilide, Bachem), was added, mixed and the OD (A₄₀₅) was monitored for 15 min, with 1 time point recorded every 12 sec using a Spectra Max 250 (Molecular Devices). The data points were used to determine the Vmax for each reaction. The standard curve was generated by plotting Vmax versus BBI concentration and was fitted to a four-parameter curve. All data fitting was done using software supplied by the manufacturer (Molecular Devices). The BBI concentration of the unknown samples was calculated from the standard curve. Alternatively, the BBI activity was measured using the same protocol but by determining bovine pancreatic chymotrypsin (Worthington) inhibition (chymotrypsin was used at the same concentration as trypsin and chymotrypsin activity was measured by adding 100 µL of a 0.4 mg/ml chymotrypsin substrate, succinyl-Ala-Ala-Pro-Phe-para-nitroanilide, Bachem).

Titers from shake flask runs (500 ml MBD medium in 2.8 L Fernbach 6 baffled flasks, 37°C, 225 rpm, harvested 60 h after of growth) typically ranged from 0.4 - 0.9 mg/ml BCE activity and 40-150 µg/ml BBI trypsin inhibitory activity. However, it is contemplated that titers likely could be improved further by optimizing the bacterial strain, culture medium and growth conditions (aeration, temperature, time of harvest, etc.).

In addition to the BCE103 fusion to wild-type BBI, fusion proteins to BBI variants and fusion proteins with various linkers between BCE103 and BBI were produced using the methods outlined above, as described in the following Examples. In addition, fusion proteins were also produced when the BBI was fused to the 2^{nd} CBD linker (BCE-cbdD-BBI; *See*, Example 4) making it possible to use the 1^{st} CBD to aid in the purification process.

### EXAMPLE 2

### Production of Peptides Substituted into the BBI Reactive Site Loops as BCE103-BBI Fusion Proteins

In this Example, experiments conducted to produce peptides substituted into the BBI reactive site loops as BCE103-BBI fusion proteins are described. The primers, as well as other sequences used in the various steps of these experiments are provided below. The sequence of 12BBIck81 from the BCE103 fusion site (at the *Bam*HI) to the end of the gene is provided in Figure 3. The CK37281 peptide sequences (ACYNLYGWTC (SEQ ID NO:9) are inserted into both the trypsin and chymotrypsin inhibitory loops.

The primers used to introduce an *Eco*RI site in the BBI gene using QuikChange® site-directed mutagenesis (Stratagene) were:
BowBeco-F
   5'-GATATGCGTCTGAATTCCTGTCATAGTGCAT (SEQ ID NO:17)
BowBeco-R
   5'-ATGCACTATGACAGGAATTCAGACGCATATC (SEQ ID NO:18)

The sequences of the DNA oligonucleotides that were annealed and cloned in the BBI gene (*Sac*I*-Eco*RI) to replace the trypsin inhibitory loop with the VegF binding peptide CK37281 were:

1BBck81+
5'-CTAAACCCTGTTGCGATCAATGCGCATGTTATAATTTGTATGGGTGGACTT
GTCGCTGCAGCGATATGCGTCTG (SEQ ID NO:19)

1BBck81-
5'- AATTCAGACGCATATCGCTGCAGCGACAAGTCCACCCATACAAATTATAA
CATGCGCATTGATCGCAACAGGGTTTAGAGCT (SEQ ID NO:20)

The sequences of the DNA oligonucleotides that were annealed and cloned in the BBI gene (*Eco*RI-*Sal*I) to replace the chymotrypsin inhibitory loop with the VegF binding peptide CK37281 were:

2BBck81+
5'-AATTCCTGTCATAGTGCCTGCAAAAGCTGCGCATGTTATAACCTGTACGGG
TGGACCTGTTTTTGCG (SEQ ID NO:21)

2BBck81-
5'-TCGACGCAAAAACAGGTCCACCCGTACAGGTTATAACATGCGCAGCTTTT
GCAGGCACTATGACAGG (SEQ ID NO:22)

The DNA sequences of the oligonucleotide pairs used to make cassettes to introduce peptides into the trypsin (*Sac*I and *Eco*R*I* restriction sites) or chymotypsin (*Eco*RI and *Sal*I restriction sites) reactive site loops of the synthetic BBI gene are provided below. These peptide coding sequences were then moved into the p2JM103BBI expression vector as *Sac*I-*Sal*I fragments.
Comstatin (1^{st} loop) 
CTAAACCCTGTTGCGATCAATGCGCATGTGTTGTTCAGGACTGGGGTCACCACC
GTTGTCGCTGCAGCGATATGCGTCTG (SEQ ID NO:23)
 and 
AATTCAGACGCATATCGCTGCAGCGACAACGGTGGTGACCCCAGTCCTGAACA
ACACATGCGCATTGATCGCAACAGGGTTTAGAGCT (SEQ ID NO:24)
 Comstatin (2^{nd} loop)
CAAAAGCTGTATCTGCGTTGTTCAGGACTGGGGTCACCACCGTTGTTTTTGCG (SEQ ID NO:25)
and 
TCGACGCAAAAACAACGGTGGTGACCCCAGTCCTGAACAACGCAGATACAGCT
TTTGCATG (SEQ ID NO:26)
 C2c(1^{st} loop) 
CTAAACCCTGTTGCGATCAATGCAGCTGTGGTCGTAAAATCCCGATCCAGTGTC
GCTGCAGCGATATGCGTCTG (SEQ ID NO:27)
 and 
AATTCAGACGCATATCGCTGCAGCGACACTGGATCGGGATTTTACGACCACAGC
TGCATTGATCGCAACAGGGTTTAGAGCT (SEQ ID NO:28)
 C3c(1^{st} loop) 
CTAAACCCTGTTGCGATCAATGCGGTTGTGCTCGTTCTAACCTGGACGAATGTC
GCTGCAGCGATATGCGTCTG (SEQ ID NO:29)
 and 
AATTCAGACGCATATCGCTGCAGCGACATTCGTCCAGGTTAGAACGAGCACAAC
CGCATTGATCGCAACAGGGTTTAGAGCT (SEQ ID NO:30)
 C4c (1^{st} loop) 
CTAAACCCTGTTGCGATCAATGCGGTTGTCAGCGTGCTCTGCCGATCCTGTGTC
GCTGCAGCGATATGCGTCTG (SEQ ID NO:31)
 and 
AATTCAGACGCATATCGCTGCAGCGACACAGGATCGGCAGAGCACGCTGACAA
CCGCATTGATCGCAACAGGGTTTAGAGCT (SEQ ID NO:32)
 C5c(1^{st} loop) 
CTAAACCCTGTTGCGATCAATGCCAGTGTGGTCGTCTGCACATGAAAACCTGTC
GCTGCAGCGATATGCGTCTG (SEQ ID NO:33)
 and 
AATTCAGACGCATATCGCTGCAGCGACAGGTTTTCATGTGCAGACGACCACACT
GGCATTGATCGCAACAGGGTTTAGAGCT (SEQ ID NO:34)
 Xa1 (2^{nd} loop) 
AATTCCTGTCATAGTGCCTGCAAAAGCTGTATCTGCGCCCGTAGTTTGCCAGCT
CAATGTTTTTGCG (SEQ ID NO:35)
 and 
TCGACGCAAAAACATTGAGCTGGCAAACTACGGGCGCAGATACAGCTTTTGCAG
GCACTATGACAGG (SEQ ID NO:36)
 hSCC1 (1^{st} loop) 
CTAAACCCTGTTGCGATCAATGCAACTGTACGTACTCAACCCCTCCACAGTGTC
GCTGCAGCGATATGCGTCTG (SEQ ID NO:37)
 and

AATTCAGACGCATATCGCTGCAGCGACACTGTGGAGGGGTTGAGTACGTACAG
TTGCATTGATCGCAACAGGGTTTAGAGCT (SEQ ID NO:38)

The DNA sequences of oligonucleotide primer pairs used to introduce peptide sequences into the trypsin or chymotrypsin reactive site loops using a QuikChange® II XL site-directed mutagenesis kit (Stratagene) are provided below. The reactions were performed as outlined by the manufacturer and described in this Example. Twenty cycles were performed with extensions of 6 minutes at 68 °C, denaturations of 50 s at 95 °C, and annealings at 55 °C for 50 s. After the cycles, a final extension was performed at 68 °C for 20 minutes.
1A (2^{nd} loop)
   CTGTATCTGCAAACGCTCAAAATCTCGTGGCTGTTTTTGCGTCGACATCAC (SEQ ID NO:39)
   and
   CGCAAAAACAGCCACGAGATTTTGAGCGTTTGCAGATACAGCTTTTGCATG (SEQ ID NO:40)
2B (2^{nd} loop)
   CTGTATCTGCTGGTATAATCAAATGACAACATGTTTTTGCGTCGACATCAC (SEQ ID NO:41)
   and
   CGCAAAAACATGTTGTCATTTGATTATACCAGCAGATACAGCTTTTGCATG (SEQ ID NO:42)
4A (2^{nd} loop)
   CTGTATCTGCCATCAACTTGGCCCGAATTCATGTTTTTGCGTCGACATCAC (SEQ ID NO:43)
   and
   CGCAAAAACATGAATTCGGGCCAAGTTGATGGCAGATACAGCTTTTGCATG (SEQ ID NO:44)
5A (2^{nd} loop)
   CTGTATCTGCCATCCGTGGGCACCGTATTCTTGTTTTTGCGTCGACATCAC (SEQ ID NO:45)
   and
   CGCAAAAACAAGAATACGGTGCCCACGGATGGCAGATACAGCTTTTGCATG (SEQ ID NO:46)
6-1 A (2^{nd} loop)
   CTGTATCTGCAATCTTCATTATCTTCAACAGTGTTTTTGCGTCGACATCAC (SEQ ID NO:47)
   and
   CGCAAAAACACTGTTGAAGATAATGAAGATTGCAGATACAGCTTTTGCATG (SEQ ID NO:48)
7A (2^{nd} loop)
   CTGTATCTGCACACCGTCTCTTTATCGCCCGTGTTTTTGCGTCGACATCAC (SEQ ID NO:49)
   and
   CGCAAAAACACGGGCGATAAAGAGACGGTGTGCAGATACAGCTTTTGCATG (SEQ ID NO:50)
8B (2^{nd} loop)
   CTGTATCTGCCTTACAGATCAATCTAAACCGTGTTTTTGCGTCGACATCAC (SEQ ID NO:51)
   and
   CGCAAAAACACGGTTTAGATTGATCTGTAAGGCAGATACAGCTTTTGCATG (SEQ ID NO:52)
9A (2^{nd} loop)
   CTGTATCTGCGTTACAACATCAATGGGCATGTGTTTTTGCGTCGACATCAC (SEQ ID NO:53)
   and
   CGCAAAAACACATGCCCATTGATGTTGTAACGCAGATACAGCTTTTGCATG (SEQ ID NO:54)
10B (2^{nd} loop)
   CTGTATCTGCCGCGCATCACCGTATGATTGGTGTTTTTGCGTCGACATCAC (SEQ ID NO:55)
   and
   CGCAAAAACACCAATCATACGGTGATGCGCGGCAGATACAGCTTTTGCATG (SEQ ID NO:56)
11-1A (2^{nd} loop)
   CTGTATCTGCTCAACACAAAAAATTCCGCAATGTTTTTGCGTCGACATCAC (SEQ ID NO:57)
   and
   CGCAAAAACATTGCGGAATTTTTTGTGTTGAGCAGATACAGCTTTTGCATG (SEQ ID NO:58)
12B (2^{nd} loop)
   CTGTATCTGCACACAATTTCGCTCTGCAACATGTTTTTGCGTCGACATCAC (SEQ ID NO:59)
   and
   CGCAAAAACATGTTGCAGAGCGAAATTGTGTGCAGATACAGCTTTTGCATG (SEQ ID NO:60)
13A (2^{nd} loop)
   CTGTATCTGCCCGGATCATGTTCCGCATCTTTGTTTTTGCGTCGACATCAC (SEQ ID NO:61)
   and
   CGCAAAAACAAAGATGCGGAACATGATCCGGGCAGATACAGCTTTTGCATG (SEQ ID NO:62)
15-1 A (2^{nd} loop)
   CTGTATCTGCTCAGGCTTTCCGCTTTCTACATGTTTTTGCGTCGACATCAC (SEQ ID NO:63)
   and
   CGCAAAAACATGTAGAAAGCGGAAAGCCTGAGCAGATACAGCTTTTGCATG (SEQ ID NO:64)
1A6 (1^{st} loop) 
TCAATGCGCATGTGAAGAGATCTGGACTATGCTTTGCCGGTGTTCCGATATGCG
TC (SEQ ID NO:65)
 and 
CGGAACACCGGCAAAGCATAGTCCAGATCTCTTCACATGCGCATTGATCGCAAC
AGG (SEQ ID NO:66)
1A6 (2^{nd} loop) 
CAAAAGCTGTGCTTGTGAAGAGATCTGGACTATGCTTTGCTTTTGCGTCGACAT
CACGG (SEQ ID NO:67)
 and 
ACGCAAAAGCAAAGCATAGTCCAGATCTCTTCACAAGCACAGCTTTTGCATGCA
CTATG (SEQ ID NO:68)
1C2 (1^{st} loop) 
TCAATGCGCATGTTGGGCCCTTACTGTCAAAACATGCCGGTGTTCCGATATGCG
TC (SEQ ID NO:69)
 and 
CGGAACACCGGCATGTTTTGACAGTAAGGGCCCAACATGCGCATTGATCGCAA
CAGG (SEQ ID NO:70)
1 C2 (2^{nd} loop) 
CAAAAGCTGTGCTTGTTGGGCCCTTACTGTCAAAACATGCTTTTGCGTCGACAT
CACGG (SEQ ID NO:71)
 and 
ACGCAAAAGCATGTTTTGACAGTAAGGGCCCAACAAGCACAGCTTTTGCATGCA
CTATG (SEQ ID NO:72)
2E2 (1^{st} loop) 
TCAATGCGCATGTCTTACAGTACTGTGGACTACATGCCGGTGTTCCGATATGCG
TC (SEQ ID NO:73)
 and 
CGGAACACCGGCATGTAGTCCACAGTACTGTAAGACATGCGCATTGATCGCAAC
AGG (SEQ ID NO:74)
2E2 (2^{nd} loop) 
CAAAAGCTGTGCTTGTCTTACAGTACTGTGGACTACATGCTTTTGCGTCGACATC
ACGG (SEQ ID NO:75)
 and 
ACGCAAAAGCATGTAGTCCACAGTACTGTAAGACAAGCACAGCTTTTGCATGCA
CTATG (SEQ ID NO:76)
2E5 (1^{st} loop) 
TCAATGCGCATGTACTCTTTGGAACAGATCTCCTTGCCGGTGTTCCGATATGCG
TC (SEQ ID NO:77)
 and 
CGGAACACCGGCAAGGAGATCTGTTCCAAAGAGTACATGCGCATTGATCGCAA
CAGG (SEQ ID NO:78)
2E5 (2^{nd} loop) 
CAAAAGCTGTGCTTGTACTCTTTGGAATCGATCTCCTTGCTTTTGCGTCGACATC
ACGG (SEQ ID NO:79)
 and 
ACGCAAAAGCAAGGAGATCGATTCCAAAGAGTACAAGCACAGCTTTTGCATGCA
CTATG (SEQ ID NO:80)
FGFns (1^{st} loop) 
TCAATGCGCATGTACAAACATCGATTCTACTCCTTGCCGGTGTTCCGATATGCG
TC (SEQ ID NO:81)
 and 
CGGAACACCGGCAAGGAGTAGAATCGATGTTTGTACATGCGCATTGATCGCAAC
AGG (SEQ ID NO:82)
FGFns (2^{nd} loop) 
CAAAAGCTGTGCTTGCACAAACATCGATTCTACTCCTTGTTTTTGCGTCGACATC
ACGG (SEQ ID NO:83)
 and 
ACGCAAAAACAAGGAGTAGAATCGATGTTTGTGCAAGCACAGCTTTTGCATGCA
CTATG (SEQ ID NO:84)
FGFkr (1^{st} loop) 
TCAATGCGCATGTACAAAAATCGATCGTACTCCTTGCCGGTGTTCCGATATGCG
TC (SEQ ID NO:85)
 and 
CGGAACACCGGCAAGGAGTACGATCGATTTTTGTACATGCGCATTGATCGCAAC
AGG (SEQ ID NO:86)
FGFkr (2^{nd} loop) 
CAAAAGCTGTGCTTGCACAAAAATCGATCGTACTCCTTGTTTTTGCGTCGACATC
ACGG (SEQ ID NO:87)
 and 
ACGCAAAAACAAGGAGTACGATCGATTTTTGTGCAAGCACAGCTTTTGCATGCA
CTATG (SEQ ID NO:88)
FGFhl (1^{st} loop) 
TCAATGCGCATGTCACCTGCAGACAACTGAAACATGCCGGTGTTCCGATATGCG
TC (SEQ ID NO:89)
 and 
CGGAACACCGGCATGTTTCAGTTGTCTGCAGGTGACATGCGCATTGATCGCAAC
AGG (SEQ ID NO:90)
FGFhl (2^{nd} loop) 
CAAAAGCTGTGCTTGCCACCTGCAGACAACTGAAACATGTTTTTGCGTCGACAT
CACGG (SEQ ID NO:91)
 and 
ACGCAAAAACATGTTTCAGTTGTCTGCAGGTGGCAAGCACAGCTTTTGCATGCA
CTATG (SEQ ID NO:92)
FGFgy (1^{st} loop) 
TCAATGCGCATGTGGCTACTTCATCCCATCGATTTGCCGGTGTTCCGATATGCG
TC (SEQ ID NO:93)
 and 
CGGAACACCGGCAAATCGATGGATGAAGTAGCCACATGCGCATTGATCGCAA
CAGG (SEQ ID NO:94)
FGFgy (2^{nd} loop) 
CAAAAGCTGTGCTTGCGGCTACTTCATCCCATCGATTTGTTTTTGCGTCGACATC
ACGG (SEQ ID NO:95)
 and 
ACGCAAAAACAAATCGATGGATGAAGTAGCCGCAAGCACAGCTTTTGCATGCA
CTATG (SEQ ID NO:96)
MM005 (1^{st} loop) 
TCAATGCGCATGTTTACGTATCCTTGCTAACAAATGCCGGTGTTCCGATATGCGT
C (SEQ ID NO:97)
 and 
CGGAACACCGGCATTTGTTAGCAAGGATACGTAAACATGCGCATTGATCGCAAC
AGG (SEQ ID NO:98)
MM005 (2^{nd} loop) 
CAAAAGCTGTGCTTGCTTACGTATCCTTGCTAACAAATGTTTTTGCGTCGACATC
ACGG (SEQ ID NO:99)
 and 
ACGCAAAAACATTTGTTAGCAAGGATACGTAAGCAAGCACAGCTTTTGCATGCA
CTATG (SEQ ID NO:100)
MM007 (1^{st} loop) 
GCGATCAATGCGCCTGCAGAACTCAACCATATCCTTTATGTCGGTGTTCCGATA
TGCGTC (SEQ ID NO:101)
 and 
GGAACACCGACATAAAGGATATGGTTGAGTTCTGCAGGCGCATTGATCGCAACA
GGGTTT (SEQ ID NO:102)
MM007 (2^{nd} loop) 
CAAAAGCTGTGCCTGCAGAACACAACCTTACCCACTTTGTTTTTGCGTCGACAT
CACGG (SEQ ID NO:103)
 and 
ACGCAAAAACAAAGTGGGTAAGGTTGTGTTCTGCAGGCACAGCTTTTGCATGCA
CTATG (SEQ ID NO:104)
MM009 (2^{nd} loop) 
CAAAAGCTGTGCCTGCCTGTTAACACCTACTCTTAACTGTTTTTGCGTCGACATC
ACGG (SEQ ID NO:105)
 and 
ACGCAAAAACAGTTAAGAGTAGGTGTTAACAGGCAGGCACAGCTTTTGCATGCA
CTATG (SEQ ID NO:106)
MM010 (1^{st} loop) 
TCAATGCGCATGCGCTCTTCCAACTCATTCTAACTGTCGGTGTTCCGATATGCG
TCT (SEQ ID NO:107)
 and 
CGGAACACCGACAGTTAGAATGAGTTGGAAGAGCGCATGCGCATTGATCGCAA
CAGG (SEQ ID NO:108)
MM010 (2^{nd} loop) 
CAAAAGCTGTGCCTGCGCGCTTCCTACACACTCTAACTGTTTTTGCGTCGACAT
CACGG (SEQ ID NO:109)
 and 
ACGCAAAAACAGTTAGAGTGTGTAGGAAGCGCGCAGGCACAGCTTTTGCATGC
ACTATG (SEQ ID NO:110)
MM017 (2^{nd} loop) 
CAAAAGCTGTGCCTGCCCTTTAGGCCTTTGCCCACCTTGTTTTGCGTCGACAT
CACGG (SEQ ID NO:111)
 and 
ACGCAAAAACAAGGTGGGCAAAGGCCTAAAGGGCAGGCACAGCTTTTGCATGC
ACTATG (SEQ ID NO:112)
FGFps1 (2^{nd} loop) 
AAGCTGTATCTGCTGGAACATCGATTCTACACCTTGTTTTGCGTCGACATCACG
G (SEQ ID NO:113)
 and 
ACGCAAAAACAAGGTGTAGAATCGATGTTCCAGCAGATACAGCTTTTGCATGCA
CT (SEQ ID NO:114)
FGFps2 (1^{st} loop) 
GCGATCAATGCATCTGTACTTGGATTGACAGTACTCCTTGTCGGTGTTCCGATAT
GCGTC (SEQ ID NO:115)
 and 
GGAACACCGACAAGGAGTACTGTCAATCCAAGTACAGATGCATTGATCGCAACA
GGGTTT (SEQ ID NO:116)
FGFps2 (2^{nd} loop) 
AAGCTGTATCTGCACATGGATCGATAGTACTCCTTGTTTTTGCGTCGACATCACG
G (SEQ ID NO:117)
 and 
ACGCAAAAACAAGGTGTAGAATCGATCCATGTGCAGATACAGCTTTTGCATGCA
CT (SEQ ID NO:118)
FGFpsB (2^{nd} loop) 
AAGCTGTATCTGTACATGGATCGATTGGACACCTTGTTTTGCGTCGACATCAC
GG (SEQ ID NO:119)
 and 
ACGCAAAAACAAGGTGTCCAATCGATCCATGTACAGATACAGCTTTTGCATGCA
CT (SEQ ID NO:120)
1A8 (2^{nd} loop) 
CAAAAGCTGCGCATGTGTTACTACAGATTGGATCGAATGTTTTTGCGTCGACAT
CACGG (SEQ ID NO:121)
 and 
ACGCAAAAACATTCGATCCAATCTGTAGTAACACATGCGCAGCTTTTGCATGCA
CTATG (SEQ ID NO:122)
1A12 (2^{nd} loop) 
CAAAAGCTGTGCCTGCCCAACACTTTGGACTCATATGTGTTTTTGCGTCGACAT
CACGGAC (SEQ ID NO:123)
 and 
ACGCAAAAACACATATGAGTCCAAAGTGTTGGGCAGGCACAGCTTTTGCATGCA
CTATGAC (SEQ ID NO:124)
1 E11 (2^{nd} loop) 
CAAAAGCTGCGCATGTTACTACTCTCAATTCCACCAATGTTTTTGCGTCGACATC
ACGG (SEQ ID NO:125)
 and 
ACGCAAAAACATTGGTGGAATTGAGAGTAGTAACATGCGCAGCTTTTGCATGCA
CTATG (SEQ ID NO:126)
TGFps1 (2^{nd} loop) 
CAAAAGCTGTCTTTGTCCGGAAAACGATAACGTTTCTCCTTGTAATTGCGTCGAC
ATCACGGACTTCTG (SEQ ID NO:127)
 and

TGTCGACGCAATTACAAGGAGAAACGTTATCGTTTTCCGGACAAAGACAGCTTT
TGCATGCACTATGAC (SEQ ID NO:128)

The DNA sequences of the oligonucleotide pair used to make the cassette to introduce the MM021 peptide into the chymotrypsin reactive site loops of the p2JM103-Ink2-BBI expression vector are provided below. The cassette was ligated into the *Sph*I and *Sal*I restriction sites in the vector.
MM021 (2^{nd} loop)
CAAAAGCTGTGCTTGTAAACACAACGTACGTCTTTTATGTTTTTGCG (SEQ ID NO:129)
and
TCGACGCAAAAACATAAAAGACGTACGTTGTGTTTACAAGCACAGCTTTTGCATG (SEQ ID NO:130)

Libraries made of cysteine constrained peptides are popular reagents (*e.g.* the commercially available PhD-C7C Phage Display Peptide Library Kit; NEB) for selecting peptides that bind to substrates of interest. BBI has two cysteine constrained reactive site loops that are structurally similar to the peptide loops displayed in various methods used to select peptide binders. So, once a cysteine constrained binding peptide has been selected, BBI is suitable for use as a scaffold to present the peptide in a binding reaction.

The VEGF binding peptide CK37281 (*See e.g.,* co-pending U.S. Provisional Patent Application Ser. No. 60/ 520,403, filed November 13, 2003) was grafted into BBI by replacing the trypsin, chymotrypsin, or both reactive site loops, with the CK37281 peptide sequence (ACYNLYGWTC)(SEQ ID NO:9) by using DNA oligonucleotide cassettes. To facilitate the construction, an *Eco*RI site was introduced in the coding region of the BBI gene (custom synthesized by Operon Technologies; *See*, Example 1) between the trypsin and chymotrypsin reactive site loops by QuikChange® site-directed mutagenesis, using methods described by the manufacturer (Stratagene) using the primers BowBeco-F and BowBeco-R, shown above (0.5 pmol of each primer was used in the QuikChange® reaction; after an initial denaturation step of 97°C for 3 minutes, 18 PCR cycles of 68°C for 12 minutes, 95°C for 30 seconds and 55°C for one minute, followed by a final extension reaction for 15 minutes at 68°C).

To replace the trypsin inhibitory peptide loop, two DNA oligonucleotides (IBBCK81+ and 1 BBCk81-) were annealed and ligated into the *Sac*I and *Eco*RI restriction sites. Likewise, to replace the chymotrypsin inhibitory peptide loop, *Eco*RI and *Sal*I sites were used for insertion of a DNA cassette made by annealing the oligonucleotides (2BBck81+ and 2BBck81-). The CK37281 peptide was grafted into both loops by inserting the CK37281 peptide in the chymotrypsin loop (using the oligonucleotides (2BBck81 + and 2BBck81-) after the trypsin loop was first replaced by the CK37281 peptide. BBI with the CK37281 peptide in the trypsin loop (1BBIck81) was moved into the pJM103BBI expression vector as a *Sac*I*-Sph*I fragment. BBI with the CK37281 in the chymotrypsin loop (2BBIck81), or both loops (12BBIck81), was moved into pJM103BBI as *Sac*I-*S*alI fragments. The correct sequences were verified by DNA sequencing (the sequence of 12BBIck81 gene is shown in Figure 3). The resulting vectors, pJM103-1BBIck81, pJM103-2BBIck81, or pJM103-12BBIck81, were used to transform *B. subtilis* BG3934comK, and the production of the BCE fusion proteins was determined as in Example 1 above.

The fusion protein running at ∼ 44 kDa was detected by SDS-PAGE to be the major protein present in the cell free broth. Although in some cases, there was significant degradation (up to 50 %) of the BBI moiety (especially after > 48 h of growth in MBD medium), as observed by the presence of a prominent protein band running at ∼ 34 kDa corresponding to the BCE103 catalytic core. In these cases, the titers of the BCE103 cellulase were similar to that measured with fusions to the wild-type BBI (Example 1), but the activity of the BBI (trypsin inhibition with 2BBIck81, or chymotrypsin inhibition with 1BBIck81) was generally about two fold less.

To reduce the proteolytic degradation of BBI variants during growth (*i.e.* decrease the amount of BCE103 cellulase core present on SDS-PAGE gels in comparison to the fusion protein), a *Bacillus subtilis* strain with nine protease genes deleted, BG6006 (*degU^{Hy}32*, *oppA*, Δ*spoIIE3501*, Δ*prE*, Δ*nprE*, Δ*epr*, Δ*ispA*, Δ*bpr*, Δ*vpr* Δ*wprA*, Δ*mpr-ybjF*, Δ*nprB*, *amyE::xyIRPxylAcomK-ermC*), was used as an expression host, and the growth temperature (35°C) and aeration (200 rpm) were reduced. With these changes, a major fusion protein band (∼ 44 kDa) was observed on SDS-PAGE gels with an insignificant band present at the molecular weight expected for the BCE catalytic core protein (∼ 34 kDa).

In addition to the CK37281 peptide, a number of other cysteine constrained peptides were produced when substituted into the trypsin and/or chymotrypsin reactive site loops of BBI fused to the C-terminus of the BCE103 cellulase. Specific examples included:
(1) Peptides designed or selected as complement antagonists, compstatin introduced into the 1^{st} or 2^{nd} reactive site loops (*See,* Sahu et al., J. Immunol., 157: 884-891, [1996]), C2c (1^{st} loop), C3c (1^{st} loop), C4c (1^{st} loop) and C5c (1^{st} loop); or peptides selected in a Factor B binding reaction 1B, 2B, 4A, 5A, 6-1A, 7A, 8B, 9A, 10B, 11-1A, 12B, 13A, and 15-1A (all in 2^{nd} loop);
(2) Peptides designed to bind to the proteases Factor Xa or stratum corenum chymotrypsin, Xa1 (2^{nd} loop) or hSCC1 (1^{st} loop), respectively;
(3) Peptides selected in FGF5 binding reactions 1A6 (1^{st} or 2nd loop), 1C2 (1^{st} or 2nd loop), 2E2 (1^{st} or 2nd loop), 2E5 (1^{st}, 2^{nd} or both loops), FGFns (1^{st} or 2^{nd} loop), FGFkr (1^{st} or 2^{nd} loop), FGFhl (1^{st} or 2^{nd} loop), FGFgy (1^{st} or 2^{nd} loop), MM005 (1^{st} or 2^{nd} loop), MM007 (1^{st}, 2^{nd} or both loops), MM009 (2^{nd} loop), MM010 (1^{st}, 2^{nd} or both loops), MM017 (2^{nd} loop), FGFps1 (2^{nd} loop), FGFps2 (1^{st}, 2^{nd} or both loops), and FGFpsB (2^{nd} loop); and
(4) Peptides selected in TGFβ-1 binding reactions 1A8 (2^{nd} loop), 1A12 (2^{nd} loop), 1 E11 (2^{nd} loop), TGFps1 (2^{nd} loop), and MM021 (2^{nd} loop).

The oligonucleotides used to introduce these peptides into either the trypsin (1^{st} loop) or chymotrypsin (2^{nd} loop) reactive site loops, and methods used to graft these peptides into BBI, are provided above. In all cases, fusion proteins were produced as determined by SDS-PAGE gels. However, with some substituted peptides, the amount of intact fusion protein was increased by reducing the proteolytic degradation as described above for the CK37281 substituted peptide.

### EXAMPLE 3

### Activation of BBI By Thiol Reducing/Oxidizing Agents

After growth, the activity of the BBI (by trysin or chymotrypsin inhibition) is typically some 5-20 times lower than what would be expected from the activity of the BCE103 cellulase measured in the cell free supernatants (the two molecules should be present at a 1:1 molar ratio in the fusion protein). An increase in the activity of BBI (measured by either trypsin or chymotrypsin inhibition) in the BCE103-BBI fusion protein can be routinely obtained by adding βME, typically concentrations of 1 - 4 mM added to the MBD growth medium about 14 h after inoculation. The trypsin or chymotrypsin inhibitory activity of BBI in the fusion protein is also lower than expected when binding peptides (*e.g.* VegF binding peptide CK37281) replace the chymotrypsin or trypsin reactive site loop, respectively. As with the wild-type BBI, the inhibitory activity can be increased by treatment with βME. Unexpectedly, other thiol reducing agents (*e.g*.,cysteine, reduced glutathione, DL-dithiothreitol and Tris[2-carboxyethyl] phosphine) had small or negligible effects on the activation of BBI during growth in these experiments. Also, additions of antioxidants (*e.g.,* ascorbic acid or DL-α-tocopherol acetate) or other adjuvants to the growth medium (*e.g.,* isoleucine, soybean oil, Tween-80), or growth at 30°C did not significantly improve the BCE103:BBI activity ratio.

Specifically, to determine the BBI activation during growth, cultures of *B. subtilis* BG6006 transformed with p2JM103-E3-2BBIck81 (See, Example 4, below) were grown in 40 ml MBD medium in 250 ml shake flasks at 37°C for 13 h. Then, the thiol reducing agents indicated on the graph in Figure 4 were added and cell supernatants harvested after 62 h of growth. The reagents 2-mercaptoethanol (BME), cysteine (Cys), reduced glutathione (Glut), and DL-dithiothreitol (DTT) were added to the growth medium to the final concentrations indicated on the graph provided in Figure 4. Concentrations of 5 mM ME can result in better BCE103:BBI activity ratios but typically result in an overall decrease in both BCE103 and BBI titers (see Figure 4), at least partially due to the reduction in bacterial growth caused by the added reagent. Titers of BCE103 and 2BBIck81 were determined using the assays described in Example 1.

BBI activation was also achieved after partial purification of the fusion proteins (*e.g.* BCE-Ink2-2BBIck81, see Example 4 below) by Q-Sepharose ion exchange chromatography.

The fusion protein was purified from cell free broth obtained from shake flasks or fermentor runs. The broth was filtered, diluted five to ten fold in water and the pH adjusted to pH 7.5 - 8.0. The diluted sample was loaded onto a column packed with Q-Sepharose resin (GE Healthcare). The column was washed with 50 mM Tris pH 7.5 and then washed again in the same buffer containing 300 mM NaCl. The fusion protein was eluted in the same buffer with 700 mM NaCl.

To activate the BBI, the pooled fusion protein fractions were diluted ten fold in Assay Buffer then treated with 2 mM βME and 0.2 mM oxidized glutathione (GSSG) with constant mixing on a stir plate or rocker platform for about 24 h at room temperature. The BBI could generally be activated to about 70-100 % of the expected trypsin inhibitory activity based on the measured concentration of the BCE103 cellulase. Although the activation method outlined above generally yielded the best results, in some cases, in order to maximize the activation of a given sample, screens were performed in 96-well plates to determine the optimal conditions. Initially, the typical conditions screened were the dilution in Assay Buffer (*e.g.,* a 2-50 fold dilution series), βME concentration (*e.g.,* series between 0.5-5 mM) and oxidized glutathione concentration (*e.g.* 0 mM then a series of 1/20 to 1/2 the ME concentration).

The activation of the fusion protein BCE-Ink2-2BBIck81 is shown in Figure 5. In this specific example, the fusion protein from a Q-Sepharose purification was diluted 1:10 in Dulbecco's PBS (Mediatech) with 0.005 % TWEEN®-80. Beta-mercaptoethanol was added to a final concentration of 3 mM and incubated overnight at room temperature on a rocker. The sample was further incubated at room temperature for about 60 h with vigorous stirring on a magnetic stir plate. The titers of the BCE103 and 2BBIck81 (before and after βME treatment) were determined by cellulase assays and trypsin inhibitory assays, respectively.

In some embodiments, such as for activating BBI or it variants in cell free broth from large volume fermentations, it is desirable to reduce the dilution and βME concentration in the activation reaction. This can be accomplished by using higher concentrations of buffer (500 mM Tris pH 8.6), or changing to zwitterionic buffers such as CHES (also CAPS, Tricine, TAPS, and other suitable zwitterionic buffers). For example, cell free broth (or fusion protein fractions purified by ion exchange chromatography) was diluted 1:1 in 375 mM CHES pH 8.6 with 0.005 % TWEEN®-80 then activated with 1 mM βME and 10 mM Na₂SO₃ and incubated with stirring at room temperature for about 24 h. BBI or its variants, as BCE103 cellulase fusion proteins, were routinely activated by this method to 70-100 % of the expected value (based on BCE103 cellulase activities).

### EXAMPLE 4

### Release of Free BBI/Variants by Cleavage of the BCE103-BBI Fusion Proteins

This Example describes experiments developed to release free BBI or its variants by cleavage of the BCE103-BBI fusion proteins.

The sequences of the DNA oligonucleotide pairs that were annealed and ligated into the *Bam*HI and *Sac*I sites of pJM103-BBI to generate potential cleavage sites during culture growth between the BCE103 catalytic domain and BBI are provided below.
BCEsubBBI (a subtilisin-type sensitive peptide sequence)
GATCCAGGTGGAGCTGCTTTAGTTGACGATGAGAGCT (SEQ ID NO:131)
and
CTCATCGTCAACTAAAGCAGCTCCACCTG (SEQ ID NO:132)
BCEcbdLBBI (a portion of the 1^{st} CBD) 
GATCCAGGTGAACCTGACCCAACTCCTCCATCTGATCCTGGAGAATACCCAGCT
TGGGACGATGAGAGCT (SEQ ID NO:133)
 and 
CTCATCGTCCCAAGCTGGGTATTCTCCAGGATCAGATGGAGGAGTTGGGTCAG
GTTCACCTG (SEQ ID NO:134)
 BCEproBBI (the entire pro peptide of BBI) 
GATCCGGCGAACCTGCGTCTGTCTAAGCTTGGCCTGCTTATGAAATCAGACCAT
CAGCACAGCAATGACGATGAGAGCT (SEQ ID NO:135)
 and 
CTCATCGTCATTGCTGTGCTGATGGTCTGATTTCATAAGCAGGCCAAGCTTAGA
CAGACGCAGGTTCGCCG (SEQ ID NO:136)
 BCEshortproBBI (a C-terminal portion of the pro peptide of BBI)
GATCCAAAATCAGACCATCAGCACAGCAATGACGATGAGAGCT (SEQ ID NO:137)
and
CTCATCGTCATTGCTGTGCTGATGGTCTGATTTTG (SEQ ID NO:138)

The sequences of the DNA oligonucleotide pair that was annealed and ligated into the *Bam*HI and *Sac*I sites of p2JM103-BBI to fuse BBI to the 2^{nd} CBD linker of BCE103 cellulase are provided below.
BCEcbdDBBI 
GATCCAGGAGAACCGGACCCAACGCCCCCAAGTGATCCAGGAGAGTATCCAGC
ATGGGATTCAAATCAAATTTACACAAATGAAATTGTGTATCATAACGGTCAGTTAT
GGCAAGCGAAATGGTGGACACAAAATCAAGAGCCAGGTGACCCATACGGTCCG
TGGGAACCACTCAAATCTGACCCAGATTCAGACGATGAGAGCT (SEQ ID
NO:139)
 and

CTCATCGTCTGAATCTGGGTCAGATTTGAGTGGTTCCCACGGACCGTATGGGTC
ACCTGGCTCTTGATTTTGTGTCCACCATTTCGCTTGCCATAACTGACCGTTATGA
TACACAATTTCATTTGTGTAAATTTGATTTGAATCCCATGCTGGATACTCTCCTGG
ATCACTTGGGGGCGTTGGGTCCGGTTCTCCTG (SEQ ID NO:140)

The peptide sequences susceptible to acid cleavage between aspartic acid and proline residues are provided below.
Linker 1 - WGDPHY (SEQ ID NO:141)(Lidell et al., J. Biol. Chem. 278:13944-51 [2003])
Linker 2 - DNNDPI (SEQ ID NO:142)(Segalas et al., FEBS Lett., 371:171-175 [1995])
Linker 3 - WADPN (SEQ ID NO:143)(Kemperman et al., Appl. Env. Microbiol., 69: 1589-1597 [2003])

Oligonucleotide primers used to introduce a *Bss*HII site into pJM103BBI by QuikChange® site-directed mutagenesis are provided below.
BCEbss-F
5'-TGGCGTTCAGCAACATGAGCGCGCAGGCTGATGATTA (SEQ ID NO:144)
BCEbss-R
5'-TAATCATCAGCCTGCGCGCTCATGTTGCTGAACGCCA (SEQ ID NO:145)

Sequences of the DNA oligonucleotides that were annealed as a cassette (*Sal*I*-Hin*dIII) to introduce *Hin*dIII and *Xho*I sites after the stop codon of BBI, to introduce a *Pac*I site after the LAT, and remove the original *Hin*dIII site are provided below.

BCEterm+
5'-GACATCACGGACTTCTGCTATGAGCCATGTAAACCAAGCGAGGACGATAA
AGAGAACTAAAAGCTTAACTCGAGGTTAACAGAGGACGGATTTCCTGAAGGAAA
TCCGTTTTTTTATTTTTAATTAAG (SEQ ID NO:146)

BCEterm-
5'-AGCTCTTAATTAAAAATAAAAAAACGGATTTCCTTCAGGAAATCCGTCCTC
TGTTAACCTCGAGTTAAGCTTTTAGTTCTCTTTATCGTCCTCGCTTGGTTTACATG
GCTCATAGCAGAAGTCCGTGATG (SEQ ID NO:147)

PCR primers used to generate the acid labile linkers provided above (*i.e*., Linker 1, Linker 2, and Linker 3) inserted between the BCE103 catalytic domain and BBI are provided below.
BCE103coreBssHII_FW
5'-CAGCAACATGAGCGCGCAGGCTG (SEQ ID NO:148)
linkerWGDPHY_RV 
5'-ATCGTCTGGATCCGGATAGTGGGGGTCTCCCCAAGATGCTGATTCTCTTAT
TTTTTCCC (SEQ ID NO:149)
 linkerDNNDPI_RV 
5'-ATCGTCTGGATCCGGTATGGGATCATTGTTGTCAGATGCTGATTCTCTTATT
TTTTCCC (SEQ ID NO:150)
 linkerVVADPN_RV

5'-ATCGTCTGGATCCGGGTTGGGATCTGCAACTACAGATGCTGATTCTCTTAT
TTTTTCCC (SEQ ID NO:151)

PCR primers used to generate the acid labile linkers provided above (*i.e.,* Linker 1, Linker 2, and Linker 3) inserted into the 1^{st} CBD linker.

BCE103corePstI_FW
GCATAAGGAT GAGTCATCTG CAGCG (SEQ ID NO:152)
LplusWGDPHY_RV 
5'-ATCGTCTGGATCCGGATAGTGGGGGTCTCCCCACGGTTCTCCTGGATCAGA
TGGCGG (SEQ ID NO:153)
 LplusDNNDPI_RV 
5'-ATCGTCTGGATCCGGTATGGGATCATTGTTGTCCGGTTCTCCTGGATCAGA
TGGCGG (SEQ ID NO:154)
 LplusVVADPN_RV

5'-ATCGTCTGGATCCGGGTTGGGATCTGCAACTACCGGTTCTCCTGGATCAGA
TGGCGG (SEQ ID NO:155)

Protein sequence of the acid labile linkers inserted between the BCE103 catalytic domain and BBI are provided below. The acid labile linkers are shown in bold type and the sequences from the first CBD domain are underlined.
Linker 1
BCE-**WGDPHY**-PDP-BBI (SEQ ID NO: 156)
Linker 2
BCE-**DNNDPI**-PDP-BBI (SEQ ID NO:157)
Linker 3
BCE-**VVADPN**-PDP-BBI (SEQ ID NO:158)
LinkerPlus 1
BCE-IPPSDPTPPSDPGEP-**WGDPHY**-PDP-BBI (SEQ ID NO:159)
LinkerPlus 2
BCE-IPPSDPTPPSDPGEP-**DNNDPI**-PDP-BBI (SEQ ID NO:160)
LinkerPlus 3
BCE-IPPSDPTPPSDPGEP-**VVADPN**-PDP-BBI (SEQ ID NO:161)

The sequences of the DNA oligonucleotide pairs that were annealed and ligated into the *Bam*HI and *Sac*I sites of pJM103-BBI to generate potential cleavage sites between the BCE103 catalytic domain and BBI during the purification process are provided, below.
BCEentBBI (Enteropeptidase cleaveage site)
GATCCAGGTGGAGACGACGATGACAAAGACGATGAGAGCT (SEQ ID NO:162)
and
CTCATCGTCTTTGTCATCGTCGTCTCCACCTG (SEQ ID NO:163)
BCEgenen1BBI (Genenase I cleavage site)
GATCCAGGTGCTGCTCATTACGACGATGAGAGCT (SEQ ID NO:164)
and
CTCATCGTCGTAATGAGCAGCACCTG (SEQ ID NO:165)

The sequences of the DNA oligonucleotide pairs that were annealed and ligated into the *Bam*HI and *Sac*I sites of pJM103-Ink2-1BBIck81 to generate potential cleavage sites between the BCE103 catalytic domain and BBI during the purification process are provided below.
BCEfurinBBI (Furin/Blisterase cleavage site)
GATCCACGTGCTAAAAGAGACGATGAGAGCT (SEQ ID NO:166)
and
CTCATCGTCTCTTTTAGCACGTG (SEQ ID NO:167)
BCEgenen2BBI (Genenase I cleavage site)
GATCCAGGCGCTGCACACTACAACGACGATGAGAGCT (SEQ ID NO:168)
and
CTCATCGTCGTTGTAGTGTGCAGCGCCTG (SEQ ID NO:169)
BCEfleBBI (Mpr cleavage site)
GATCCATTCCTTGAAGACGATGAGAGCT (SEQ ID NO:170)
and
CTCATCGTCTTCAAGGAATG (SEQ ID NO:171)

Sequences of the oligonucleotide primer pairs used to introduce the E and E3 linkers in Linker 2 by QuikChange site-directed mutagenensis (Stratagene) are provided below.
BCE-Elnk-BBI (Mpr cleavage site)
CCCATACCGGAGCCAGACGATGAGAGCTC (SEQ ID NO:172)
and
CATCGTCTGGCTCCGGTATGGGATCATTGTTG (SEQ ID NO:173)

The protein sequence of the E3 linker between the BCE103 catalytic domain and BBI was DNNDPIPEP**DDESFNMPIPEP** (SEQ ID NO:174). In this sequence, the E Linker is underlined and the sequence generated by faulty recombination in *E. coli* is shown in bold type. Cleavage by Mpr (or V8 protease) can occur after any of the three glutamic acids present in the E3 Linker. Thus, the structure was BCE-(SEQ ID NO:174) -BBI

The sequences of the DNA oligonucleotide pairs that were annealed and ligated into the *Bam*HI and *Sac*I sites of p2JM103-Ink2-2BBIck81 to generate potential Genenase I cleavage sites between the BCE103 catalytic domain and BBI are provided below.
BCEgenen3BBI 
GATCCAGGCGCTGCACACTACAAATCAGACCATCAGCACAGCAATGACGATGA
GAGCT (SEQ ID NO:175)
 and
CTCATCGTCATTGCTGTGCTGATGGTCTGATTTGTAGTGTGCAGCGCCTG (SEQ ID NO:176)
BCEgenen4BBI
GATCCAGGCGCTGCACACTACGTAGAATTTCAAGACGATGAGAGCT (SEQ ID NO:177)
and
CTCATCGTCTTGAAATTCTACGTAGTGTGCAGCGCCTG (SEQ ID NO:178)

The protein sequence of a Genenase I sensitive cleavage site (also acid and Mpr sensitive) inserted between the BCE103 catalytic domain and BBI was DNNDPIPDP**GAAHYVEFQ** (SEQ ID NO:179). The Genenase I site (Gen4 Linker) is in bold type (cleavage occurs between the tyrosine and valine) (NEB) and Linker 2 is underlined. Cleavage by Mpr can also occur after the glutamic acid that follows the valine in the Gen4 linker. The sequence used herein was BCE-SEQ ID NO:179)-BBI

Cleavage sites in the BCE103-Ink2-2BBIck81 fusion protein are indicated below. The C-terminal seven amino acids of the BCE103 catalytic domain (underlined), linker 2 sequence (bold type), and 2BBIck81 sequences are shown. The acid/heat labile Asp-Pro bonds are indicated with solid headed arrows and the Mpr sensitive bonds after glutamic acids are indicated with line headed arrows.

...KIRESASDNNDPIPDPDDESSKPCCDQCACTKSNPPQCRCSDMRLNSCHSACK
SCACYNLYGWTCFCVDITDFCYEPCKPSEDDKEN (SEQ ID NO:180)

In order to isolate free BBI or its variants, the BBI moiety needs to be cleaved from the BCE103-BBI fusion protein. In some embodiments, this is accomplished during growth, by proteases intrinsically produced by *B. subtilis.* In some alternative embodiments, this cleavage occurs after growth, during the purification process (*e.g.* by acid/heat or proteolytic cleavage). Linkers potentially susceptible to cleavage during growth were designed (*See,* above, sub, cbdL, pro, shortpro, and cbdD) and cloned into the pJM103BBI or p2JM103BBI expression vectors as *Bam*HI*-Sac*I cassettes. The production of fusion protein versus BCE103 catalytic domain was analyzed on SDS-PAGE gels as described in Example 1.

Little cleavage of the fusion protein was observed for all these linkers except with the pro linker, which was nearly completely cleaved so that very little intact fusion protein was observed on gels, although there was a large band corresponding to the BCE103 catalytic core. Unfortunately, this cleavage during growth resulted in negligible BBI activity measured in cell free supernatants and no BBI band could be identified on SDS-PAGE gels. Although it is not intended that the present invention be limited to any particular mechanism or theory, it is possible that the BBI is particularly sensitive to proteolytic degradation in its inactive form. Thus, cleavage during the purification process after activation is generally preferred.

In some embodiments, the bonds between aspartic acid and proline residues are cleaved by heat treatment at acidic pH as known in the art (*See e.g.,* Landon, Meth. Enzymol., 47:145-149 [1977]). The 1^{st} CBD linker in the BCE103 cellulase has three Asp-Pro dipeptide sequences *(See,* Figure 1) with the potential to be cleaved by acid/heat treatment. However, cleavage by acid/heat treatment at these sites was found to be inefficient. Protein sequences that are especially labile to acid/heat have been described in the literature, three of such sequences are WGDPHY (SEQ ID NO:141), DNNDPI (SEQ ID NO:142), and WADPN (SEQ ID NO:143)(*i.e*., Linkers 1, 2 and 3).

Before these acid labile linkers were introduced into the BCE103-BBI expression vector, pJM103BBI, a *Bss*HII site was introduced by QuikChange® XL (Stratagene) mutagenesis (using the manufacturer's methods; and described in Example 2 above, except 8 minute extension and 1 minute denaturation steps were used) in the *aprE* signal sequence coding region using the oligonucleotide primers BCEbss-F and BCEbss-R (provided above). Then, *Hin*dIII and *Xho*I sites were inserted in front of the LAT terminator (after the BBI stop codon) and a Pa*c*I site was added after the terminator (the original *Hind*III site after the LAT terminator was removed) by inserting an oligonucleotide cassette (BCEterm+ and BCEterm-; provided above) into the *Sal*I and the original *Hin*dIII sites. This new vector was called "p2JM103BBI."

The acid labile linker fragments were generated by PCR, using forward primer BCE103coreBssHII_FW with each of the reverse primers, linker WGDPHY_R, linker DNNDPI_RV, or linkerVVADPN_RV (the sequences of which are all provided above) and p2JM103BBI as the template (see Example 1 for the PCR protocol). The PCR fragments of 970 bp were digested with *Bam*HI and *Pst*I*,* the 154 bp fragments encoding the acid linker fragments were isolated from an agarose gel after electrophoresis, and ligated into the p2JM103 vector digested with *Bam*HI and *Pst*I that had also been purified from a gel. The linker sequences in the final expression vectors, p2JM1031nk1-BBI, p2JM103Ink2-BBI and p2JM103Ink3-BBI, were verified by DNA sequencing.

Competent *B. subtilis* strain BG3934comK or BG6006 were transformed with the plasmids, colonies selected on 5 µg/ml chloramphenicol LA plates and amplified to 25µg/ml chloramphenicol as described in Example 1.

Similarly, the acid labile linkers were inserted into the first CBD linker. Specifically, PCR fragments were generated using the forward primer BCE103corePstI_FW with the reverse primers LplusWGDPHY_RV, LplusDNNDPI_RV, or LplusVVADPN_RV (*See above,* for the sequences) with p2JM103BBI as a template. The PCR fragments of about 150 bp were digested with *Bam*HI and *Pst*I, purified and ligated to the p2JM103BBI vector digested with *Bam*HI and *Pst*I. The correct sequences were verified by DNA sequencing and the plasmids p2JM103pIInk1-BBI, p2JM103pIInk2-BBI and p2JM103piInk3-BBI were used to transform *B. subtilis* strains as described above.

After growth in MBD medium, the fusion proteins were purified by ion exchange chromatography essentially as described above (*See*, Example 2). The fusion protein was cleaved by treatment at 55°C for 16 h in 10% formic acid. The BCE103 catalytic domain precipitated during the acid treatment and was removed by centrifugation. The free BBI in the supernatant was dried overnight on a SpeedVac. The sample was suspended in 50 mM Tris pH 8 before loading on the SDS-PAGE gel. By analysis of the protein stained SDS-PAGE gels, it was observed that acid cleavage was much more efficient in the fusion proteins where Linker 2 was inserted between the BCE103 catalytic domain and BBI (BCE-DNNDPI-PDP-BBI). This linker was found to be cleaved in a couple of hours at 75°C in 20 mM glycine pH 2.

In alternative embodiments, the fusion protein was cleaved by treatment with a protease during the purification process. Linkers were designed with cleavage sites for glutamic acid specific proteases (*e.g*., Mpr or V8 protease), Furin/blisterase, Genenase I, and Enteropeptidase (Enterokinase). These linkers were introduced as oligonucleotide cassettes (*See above,* for the sequences) between the BCE103 catalytic core and BBI in the expression vector using the *Bam*HI and *Sac*I sites (*See*, Figure 1). In the coding region of the original expression vector (pJM103BBI), there is a glutamic acid residue in the 1^{st} CBD domain and at the third residue in BBI *(See,* Figure 1), which is contemplated to be susceptible to cleavage by glutamic acid specific proteases such as *B. subtilis* Mpr (BsMpr) or V8 protease. However, neither BsMpr nor V8 protease were found to cleave the BCE-BBI fusion protein very efficiently at these sites. Thus, it was necessary to design other linkers that were susceptible to cleavage by these proteases.

The six acid labile linkers described above were tested for cleavage by BsMpr. These fusion proteins were cleaved by treatment for 16 h with 16 µg of BsMpr at room temperature. After cleavage, the BCE103 catalytic domain was precipitated by the addition of 10 % formic acid and removed by centrifugation. The free BBI in the supernatant was dried overnight on a SpeedVac. The sample was suspended in 50 mM Tris pH 8, before loading on the SDS-PAGE. Similar to the acid cleavage, the BCE-DNNDPI-PDP-BBI (Linker 2) fusion protein was much more efficiently cleaved by BsMpr than any of the other linkers. Therefore, BBI and its variants were found to be effectively released from the BCE-DNNDPI-PDP-BBI fusion protein either by acid/heat treatment or proteolytic digestion with a glutamic acid specific protease such as BsMpr. Several other linkers designed for cleaved by Mpr (*e.g.,* E, E3 linker, and fle, provided above) were tested but none of them had any advantages over Linker 2 (the E3 linker was generated by faulty recombination in *E. coli* after transformation with the QuikChange® site-directed mutagensis reaction designed to construct the E linker). As shown above, there are two acid/heat labile cleavage sites in Linker 2 and three sites sensitive to cleavage by Mpr.

Linkers designed for cleavage by Furin or Blisterase (NEB) (BCEfurinBBI), or Enteropeptidase (Enterokinase, NEB) (BCEentBBI) were tested, but none of these sequences were cleaved efficiently by the appropriate protease. Four linkers were also designed (BCEgenen1BBI, BCEgenen2BBI, BCEgenen3BBI, and BCEgenen4BBI) and tested for cleavage by Genenase I (NEB). Efficient cleavage of the fusion protein was observed only with the Gen4 Linker (BCEgenen4BBI). BsMpr was also found to efficiently cleave the Gen4 linker.

After activation of the purified BCE-Ink2-2BBlck81 fusion protein, cleavage by BsMpr does not go to completion as judged by SDS-PAGE gels. However, it was discovered that complete cleavage after activation of BCE-BBI fusion proteins with Linker 2 (or the Gen4 linker) can be accomplished by using the Mpr protease isolated from *Bacillus licheniformis* (BIMpr). While it is not intended that the present invention be limited to any particular mechanism, cleavage after the third amino acid in mature BBI appeared to be more sensitive to BIMpr while cleavage after the sixth amino acid from the C-terminus of BBI is more sensitive to BsMpr cleavage.

In some embodiments, after cleavage, the BBI is purified away from the BCE103 catalytic domain by selective acid precipitation (pH 3 or lower) of the BCE103 catalytic domain as described above, ion exchange chromatography (*See,* Example 5), or by selective binding of BBI on an anhydrotrypsin-agarose (Sigma) column loaded in 50 mM Tris pH 8.0, washed with 50 mM Tris pH 8.0 with 150 mM NaCl, then eluting bound BBl with 50 mM glycine pH 2.2 with 300 mM NaCl).

### EXAMPLE 5

### Binding of BBlck81 to VegF

In this Example, experiments conducted to assess the binding of BBlck81 to VegF are described. The BCE103-Ink2-2BBlck81 fusion protein was produced in *B*. *subtilis* as described in Example 2. The fusion protein was purified, and the BBI trypsin inhibitory activity was increased by treatment with βME and oxidized glutathione as described in Example 3. The fusion protein was cleaved by BsMpr protease (*See,* Example 4) and the free 2BBlck81 was purified from the BCE103 catalytic domain by ion exchange chromatography using a Q-Sepharose column.

Briefly, after cleavage, the pH of the cleaved sample was adjusted to 5.5, the sample was then loaded onto the column (equilibrated with 25 mM MES pH 5.5). The free 2BBlck81 was washed through the column using 25 mM sodium acetate pH 5.0 while the BCE103 catalytic core remained bound to the resin. The 2BBlck81 fraction was concentrated by ultrafiltration and analyzed using an electrochemiluminescence (ECL) based binding assay (BioVeris). The Anti-VegF antibody (Santa Cruz) and VegF (PeproTech) were labeled with the electrochemiluminescent dye and biotin, respectively, as described by the manufacturer (BioVeris). All materials were in Dulbecco's PBS (Mediatech) supplemented with 0.1 % TWEEN®-80. An initial dilution series of Anti-VegF antibody (125, 250 and 500 ng/ml) and VegF (100, 150, 200 and 250 ng/ml) were tested in the binding assay to determine the concentrations of each that would give a robust ECL signal.

For testing 2BBlck81 binding, 50 µL of 500 ng/ml ECL labeled Anti-VegF antibody, 50 µL of 250 ng/ml biotinylated VegF and 100 µL 2BBlck81 (series of 12.5, 15, 31.25, 62.5, 125, 250 or 500 ng/ml) were incubated at room temperature for 2 h with shaking. Then, 50 µL of 0.2 mg/ml streptavidin coated beads were added and the reaction was incubated at room temperature for 30 minutes. The ECL signal was measured using a BioVeris M8/384 Analyzer as described by the manufacturer (BioVeris). As shown in Figure 6, the ECL signal decreased as increasing concentrations of 2BBlck81 displaced more of the labeled Anti-VegF antibody bound to VegF attached to the magnetic beads.

Thus, the CK37281 peptide when grafted onto the chymotrypsin inhibitory loop of BBI (2BBlck81) competed with the Anti-VegF antibody for binding to VegF at micromolar concentrations. In fact, 2BBlck81 competed for VegF binding better than the synthesized CK37281 peptide itself (*See*, Figure 6). The CK37281 peptide inserted into the trypsin inhibitory loop, 1BBlck81, also competed with the Anti-VegF antibody in the BioVeris assay. Thus, BBl was found to be useful as a scaffold to present active binding peptides selected by various screening methods.

### EXAMPLE 6

### Use of Alternative Fusion Partners for the Production of 2BBlck81

In this Example, experiments conducted to evaluate alternative fusion partners are described. The DNA sequence of the oligonucleotide primers used to amplify the *dsbC* gene (*E. coli*) from pET-40b(+) are provided below. These primers generate a *Bss*HII site at the 5' end and a *Bam*HI at the 3' end for cloning into p2JM103-Gen4-2BBlck81.
DsbCBBI-F
AACATGAGCGCGCAGGCTGATGACGCGGCAATTCAACAAACGTTAG (SEQ ID NO:181)
DsbCBBI-R

TCGTCTGGATCCGGTATGGGATCATTGTTGTCACCAGAACCACTAGTTGATCCT
TTACCGCTGGTCATTTTTTGGTG (SEQ ID NO:182)

The DNA sequences of the oligonucleotides that were annealed together to make a cassette (*Alw*44I*-Bam*HI) for fusing the *P. mendocina* cutinase gene to BBI with Linker 2, are provided below.
CutinaseBBI+ 
TGCACTTCTCTGCTTTGGTCTGTTGAACGCAGAGGTCTTGACAACAATGATCCTA
TTCCG (SEQ ID NO:183)
 CutinaseBBI-

GATCCGGAATAGGATCATTGTTGTCAAGACCTCTGCGTTCAACAGACCAAAGCA
GAGAAG (SEQ ID NO:184)

Because the BBI moiety has seven disulfide bonds, it is contemplated that higher titers of active BBI will be obtained using fusion proteins other than the BCE103 cellulase catalytic domain. For example, in some embodiments, compositions such as thiol-disulfide oxidoreductases and/or protein disulfide isomerases find use as fusion proteins to help produce correctly folded BBI moieties. In this embodiment, no additional activation step is needed under most circumstances. In additional embodiments, other proteins produced at high titers in *B. subtilis* also find use as fusion partners. For example, the thermostable protein disulfide isomerase from the fungus *Humicola insolens* (hiPDl) has been used as a fusion partner to produce the light chain of immunoglobulin G (2 disulfides) in *Bacillus brevis* (*See,* Kajino et al., Appl. Env. Microbiol., 66:638-642 [2000]).

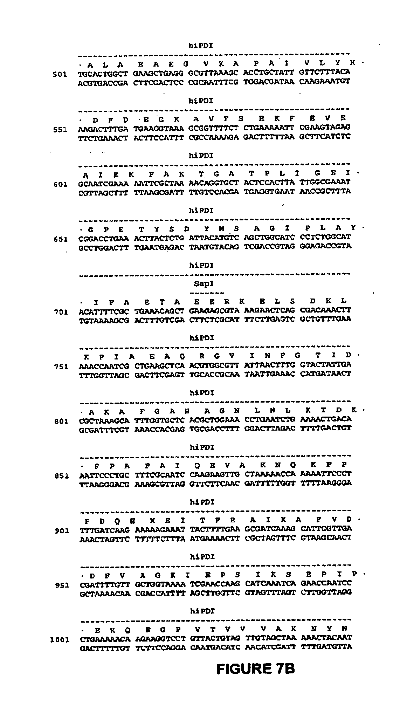

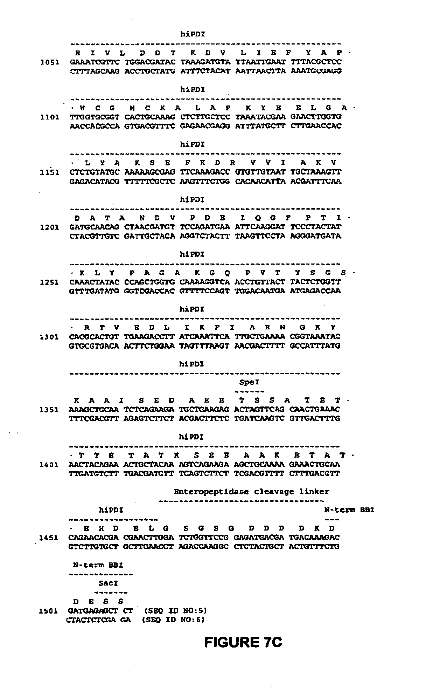

To determine whether hiPDl could be a better fusion partner than BCE103 for the production of BBI, this hiPDl gene was synthesized (DNA2.0) and cloned into the expression vector, p2JM103-Ink2-2BBlck81 (*See*, Example 4) as a *Bss*HII- *Sac*I fragment. In designing the synthetic gene, codons occurring with high frequency in highly expressed *B. subtilis* genes were selected except in cases where restriction sites were introduced or deleted. In the final construction, the N-terminus of the mature hiPDl gene was fused to the AprE signal sequence and the C-terminus was fused to a linker with an Enteropeptidase cleaveage site (Kajino et al., Appl. Env. Microbiol., 66:638-642 [2000]), which in turn was fused to 2BBlck81 (*See,* Figure 7). This expression vector, p2JM-PDl-EK-2BBlck81, was used to transform *B. subtilis* BG6006 and the production of the fusion protein was determined in MBD medium (as described in Example 1) with or without 2 mM BME added 14 h after inoculation.

As determined by SDS-PAGE gels, the production of the PDI-2BBlck81 fusion protein was typically somewhat less than the BCE-2BBck81 grown under identical conditions. The BBI titers (trypsin inhibition) measured from the PDI-2BBlck81 cell free supernatants were also typically less than the BCE-2BBlck81 fusion. As with fusions to BCE103, the measured activities of BBI when fused to PDI were higher when grown in 2 mM BME and the BBI activity was increased by the addition of BME to the cell free supernatants after growth when grown in BME free medium (as described in Example 3). Thus, the thiol-disulfide oxidoreductase activity of PDI does not seem to significantly improve the titers of active 2BBlck81 in the fusion protein or obviate the need for activation of the BBI molecule.

In order to increase the reduction potential of the fusion protein, which was contemplated to improve the BBI titers during growth, DsbC from *Escherichia coli* was used as a fusion partner for 2BBlck81. The *dsbC* gene was amplified by PCR using Herculase Enhanced DNA polymerase as described by the manufacturer (Stratagene) using DsbCBBI-F and DsbCBBl-R as primers (sequences shown above) and pET-40b(+) (Novagen) as a template. The isolated PCR fragment was cloned into the vector p2JM103-Gen4-2BBlck81 (*See*, Example 4) as a *Bss*HII-*Bam*HI fragment. The correct sequence of the fusion gene was verified by DNA sequencing. In this case, the titers of the DsbC-2BBlck81 fusion protein were significantly lower than the BCE-2BBlck81 fusion protein as judged on SDS-PAGE gels and the titers of the active 2BBlck81 measured by trypsin inhibition were much lower as well.

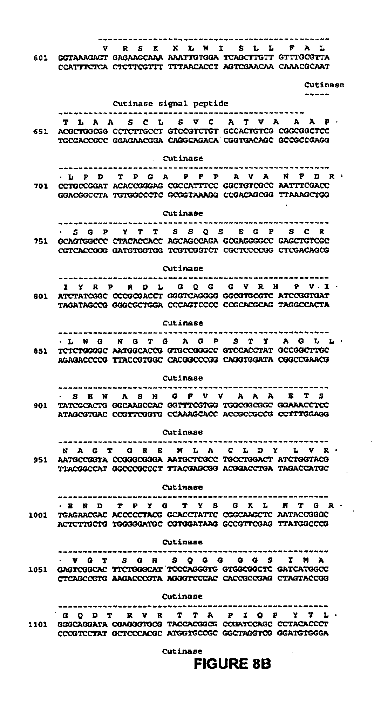

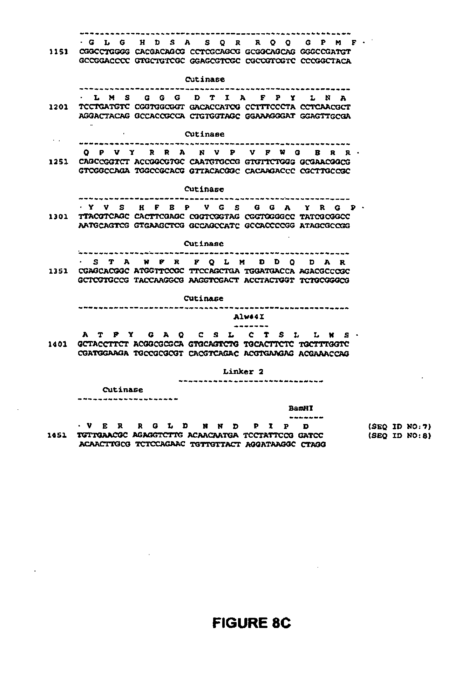

Other proteins that are produced at high titers in *B. subtilis* find use as fusion partners for the production of BBl. One such protein is the cutinase from *Pseudomonas mendocina,* which has been expressed at high titers utilizing the *aprE* promoter from *B. subtilis* (*See e.g.,* U.S. Pat. No. 5,429,950). The *aprE*-cutinase gene fusion as an *Eco*RI-*Alw*44I fragment (from pAK-15) was ligated with an *Alw*44I-*Bam*HI linker oligonucleotide cassette (*See*, sequence above) into the p2JM103-Ink2-2BBlck81 (*See*, Example 4) that had been cut with *Eco*RI and *Bam*HI*.* This cutinase-linker2-2BBlck81 expression vector (*See*, Figure 8 for the *Eco*RI-*Bam*HI *aprE-*cutinase-linker2 sequence) was used to transform *B. subtilis* BG6006 cells and the fusion protein was produced in MBD medium as described previously for the other fusion proteins (*See,* Example 1). In this case, the cutinase-linker2-2BBlck81 fusion protein was not the major band observed on SDS-PAGE gels and the measured lipase titers (as measured using the methods provided in U.S. Pat. No. 5,429,950) and BBl titers were much less (ca. 20 fold) than found with the BCE-2BBlck81 fusion protein. Also, the BBl titers in the cutinase fusion protein were not improved significantly when 3 mM ME was added to the growth medium. Thus, the highest titers of active 2BBlck81 was consistently obtained by activation of the BCE-2BBlck81 fusion protein. Nonetheless, it is contemplated that various fusion partners will find use in the present invention.

### Example 7

### Expression of truncated BBI-AV (BBlt-AV; SEQ ID NO:187)

This Example describes experiments that were performed to improve the production of the 2BBlck81 BBl protein (BBI-AV; SEQ ID NO:186) DDESSKPCCDQCACTKSNPPQCRCSDMRLNSCHSACKSCACYNLYGWTCFCVDI TDFCYEPCKPSEDDKEN (SEQ ID NO:18).

The Examples above and the journal article of Vogtentanz et al., 2007 (Protein Expr Purif 55:40-52 [2007]) describe a method for producing a fusion protein that comprises the soybean Bowman-Birk protease inhibitor (BBl) fused to the C-terminus of the BCE103 cellulase catalytic domain (BCE). As described above, this system has been used to produce BBl molecules with various variant peptides replacing the trypsin and/or chymotrypsin inhibitory loops. For example, a BBl molecule, BBl-AV (SEQ ID NO:186) containing the VEGF binding peptide, CK37281 (ACYNLYGWTC; SEQ ID NO:9), that replaces the native chymotrypsin inhibitory loop and the residue immediately N-terminal of the flanking cysteine residue (ICALSYPAQC; SEQ ID NO:388) was produced, purified and shown to compete in an electrochemiluminescent (ECL) based binding assay (BioVeris) with a monclonal antibody for binding to VEGF. However, the recovered yield of the variant BBI-AV was less than that of the wild-type BBl most likely due to an initial higher percentage of molecules with incorrectly formed disulfide bonds.

A truncated form of the BBI-AV of SEQ ID NO:186 (*i.e.* BBlt-AV of SEQ ID NO:187)
DDESSKPCCDQCACTKSNPPQCRCSDMRLNSCHSACKSCACYNLYGWTCFCVDI TDFCYEPCKPSE (SEQ ID NO:187), which lacks the 5 C-terminal amino acids of SEQ ID NO:186 was shown to bind better than the full-length molecule (*i.e.* untruncated molecule) to VEGF in an ECL (BioVeris) assay using a labeled monoclonal antibody against VEGF as a competitor. The C-truncated version of BBI-AV, called BBlt-AV (SEQ ID NO:187), can be produced during the purification process by trimming the C-terminus with GluBL protease (glutamyl endopeptidase I from *Bacillus licheniformis*). Alternatively, the BBlt-AV can be obtained by introducing a stop codon using an engineered oligonucleotide cassette as follows. The following BBl-trunc+ and BBI-trunc- oligonucleotides were annealed to generate an oligonucleotide cassette
BBl-trunc+
5'-TCGACATCACGGACTTCTGCTATGAACCATGTAAACCAAGCGAGTAAA (SEQ ID NO:188).
and
BBI -trunc-5'-AGCTTTTACTCGCTTGGTTTACATGGTTCATAGCAGAAGTCCGTGATG (SEQ ID NO:189)

The oligonucleotide cassette was then ligated into the *Sal*I and *Hin*dIII sites of the expression vector p2JM103-Ink2-2BBlck81, and the correct sequence was verified and the resulting expression vector was called p2JM103-Ink2-BBlt-AV.

To improve the expression, a polynucleotide sequence (SEQ ID NO:190 GCTGGTAAA) encoding the first three amino acids of the AprE pro-peptide, AGK (SEQ ID NO:191) (U.S. Patent No. 5,429,950), was inserted between the end of the AprE signal sequence and the start of the mature BCE103 cellulase using a QuikChange® site-directed mutagenesis kit (Stratagene). The site directed mutagenesis was performed essentially as described by the manufacturer using p2JM103-lnk2-BBlt-AV as a template with the oligonucleotide primers:
BCE-AGK-F:
   5'-GCGCGCAGGCAGCTGGTAAAGATGATTATTCAGTTGTAGA (SEQ ID NO:192)
   and
BCE-AGK-R:
   5'-AATAATCATCTTTACCAGCTGCCTGCGCGCTCATGTTGCT (SEQ ID NO:193)

The correct insertion was verified by DNA sequencing and the resulting expression vector was called p2JMagk103-Ink2-BBlt-AV (SEQ ID NO:194).

GAATTCTCCATTTTCTTCTGCTATCAAAATAACAGACTCGTGATTTTCCAAACGA
GCTTTCAAAAAGCCTCTGCCCCTTGCAAATCGGATGCCTGTCTATAAAATTCCC
GATATTGGTTAAACAGCGGCGCAATGGCGGCCGCATCTGATGTCTTTGCTTGGC
GAATGTTCATCTTATTTCTTCCTCCCTCTCAATAATTTTTTCATTCTATCCCTTTTC
TGTAAAGTTTATTTTTCAGAATACTTTTATCATCATGCTTTGAAAAAATATCACGAT
AATATCCATTGTTCTCACGGAAGCACACGCAGGTCATTTGAACGAATTTTTTCGA
CAGGAATTTGCCGGGACTCAGGAGCATTTAACCTAAAAAAGCATGACATTTCAG
CATAATGAACATTTACTCATGTCTATTTTCGTTCTTTTCTGTATGAAAATAGTTATT
TCGAGTCTCTACGGAAATAGCGAGAGATGATATACCTAAATAGAGATAAAATCAT
CTCAAAAAAATGGGTCTACTAAAATATTATTCCATCTATTACAATAAATTCACAGA
ATAGTCTTTTAAGTAAGTCTACTCTGAATTTTTTTAAAAGGAGAGGGTAAAGAGT
GAGAAGCAAAAAATTGTGGATCAGCTTGTTGTTTGCGTTAACGTTAATCTTTACG
ATGGCGTTCAGCAACATGAGCGCGCAGGCAGCTGGTAAAGATGATTATTCAGTT
GTAGAGGAACATGGGCAACTAAGTATTAGTAACGGTGAATTAGTCAATGAACGA
GGCGAACAAGTTCAGTTAAAAGGGATGAGTTCCCATGGTTTGCAATGGTACGGT
CAATTTGTAAACTATGAAAGCATGAAATGGCTAAGAGATGATTGGGGAATAACTG
TATTCCGAGCAGCAATGTATACCTCTTCAGGAGGATATATTGACGATCCATCAGT
AAAGGAAAAAGTAAAAGAGACTGTTGAGGCTGCGATAGACCTTGGCATATATGT
GATCATTGATTGGCATATCCTTTCAGACAATGACCCGAATATATATAAAGAAGAA
GCGAAGGATTTCTTTGATGAAATGTCAGAGTTGTATGGAGACTATCCGAATGTG
ATATACGAAATTGCAAATGAACCGAATGGTAGTGATGTTACGTGGGACAATCAA
ATAAAACCGTATGCAGAAGAAGTGATTCCGGTTATTCGTGACAATGACCCTAATA
ACATTGTTATTGTAGGTACAGGTACATGGAGTCAGGATGTCCATCATGCAGCCG
ATAATCAGCTTGCAGATCCTAACGTCATGTATGCATTTCATTTTTATGCAGGAAC
ACATGGACAAAATTTACGAGACCAAGTAGATTATGCATTAGATCAAGGAGCAGC
GATATTTGTTAGTGAATGGGGGACAAGTGCAGCTACAGGTGATGGTGGTGTTT
TTTAGATGAAGCACAAGTGTGGATTGACTTTATGGATGAAAGAAATTTAAGCTGG
GCCAACTGGTCTCTAACGCATAAGGATGAGTCATCTGCAGCGTTAATGCCAGGT
GCAAATCCAACTGGTGGTTGGACAGAGGCTGAACTATCTCCATCTGGTACATTT
GTGAGGGAAAAAATAAGAGAATCAGCATCTGACAACAATGATCCCATACCGGAT
CCAGACGATGAGAGCTCTAAACCCTGTTGCGATCAATGCGCATGTACGAAATCA
AATCCTCCACAGTGTCGGTGTTCCGATATGCGTCTGAATTCCTGTCATAGTGCC
TGCAAAAGCTGCGCATGTTATAACCTGTACGGGTGGACCTGTTTTTGCGTCGAC
ATCACGGACTTCTGCTATGAACCATGTAAACCAAGCGAGTAAAAGCTTAACTCG
AGGTTAACAGAGGACGGATTTCCTGAAGGAAATCCGTTTTTTTATTTTTAATTAA
GAGCTTGGCGTAATCATGGTCATAGCTGTTTCCTGTGTGAAATTGTTATCCGCTC
ACAATTCCACACAACATACGAGCCGGAAATAAAGTGTAAAGCCTGGGGTGCCTA

ATGAGTGAGCTAACTCACATTAATTGCGTTGCGCTCACTGCCCGCTTTCCAGTC
GGGAAACCTGTCGTGCCAGCTGCATTAATGAATCGGCCAACGCGCGGGGAGAG
GCGGTTTGCGTATTGGGCGCTCTTCCGCTTCCTCGCTCACTGACTCGCTGCGC
TCGGTCGTTCGGCTGCGGCGAGCGGTATCAGCTCACTCAAAGGCGGTAATACG
GTTATCCACAGAATCAGGGGATAACGCAGGAAAGAACATGTGAGCAAAAGGCC
AGCAAAAGGCCAGGAACCGTAAAAAGGCCGCGTTGCTGGCGTTTTTCCATAGG
CTCCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCC
TTCGGGAAGCGTGGCGCTTTCTCATAGCTCACGCTGTAGGTATCTCAGTTCGGT
GTAGGTCGTTCGCTCCAAGCTGGGCTGTGTGCACGAACCCCCCGTTCAGCCCG
ACCGCTGCGCCTTATCCGGTAACTATCGTCTTGAGTCCAACCCGGTAAGACACG
ACTTATCGCCACTGGCAGCAGCCACTGGTAACAGGATTAGCAGAGCGAGGTAT
GTAGGCGGTGCTACAGAGTTCTTGAAGTGGTGGCCTAACTACGGCTACACTAG
AAGAACAGTATTTGGTATCTGCGCTCTGCTGAAGCCAGTTACCTTCGGAAAAAG
AGTTGGTAGCTCTTGATCCGGCAAACAAACCACCGCTGGTAGCGGTGGTTTTTT
TGTTTGCAAGCAGCAGATTACGCGCAGAAAAAAAGGATCTCAAGAAGATCCTTT
GATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAAT
GAAGTTTTAAATCAATCTAAAGTATATATGAGTAAACTTGGTCTGACAGTTACCAA
TGCTTAATCAGTGAGGCACCTATCTCAGCGATCTGTCTATTTCGTTCATCCATAG
TTGCCTGACTCCCCGTCGTGTAGATAACTACGATACGGGAGGGCTTACCATCTG
GCCCCAGTGCTGCAATGATACCGCGAGACCCACGCTCACCGGCTCCAGATTTA
TCAGCAATAAACCAGCCAGCCGGAAGGGCCGAGCGCAGAAGTGGTCCTGCAAC
TTTATCCGCCTCCATCCAGTCTATTAATTGTTGCCGGGAAGCTAGAGTAAGTAGT
TCGCCAGTTAATAGTTTGCGCAACGTTGTTGCCATTGCTACAGGCATCGTGGTG
TCACGCTCGTCGTTTGGTATGGCTTCATTCAGCTCCGGTTCCCAACGATCAAGG
CGAGTTACATGATCCCCCATGTTGTGCAAAAAAGCGGTTAGCTCCTTCGGTCCT
CCGATCGTTGTCAGAAGTAAGTTGGCCGCAGTGTTATCACTCATGGTTATGGCA
GCACTGCATAATTCTCTTACTGTCATGCCATCCGTAAGATGCTTTTCTGTGACTG
GTGAGTACTCAACCAAGTCATTCTGAGAATAGTGTATGCGGCGACCGAGTTGCT
CTTGCCCGGCGTCAATACGGGATAATACCGCGCCACATAGCAGAACTTTAAAAG
TGCTCATCATTGGAAAACGTTCTTCGGGGCGAAAACTCTCAAGGATCTTACCGC
TGTTGAGATCCAGTTCGATGTAACCCACTCGTGCACCCAACTGATCTTCAGCAT
CTTTTACTTTCACCAGCGTTTCTGGGTGAGCAAAAACAGGAAGGCAAAATGCCG
CAAAAAAGGGAATAAGGGCGACACGGAAATGTTGAATACTCATACTCTTCCTTTT
TCAATATTATTGAAGCATTTATCAGGGTTATTGTCTCATGAGCGGATACATATTTG

AATGTATTTAGAAAAATAAACAAATAGGGGTTCCGCGCACATTTCCCCGAAAAGT
GCCACCTGACGTCTAAGAAACCATTATTATCATGACATTAACCTATAAAAATAGG
CGTATCACGAGGCCCTTTCGTCTCGCGCGTTTCGGTGATGACGGTGAAAACCT
CTGACACATGCAGCTCCCGGAGACGGTCACAGCTTGTCTGTAAGCGGATGCCG
GGAGCAGACAAGCCCGTCAGGGCGCGTCAGCGGGTGTTGGCGGGTGTCGGG
GCTGGCTTAACTATGCGGCATCAGAGCAGATTGTACTGAGAGTGCACCATAGAT
CTGGAGCTGTAATATAAAAACCTTCTTCAACTAACGGGGCAGGTTAGTGACATTA
GAAAACCGACTGTAAAAAGTACAGTCGGCATTATCTCATATTATAAAAGCCAGTC
ATTAGGCCTATCTGACAATTCCTGAATAGAGTTCATAAACAATCCTGCATGATAA
CCATCACAAACAGAATGATGTACCTGTAAAGATAGCGGTAAATATATTGAATTAC
CTTTATTAATGAATTTTCCTGCTGTAATAATGGGTAGAAGGTAATTACTATTATTA
TTGATATTTAAGTTAAACCCAGTAAATGAAGTCCATGGAATAATAGAAAGAGAAA
AAGCATTTTCAGGTATAGGTGTTTTGGGAAACAATTTCCCCGAACCATTATATTT
CTCTACATCAGAAAGGTATAAATCATAAAACTCTTTGAAGTCATTCTTTACAGGA
GTCCAAATACCAGAGAATGTTTTAGATACACCATCAAAAATTGTATAAAGTGGCT
CTAACTTATCCCAATAACCTAACTCTCCGTCGCTATTGTAACCAGTTCTAAAAGC
TGTATTTGAGTTTATCACCCTTGTCACTAAGAAAATAAATGCAGGGTAAAATTTAT
ATCCTTCTTGTTTTATGTTTCGGTATAAAACACTAATATCAATTTCTGTGGTTATA
CTAAAAGTCGTTTGTTGGTTCAAATAATGATTAAATATCTCTTTTCTCTTCCAATT
GTCTAAATCAATTTTATTAAAGTTCATTTGATATGCCTCCTAAATTTTTATCTAAAG
TGAATTTAGGAGGCTTACTTGTCTGCTTTCTTCATTAGAATCAATCCTTTTTTAAA
AGTCAATATTACTGTAACATAAATATATATTTTAAAAATATCCCACTTTATCCAATT
TTCGTTTGTTGAACTAATGGGTGCTTTAGTTGAAGAATAAAAGACCACATTAAAA
AATGTGGTCTTTTGTGTTTTTTTAAAGGATTTGAGCGTAGCGAAAAATCCTTTTCT
TTCTTATCTTGATAATAAGGGTAACTATTGCCGGATCGTCCTCAGGAGTAGGCG
ACATCGCTAAATAATGATCTATGCGGTGTGAAATACCGCACAGATGCGTAAGGA
GAAAATACCGCATCAGGCGCCATTCGCCATTCAGGCTGCGCAACTGTTGGGAA
GGGCGATCGGTGCGGGCCTCTTCGCTATTACGCCAGCTGGCGAAGGGGGAT
GTGCTGCAAGGCGATTAAGTTGGGTAACGCCAGGGTTTTCCCAGTCACGACGT
TGTAAAACGACGGCCAGT (SEQ ID NO:194)

The p2JMagk103-Ink2-BBlt-AV expression vector was used to transform bacterial cells to express BBlt-AV fused to the BCE protein (SEQ ID NO:195)

AGKDDYSVVEEHGQLSISNGELVNERGEQVQLKGMSSHGLQWYGQFVNYESMK
WLRDDWGITVFRAAMYTSSGGYIDDPSVKEKVKETVEAAIDLGIYVIIDWHILSDNDP
NIYKEEAKDFFDEMSELYGDYPNVIYEIANEPNGSDVTWDNQIKPYAEEVIPVIRDND

PNNIVIVGTGTWSQDVHHAADNQLADPNVMYAFHFYAGTHGQNLRDQVDYALDQG
AAIFVSEWGTSAATGDGGVFLDEAQVWIDFMDERNLSWANWSLTHKDESSAALMP
GANPTGGWTEAELSPSGTFVREKIRESASDNNDPIPDPDDESSKPCCDQCACTKS
NPPQCRCSDMRLNSCHSACKSCACYNLYGWTCFCVDITDFCYEPCKPSE (SEQ ID
NO:195)

### EXAMPLE 8

### BBlt-AV Site Saturation Libraries

This example describes experiments that were performed to generate modified variant BBPls derived from the unmodified BBIt-AV by constructing site saturation libraries comprising substitutions at each of the 39 amino acid positions of the BBIt-AV molecule (SEQ ID NO:187) shown in Figure 9.

BBlt-AV site-saturation libraries were created essentially as described by Amin et al. (Biotechniques 35: 1134-1140, [2003]) using a modified version of the QuikChange multi site-directed mutagenesis (QCMS) kit from Stratagene (La Jolla, CA) as follows. Overlapping forward and reverse primers with the NNS codon in the middle and 17 - 20 flanking bases were designed for each chosen site, and the sequences for each forward (F) and reverse (R) primer at each of the 66 amino acid positions in the BBI-AV molecule are shown in Table 1. Each mutagenesis reaction contained 50 - 100 ng template plasmid (p2JMagk103-Ink2-BBlt-AV; SEQ ID NO: 194), 0.5 µl forward primer (25 µM), 0.5 µl reverse primer (25 µM), 1 µl dNTP's (QCMS kit), 2.5 µl 10x QCMS reaction buffer, 18.5 µl deionized water, and 1 µl of enzyme blend (QCMS kit), for a total volume of 25 µl. For BBI-AV libraries at residues 10, 11, 13 and 25, only 1µL of the forward primer (25 M) was used without the reverse primer. For libraries at residues 6, 37, 38, 50 and 53, the mutagenesis reaction was carried out with 0.5 µl of the forward mutagenic primer in combination with another primer (5'-CTATGCGGCATCAGAGCAGATTGTAC; SEQ ID NO:328 complementary to a sequence in the vector. For the mutagenesis reaction, the thermocycler program used was 1 cycle at 95° for 2 min., followed by 29 cycles of 95°C for 30 sec, 55°C for 1 min., and 68°C for 10 minutes (MJ Research thermocycler). The template DNA was digested by the addition of 0.5 -1 µl *Dpn*I (from the QCMS kit) and incubation at 37°C for 1 - 4 hours, followed by another addition of 0.5 - 1 µl *Dpn*I and another incubation at 37°C for 1-4 hours. The template DNA was obtained from *E. coli dam⁺* strains, such as TOP10 (Invitrogen, Carlsbad, CA, USA), to ensure that it would be susceptible to *DpnI* digestion.

For efficient transformation of *B. subtilis,* the DNA in each mutagensis reaction was amplified by rolling circle amplification (RCA) using the Templiphi kit from Amersham. Specifically, 1µL of the undiluted QCMS reaction was mixed with 5µL of sample buffer from the Templiphi kit. The mixture was heated for 3 minutes at 95 °C to denature the DNA. The reaction was placed on ice to cool for 2 minutes. Then, 5 µL of reaction buffer and 0.2 µL of phi29 polymerase were added to the reaction and incubated at 30°C in a MJ Research thermocycler for 5 hours. Finally, the phi29 enzyme in the reaction was heat inactivated by incubation at 65°C for 10 min in a MJ Research thermocycler. The amplified DNA was diluted 10 -100 fold and 1 µL was used to transform 100 µL of competent *B. subtilis* BG6006 cells (Vogtentanz et al., 2007). Aliquots of 25 µL or 75 µL of the transformation mixture were plated on Luria Agar plates supplemented with 5 µg/ml chloramphenicol. After growth, individual transformants were picked to inoculate 200 µl of Luria broth supplemented with 5 µg/ml chloramphenicol in each well of a 96-well microtiter plate. The cultures were grown in a humidified and aerated box at 37 °C and 270 rpm (Innova 4230 incubator, New Brunswick Scientific). After growth, 80 µl of 50 % glycerol were added to each well and mixed. A duplicate plate was made by removing 140 µl of the culture from each well and placing it in a new microtiter plate. Both plates were stored at -80 °C. One plate was saved as a master and the other was submitted for DNA sequencing (Cogenics, Huston, TX) of the individual mutants. For DNA sequencing, an overnight culture of each colony grown in a microtiter plate was diluted 10 - 40 fold and 2 µL was used in a PCR reaction with Amersham Biosciences Ready-to-go PCR beads and the primers BBI-PCR-F and BBI-PCR-R. The thermo-cycling conditions were one cycle at 95 °C for 5 minutes, followed by 30 cycles of 95 °C for 1 minute, 55 °C for 1 minute and 72 °C for 1 minute, and a final cycle at 72 °C for 7 minutes. The PCR products were treated with Exonuclease I and shrimp alkaline phosphatase (EXOSAP-IT, Amersham Biosciences) or they were column purified before sequencing with the M13 reverse primer.

**TABLE 1.**

| **NNS mutagenesis primers** | | |
|---|---|---|
| **Primer name** | **Primer Sequence** | **SEQ ID NO:** |
| **Forward Primers** | | |
| BBl1F | GATCCCATACCGGATCCANNSGATGAGAGCTCTAAACC | 196 |
| BBl2F | CCATACCGGATCCAGACNNSGAGAGCTCTAAACCCTG | 197 |
| BBl3F | CATACCGGATCCAGACGATNNSAGCTCTAAACCCTGTTG | 198 |
| BBl4F | CGGATCCAGACGATGAGNNSTCTAAACCCTGTTGCGATC | 199 |
| BBl5F | GATCCAGACGATGAGAGCNNSAAACCCTGTTGCGATCAATG | 200 |
| BBl6F | CAGACGATGAGAGCTCTNNSCCCTGTTGCGATCAATG | 201 |
| BBl7F | GACGATGAGAGCTCTAAANNSTGTTGCGATCAATGCGC | 202 |
| BBl8F | GATGAGAGCTCTAAACCCNNSTGCGATCAATGCGCATG | 203 |
| BBl9F | GAGAGCTCTAAACCCTGTNNSGATCAATGCGCATGTAC | 204 |
| BBl10F | GCTCTAAACCCTGTTGCNNSCAATGCGCATGTACGAAATC | 205 |
| BBl11F | CTAAACCCTGTTGCGATNNSTGCGCATGTACGAAATC | 206 |
| BBl12F | CTAAACCCTGTTGCGATCAANNSGCATGTACGAAATCAAATC | 207 |
| BBl13F | CCTGTTGCGATCAATGCNNSTGTACGAAATCAAATCC | 208 |
| BBl14F | GTTGCGATCAATGCGCANNSACGAAATCAAATCCTCC | 209 |
| BBl15F | GCGATCAATGCGCATGTNNSAAATCAAATCCTCCACAG | 210 |
| BBl16F | GATCAATGCGCATGTACGNNSTCAAATCCTCCACAGTG | 211 |
| BBl17F | CAATGCGCATGTACGAAANNSAATCCTCCACAGTGTCG | 212 |
| BBl18F | GCGCATGTACGAAATCANNSCCTCCACAGTGTCGGTG | 213 |
| BBl19F | CATGTACGAAATCAAATNNSCCACAGTGTCGGTGTTC | 214 |
| BBl20F | GTACGAAATCAAATCCTNNSCAGTGTCGGTGTTCCGATAT | 215 |
| BBl21F | CGAAATCAAATCCTCCANNSTGTCGGTGTTCCGATATG | 216 |
| BBl22F | GAAATCAAATCCTCCACAGNNSCGGTGTTCCGATATGCG | 217 |
| BBl23F | CAAATCCTCCACAGTGTNNSTGTTCCGATATGCGTCTG | 218 |
| BBl24F | CAAATCCTCCACAGTGTCGGNNSTCCGATATGCGTCTGAATTC | 219 |
| BBl25F | CTCCACAGTGTCGGTGTNNSGATATGCGTCTGAATTC | 220 |
| BBl26F | CACAGTGTCGGTGTTCCNNSATGCGTCTGAATTCCTG | 221 |
| BBl27F | CAGTGTCGGTGTTCCGATNNSCGTCTGAATTCCTGTCATAG | 222 |
| BBl28F | GTCGGTGTTCCGATATGNNSCTGAATTCCTGTCATAG | 223 |
| BBl29F | GGTGTTCCGATATGCGTNNSAATTCCTGTCATAGTGC | 224 |
| BBl30F | GTTCCGATATGCGTCTGNNSTCCTGTCATAGTGCCTG | 225 |
| BBl31F | CCGATATGCGTCTGAATNNSTGTCATAGTGCCTGCAAAAG | 226 |
| BBl32F | GATATGCGTCTGAATTCCNNSCATAGTGCCTGCAAAAG | 227 |
| BBl33F | ATATGCGTCTGAATTCCTGTNNSAGTGCCTGCAAAAGCTG | 228 |
| BBl34F | GTCTGAATTCCTGTCATNNSGCCTGCAAAAGCTGCGC | 229 |
| BBl35F | CTGAATTCCTGTCATAGTNNSTGCAAAAGCTGCGCATG | 230 |
| BBl36F | GAATTCCTGTCATAGTGCCNNSAAAAGCTGCGCATGTTATAA | 231 |
| BBl37F | CCTGTCATAGTGCCTGCNNSAGCTGCGCATGTTATAAC | 232 |
| BBl38F | GTCATAGTGCCTGCAAANNSTGCGCATGTTATAACCTG | 233 |
| BBl39F | CATAGTGCCTGCAAAAGCNNSGCATGTTATAACCTGTAC | 234 |
| BBl40F | GTGCCTGCAAAAGCTGCNNSTGTTATAACCTGTACGG | 235 |
| BBl41F | CCTGCAAAAGCTGCGCANNSTATAACCTGTACGGGTG | 236 |
| BBl42F | GCAAAAGCTGCGCATGTNNSAACCTGTACGGGTGGAC | 237 |
| BBl43F | CAAAAGCTGCGCATGTTATNNSCTGTACGGGTGGACCTG | 238 |
| BBl44F | GCTGCGCATGTTATAACNNSTACGGGTGGACCTGTTTTG | 239 |
| BBl45F | GCGCATGTTATAACCTGNNSGGGTGGACCTGTTTTTG | 240 |
| BBl46F | CATGTTATAACCTGTACNNSTGGACCTGTTTTTGCGTC | 241 |
| BBl47F | GTTATAACCTGTACGGGNNSACCTGTTTTTGCGTCGAC | 242 |
| BBl48F | GTTATAACCTGTACGGGTGGNNSTGTTTTTGCGTCGACATC | 243 |
| BBl49F | ATAACCTGTACGGGTGGACCNNSTTTTGCGTCGACATCAC | 244 |
| BBl50F | CTGTACGGGTGGACCTGTNNSTGCGTCGACATCACGGAC | 245 |
| BBl51F | GTACGGGTGGACCTGTTTTNNSGTCGACATCACGGACTTC | 246 |
| BBl52F | GGTGGACCTTTTTGCNNSGACATCACGGACTTCTG | 247 |
| BBl53F | GGACCTGTTTTTGCGTCNNSATCACGGACTTCTGCTATG | 248 |
| BBl54F | CCTGTTTTTGCGTCGACNNSACGGACTTCTGCTATGAAC | 249 |
| BBl55F | GTTTTTGCGTCGACATCNNSGACTTCTGCTATGAACC | 250 |
| BBl56F | GTTTTTGCGTCGACATCACGNNSTTCTGCTATGAACCATG | 251 |
| BBl57F | GCGTCGACATCACGGACNNSTGCTATGAACCATGTAAAC | 252 |
| BBl58F | GTCGACATCACGGACTTCNNSTATGAACCATGTAAACC | 253 |
| BBl59F | GACATCACGGACTTCTGCNNSGAACCATGTAAACCAAG | 254 |
| BBl60F | CATCACGGACTTCTGCTATNNSCCATGTAAACCAAGCGAG | 255 |
| BBl61F | CGGACTTCTGCTATGAANNSTGTAAACCAAGCGAGTAAAA | 256 |
| BBl62F | GACTTCTGCTATGAACCANNSAAACCAAGCGAGTAAAAG | 257 |
| BBl63F | CTTCTGCTATGAACCATGTNNSCCAAGCGAGTAAAAGCTTAA | 258 |
| BBl64F | GCTATGAACCATGTAAANNSAGCGAGTAAAAGCTTAAC | 259 |
| BBl65F | CTATGAACCATGTAAACCANNSGAGTAAAAGCTTAACTC | 260 |
| BBl66F | GAACCATGTAAACCAAGCNNSTAAAAGCTTAACTCGAG | 261 |

| **Reverse Primers** | | |
|---|---|---|
| BBl1R | GGTTTAGAGCTCTCATCSNNTGGATCCGGTATGGGATC | 262 |
| BBl2R | CAGGGTTTAGAGCTCTCSNNGTCTGGATCCGGTATGG . | 263 |
| BBl3R | CAACAGGGTTTAGAGCTSNNATCGTCTGGATCCGGTATG | 264 |
| BBl4R | GATCGCAACAGGGTTTAGASNNCTCATCGTCTGGATCCG | 265 |
| BBl5R | CATTGATCGCAACAGGGTTTSNNGCTCTCATCGTCTGGATC | 266 |
| BBl6R | CATTGATCGCAACAGGGSNNAGAGCTCTCATCGTCTG | 267 |
| BBl7R | GCGCATTGATCGCAACASNNTTTAGAGCTCTCATCGTC | 268 |
| BBl8R | CATGCGCATTGATCGCASNNGGGTTTAGAGCTCTCATC | 269 |
| BBl9R | GTACATGCGCATTGATCSNNACAGGGTTTAGAGCTCTC | 270 |
| BBl10R | GATTTCGTACATGCGCATTGSNNGCAACAGGGTTTAGAGC | 271 |
| BBl11R | GATTTCGTACATGCGCASNNATCGCAACAGGGTTTAG | 272 |
| BBl12R | GATTTGATTTCGTACATGCSNNTTGATCGCAACAGGGTTTAG | 273 |
| BBl13R | GGATTTGATTTCGTACASNNGCATTGATCGCAACAGG | 274 |
| BBl14R | GGAGGATTTGATTTCGTSNNTGCGCATTGATCGCAAC | 275 |
| BBl15R | CTGTGGAGGATTTGATTTSNNACATGCGCATTGATCGC | 276 |
| BBl16R | CACTGTGGAGGATTTGASNNCGTACATGCGCATTGATC | 277 |
| BBl17R | CGACACTGTGGAGGATTSNNTTTCGTACATGCGCATTG | 278 |
| BBl18R | CACCGACACTGTGGAGGSNNTGATTTCGTACATGCGC | 279 |
| BBl19R | GAACACCGACACTGTGGSNNATTTGATTTCGTACATG | 280 |
| BBl20R | ATATCGGAACACCGACACTGSNNAGGATTTGATTTCGTAC | 281 |
| BBl21R | CATATCGGAACACCGACASNNTGGAGGATTTGATTTCG | 282 |
| BBl22R | CGCATATCGGAACACCGSNNCTGTGGAGGATTTGATTTC | 283 |
| BBl23R | CAGACGCATATCGGAACASNNACACTGTGGAGGATTTG | 284 |
| BBl24R | GAATTCAGACGCATATCGGASNNCCGACACTGTGGAGGATTTG | 285 |
| BBl25R | GAATTCAGACGCATATCSNNACACCGACACTGTGGAG | 286 |
| BBl26R | CAGGAATTCAGACGCATSNNGGAACACCGACACTGTG | 287 |
| BBl27R | CTATGACAGGAATTCAGACGSNNATCGGAACACCGACACTG | 288 |
| BBl28R | CTATGACAGGAATTCAGSNNCATATCGGAACACCGAC | 289 |
| BBl29R | GCACTATGACAGGAATTSNNACGCATATCGGAACACC | 290 |
| BBl30R | CAGGCACTATGACAGGASNNCAGACGCATATCGGAAC | 291 |
| BBBl31R | CTTTTGCAGGCACTATGACASNNATTCAGACGCATATCGG | 292 |
| BBl32R | CTTTTGCAGGCACTATGSNNGGAATTCAGACGCATATC | 293 |
| BBl33R | CAGCTTTTGCAGGCACTSNNACAGGAATTCAGACGCATAT | 294 |
| BBl34R | GCGCAGCTTTTGCAGGCSNNATGACAGGAATTCAGAC | 295 |
| BBl35R | CATGCGCAGCTTTTGCASNNACTATGACAGGAATTCAG | 296 |
| BBl36R | TTATAACATGCGCAGCTTTTSNNGGCACTATGACAGGAATTC | 297 |
| BBl37R | GTTATAACATGCGCAGCTSNNGCAGGCACTATGACAGG | 298 |
| BBl38R | CAGGTTATAACATGCGCASNNTTTGCAGGCACTATGAC | 299 |
| BBl39R | GTACAGGTTATAACATGCSNNGCTTTTGCAGGCACTATG | 300 |
| BBl40R | CCGTACAGGTTATAACASNNGCAGCTTTTGCAGGCAC | 301 |
| BBl41R | CACCCGTACAGGTTATASNNTGCGCAGCTTTTGCAGG | 302 |
| BBl42R | GTCCACCCGTACAGGTTSNNACATGCGCAGCTTTTGC | 303 |
| BBl43R | CAGGTCCACCCGTACAGSNNATAACATGCGCAGCTTTTG | 304 |
| BBl44R | CAAAAACAGGTCCACCCGTASNNGTTATAACATGCGCAGC | 305 |
| BBl45R | CAAAAACAGGTCCACCCSNNCAGGTTATAACATGCGC | 306 |
| BBl46R | GACGCAAAAACAGGTCCASNNGTACAGGTTATAACATG | 307 |
| BBl47R | GTCGACGCAAAAACAGGTSNNCCCGTACAGGTTATAAC | 308 |
| BBl48R | GATGTCGACGCAAAAACASNNCCACCCGTACAGGTTATAAC | 309 |
| BBl49R | GTGATGTCGACGCAAAASNNGGTCCACCCGTACAGGTTAT | 310 |
| BBl50R | GTCCGTGATGTCGACGCASNNACAGGTCCACCCGTACAG | 311 |
| BBl51R | GAAGTCCGTGATGTCGACSNNAAAACAGGTCCACCCGTAC | 312 |
| BBl52R | CAGAAGTCCGTGATGTCSNNGCAAAAACAGGTCCACC | 313 |
| BBl53R | CATAGCAGAAGTCCGTGATSNNGACGCAAAAACAGGTCC | 314 |
| BBl54R | GTTCATAGCAGAAGTCCGTSNNGTCGACGCAAAAACAGG | 315 |
| BBl55R | GGTTCATAGCAGAAGTCSNNGATGTCGACGCAAAAAC | 316 |
| BBl56R | CATGGTTCATAGCAGAASNNCGTGATGTCGACGCAAAAAC | 317 |
| BBl57R | GTTTACATGGTTCATAGCASNNGTCCGTGATGTCGACGC | 318 |
| BBl58R | GGTTTACATGGTTCATASNNGAAGTCCGTGATGTCGAC | 319 |
| BBl59R | CTTGGTTTACATGGTTCSNNGCAGAAGTCCGTGATGTC | 320 |
| BBl60R | CTCGCTTGGTTTACATGGSNNATAGCAGAAGTCCGTGATG | 321 |
| BBl61R | TTTTACTCGCTTGGTTTACASNNTTCATAGCAGAAGTCCG | 322 |
| BBl62R | CTTTTACTCGCTTGGTTTSNNTGGTTCATAGCAGAAGTC | 323 |
| BBl63R | TTAAGCTTTTACTCGCTTGGSNNACATGGTTCATAGCAGAAG | 324 |
| BBl64R | GTTAAGCTTTTACTCGCTSNNTTTACATGGTTCATAGC | 325 |
| BBl65R | GAGTTAAGCTTTTACTCSNNTGGTTTACATGGTTCATAG | 326 |
| BBl66R | CTCGAGTTAAGCTTTTASNNGCTTGGTTTACATGGTTC | 327 |

The cultures in each master plate were thawed and a pin replicator was used to inoculate a new sterile microtiter plate containing 150 µl of Luria broth with 5 µg/ml chloramphenicol in each well. The plates were grown for ~ 10 hrs in a humidified and aerated box as described above. For controls, the cultures in two wells were replaced with BBl and BBI-AV cultures that had been grown concurrently in 15 ml culture tubes (5 ml of Luria broth with 5 µg/ml chloramphenicol). After the cultures were grown, the plate was used to inoculate two duplicate 96-well filter plates (Millipore, MSGV2210) containing 150 µl of MBD medium (Vogtentanz et al., 2007) with 5 µg/ml chloramphenicol in each well by using a pin replicator. The duplicate plates were grown for ~ 14 hours (as above, with plastic spacers placed between the stacked plates to allow for efficient air flow around the plates) and then 2-mecaptoethanol was added to each well in one of the plates to a final concentration of 2 mM. The plates were then grown for an additional - 46 hours. The 96-well filter plates containing the culture broth (with or without added 2-mecaptoethanol) were then assayed for BBl and BCE activity.

### EXAMPLE 9

### Primary Screening of BBlt-AV Variants: BBI and BCE Activity Assays to Determine Variants Having the Highest BBlt-AV:BCE Activity Ratio

In this Example, methods are provided for assessing the effect of the amino acid substitutions introduced into the modified variant BBlt-AVs generated according to the site-saturation methods. The trypsin inhibitory activity of the modified BBIT-AV generated by the site-saturation methods described above was measured and compared to the trypsin inhibitory activity of the control wild-type BBI (SEQ ID NO:13; BBI-wt or sBBl) and the control unmodified BBlt-AV (SEQ ID NO:187). It is not intended that the present invention be limited to these specific methods, as other suitable methods find use.

### TRYPSIN INHIBITORY ASSAY: BBl ACTIVITY DETERMINATION

The relative concentration of active control and modified BBlt-AV's was determined by a trypsin inhibitory assay using purified sBBl as a standard as described above. For some samples, the active BBI concentration was measured by residual trypsin activity according to the method described by Flecker (Flecker, P. FEBS Lett. 252:153-157 [1989]; Vogtentanz *et al. supra*).

Libraries were analyzed using dilutions of cultures grown in 96-well plates. Typically, the culture broth from each well was diluted (200 fold) in Assay Buffer (100 mM Tris pH 8.6, 0.005% Tween 80) and transferred to a second microtiter plate (CoStar 9017, Corning, Inc., Corning, NY). Then, 20 µl of the diluted samples were transferred to a third microtiter plate. A standard curve was created by adding 20, 10; 5 or 2.5 µl of the diluted sBBl (SEQ ID NO:13) control culture to empty wells. Then, 80 µL of 50 ng/ml trypsin (bovine pancreatic trypsin, Worthington Biochemical Corp., Lakewood, NJ) (prepared from a 1 mg/mL stock solution diluted into Assay Buffer) was added to each well of the plate. The reactions were mixed and incubated 15 min at room temperature. After the incubation, 100 µL of 0.5 mg/ml trypsin substrate (succinyl-Ala-Ala-Pro-Arg-para-nitroanilide, Bachem Bioscience, Inc., King of Prussia, PA), diluted in Assay Buffer from a 100 mg/ml solution in DMSO, was added to each well, mixed, and the absorbance at 405 nm was monitored for 15 min, with 1 time point recorded every 12 sec using a Spectra Max 250 (Molecular Devices). The data points were used to determine the rate for each reaction. The standard curve was generated by plotting the reaction rate versus the BBI volume and was fitted to a four-parameter equation. All data fitting was done using software supplied by the manufacturer (Molecular Devices Corp., Sunnyvale, CA). The activity of each BBlt-AV variant was calculated from the standard curve. Thus, the activities of all modified variant proteins were determined relative to the activity in the sBBl control (BBI-WT) culture. If the majority of the data did not fall near the IC₅₀ of the standard curve, a new dilution plate was made and assayed.

### CELLULASE ENZYME ACTIVITY ASSAY: BCE ASSAY

The concentration of BCE was determined by an activity assay using purified BCE as a standard as described above and by (Vogtentanz et al. 2007). For the analysis of libraries grown in 96-well microtiter plates, 20 µl of culture broth were transferred to each well of a new microtiter plate. A standard curve was created by adding 20, 10, 5 or 2.5 µl of the diluted BBl-WT control culture to these empty wells. Then, 180 µl of the BCE substrate (0.25 µg/ml in Assay Buffer), 4-nitrophenyl β-D-cellobioside (Sigma, St. Louis, MO), was added to the plate. The plate was mixed and the absorbance at 405 nm monitored for 15 min, with 1 time point recorded every 12 s using a Spectra Max 250 (Molecular Devices Corp., Sunnyvale, CA). The data points were used to determine the rate for each reaction. The standard curve was generated by plotting rate versus culture volume and was fit to a quadratic curve. The BCE concentration in each variant culture was determined relative to the standard control culture.

### Enzyme Activity Ratio: BBlt-AV:BCE ratios

From the microtiter plate assay results, the BBlt-AV:BCE activity ratio of each well was determined by dividing the relative BBlt-AV trypsin inhibitory activity values by the relative BCE activity values. The data were sorted based on the BBlt-AV:BCE activity ratio. Modified variants with the highest BBlt-AV:BCE activity ratios were selected and subjected to a secondary screening described below.

### Analysis of Enzyme Activity ratio BBlt-AV:BCE -Secondary Screening of Variants

In this example, modified BBlt-AVs having improved BBlt-AV:BCE activity relative to that of the control unmodified BBlt-AV when grown in the presence or absence of a reducing agent (2-mercaptoethanol), as determined in the primary screen, were arrayed and assayed in quadruplicate in a secondary screen. Initially, only the polynucleotides encoding each of the modified BBlt-AVs having the greatest BBlt-AV:BCE ratio as determined in the secondary screen were sequenced. Later, all BBlt-AV clones were sequenced.

### Quadruplicate testing in microtiter plates - secondary screen

For each plate, twenty-two samples of cultures containing modified BBlt-AVs selected from the initial screen, and two samples of control cultures containing sBBl and unmodified BBlt-AV, were grown for ~ 10 hrs in 5 ml of Luria broth with 5 ppm chloramphenicol (37 °C, 250 rpm) in 15 ml culture tubes. Then, 150 µl of each of the 24 cultures were arrayed in quadruplicate in a 96-well microtiter plate. This arrayed plate was then used to inoculate duplicate filter plates (Millipore, MSGV2210) containing 150 µl of MBD medium with 5 µg/ml chloramphenicol per well by using a pin replicator. The plated cultures were grown (in the presence and absence of 2-β-mercaptoethanol (βME), and the BBI-AV:BCE ratios were determined and analyzed as described above.

Figure 10 shows an example of the effect of all possible 19 amino acid subsitutions at position 29 of SEQ ID NO:187 on the trypsin inhibitory activity of the modified variant BBlt. The data show the ratio of BBI and BCE activities determined from quadruplicate measurements of BCE and trypsin activities of the modified BBI-AVs having a single amino acid substitution at position 29 of SEQ ID NO:187.

### EXAMPLE 10

### Selection for improved modified BBlt-AVs with known amino acid substitutions

In this example, site saturation libraries were constructed to generate a complete set of amino acid substitutions at sites identified in the initial screen that resulted in BBIt-AVs having improved trypsin inhibitory activity when compared to the trypsin inhibitory activity of the unmodified parent BBI-AV. In addition, a complete set of amino acid substitutions at sites that had not been previously substituted with all possible 19 amino acids were generated by site-directed mutagenesis. A total of 39 site saturation libraries were generated and quadruplicate activity measurements of BBI trypsin inhibitory activity and BCE activity were analyzed to determine the single substitutions that resulted in modified BBlt-AV molecules having trypsin inhibitory activity that was greater than that of the control BBI molecules *i.e*. sBBl and unmodified BBlt-AV. The positions of the amino acids that were substituted in the unmodified BBlt-AV are shown in Figure 9.

### Construction of a second set of site-saturation libraries at sites 18, 38 and 61

First, to aid library construction, a synthetic gene was synthesized to replace the *Eco*RI site with a *Sph*I site between the region coding for the trypsin inhibitory loop and the region encoding the chymotypsin inhibitory loop in the 2BBlck81 coding region (the location of the EcoRI site is shown in Figure 3). The synthetic gene sequence is: 5'-GGATCCAGACGATGAGAGCTCTAAACCTTGTTGCGATCAATGCGCTTGTACAAA ATCAAACCCTCCACAATGTCGTTGTTCTGATATGCGTTTAAATAGCTGTCATTCT GCATGCAAATCATGTGCTTGCTATAACCTTTACGGTTGGACATGTTTCTGCGTCG ACATCACTGACTTCTGCTATGAACCATGTAAACCTTCTGAATAAAAGCTT(SEQ ID NO:329). This synthetic gene was cloned into p2JMagk103-lnk2-BBlt-AV as a BamHl-Hindlll fragment and the resulting vector was called p2JMagk103-lnk2-BBlt-AVsph.

Site-saturation libraries were constructed at positions 18, 37 and 61 using oligonucleotide cassettes as follows.

A site-saturation library at site 18 was constructed by annealing oligonucleotides 5'-GTACAAAATCANNSCCTCCACAATGTCGTTGTTCTGATATGCGTTTAAATAGCTG TCATTCTGCATG (SEQ ID NO:330) and 5'-CAGAATGACAGCTATTTAAACGCATATCAGAACAACGACATTGTGGAGGSNNTG ATTTT (SEQ ID NO:331) and ligating the resulting cassette into the BsrGl and Sphl sites of p2JMagk103-lnk2-BBl-AVsph.

A site-saturation library at site 37 was constructed by annealing the oligonucleotides 5'-TCGACGCAGAAACATGTCCAACCGTAAAGGTTATAGCAAGCACATGASNNGCAT G (SEQ ID NO:332) and 5'-CNNSTCATGTGCTTGCTATAACCTTTACGGTTGGACATGTTTCTGCG (SEQ ID NO:333) and ligating the resulting cassette into the Sall and Sphl sites of p2JMagk103-Ink2-BBl-AVsph.

The site-saturation library at position 61 was made by annealing oligonucleotide 5'-TCGACATCACTGACTTCTGCTATGAANNSTGTAAACCTTCTGAATAAA (SEQ ID NO:334) with the two oligonucleotides 5'-TTCATAGCAGAAGTCAGTGATG (SEQ ID NO:335) and 5'-AGCTTTTATTCAGAAGGTTTACA (SEQ ID NO:336) and ligating this cassette into the Sall and Hindlll sites of p2JMagk103-Ink2-BBIt-AVsph.

The ligation mixes were used to transform *E. coli* TOP10 cells (Invitrogen). Ninety six transformants were selected, and the amino acid substitution present in each clone was determined by DNA sequencing. Plasmids isolated from each single amino acid substitution were then used to transform *B. subtilis.*

### Generation of amino acid substitutions by site directed mutagenesis

Amino acid substitutions at selected sites were constructed by ligating an oligonucleotide or an oligonucleotide cassette into the of p2JMagk103-lnk2-BBl-AVsph vector using the appropriate restriction sites. The substitutions, oligonucleotide sequences and restriction sites used are shown in Table 2.

**TABLE 2**

| **Oligonucleotides for site directed muatagenesis** | | |
|---|---|---|
| **Amino Acid Substitution(s)** | **Oligonucleotide Pairs Sequence(s)** | **Restriction Sites** |
| A13Y | 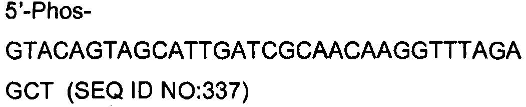 | SacI & BsrGl |
| R23D,E | 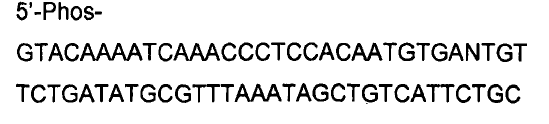 | BsrGI & Sphl |
| | ATG (SEQ ID NO:338) | |
| | 5'- CAGAATGACAGCTATTTAAACGCATATCAGAA CANTCACATTGTGGAGGGTTTGATTTT (SEQ ID NO:339) | |
| S25M | 5'-Phos- GTACAAAATCAAACCCTCCACAATGTCGTTGT ATGGATATGCGTTTAAATAGCTGTCATTCTGC ATG (SEQ ID NO SEQ ID NO:340) | BsrGl & Sphl |
| | 5'- CAGAATGACAGCTATTTAAACGCATATCCATA CAACGACATTGTGGAGGGTTTGATTTT (SEQ ID NO:341) | |
| S25W | 5'-Phos- GTACAAAATCAAACCCTCCACAATGTCGTTGT TGGGATATGCGTTTAAATAGCTGTCATTCTGC ATG (SEQ ID NO:342) | BsrGI & Sphl |
| | 5'- CAGAATGACAGCTATTTAAACGCATATCCCAA CAACGACATTGTGGAGGGTTTGATTTT (SEQ ID NO:343) | |
| S25A,V,G | 5'-Phos- GTACAAAATCAAACCCTCCACAATGTCGTTGT GBAGATATGCGTTTAAATAGCTGTCATTCTGC ATG (SEQ ID NO:344) | BsrGl & Sphl |
| | 5'- CAGAATGACAGCTATTTAAACGCATATCTVCA CAACGACATTGTGGAGGGTTTGATTTT (SEQ | |
| | ID NO:345) | |
| S25N,Q,K,H | 5'-Phos- GTACAAAATCAAACCCTCCACAATGTCGTTGT MAWGATATGCGTTTAAATAGCTGTCATTCTGC ATG (SEQ ID NO:346) | BsrGl & Sphl |
| | 5'- CAGAATGACAGCTATTTAAACGCATATCWTKA CAACGACATTGTGGAGGGTTTGATTTT (SEQ ID NO:347) | |
| M27W | 5'-Phos- GTACAAAATCAAACCCTCCACAATGTCGTTGT TCTGATTGGCGTTTAAATAGCTGTCATTCTGC ATG (SEQ ID NO:348) | BsrGI & SphI |
| | 5'- CAGAATGACAGCTATTTAAACGCCAATCAGAA CAACGACATTGTGGAGGGTTTGATTTT (SEQ ID NO:349) | |
| M27H | 5'-Phos- GTACAAAATCAAACCCTCCACAATGTCGTTGT TCTGATCACCGTTTAAATAGCTGTCATTCTGC ATG (SEQ ID NO:350) | BsrGI & SphI |
| | 5'- CAGAATGACAGCTATTTAAACGGTGATCAGAA CAACGACATTGTGGAGGGTTTGATTTT (SEQ ID NO:351) | |
| M27G,A,V,D | 5'-Phos- GTACAAAATCAAACCCTCCACAATGTCGTTGT TCTGATGNTCGTTTAAATAGCTGTCATTCTGC ATG (SEQ ID NO:352) | BsrGl & Sphl |
| | 5'- CAGAATGACAGCTATTTAAACGANCATCAGAA CAACGACATTGTGGAGGGTTTGATTTT (SEQ ID NO:353) | |
| M27F,l | 5'-Phos- GTACAAAATCAAACCCTCCACAATGTCGTTGT TCTGATWTTCGTTTAAATAGCTGTCATTCTGC ATG (SEQ ID NO:354) | BsrGl & Sphl |
| | 5'- CAGAATGACAGCTATTTAAACGAAWATCAGAA CAACGACATTGTGGAGGGTTTGATTTT (SEQ ID NO:355) | |
| L29I | 5'-Phos- GTACAAAATCAAACCCTCCACAATGTCGTTGT TCTGATATGCGTATTAATAGCTGTCATTCTGC ATG (SEQ ID NO:356) | BsrGI & SphI |
| | 5'- CAGAATGACAGCTATTAATACGCATATCAGAA CAACGACATTGTGGAGGGTTTGATTTT (SEQ ID NO:357) | |
| L29D,E | 5'-Phos- GTACAAAATCAAACCCTCCACAATGTCGTTGT TCTGATATGCGTGANAATAGCTGTCATTCTGC ATG (SEQ ID NO:358) | BsrGl & Sphl |
| | 5′- CAGAATGACAGCTATTNTCACGCATATCAGAA CAACGACATTGTGGAGGGTTTGATTTT (SEQ ID NO:359) | |
| S31M | 5'-Phos- GTACAAAATCAAACCCTCCACAATGTCGTTGT TCTGATATGCGTTTAAATATGTGTCATTCTGCA TG (SEQ ID NO:360) | BsrGl & SphI |
| | 5' CAGAATGACACATATTTAAACGCATATCAGAA CAACGACATTGTGGAGGGTTTGATTTT (SEQ ID NO:361) | |
| S31D,E,Y | 5'-Phos- GTACAAAATCAAACCCTCCACAATGTCGTTGT TCTGATATGCGTTTAAATKAWTGTCATTCTGC ATG (SEQ ID NO:362) | BsrGl & Sphl |
| | 5'- CAGAATGACAWTMATTTAAACGCATATCAGAA CAACGACATTGTGGAGGGTTTGATTTT (SEQ ID NO:363) | |
| S31Q,H | 5'-Phos- GTACAAAATCAAACCCTCCACAATGTCGTTGT TCTGATATGCGTTTAAATCANTGTCATTCTGC ATG (SEQ ID NO:364) | BsrGI & SphI |
| | 5'- CAGAATGACANTGATTTAAACGCATATCAGAA CAACGACATTGTGGAGGGTTTGATTTT (SEQ ID NO:365) | |
| S34I | 5'-Phos- GTACAAAATCAAACCCTCCACAATGTCGTTGT TCTGATATGCGTTTAAATAGCTGTCATATCGC ATG (SEQ ID NO:366) | BsrGI & SphI |
| | 5'- CGATATGACAGCTATTTAAACGCATATCAGAA CAACGACATTGTGGAGGGTTTGATTTT (SEQ ID NO:367) | |
| S34W | 5'-Phos- GTACAAAATCAAACCCTCCACAATGTCGTTGT TCTGATATGCGTTTAAATAGCTGTCATTGGGC ATG (SEQ ID NO:368) | BsrGl & Sphl |
| | 5'- CCCAATGACAGCTATTTAAACGCATATCAGAA CAACGACATTGTGGAGGGTTTGATTTT (SEQ ID NO:369) | |
| S34R,Q | 5'-Phos- GTACAAAATCAAACCCTCCACAATGTCGTTGT TCTGATATGCGTTTAAATAGCTGTCATCRAGC ATG (SEQ ID NO:370) | BsrGl & SphI |
| | 5'- CTYGATGACAGCTATTTAAACGCATATCAGAA CAACGACATTGTGGAGGGTTTGATTTT (SEQ ID NO:371) | |
| S34D,E | 5'-Phos- GTACAAAATCAAACCCTCCACAATGTCGTTGT TCTGATATGCGTTTAAATAGCTGTCATGANGC ATG (SEQ ID NO:372) | BsrGl & Sph |
| | 5'- CNTCATGACAGCTATTTAAACGCATATCAGAA CAACGACATTGTGGAGGGTTTGATTTT (SEQ ID NO:373) | |
| A40V,D | 5'-Phos- TCGACGCAGAAACATGTCCAACCGTAAAGGT | SphI & SalI |
| | TATAGCAAWCACATGATTTGCATG (SEQ ID NO:374) | |
| | 5'- CAAATCATGTGWTTGCTATAACCTTTACGGTT GGACATGTTTCTGCG (SEQ ID NO:375) | |
| A40H,P | 5'-Phos- TCGACGCAGAAACATGTCCAACCGTAAAGGT TATAGCAAKGACATGATTTGCATG (SEQ ID NO:376) | Sphl & Sall |
| | 5'- CAAATCATGTCMTTGCTATAACCTTTACGGTT GGACATGTTTCTGCG (SEQ ID NO:377) | |
| A40Y,W | 5'-Phos- TCGACGCAGAAACATGTCCAACCGTAAAGGT TATAGCAMYAACATGATTTGCATG (SEQ ID NO:378) | Sphl & Sall |
| | 5'- CAAATCATGTTRKTGCTATAACCTTTACGGTT GGACATGTTTCTGCG (SEQ ID NO:379) | |
| F50D | 5'-Phos- TCGACGCAATCACATGTCCAACCGTAAAGGTT ATAGCAAGCACATGATTTGCATG (SEQ ID NO:380) | SphI & SalI |
| | 5'- CAAATCATGTGCTTGCTATAACCTTTACGGTT GGACATGTGATTGCG (SEQ ID NO:381) | |
| F50Q,P | 5'-Phos - | SphI & SalI |
| | TCGACGCATKGACATGTCCAACCGTAAAGGTT ATAGCAAGCACATGATTTGCATG (SEQ ID NO:382) | |
| | 5'- CAAATCATGTGCTTGCTATAACCTTTACGGTT GGACATGTCMATGCG (SEQ ID NO:383) | |
| F50S,R | 5'-Phos- TCGACGCAKCTACATGTCCAACCGTAAAGGTT ATAGCAAGCACATGATTTGCATG (SEQ ID NO:384) | Sphl & Sall |
| | 5'- CAAATCATGTGCTTGCTATAACCTTTACGGTT GGACATGTAGMTGCG (SEQ ID NO:385) | |
| V52D | 5'-Phos- CAAATCATGTGCTTGCTATAACCTTTACGGTT GGACATGTTTCTGCGATGACATCACTGACTTC TGCTATGAACCATGTAAACCTTCTGAATAAA (SEQ ID NO:386) | SphI & HindIII |
| | 5'- AGCTTTTATTCAGAAGGTTTACATGGTTCATA GCAGAAGTCAGTGATGTCATCGCAGAAACAT GTCCAACCGTAAAGGTTATAGCAAGCACATGA TTTGCATG (SEQ ID NO:387) | |

### Secondary screening of individual amino acid substitutions: quadruplicate testing in microtiter plates

For each chosen site, clones were selected corresponding to individual amino acid substitutions. These clones were grown, along with cultures of BBI-WT, BBlt-AV and BBlt-AV-F50T as controls, in 5 ml of Luria broth with 5 ppm chloramphenicol (37 °C, 250 rpm) in 15 ml culture tubes for - 10 hrs at 37 °C. One hundred and fifty microliter aliquots of each the cultures were arrayed in the wells of a 96-well microtiter plate, 4 wells for each amino acid substitution and each control. Each culture grown in the arrayed plate was then used to inoculate duplicate filter plates (Millipore, MSGV2210) containing 150 µl of MBD medium with 5 µg/ml chloramphenicol per well by using a pin replicator. The cultures in the plates were grown in the presence and absence of 2-mercaptoethanol and analyzed as described above. BBlt-AV-F50T is a modified BBlt-AV that was identified as having an initial BBlt-AV:BCE activity ratio prior to activation that was comparable to that of the unmodified BBlt-AV parent, but which has a three-fold greater BBlt-AV:BCE activity ratio than that of the unmodified BBlt-AV after activation with a reducing agent such as 2-mercaptoethanol.

For each site, the BBI:BCE activity ratio was determined for each well and the average values for the quadruplicate measurements was calculated. The activity ratios for the individual amino acid substitutions were compared to the ratios calculated for unmodified parent BBlt-AV (SEQ ID NO:187), the wild-type BBI (SEQ ID NO:13) and the modified BBlt-AV named BBlt-AV-F50T.

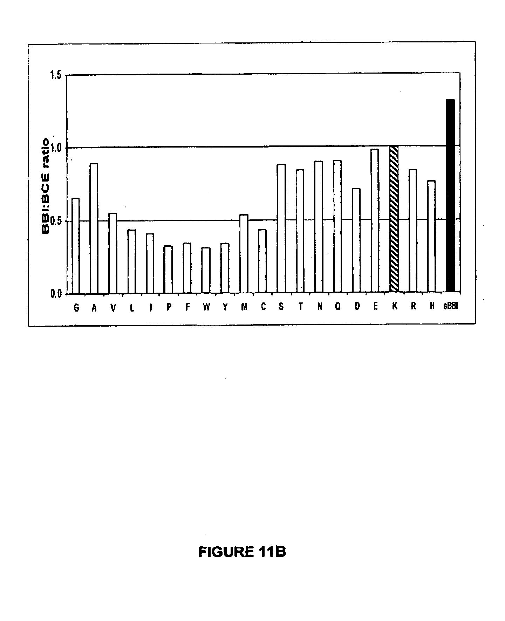

Figures 11A and 11B show the average of the quadruplicate determinations of the BBI:BCE ratio determined in the presence of 2-mercaptoethanol (BME) relative to that determined for the sBBl for each of the substitutions at positions 50 and 37, respectively.

Overall, the data show single amino acid substitutions made at 16 of the 66 positions/sites in the parent BBlt-AV molecule resulted in BBlt-AV variants that had trypsin inhibitory activity equal or greater than that of the precursor BBlt-AV (data summarized in Table 3). Of all the single site substitutions, F50R resulted in the best BBIt-AV:BCE- activity ratio, while several substitutions at position 50 produced modified variant BBlt-AVs having a four-fold greater BBlt-AV:BCE activity ratios than that of the precursor unmodified BBlt-AV, and that were comparable to that of the wild-type inhibitor (Figure 11A-B).

These data show that a single substitution in the BBI-AV molecule can have a significant effect on the activity of the BBI-AV.

**TABLE 3**

| **Substitutions that result in modified BBlt-AV molecules with greater BBI:BCE activity ratios than the unmodified BBlt-AV** | | |
|---|---|---|
| **Position** | **Native Amino Acid** | **Improved Substitutions (with BME)** |
| 1 | D | A, P, |
| 4 | S | V (4D13 insert) * |
| 5 | S | P, A |
| 11 | Q | G |
| 13 | A | Y, I, F, M, L V, K, R |
| 18 | N | I, V |
| 25 | S | K N, W, I, A, R |
| 27 | M | H, R, K, V, A, Q |
| 29 | L | R, K, P |
| 31 | S | Q, H, E, A, R, W, K, T |
| 38 | S | N, K, R |
| 40 | A | H, K, Q, R, Y |
| 50 | F | R, Q, K, T, V, M, S |
| 52 | V | K, T, R , Q, L, H, A, M, S, E |
| 55 | T | M |
| 65 | S | E D |

| | | |
|---|---|---|
| * The 4D13 insert is a 13 amino acid sequence that was added to the N-terminus of the BBI protein as described below. | | |

### EXAMPLE 11

### Selection of modified variants having improved purification yield

In this example, the methods described were used to identify modified BBlt-AVs containing single amino acid substitutions that retained trypsin inhibitory activity while being produced at yields greater than the corresponding unmodified precursor BBlt-AV.

Modified BBI-AVs that had been selected for having the greatest BBlt-AV:BCE activity ratio in the quadruplicate plate screens were further tested in a shake flask screen that was designed to mimic the acid/heat treatment that is used during the purification process to cleave the BBlt-AV from the BCE:BBlt-AV fusion protein. Thus, modified BBlt-AVs that retained a BBI:BCE activity ratio greater than that of the unmodified BBI-AV following acid/heat treatment were identified as modified BBlt-AVs that would be produced at yields greater than that of the unmodified precursor BBlt-AV.

After activation with reducing agent, *e.g*.2-mercaptoethanol, a higher BBlt-AV:BCE activity ratio indicates that a given amino acid substitution has significantly increased the fraction of molecules with at least a correctly folded trypsin inhibitory loop. However, two different amino acid substitutions with similar BBlt-AV:BCE activity ratios could have somewhat different total yields after purification. For example, V52L and M27A activate to similar levels but M27A has about 40 % better yield after acid/heat treatment. Thus, variants selected in the first screens as "good activators" were further evaluated by a second criterion that was developed to better predict purification yield.

### Shake flask screen: testing for the purification yield

To increase expression, the gene copy of the selected clones was amplified by sequentially streaking colonies on Luria agar plates with 25 µg/ml chloramphenicol until the growth rate was similar to growth on Luria agar plates without chloramphenicol. Cultures of the selected variants and controls were grown in 3 ml Luria broth with 25 µg/ml chloramphenicol in 15 ml tubes for ~ 10 h. These cultures (30 µ) were then used to inoculate 30 ml of MBD medium in 250 ml baffled shake flasks and grown at 37 °C and 225 rpm for ~ 60 h.

For selection of the variants with the best yields, the culture broths were processed as follows. The broth was first activated by mixing 20 ml of broth with 20 ml of 0.25 M glycine buffer (pH 9.3) and the pH was adjusted to 9.0 with 50% NaOH. Then, 2-mercaptoethanol was added to the diluted cultures to a final concentration of 2 mM and the cultures were incubated overnight at room temperature with gentle shaking. The BBlt-AV moiety was then cleaved from the fusion protein by acid/heat treatment, which was accomplished by first adjusting the pH of the activated broth to pH 1.9 - 2.0 with sulfuric acid, and followed by incubation at 60 °C for 16 hours, with shaking. The BBlt-AV-BCE fusion protein and the BCE catalytic domain are not soluble at pH 2, whereas the free BBI species is soluble. After the cleavage reaction, the insoluble material was removed by centrifugation (5 min. at 3,000 rpm) and the supernatant was analyzed for trypsin inhibitory activity as described above. The BBI activity was also determined for the starting material and for the samples taken after activation. The BCE activity of the starting material was also determined as described above. The BBlt-AV:BCE activity ratios were then calculated for each step in process. The modified BBlt-AVs produced at the highest yields following the acid/heat treatment were selected for further study.

Amino acid substitutions were found at sites 13, 25, 27, 29, 31, 40, 50 and 52 that significantly improved the BBlt-AV yield after acid/heat treatment. Other substitutions with improved yields after acid/heat treatment were D1 C (when not activated), S4V, S5P, Q11G, S38N and S65E. An additional variant that was produced by an unexpected duplication of a primer (during the QuikChange reaction used to make the site-saturation library at position 4) also had a higher BBlt-AV:BCE activity ratio after acid/heat treatment. In this variant, the amino acid sequence, DDEPSKPCCDPDP (SEQ ID NO:389) (called the 4D13 insert), was inserted between the linker and the N-terminus of BBlt-AV (SEQ ID NO:187) to generate a modified BBlt-AV fused to the linker in SEQ ID NO:391.

DNNDPIPDPddepskpccdpdpDDESSKPCCDQCACTKSNPPQCRCSDMRLNSCHSA
CKSCACYNLYGWTCFCVDITDFCYEPCKPSE (SEQ ID NO:391).

The linker is shown in italicized letters, the insert is written in lower case letters and the BBlt-AV corresponding to SEQ ID NO:187 is in bold letters. The sequence of the 4D13 modified BBtl-AV is:

DDEPSKPCCDPDPDDESSKPCCDQCACTKSNPPQCRCSDMRLNSCHSACKSCAC
YNLYGWTCFCVDITDFCYEPCKPSE (SEQ ID NO:390).

The synthetic gene encoding 4D13-BBlt-AV of SEQ ID NO:390 is:

GGATCCAGATGACGAACCGAGCAAACCTTGCTGTGATCCAGACCCTGACGATG
AGAGCTCTAAACCCTGTTGCGATCAATGCATTTGTACGAAATCAAATCCTCCACA
GTGTCGGTGTTCCGATATGCGTCCGAATTCCTGTCATAGTGCCTGCAAAAGCTG
CAAGTGTTATAACCTGTACGGGTGGACCTGTACATGCGCCGACATCACGGACTT
CTGCTATGAACCATGTAAACCAAGCGAGTAAAAGCTT (SEQ ID NO:394).

### EXAMPLE 12

### Combinations of Mutations

In this example, the single amino acid substitutions that resulted in BBlt-AVs having improved BBI:BCE activity ratios in either the plate screens or in the shake flask screen were combined to generate modified BBlt-AVs that have greater BBlt-AV:BCE activity ratios and/or production yields than those of modified BBlt-AVs carrying single amino acid substitutions.

Following the primary and secondary screens described in Examples 9 and 10 above, three modified BBlt-AVs each containing a single amino acid substitution A13I, F50T and V52A, respectively, were identified as having an improved BBI:BCE activity ratio relative to the respective control. A library was constructed to select for the best combination of these three substitutions. The library was made by the QuikChange® mutagenesis protocol described above using equimolar concentrations of the following primers in the reaction mixture: primer 5'-CTGTTGCGATCAATGCATTTGTACGAAATC (SEQ ID NO:395) was used to generate the A13l substitution, primer 5'-CTGTACGGGTGGACCTGTACATGCGYCGACATCACGGACTTC (SEQ ID NO:396) was used to generate the F50T and F50T-V52A substitutions, and primer 5'-GACCTGTTTTTGCGYCGACATCACGGAC (SEQ ID NO:397) was used to generate the V52A substitution in the reaction mixture. Clones were selected and screened in microtiter plates and screened as described above.
The modified double variant BBlt-AV containing the F50T and the V52A substitutions (BBlt-AV-F50T-V52A: SEQ ID NO:595) was obtained, and determined to have the polynucleotide sequence of SEQ ID NO:398.

### BBlt-AV-F50T-V52A

GGATCCAGACGATGAGAGCTCTAAACCCTGTTGCGATCAATGCGCATGTACGAA
ATCAAATCCTCCACAGTGTCGGTGTTCCGATATGCGTCTGAATTCCTGTCATAGT
GCCTGCAAAAGCTGCGCATGTTATAACCTGTACGGGTGGACCTGTACATGCGC
CGACATCACGGACTTCTGCTATGAACCATGTAAACCAAGCGAGTAAAGCTT
(SEQ ID NO:398)

DPDDESSKPCCDQCACTKSNPPQCRCSDMRLNSCHSACKSCACYNLYGWTCTCA
DITDFCYEPCKPSE (SEQ ID NO:595).

### Generation of modified BBlt-AVs containing three, four and five amino acid substitutions

Combinations of four productive substitutions, A13I, S25L, L29P and A40K, identified in the screens described in Examples 9 and 10 above were made in the BBlt-AV-F50T-V52A double variant. These substitutions were combined by using synthetic genes that were cloned into the BamHl and Hindlll sites of the p2JMagk103lnk2BBlt-AV vector. The polynucleotide sequences encoding the corresponding amino acid sequences for the modified BBlt-AVs comprising combinations of substitutions are given below.
BBlt-AV-A13I-S25L-F50T-V52A 
GGATCCAGACGATGAGAGCTCTAAACCCTGTTGCGATCAATGCATTTGTACGAA
ATCAAATCCTCCACAGTGTCGGTGTCTTGATATGCGTCTGAATTCCTGTCATAGT
GCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:399)
 
DPDDESSKPCCDQCICTKSNPPQCRCLDMRLNSCHSACKSCACYNLYGWTCTCA
DITDFCYEPCKPSE (SEQ ID NO:596)
 BBlt-AV-A13I-S25L-L29P-F50T-V52A 
GGATCCAGACGATGAGAGCTCTAAACCCTGTTGCGATCAATGCATTTGTACGAA
ATCAAATCCTCCACAGTGTCGGTGTCTTGATATGCGTCCGAATTCCTGTCATAGT
GCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:400)
 
DPDDESSKPCCDQCICTKSNPPQCRCLDMRPNSCHSACKSCACYNLYGWTCTCA
DITDFCYEPCKPSE (SEQ ID NO:597).
 BBlt-AV-A13I-S25L-L29P-A40K-F50T-V52A 
GGATCCAGACGATGAGAGCTCTAAACCCTGTTGCGATCAATGCATTTGTACGAA
ATCAAATCCTCCACAGTGTCGGTGTCTTGATATGCGTCCGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:401)
 
DPDDESSKPCCDQCICTKSNPPQCRCLDMRPNSCHSACKSCKCYNLYGWTCTCA
DITDFCYEPCKPSE (SEQ ID NO:598)
 BBlt-AV-A13I-S25L-A40K-F50T-V52A 
GGATCCAGACGATGAGAGCTCTAAACCCTGTTGCGATCAATGCATTTGTACGAA
ATCAAATCCTCCACAGTGTCGGTGTCTTGATATGCGTCTGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:402)
 
DPDDESSKPCCDQCICTKSNPPQCRCLDMRLNSCHSACKSCKCYNLYGWTCTCA
DITDFCYEPCKPSE (SEQ ID NO:599)
 BBlt-AV-A13I-L29P-F50T-V52A 
GGATCCAGACGATGAGAGCTCTAAACCCTGTTGCGATCAATGCATTTGTACGAA
ATCAAATCCTCCACAGTGTCGGTGTTCCGATATGCGTCCGAATTCCTGTCATAG
TGCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:403)
 
DPDDESSKPCCDQCICTKSNPPQCRCSDMRPNSCHSACASCACYNLYGWTCTCA
DITDFCYEPCKPSE (SEQ ID NO:600)
 BBlt-AV-A13I-L29P-A40K-F50T-V52A (TA5) 
GGATCCAGACGATGAGAGCTCTAAACCCTGTTGCGATCAATGCATTTGTACGAA
ATCAAATCCTCCACAGTGTCGGTGTTCCGATATGCGTCCGAATTCCTGTCATAG
TGCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:404)
 
DPDDESSKPCCDQCICTKSNPPQCRCSDMRPNSCHSACKSCKCYNLYGWTCTCA
DITDFCYEPCKPSE (SEQ ID NO:601)
 BBlt-AV-A13I-A40K-F50T-V52A 
GGATCCAGACGATGAGAGCTCTAAACCCTGTTGCGATCAATGCATTTGTACGAA
ATCAAATCCTCCACAGTGTCGGTGTTCCGATATGCGTCTGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:405)
 
DPDDESSKPCCDQCICTKSNPPQCRCSDMRLNSCHSACKSCKCYNLYGWTCTCA
DITDFCYEPCKPSE (SEQ ID NO:602)
 BBlt-AV-S25L-F50T-V52A 
GGATCCAGACGATGAGAGCTCTAAACCCTGTTGCGATCAATGCGCATGTACGAA
ATCAAATCCTCCACAGTGTCGGTGTCTTGATATGCGTCTGAATTCCTGTCATAGT
 
GCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:406)
 
DPDDESSKPCCDQCACTKSNPPQCRCLDMRLNSCHSACKSCACYNLYGWTCTCA
DITDFCYEPCKPSE (SEQ ID NO:603)
 BBlt-AV-S25L-L29P-F50T-V52A 
GGATCCAGACGATGAGAGCTCTAAACCCTGTTGCGATCAATGCGCATGTACGAA
ATCAAATCCTCCACAGTGTCGGTGTCTTGATATGCGTCCGAATTCCTGTCATAGT
GCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:407)
 
DPDDESSKPCCDQCACTKSNPPQCRCLDMRPNSCHSACKSCACYNLYGWTCTCA
DITDFCYEPCKPSE (SEQ ID NO:604)
 BBlt-AV-S25L-L29P-A40K-F50T-V52A 
GGATCCAGACGATGAGAGCTCTAAACCCTGTTGCGATCAATGCGCATGTACGAA
ATCAAATCCTCCACAGTGTCGGTGTCTTGATATGCGTCCGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:408)
 
DPDDESSKPCCDQCACTKSNPPQCRCLDMRPNSCHSACKSCKCYNLYGWTCTCA
DITDFCYEPCKPSE (SEQ ID NO:605)
 BBlt-AV-S25L-A40K-F50T-V52A 
GGATCCAGACGATGAGAGCTCTAAACCCTGTTGCGATCAATGCGCATGTACGAA
ATCAAATCCTCCACAGTGTCGGTGTCTTGATATGCGTCTGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:409)
 
DPDDESSKPCCDQCACTKSNPPQCRCLDMRLNSCHSACKSCKCYNLYGWTCTCA
DITDFCYEPCKPSE (SEQ ID NO:606)
 BBlt-AV-L29P-F50T-V52A 
GGATCCAGACGATGAGAGCTCTAAACCCTGTTGCGATCAATGCGCATGTACGAA
ATCAAATCCTCCACAGTGTCGGTGTTCCGATATGCGTCCGAATTCCTGTCATAG
TGCCTGCAAAAGCTGCGCATGTTATAACCTGTACGGGTGGACCTGTACATGCGC
CGACATCACGGACTTCTGCTATGAACCATGTAAACCAAGCGAGTAAAAGCTT
(SEQ ID NO:410)
 
DPDDESSKPCCDQCACTKSNPPQCRCSDMRPNSCHSACKSCACYNLYGWTCTC
ADITDFCYEPCKPSE (SEQ ID NO:607)
 BBlt-AV-L29P-A40K-F50T-V52A 
GGATCCAGACGATGAGAGCTCTAAACCCTGTTGCGATCAATGCGCATGTACGAA
ATCAAATCCTCCACAGTGTCGGTGTTCCGATATGCGTCCGAATTCCTGTCATAG
TGCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:411)
 
DPDDESSKPCCDQCACTKSNPPQCRCSDMRPNSCHSACKSCKCYNLYGWTCTC
ADITDFCYEPCKPSE (SEQ ID NO:608)
 BBlt-AV-A40K-F50T-V52A

GGATCCAGACGATGAGAGCTCTAAACCCTGTTGCGATCAATGCGCATGTACGAA
ATCAAATCCTCCACAGTGTCGGTGTTCCGATATGCGTCTGAATTCCTGTCATAGT
GCCTGCAAAAGCTGCAAGTGTTATAACCTGTACGGGTGGACCTGTACATGCGC
CGACATCACGGACTTCTGCTATGAACCATGTAAACCAAGCGAGTAAAAGCTT
(SEQ ID NO:412)

DPDDESSKPCCDQCACTKSNPPQCRCSDMRLNSCHSACKSCKCYNLYGWTCTCA
DITDFCYEPCKPSE (SEQ ID NO:609)

### Generation of modified BBlt-AVs containing six and seven amino acid substitutions

Additional modified BBlt-AV were generated to comprise variations including the 4D13 insertion, single amino acid substitutions D1 C, S4V, S5P, Q11G, l13L, S25R, M27R, P29K, S31A, S31 R, S38N, T50K, A52T, S65E, and double amino acid substitutions including S25R-S31 R and S25R-S31 with substitutions present in a quintuple variant (BBlt-AV-A13I-L29P-A40K-F50T-V52A) encoded by the polynucleotide of SEQ ID NO:404.

The 4D13 peptide was inserted into the p2JM103 based vector constructed for the expression of BBlt-AV-A13I-L29P-A40K-F50T-V52A (SEQ ID NO:404) with an oligonucleotide cassette having BamHl and SacI restriction sites on the ends using the oligonucleotides:
5'-GATCCAGATGACGAACCGAGCAAACCTTGCTGTGATCCAGACCCTGACGATGA GAGCT (SEQ ID NO:392) and
5'-CTCATCGTCAGGGTCTGGATCACAGCAAGGTTTGCTCGGTTCGTCATCTG (SEQ ID NO:393).

Amino acid sequence of BBlt-AV-4D13 insert-A13I-L29P-A40K-F50T-V52A: DPDDEPSKPCCDPDPDDESSKPCCDQCICTKSNPPQCRCSDMRPNSCHSACKSC KCYNLYGWTCTCADITDFCYEPCKPSE (SEQ ID NO:413)

The other variants were made by cloning synthetic genes into the BamHI and Hindlll sites of p2JM103-lnk2. The polynucleotide sequences of each of the synthetic genes and resulting modified amino acid sequences of the modified variant BBlt-AVs are given in Table 4.

**TABLE 4**

| **BBlt-AVs comprising substitutions/modifications based on** the BBlt-AV-A13I-L29P-A40K-F50T-V52A **variant (SEQ ID NO:601)** | | |
|---|---|---|
| **Amino Acid Substitutions in BBlt-AV** | **Synthetic Gene Sequence Encoding the BBlt-AV Variant** | **Amino Acid Sequence of the BBlt-AV Variant** |
| BBlt-AV-D1C-A13I-L29PA40K-F50T-V52A | GGATCCATGCGATGAGAGCTCTAAACCTTGT TGCGATCAATGCATTTGTACTAAATCAAACCC TCCACAATGTCGTTGTTCTGATATGCGTCCTA ATTCATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:414) | DPCDESSKPCCDQ CICTKSNPPQCRCS DMRPNSCHSACKS CKCYNLYGWTCTCA DITDFCYEPCKPSE (SEQ ID NO:611) |
| BBlt-AV-S4V-A13I-L29P-A40K-F50T-V52A | GGATCCAGACGATGAGGTTTCTAAACCTTGT TGCGATCAATGCATTTGTACTAAATCAAACCC TCCACAATGTCGTTGTTCTGATATGCGTCCTA ATTCATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:415) | DPDDEVSKPCCDQ CICTKSNPPQCRCS DMRPNSCHSACKS CKCYNLYGWTCTCA DITDFCYEPCKPSE (SEQ ID NO:612) |
| BBlt-AV-S5P-A13I-L29P-A40K-F50T-V52A | GGATCCAGACGATGAGAGCCCTAAACCTTGT TGCGATCAATGCATTTGTACTAAATCAAACCC TCCACAATGTCGTTGTTCTGATATGCGTCCTA ATAGCTGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:416) | DPDDESPKPCCDQ CICTKSNPPQCRCS DMRPNSCHSACKS CKCYNLYGWTCTCA DITDFCYEPCKPSE (SEQ ID NO:613) |
| BBIt-AV-Q11G-A13I-L29P-A40K-F50T-V52A | GGATCCAGACGATGAGAGCTCTAAACCTTGT TGCGATGGCTGCATTTGTACTAAATCAAACC CTCCACAATGTCGTTGTTCTGATATGCGTCCT AATTCATGTCATTCTGCATGCAAATCATGTAA ATGCTATAACCTTTACGGTTGGACATGTACAT GCGCAGATATCACTGACTTCTGCTATGAACC ATGTAAACCTTCTGAATAAAAGCTT (SEQ ID NO:417) | DPDDESSKPCCDG CICTKSNPPQCRCS DMRPNSCHSACKS CKCYNLYGWTCTCA DITDFCYEPCKPSE (SEQ ID NO:614) |
| BBlt-AV-A13L-L29PA40K-F50T-V52A | GGATCCAGACGATGAGAGCTCTAAACCTTGT TGCGATCAATGCCTTTGTACTAAATCAAACCC TCCACAATGTCGTTGTTCTGATATGCGTCCTA ATTCATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:418) | DPDDESSKPCCDQ CLCTKSNPPQCRCS DMRPNSCHSACKS CKCYNLYGWTCTCA DITDFCYEPCKPSE (SEQ ID NO:615) |
| BBlt-AV-A13I-S25R-L29PA40K-F50T-V52A | GGATCCAGACGATGAGAGCTCTAAACCTTGT TGCGATCAATGCATTTGTACTAAATCAAACCC TCCACAATGTCGTTGTAGAGATATGCGTCCT AATAGCTGTCATTCTGCATGCAAATCATGTAA | DPDDESSKPCCDQ CICTKSNPPQCRCR DMRPNSCHSACKS CKCYNLYGWTCTCA |
| | ATGCTATAACCTTTACGGTTGGACATGTACAT GCGCAGATATCACTGACTTCTGCTATGAACC ATGTAAACCTTCTGAATAAAAGCTT (SEQ ID NO:419) | DITDFCYEPCKPSE (SEQ ID NO:616) |
| BBlt-AV-A13I-S25R-L29P-S31A-A40K-F50T-V52A | GGATCCAGACGATGAGAGCTCTAAACCTTGT TGCGATCAATGCATTTGTACTAAATCAAACCC TCCACAATGTCGTTGTAGAGATATGCGTCCT AATGCTTGTCATTCTGCATGCAAATCATGTAA ATGCTATAACCTTTACGGTTGGACATGTACAT GCGCAGATATCACTGACTTCTGCTATGAACC ATGTAAACCTTCTGAATAAAAGCTT (SEQ ID NO:420) | DPDDESSKPCCDQ CICTKSNPPQCRCR DMRPNACHSACKS CKCYNLYGWTCTCA DITDFCYEPCKPSE (SEQ ID NO:617) |
| BBlt-AV-A13I-S25R-L29P-S31R-A40K-F50T-V52A | GGATCCAGACGATGAGAGCTCTAAACCTTGT TGCGATCAATGCATTTGTACTAAATCAAACCC TCCACAATGTCGTTGTAGAGATATGCGTCCT AATCGCTGTCATTCTGCATGCAAATCATGTAA ATGCTATAACCTTTACGGTTGGACATGTACAT GCGCAGATATCACTGACTTCTGCTATGAACC ATGTAAACCTTCTGAATAAAAGCTT (SEQ ID NO:421) | DPDDESSKPCCDQ CICTKSNPPQCRCR DMRPNRCHSACKS CKCYNLYGWTCTCA DITDFCYEPCKPSE (SEQ ID NO:618) |
| BBlt-AV-A13I-M27R-L29PA40K-F50T-V52A | GGATCCAGACGATGAGAGCTCTAAACCTTGT TGCGATCAATGCATTTGTACTAAATCAAACCC TCCACAATGTCGTTGTTCTGATAGACGTCCTA ATTCATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:422) | DPDDESSKPCCDQ CICTKSNPPQCRCS DRRPNSCHSACKSC KCYNLYGWTCTCAD ITDFCYEPCKPSE (SEQ ID NO:619) |
| BBlt-AV-A13I-L29K-A40K-F50T-V52A | GGATCCAGACGATGAGAGCTCTAAACCTTGT TGCGATCAATGCATTTGTACTAAATCAAACCC TCCACAATGTCGTTGTTCTGATATGCGTAAAA ATTCATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:423) | DPDDESSKPCCDQ CICTKSNPPQCRCS DMRKNSCHSACKS CKCYNLYGWTCTCA DITDFCYEPCKPSE (SEQ ID NO:620) |
| BBlt-AV-A13I-L29P-S31A-A40K-F50T-V52A | GGATCCAGACGATGAGAGCTCTAAACCTTGT TGCGATCAATGCATTTGTACTAAATCAAACCC TCCACAATGTCGTTGTTCTGATATGCGTCCTA ATGCTTGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:424) | DPDDESSKPCCDQ CICTKSNPPQCRCS DMRPNACHSACKS CKCYNLYGWTCTCA DITDFCYEPCKPSE (SEQ ID NO:621) |
| BBlt-AV-A13I-L29P-S31 R-A40K-F50T-V52A | GGATCCAGACGATGAGAGCTCTAAACCTTGT TGCGATCAATGCATTTGTACTAAATCAAACCC TCCACAATGTCGTTGTTCTGATATGCGTCCTA ATAGATGTCATTCTGCATGCAAATCATGTAAA TGCTATAACCTTTACGGTTGGACATGTACATG CGCAGATATCACTGACTTCTGCTATGAACCAT GTAAACCTTCTGAATAAAAGCTT (SEQ ID NO:425) | DPDDESSKPCCDQ CICTKSNPPQCRCS DMRPNRCHSACKS CKCYNLYGWTCTCA DITDFCYEPCKPSE (SEQ ID NO:622) |
| BBlt-AV-A13I-L29P-S38N-A40K-F50T-V52A | GGATCCAGACGATGAGAGCTCTAAACCTTGT TGCGATCAATGCATTTGTACTAAATCAAACCC TCCACAATGTCGTTGTTCTGATATGCGTCCTA ATAGCTGTCATTCTGCATGCAAAAACTGTAAA TGCTATAACCTTTACGGTTGGACATGTACATG CGCAGATATCACTGACTTCTGCTATGAACCAT GTAAACCTTCTGAATAAAAGCTT (SEQ ID NO:426) | DPDDESSKPCCDQ CICTKSNPPQCRCS DMRPNSCHSACKN CKCYNLYGWTCTCA DITDFCYEPCKPSE (SEQ ID NO:623) |
| BBlt-AV-A13I-L29P-A40K-F50K-V52A | GGATCCAGACGATGAGAGCTCTAAACCTTGT TGCGATCAATGCATTTGTACTAAATCAAACCC TCCACAATGTCGTTGTTCTGATATGCGTCCTA ATTCATGTCATTCTGCATGCAAATCATGTAAA TGCTATAACCTTTACGGTTGGACATGTAAATG CGCAGATATCACTGACTTCTGCTATGAACCAT GTAAACCTTCTGAATAAAAGCTT (SEQ ID NO:427) | DPDDESSKPCCDQ CICTKSNPPQCRCS DMRPNSCHSACKS CKCYNLYGWTCKC ADITDFCYEPCKPSE (SEQ ID NO:624) |
| BBlt-AV-A13I-L29P-A40K-F50T-V52T | GGATCCAGACGATGAGAGCTCTAAACCTTGT TGCGATCAATGCATTTGTACTAAATCAAACCC TCCACAATGTCGTTGTTCTGATATGCGTCCTA ATTCATGTCATTCTGCATGCAAATCATGTAAA | DPDDESSKPCCDQ CICTKSNPPQCRCS DMRPNSCHSACKS CKCYNLYGWTCTCT |
| | TGCTATAACCTTTACGGTTGGACATGTACATG CACAGATATCACTGACTTCTGCTATGAACCAT GTAAACCTTCTGAATAAAAGCTT (SEQ ID NO:428) | DITDFCYEPCKPSE (SEQ ID NO:625) |
| BBlt-AV-A13I-L29P-A40K-F50T-V52AS65E | GGATCCAGACGATGAGAGCTCTAAACCTTGT TGCGATCAATGCATTTGTACTAAATCAAACCC TCCACAATGTCGTTGTTCTGATATGCGTCCTA ATAGCTGTCATTCTGCATGCAAATCATGTAAA TGCTATAACCTTTACGGTTGGACATGTACATG CGCAGATATCACTGACTTCTGCTATGAACCAT GTAAACCTGAAGAATAAAAGCTT (SEQ ID NO:429) | DPDDESSKPCCDQ CICTKSNPPQCRCS DMRPNSCHSACKS CKCYNLYGWTCTCA DITDFCYEPCKPEE (SEQ ID NO:626) |

The BBI:BCE ratio of the modified variant BBlt-AVs was determined as described above, and the yield for each of the modified variant BBlt-AVs was calculated. The modified BBlt-AVs BBlt-AV-A13I-S25R-L29P-S31R-A40K-F50T-V52A (SEQ ID NO:618), BBlt-AV-A13I-S25R-L29P-S31A-A40K-F50T-V52A (SEQ ID NO:617), BBlt-AV-A13I-S25R-L29P-A40K-F50T-V52A (SEQ ID NO:616), BBlt-AV-A13I-L29P-S31R-A40K-F50T-V52A (SEQ ID NO:622) had significantly better yields after acid/heat treatment than quintuple BBlt-AV-A13I-L29P-A40K-F50T-V52A (SEQ ID NO:601), while BBlt-AV-A13I-L29P-A40K-F50K-V52A (SEQ ID NO:624), BBlT-AV-A13L-L29P-A40K-F50T-V52A (SEQ ID NO:615) and BBlt-AV-4D13 insert -A13I-L29P-A40K-F50T-V52A (SEQ ID NO:413) had slightly better yields than BBlt-AV-A13I-L29P-A40K-F50T-V52A (SEQ ID NO:601). The modified BBlt-AV-A13I-L29PA40K-F50T-V52A-S65E (SEQ ID NO:626) and BBlt-AV-A13I-L29P-S38N-A40K-F50T-V52A (SEQ ID NO:623) had similar yields as BBlt-AV-A13I-L29P-A40K-F50T-V52 (SEQ ID NO:624), BBlt-AV-A13I-M27R-L29P-A40K-F50T-V52A (SEQ ID NO:619), BBlt-AV-A13I-L29P-S31A-A40K-F50T-V52A (SEQ ID NO:621) and BBlT-AV-A13I-L29P-A40K-F50T-V52T (SEQ ID NO:625) somewhat less, while BBlt-AV-S4V-A13I-L29P-A40K-F50T-V52A (SEQ ID NO:612), BBlt-AV-Q11G-A13I-L29PA40K-F50T-V52A (SEQ ID NO:614), BBlt-AV-A13I-L29K-A40K-F50T-V52A (SEQ ID NO:620) and BBlt-AV-S5P-A13I-L29P-A40K-F50T-V52A (SEQ ID NO:611) all had significantly lower yields than BBlt-AV-A13I-L29P-A40K-F50T-V52A (SEQ ID NO:601), after acid-heat treatment. The yield of BBlt-AV-D1C-A13I-L29P-A40K-F50T-V52A (SEQ ID NO:611) in the absence of activation was also much lower than BBlt-AV-A131-L29P-A40K-F50T-V52A (SEQ ID NO:601). However, the yield after acid/heat treatment of BBlt-AV-D1C-A13I-L29P-A40K-F50T-V52A (SEQ ID NO:611) when not activated was about two fold higher than BBIt-AV-A13I-L29P-A40K-F50T-V52A (SEQ ID NO:601) when activated but similar to BBIt-AV-A13I-L29P-A40K-F50T-V52A (SEQ ID NO:601) when it was not activated. Thus, no substitutions were found that could produce high yields without activation.

### Generation of modified BBI-AVs containing eight amino acid substitutions: octuple variants

Following the testing of the quintuple-based modified BBI-AVs, additional modified variant BBIs (octuple variants) were generated to contain eight amino acid substitutions. The activity and purification yield of the octuple variants were determined as described above. Construction of the octuple modified BBIt-AVs was achieved as described above using the synthetic genes shown in Table 5.

**TABLE 5**

| **Octuple Modified Variant BBIt-AVs** | | |
|---|---|---|
| **Amino Acid Substitutions** | **Synthetic Gene Sequence Encoding the Octuple Variant** | **Amino Acid Sequence of the BBIt-AV Variant** |
| BBIt-AV-A13IS25R-M27A-L29P-S31A-A40H-F50K-V52T (KT8) | GGATCCAGACGATGAGAGCTCTAAACCTT GTTGCGATCAATGCATCTGTACAAAATCA AACCCTCCACAATGTCGTTGTAGAGATGC TCGTCCTAATGCATGTCATTCTGCATGCA AATCATGTCACTGCTATAACCTTTACGGTT GGACATGTAAATGCACAGACATCACTGAC TTCTGCTATGAACCATGTAAACCTTCTGAA TAAAAGCTT (SEQ ID NO:486) | DPDDESSKPCCDQCICTKSNP PQCRCRDARPNACHSACKSC HCYNLYGWTCKCTDITDFCYE PCKPSE (SEQ ID NO:627) |
| BBIt-AV-A13IS25K-M27A-L29R-S31E-A40K-F50Q-V52Q (QQ8) | GGATCCAGACGATGAGAGCTCTAAACCTT GTTGCGATCAATGCATCTGTACAAAATCA AACCCTCCACAATGTCGTTGTAAAGATGC TCGTAGAAATGAATGTCATTCTGCATGCA AATCATGTAAATGCTATAACCTTTACGGTT GGACATGTCAATGCCAAGACATCACTGAC TTCTGCTATGAACCATGTAAACCTTCTGAA TAAAAGCTT (SEQ ID NO:487) | DPDDESSKPCCDQCICTKSNP PQCRCKDARRNECHSACKSC KCYNLYGWTCQCQDITDFCYE PCKPSE (SEQ ID NO:628) |
| BBIt-AV-A13I-S25K-M27R-L29E-S31A-A40H-F50R-V52K (RK8) | GGATCCAGACGATGAGAGCTCTAAACCTT GTTGCGATCAATGCATCTGTACAAAATCA AACCCTCCACAATGTCGTTGTAAAGATAG ACGTGAAAATGCTTGTCATTCTGCATGCA AATCATGTCACTGCTATAACCTTTACGGTT GGACATGTAGATGCAAAGACATCACTGAC TTCTGCTATGAACCATGTAAACCTTCTGAA TAAAAGCTT (SEQ ID NO:488) | DPDDESSKPCCDQCICTKSNP PQCRCKDRRENACHSACKSC HCYNLYGWTCRCKDITDFCYE PCKPSE (SEQ ID NO:629) |
| BBIt-AV-A13IS25K-M27A-L29R-S31A-A40H-F50R-V52L (RL8) | GGATCCAGACGATGAGAGCTCTAAACCTT GTTGCGATCAATGCATCTGTACAAAATCA AACCCTCCACAATGTCGTTGTAAAGATGC TCGTAGAAATGCTTGTCATTCTGCATGCA AATCATGTCACTGCTATAACCTTTACGGTT GGACATGTAGATGCTTAGACATCACTGAC TTCTGCTATGAACCATGTAAACCTTCTGAA TAAAAGCTT (SEQ ID NO:489) | DPDDESSKPCCDQCICTKSNP PQCRCKDARRNACHSACKSC HCYNLYGWTCRCLDITDFCYE PCKPSE (SEQ ID NO:630) |
| BBIt-AV-A13IS25K-M27Q-L29P-S31 E-A40H-F50R-V52Q (RQ8) | GGATCCAGACGATGAGAGCTCTAAACCTT GTTGCGATCAATGCATCTGTACAAAATCA AACCCTCCACAATGTCGTTGTAAAGATCA ACGTCCTAATGAATGTCATTCTGCATGCA AATCATGTCACTGCTATAACCTTTACGGTT GGACATGTAGATGCCAAGACATCACTGAC TTCTGCTATGAACCATGTAAACCTTCTGAA TAAAAGCTT (SEQ ID NO:490) | DPDDESSKPCCDQCICTKSNP PQCRCKDQRPNECHSACKSC HCYNLYGWTCRCQDITDFCYE PCKPSE (SEQ ID NO:631) |

The best octuple variants selected in the shake flask screen were tested in the purification process essentially as described above, and including the following changes: 1) after growth, the culture was two fold diluted in glycine buffer (125 mM final concentration and pH adjusted to 9.0) instead of CHES buffer; 2) samples were activated with 2 mM 2-mercaptoethanol only (no sodium sulfite was added; 3) the acid precipitated fusion protein was resuspended in 100 ml of 125 mM glycine (rather than 350 ml of 40 mM glycine); and 4) the pellet collected after the acid/heat treatment was washed with 10 ml water, filtered, and this "washed pellet" filtrate was combined with the original filtrate. The final filtrates were concentrated and trimmed with Glu-BL as described above

The results show that octuple variant BBIt-AV molecules QQ8 (SEQ ID NO:628) and KT8 (SEQ ID NO:627) resulted in purification yields that were greater than that of the quintuple modified variant BBIt-AV-A13I-L29P-A40K-F50T-V52A variant (SEQ ID NO:601). The modified octuple variants BBIt-AV-A13I-S25K-M27A-L29R-S31A-A40H-F50R-V52L (RL8; SEQ ID NO:630), BBIt-AV-A13I-S25K-M27A-L29R-S31E-A40K-F50Q-V52Q (QQ8; SEQ ID NO:628) and BBIt-AV-A13I-S25R-M27A-L29P-S31A-A40H-F50K-V52T (KT8; SEQ ID NO:627) resulted in yields that were better than the wild-type sBBI molecule/protein (Figure 13).

### Example 13

### Improved production of BBI scaffolds carrying FGF5 or TGFβ1 binding peptides

In this example, the same amino acid substitutions that resulted in modified variant BBIt-AVs having improved trypsin inhibitory activity and/or purification yields (see Examples 7-13) were tested for improving the trypsin inhibitory activity and/or the purification yield of variant BBI scaffolds in which the chymotrypsin loop was replaced with either an FGF-5 or a TGFβ1 binding peptide.

### Generation of variant and modified variant BBI-FGF-5 proteins:

Modified variant BBIt-FGF-5 protease inhibitors comprising the combination of substitutions A13I-L29P-F50T-V52A (all with alanine at position 40) and either the FGF-5 binding peptide MM007: RTQPYPL (SEQ ID NO:670), or FGF-5 binding peptide FGFps2: TWIDSTP (SEQ ID NO:671) in place of the chymotrypsin loop, were constructed by ligating a synthetic gene into the *Bam*HI and *Hin*dIII sites of the vector p2JMagk103-Ink2-BBIt-AV. The amino acid sequences and DNA sequences of the synthetic genes encoding the resulting modified variant MM007-Q-BBIt-FGF-5 (SEQ ID NO:432; SEQ ID NO:433) and the modified variant FGFps2-Q-BBIt-FGF-5, respectively (SEQ ID NO:434 SEQ ID NO:435) are as follows.
MM007-Q-BBIt-FGF-5 synthetic gene 
GGATCCAGACGATGAGAGCTCTAAACCTTGTTGCGATCAATGCATTTGTACTAA
ATCAAATCCTCCACAATGTCGTTGTTCTGATATGCGTCCTAATAGCTGTCATTCT
GCATGCAAATCATGTGCTTGCCGTACTCAACCATACCCTCTTTGTACATGCGCA
 
GACATCACTGACTTCTGCTATGAACCATGTAAACCATCTGAATAAAAGCTT (SEQ
ID NO:433)
 MM007-Q-BBIt-FGF-5 protein 
DPDDESSKPCCDQCICTKSNPPQCRCSDMRPNSCHSACKSCACRTQPYPLCTCA
DITDFCYEPCKPSE (SEQ ID NO:432)
 FGFps2-Q-BBIt-FGF-5

GGATCCAGACGATGAGAGCTCTAAACCTTGTTGCGATCAATGCATTTGTACTAA
ATCAAATCCTCCACAATGTCGTTGTTCTGATATGCGTCCTAATAGCTGTCATTCT
GCATGCAAATCATGTGCTTGCACTTGGATTGATTCAACACCATGTACATGCGCA
GACATCACTGACTTCTGCTATGAACCATGTAAACCATCTGAATAAAAGCTT (SEQ
ID NO:435)

DPDDESSKPCCDQCICTKSNPPQCRCSDMRPNSCHSACKSCACTWIDSTPCTCAD
ITDFCYEPCKPSE (SEQ ID NO:434)

### Generation of variant and modified variant BBI-TGFβ proteins:

The chymotrypsin inhibitory loop of the BBIt-AV of SEQ ID NO:187 was replaced with the TGFβ1 binding peptide PEN3: CLCPENINVLPCN (SEQ ID NO:436), the TGFβ1 binding peptide MM021W: CICKHNVDWLCF (SEQ ID NO:437) or the TGFβ1 binding peptide WTQ: CICWTQHIHNCF (SEQ ID NO:438) to generate variant BBPIs according to the method described in Example 2, as follows.
Oligonucleotide pairs were used to make cassettes to replace the FGFhl binding peptide in the chymotrypsin reactive site loop encoded by the p2JM103-Ink2-2BBI-FGFhl expression vector with the TGFβ1 binding peptides, PEN3, MM021W or WTQ. The p2JM103-Ink2-2BBI-FGFhl was constructed using the primers (SEQ ID NO:91 and SEQ ID NO:92) and QuikChange® method as described in Example 2. The TGFβ binding cassettes were ligated into the *Sph*I and *Sal*I restriction sites in the vector p2JM103Ink2-2BBI-FGFhl to construct the variant BBI molecules PEN3-BBIt-TGFβ, MM021W-BBIt-TGFβ and WTQ-BBIt-TGFβ, respectively. The DNA sequences of the oligonucleotides used to make the cassettes are shown below.
PEN3 (2^{nd} loop)
CAAAAGCTGTCTTTGTCCTGAAAATATTAACGTTCTTCCTTGTAACTGCG (SEQ ID NO:439)
And 
TCGACGCAGTTACAAGGAAGAACGTTAATATTTTCAGGACAAAGACAGCTTTTG
CATG (SEQ ID NO:440)
 MM021W (2^{nd} loop)
CAAATCATGCATTTGTAAACACAACGTAGATTGGTTATGTTTTTGCG(SEQID NO:129)
And
TCGACGCAAAAACATAACCAATCTACGTTGTGTTTACAAATGCATGATTTGCATG (SEQ ID NO:130)
WTQ (2^{nd} loop)
CAAATCATGCATTTGTTGGACACAACATATCCACAACTGTTTTTGCG (SEQ ID NO:441)
And
TCGACGCAAAAACAGTTGTGGATATGTTGTGTCCAACAAATGCATGATTTGCATG (SEQ ID NO:442)

In addition, modified variant BBPI-TGFβ1 variants comprising the combination of substitutions A13I-L29P-V52A (all also have alanine at position 40 and threonine at position 50) and the TGFβ1 binding peptide PEN3-Q: CPENINVLPC (SEQ ID NO:672), MM021W-Q: CKHNVDWLC (SEQ ID NO:673) or WTQ-Q: CWTQHIHNC (SEQ ID NO:674) in place of the chymotrypsin loop, were constructed by ligating a synthetic gene into the *Bam*HI and *Hin*dIII sites of the vector p2JMagk103-Ink2-BBIt-AV. The amino acid sequences and DNA sequences of the synthetic genes encoding the resulting modified variant PEN3-Q -BBIt-TGFβ1 (SEQ ID NO:443; SEQ ID NO:444), the modified variant MM021W-BBI-TGF β1 (SEQ ID NO:445; SEQ ID NO:446), and the modified variant WTQ -BBI-TGFβ1 (SEQ ID NO:447; SEQ ID NO:448) are as follows.
PEN3-Q-BBIt-TGFβ1 (SEQ ID NO:443) 
DPDDESSKPCCDQCICTKSNPPQCRCSDMRPNSCHSACKSCACPENINVLPCTCA
DITDFCYEPCKPSE
 PEN3-Q-BBIt-TGFβ1 (SEQ ID NO:444) 
GGATCCAGACGATGAGAGCTCTAAACCTTGTTGCGATCAATGCATTTGTACTAA
ATCAAATCCTCCACAATGTCGTTGTTCTGATATGCGTCCTAATAGCTGTCATTCT
GCATGCAAATCATGTGCTTGCCCAGAAAACATCAACGTTCTTCCTTGTACATGC
GCAGACATCACTGACTTCTGCTATGAACCATGTAAACCATCTGAATAAAGCTT
 MM021W-Q-BBIt-TGFβ1 (SEQ ID NO:445) 
DPDDESSKPCCDQCICTKSNPPQCRCSDMRPNSCHSACKSCACKHNVDWLCTCA
DITDFCYEPCKPSE
 MM021W-Q-BBIt-TGFβ1 (SEQ ID NO:446) 
GGATCCAGACGATGAGAGCTCTAAACCTTGTTGCGATCAATGCATTTGTACTAA
ATCAAATCCTCCACAATGTCGTTGTTCTGATATGCGTCCTAATAGCTGTCATTCT
GCATGCAAATCATGTGCTTGCAAACATAACGTTGATTGGCTTTGTACATGCGCA
GACATCACTGACTTCTGCTATGAACCATGTAAACCATCTGAATAAAAGCTT
 WTQ-Q-BBIt-TGFβ1 (SEQ ID NO:447) 
DPDDESSKPCCDQCICTKSNPPQCRCSDMRPNSCHSACKSCACKHNVDWLCTCA
DITDFCYEPCKPSE
 WTQ-Q-BBIt-TGFβ1 (SEQ ID NO:448)

GGATCCAGACGATGAGAGCTCTAAACCTTGTTGCGATCAATGCATTTGTACTAA
ATCAAATCCTCCACAATGTCGTTGTTCTGATATGCGTCCTAATAGCTGTCATTCT
GCATGCAAATCATGTGCTTGCTGGACACAACATATCCACAACTGTACATGCGCA
GACATCACTGACTTCTGCTATGAACCATGTAAACCATCTGAATAAAAGCTT

The ten vectors comprising the variant and modified variant BBIs containing an FGF-5 or a TGFβ binding peptide were used to transform *B. subtilis* BG6006 host cells. The transformants were grown and the engineered BBIs produced by the host cells were tested for trypsin inhibitory activities and production yields as described above.

Figure 14 shows the trypsin inhibitory activity of the unmodified variant parent MM007-PT-BBIt-FGF-5 (SEQ ID NO:678): 
(DDESSKPCCDQCACTKSNPPQCRCSDMRLNSCHSACKSCAC RTQPYPL
CFCVDITDFCYEPCKPSE; SEQ ID NO:678),
 the unmodified variant parent FGFps2-PT-BBIt-FGF5 (SEQ ID NO:679): 
(DDESSKPCCDQCACTKSNPPQCRCSDMRLNSCHSACKSCAC TWIDSTP
CFCVDITDFCYEPCKPSE; SEQ ID NO:679),
 the unmodified variant parent PEN3-PT-BBIt-TGFβ (SEQ ID NO:680):
(DDESSKPCCDQCACTKSNPPQCRCSDMRLNSCHSACKSCAC PENINVLP CFCVDITDFCYEPCKPSE; SEQ ID NO:680),
the unmodified variant parent WTQ-PT- TGFβ (SEQ ID NO:681):
(DDESSKPCCDQCACTKSNPPQCRCSDMRLNSCHSACKSCAC WTQHIHNCF CFCVDITDFCYEPCKPSE; SEQ ID NO:681),
and the unmodified variant parent MM0021W-PT-TGFβ (SEQ ID NO:682): (DDESSKPCCDQCACTKSNPPQCRCSDMRLNSCHSACKSCAC KHNVDWL CFCVDITDFCYEPCKPSE; SEQ ID NO:682), (designated PT in Figure 14) and the corresponding modified variant MM007-Q-BBIt-FGF-5 (SEQ ID NO:432), the modified variant FGFps2-Q-BBIt-FGF5 (SEQ ID NO:434), the modified variant PEN3-Q-BBIt-TGFβ1 (SEQ ID NO:443), the modified variant WTQ-Q-BBIt- TGFβ1 (SEQ ID NO:445), and the modified variant MM0021W-Q-BBIt-TGFβ1 (SEQ ID NO:447) proteins (designated Q in Figure 14) measured after growth, after activation with 2-mercaptoethanol and after acid/heat treatment. The data show that the combination of the amino acids 13I-29P-40A- 50T-52A when present in the BBIt scaffold carrying FGF-5 or TGFβ1 binding peptides improve the trypsin inhibitory activity after growth and activation, and the production yield after acid/heat treatment when compared to the corresponding parent BBI scaffold carrying the binding peptides.

The data also evidences that substitutions that were shown to improve the trypsin inhibitory activity and/or purification yield of a modified variant BBI in which the chymotrypsin loop had been replaced with a VEGF binding peptide are not specific to BBI-AVs as the same substitutions improve the trypsin inhibitory activity and/or the production yield of BBIs in which the chymotrypsin loop was replaced by other binding peptides *e.g.* FGF-5 binidng peptides and TGFβ1 binding peptides.

Therefore, the amino acid substitutions made in the BBI scaffold that increase the production yield and/or the trypsin activity of BBIt-AV are expected to be generally applicable to BBI scaffolds carrying other binding peptides.

### Example 14

### Performance of Bowman Birk Inhibtor scaffolds

In this example, the ability to predict the effect of amino acid substitutions on the trypsin activity and/or production yield of a variant Bowman Birk Inhibitor e.g. BBIt-AV (SEQ ID NO:187) was tested by substituting amino acids that are not conserved across Bowman Birk Inhibitor sequences from *Dolichos biflorus* (BBdb, Acc. No. AAK97765; SEQ ID NO:449)
PSESSKPCCDQCACTKSIPPQCRCTDVRLNSCHSACSSCVCTFSIPAQCVCVDMK DFCYEPCK (SEQ ID NO:449), from *Glycine max* (soybean) protease inhibitor IV or D-II (BBsb3, Acc. No. P01064; SEQ ID NO: 450)
DDEYSKPCCDLCMCTRSMPPQCSCEDIRLNSCHSDCKSCMCTRSQPGQCRCLDT NDFCYKPCKSRDD (SEQ ID NO:450), and from *Torresea (Amburana) cearensis* (BBtc, Acc. No. P83283; SEQ ID NO:451)
SSKWEACCDRCACTKSIPPQCHCADIRLNSCHSACESCACTHSIPAQCRCFDITDF CYKPCSG (SEQ ID NO:451), into the BBIt-AV scaffold.

Variant Bowman Birk Inhibitors were generated by replacing the second protease inhibitory loop of the Bowman Birk Inhibitor from *Dolichos biflorus* (BBdb, Acc. No. AAK97765; SEQ ID NO:449), the second protease inhibitory loop of the Bowman Birk Inhibitor from *Glycine max* (soybean) protease inhibitor IV or D-II (BBsb3, Acc. No. P01064; SEQ ID NO: 450) and the second protease inhibitory loop of the Bowman Birk Inhibitor from *Torresea (Amburana) cearensis* (BBtc, Acc. No. P83283; SEQ ID NO:451) with the VEGF binding peptide CK37281 (ACYNLYGWTC; SEQ ID NO:9) to generate the corresponding variant BBdb-AV (SEQ ID NO:452), BBsb3-AV (SEQ ID NO:453), and BBtc-AV (SEQ ID NO:454).

The sequences were aligned, as shown in Figure 15, and amino acids at positions equivalent to positions 11, 13, 18, 23, 25, 27, 35, 37, 52, 54 and 55 of the BBPI of SEQ ID NO:187 (BBIt-AV) were identified as not being conserved across the four inhibitors, and were chosen to modify the BBIt-AV scaffold.

The synthetic genes encoding BBdb-AV (SEQ ID NO:455) and BBsb3-AV (SEQ ID NO:456) were ligated into the p2JMagk103-Ink2-BBIt-AV as *Bam*HI*-Hin*dIII fragments. For cloning the BBtc-AV gene, the gene was first digested with *Bfr*I, the overhang filled in with T4 DNA polymerase and dNTP's, the DNA purified, and then digested with *Bam*HI. This fragment was ligated into the p2JMagk103-Ink2-BBIt-AV vector that had been digested with *Hin*dIII, the overhang filled in with T4 DNA polymerase and dNTP's, the DNA purified, and then digested with *Bam*HI. The DNA sequences and the corresponding amino acid sequences of the variant PIs are as follows.
BBdb-AV 
GGATCCTTCTGAGAGCTCTAAACCATGCTGTGATCAATGCGCTTGTACAAAATCT
ATCCCTCCACAATGCCGTTGCACTGATGTTCGTCTTAACTCATGTCACTCTGCAT
GCAGCTCATGCGCTTGTTACAACCTTTACGGTTGGACATGCGTTTGCGTCGACA
TGAAAGATTTCTGCTACGAACCTTGTAAATAAAAGCTT (SEQ ID NO:455)
 BBdb-AV (SEQ ID NO:452) 
DPSESSKPCCDQCACTKSIPPQCRCTDVRLNSCHSACSSCACYNLYGWTCVCVD
MKDFCYEPCK
 BBsb3-AV 
GGATCCAGATGACGAATACTCTAAACCTTGCTGTGATCTTTGCATGTGTACACGT
TCTATGCCACCTCAATGCTCATGTGAAGACATCCGCCTTAACTCTTGCCACTCA
GATTGCAAAAGCTGCGCTTGTTACAACCTTTACGGTTGGACATGCCGTTGTTTA
GATACTAACGATTTCTGCTACAAACCTTGCAAATCTCGTGATGATTAAAGCTT
(SEQ ID NO:456)
 BBsb3-AV AA (SEQ ID NO:453) 
DPDDEYSKPCCDLCMCTRSMPPQCSCEDIRLNSCHSDCKSCACYNLYGWTCRCL
DTNDFCYKPCKSRDD
 BBtc-AV 
GGATCCTTCTTCAAAATGGGAAGCTTGCTGTGATCGTTGCGCATGCACAAAATC
TATCCCTCCACAATGCCACTGCGCTGATATCCGTCTTAACTCATGCCATTCTGCA
TGCGAAAGCTGCGCTTGTTACAACCTTTACGGTTGGACATGCCGTTGCTTCGAT
ATCACTGATTTCTGTTACAAACCTTGCTCTGGCTAAAAGCTTAAAGGAGACCGT
TAATCTAAAATCATTATTTGAGGCCCGAGCTTAAAGCTTAAG (SEQ ID NO:457)
 BBtc-AV AA (SEQ ID NO:454)

DPSSKWEACCDRCACTKSIPPQCHCADIRLNSCHSACESCACYNLYGWTCRCFDI
TDFCYKPCSG

The three expression vectors comprising the variant inhibitor sequences were used to transform *B. subtilis* host cells BG6006.

Typically, substitutions at the N-terminal and C-terminal ends, which are outside of the first disulfide bond (C8-C62), have small effects on the trypsin inhibitory activity and/or purification yield. Thus, only substitutions of amino acids at positions between C8 and C62 were studied. The substitutions targeted in this study are shown in Figure 15 (bold and underlined).

The individual amino acid substitutions at positions 11, 13, 18, 23, 25, 27, 35, 37, 50, 52, 54, 55, and 60 in BBIt-AV (SEQ ID NO:187) were constructed as described in Examples 8 and 10. These substitutions were analyzed by screening in microtiter plates (with and without reducing agent) as described in Examples 9 and 10. The results are summarized in Table 6 below

**TABLE 6**

| **Effect of single amino acid substitutions present in the BBtc-AV, BBdb-AV and BBsb3-AV scaffolds on the trypsin activity of the modified variant BBIt-AV** | | | |
|---|---|---|---|
| **Position and amino acid present in the BBIt-AV scaffold** | **Amino acid present in the alternative scaffold and relative activity of the amino acid substitution** | | |
| | **BBItc-AV (SEQ ID NO:454)** | **BBIdb-AV (SEQ ID NO:452)** | **BBIsb3-AV (SEQ ID NO:453)** |
| Q11 | R (+/-) | wt* | L (+/-)** |
| A13 | wt | wt | M (+)*** |
| N18 | I (+++) | I (+++) | M (+/-) |
| R23 | H (-) | wt | S (---) |
| S25 | A (+) | T (+/-) | E (---) |
| M27 | I (+/-) | V (+/-) | I (+/-) |
| A35 | wt | wt | D (+/-) |
| K37 | E (+/-) | S (+/-) | wt |
| F50 | R (++) | V (+) | R (++) |
| V52 | F (+/-) | wt | L (+) |
| I54 | wt | M (-) | T (-)**** |
| T55 | wt | K (-) | N (+/-) |
| E60 | K (+/-) | wt | K (+/-) |

| | | | |
|---|---|---|---|
| * wt indicates that the amino acid at this position is the same as in the BBIt-AV scaffold (SEQ ID NO:187) ** (+/-) indicates that the relative BBI:BCE activity ratio in the presence of reducing agent is not significantly different than that of BBIt-AV. *** (+) indicates that the the relative BBI:BCE activity ratio in the presence of reducing agent is greater than that of BBIt-AV; the greater the number of "+", the greater the difference. **** (-)indicates that the the relative BBI:BCE activity ratio in the presence of reducing agent is less than that of BBIt-AV; the greater the number of "-", the greater the difference. | | | |

Assuming the effects of the amino acid substitutions are additive, the data provided in Table 6 predicts that the variant BBItc-AV would perform significantly better than BBIt-AV, while BBIsb3-AV, should perform significantly worse than BBIt-AV. In addition, one would predict that BBIdb-AV would perform somewhat better than BBIt-AV in terms of activation with reducing agent.

To test these predictions, the trypsin activity and production yield of BBIdb-AV, BBIsb3-AV and BBItc-AV were tested using the Shake Flask Screen described in Example 11 and their activity compared to that of the unmodified variant BBIt-AV.

The data shown in Figure 16 show that, as predicted, BBItc-AV had the highest trypsin inhibitory activity after activation, and BBIdb-AV inhibitory activity was also greater than that of the BBIt-AV inhibitor but less than that of the BBItc-AV inhibitor. In addition, and as predicted, BBIsb3-AV had very low trypsin inhibitory activity and did not activate with reducing agent (inhibitory activity was too small to be shown in Figure 16). Furthermore, not only did BBItc-AV and BBIdb-AV have higher inhibitory activities after activation than BBIt-AV, they also had higher inhibitory activities after acid/heat treatment indicating improved production yields.

Therefore, the results indicate that activity data obtained when testing single amino acid substitutions in BBIt-AV can be used to predict the performance of other Bowman Birk Inhibitors when used as scaffolds with binding peptides replacing the second protease inhibitory loop

### Example 15

### Binding of modified variant BBIs to target proteins

In this example, the capacity of variant peptides to retain their ability to bind the corresponding target proteins was tested when the variant peptides were grafted into a modified Bowman Birk Inhibitor scaffold to replace the chymotrypsin loop.

The construction of the expression vector for BBI is described in Example 1 and the constructions of vectors for variant BBIs containing the VEGF-binding peptide CK37281 (SEQ ID NO:9), the FGF-5-binding peptide MM007 (SEQ ID NO:430) or the FGF-5-binding peptide FGF5ps2 (SEQ ID NO:431) in place of the chymotrypsin inhibitory sequence are described in Example 13 above. The method for the construction of expression vectors encoding for the modified variant BBIt-AVs having various combinations of amino acid substitution is described in Example 12 and the method for constructing the FGF5 and TGFβ-binding BBIs is described in Example 13.

VEGF-binding peptides named VegK (KYYLYWW ; SEQ ID NO:458), VegT (TLWKSYW ; SEQ ID NO:459) and VegKD (KYDLYWW ;SEQ ID NO: 460) were introduced into the chymotrypsin inhibitory loop by ligating oligonucleotide cassettes into the *Sph*I and *Sal*I sites of p2JM103-Ink2-2BBIck81. The sequences of the oligonucleotides used to generate the modified variant VEGK and VEGT BBPIs are shown below.
VegK:
CAAATCTTGCGCATGTAAATATTACCTTTACTGGTGGTGTTTTTGCG (SEQ ID NO:461)
And 
TCGACGCAAAAACACCACCAGTAAAGGTAATATTTACATGCGCAAGATTTGCAT
G (SEQ ID NO:462)
 BBIT-VEGK 
DPDDESSKPCCDQCICTKSNPPQCRCRDARPNACHSACKSCACKYYLYWWCKCT
DITDFCYEPCKPSE (SEQ ID NO:640)
 VegT:
CAAATCTTGCGCGTGCACACTTTGGAAATCTTACTGGTGTTTTTGCG (SEQ ID NO:463) And 
TCGACGCAAAAACACCAGTAAGATTTCCAAAGTGTGCACGCGCAAGATTTGCAT
G (SEQ ID NO:464)
 BBIt-VEGT 
DPDDESSKPCCDQCICTKSNPPQCRCRDARPNACHSACKSCAC TLWKSYW
CKCTDITDFCYEPCKPSE (SEQ ID NO:641)
 VegKD:
CAAATCTTGCATCTGTAAATATGATCTTTACTGGTGGTGTTTTTGCG (SEQ ID NO:465)
And
TCGACGCAAAAACACCACCAGTAAAGATCATATTTACAGATGCAAGATTTGCATG (SEQ ID NO:466)

The VEGF-binding peptide named VegKD was introduced into the variant BBI-A13I-S25K-L29P-V52K by ligating a synthetic gene (VegKD-Q) into the *Bam*HI and *Hin*dIII sites of p2JMagk103-Ink2-2BBIck81. The sequence of the synthetic gene is shown and encoded BBPI are shown below.
VegKD-Q:

GGATCCAGACGATGAGAGCTCTAAACCTTGTTGCGATCAATGCATCTGTACAAA
ATCAAACCCTCCACAATGTCGTTGTAAAGATATGCGTCCTAATAGCTGTCATTCT
GCATGCAAATCATGTATCTGCAAATATGACCTTTACTGGTGGTGTTTCTGCAAAG
ACATCACTGACTTCTGCTATGAACCATGTAAACCTTCTGAATAAAAGCTT (SEQ
ID NO:467)

DPDDESSKPCCDQCICTKSNPPQCRCRDARPNACHSACKSCACKYDLYWWCKCT
DITDFCYEPCKPSE (SEQ ID NO:642)

The VEGF-binding peptides V1 (SKHSQIT; SEQ ID NO:468), V2 (KTNPSGS; SEQ ID NO:469), V3 (RPTGHSL; SEQ ID NO:470), V4 (KHSAKAE; SEQ ID NO:471), V5 (KPSSASS; SEQ ID NO:472) and V6 (PVTKRVH;SEQ ID NO:473), and the TNF - binding peptides T1 (RYWQDIP; SEQ ID NO:474), T2 (APEPILA; SEQ ID NO:475) and T3 (DMIMVSI; SEQ ID NO:476), were introduced into the chymotrypsin inhibitory loop of a modified BBIt containing amino acid substitutions A13I-S25R-M27A-L29P-S31A-I40A-F50K-V52T by ligating synthetic genes into the *Bam*HI and *Hin*dIII sites of p2JMagk103-Ink2-2BBIck81. The DNA sequences of the synthetic genes encoding the resulting modified BBIs are shown below.
BBIt-AV-V1: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAGCAAACACTCTCAAATTACTTGTAAATGCACAG
ACATCACTGACTTCTGCTATGAACCATGTAAACCTTCTGAATAAAGCTT (SEQ
ID NO:477)
 
DPDDESSKPCCDQCICTKSNPPQCRCRDARPNACHSACKSCACSKHSQITCKCTDI
TDFCYEPCKPSE (SEQ ID NO:491)
 BBIt-AV-V2: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAAAACAAACCCAAGCGGTTCTTGTAAATGCACA
GACATCACTGACTTCTGCTATGAACCATGTAAACCTTCTGAATAAAGCTT (SEQ
ID NO:478)
 
DPDDESSKPCCDQCICTKSNPPQCRCRDARPNACHSACKSCACKTNPSGSCKCT
DITDFCYEPCKPSE (SEQ ID NO:632)
 BBIt-AV-V3: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAGACCAACTGGTCACAGCCTTTGTAAATGCACA
GACATCACTGACTTCTGCTATGAACCATGTAAACCTTCTGAATAAAGCTT (SEQ
ID NO:479)
 
DPDDESSKPCCDQCICTKSNPPQCRCRDARPNACHSACKSCACRPTGHSLCKCT
DITDFCYEPCKPSE (SEQ ID NO:633)
 BBIt-AV-V4: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAAACACAGCGCTAAAGCAGAATGTAAATGCACA
GACATCACTGACTTCTGCTATGAACCATGTAAACCTTCTGAATAAAGCTT (SEQ
ID NO:480)
 
DPDDESSKPCCDQCICTKSNPPQCRCRDARPNACHSACKSCACKHSAKAECKCT
DITDFCYEPCKPSE (SEQ ID NO:634)
 BBIt-AV-V5: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAAACCAAGCTCTGCTTCATCTTGTAAATGCACAG
 
ACATCACTGACTTCTGCTATGAACCATGTAAACCTTCTGAATAAAAGCTT (SEQ
ID NO:481)
 
DPDDESSKPCCDQCICTKSNPPQCRCRDARPNACHSACKSCACKPSSASSCKCTD
ITDFCYEPCKPSE (SEQ ID NO:635)
 BBIt-AV-V6: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCCCAGTTACTAAAAGAGTACACTGTAAATGCACA
GACATCACTGACTTCTGCTATGAACCATGTAAACCTTCTGAATAAAGCTT (SEQ
ID NO:482)
 
DPDDESSKPCCDQCICTKSNPPQCRCRDARPNACHSACKSCACPVTKRVHCKCT
DITDFCYEPCKPSE (SEQ ID NO:636)
 BBIt-TNF-T1: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCAGATACTGGCAAGATATTCCATGTAAATGCACA
GACATCACTGACTTCTGCTATGAACCATGTAAACCTTCTGAATAAAGCTT (SEQ
DINO:483)
 
DPDDESSKPCCDQCICTKSNPPQCRCRDARPNACHSACKSCACRYWQDIPCKCT
DITDFCYEPCKPSE (SEQ ID NO;637)
 BBIt-TNF-T2: 
GGATCCAGACGATGAGAGCTCTAAACCTTGTTGCGATCAATGCATCTGTACAAA
ATCAAACCCTCCACAATGTCGTTGTAGAGATGCTCGTCCTAATGCATGTCATTCT
GCATGCAAATCATGTGCTTGCGCACCAGAACCTATTCTTGCTTGTAAATGCACA
GACATCACTGACTTCTGCTATGAACCATGTAAACCTTCTGAATAAAGCTT (SEQ
ID NO:484)
 
DPDDESSKPCCDQCICTKSNPPQCRCRDARPNACHSACKSCACAPEPILACKCTDI
TDFCYEPCKPSE (SEQ ID NO:638)
 BBIt-TNF-T3:

GGATCCAGACGATGAGaAGCTCTAAACCTTGTTGCGATCAATGCATCTGTACAA
AATCAAACCCTCCACAATGTCGTTGTAGAGATGCTCGTCCTAATGCATGTCATTC
TGCATGCAAATCATGTGCTTGCGATATGATTATGGTTAGCATCTGTAAATGCACA
GACATCACTGACTTCTGCTATGAACCATGTAAACCTTCTGAATAAAGCTT (SEQ
ID NO:485)

DPDDESSKPCCDQCICTKSNPPQCRCRDARPNACHSACKSCACDMIMVSICKCTDI
TDFCYEPCKPSE (SEQ ID NO:639)

The resulting vectors were used to transform *B. subtilis* BG6006 host cells. Cultures were grown in 500 ml of MBD medium and the BBI species were purified essentially as described above and by Vogtentanz et al., 2007 (Protein Expr Purif 55:40-52 [2007]). BioVeris binding assays were performed essentially as outlined in Example 5 and by the manufacturer. Binding of the modified variant BBIs to their respective target proteins was measured using a BioVeris® binding assay, which is a competition assay that determines the binding of the modified variant BBIs to its target protein (e.g. VEGF, FGF5, TGFβ and TNFα) in the presence or absence of a labeled competitor. The competitor can be a labeled monoclonal antibody raised against the target protein or a labeled native receptor of the target protein. The electrochemiluminesent binding assays were performed essentially as described in Example 5. The apparent IC50s were determined from binding data by determining the concentration of the BBI species needed to reduce the BioVeris signal by one half. BBI scaffolds without the target binding peptide in the chymotrypsin inhibitory loop were used as negative controls.

The results are summarized in Table 7 below.

**TABLE 7**

| **Binding activity of modified variant Bowman Birk Inhibitors in which the chymotrypsin loop was replaced by a VEGF-, FGF-5-, TGFβ- or TNFα-binding variant peptide** | | | | | |
|---|---|---|---|---|---|
| **Target Protein** | **Variant Peptide Name^{•}** | **Variant Peptide Sequence^{▲}** | **Bowman Birk Inhibitor Scaffold** | **Resulting Modified Variant Bowman Birk Inhibitor** | **BioVeris Binding*** |
| VEGF | CK37281 | YNLYGWT (SEQ ID NO:9) | BBIt | BBIt-AV of SEQ ID NO:187 | +++ |
| VEGF | CK37281 | YNLYGWT (SEQ ID NO:9) | BBIt-A13I-A40K-F50T-V52A | BBIt-AV of SEQ ID NO:602 | +++ |
| VEGF | CK37281 | YNLYGWT (SEQ ID NO:9) | BBIt-A13I-L29P-A40K-F50T-V52A | BBIt-AV of SEQ ID NO:601 | +++ |
| VEGF | CK37281 | YNLYGWT (SEQ ID NO:9) | BBIt-A13I-S25R-M27A-L29P-S31A-A40H-F50K-V52T (KT8) | BBIt-AV of SEQ ID NO:627 | +++ |
| VEGF | CK37281 | YNLYGWT (SEQ ID NO:9) | BBIt-A13I-S25K-M27A-L29R-S31E-A40K-F50Q-V52Q (QQ8) | BBIt-AV of SEQ ID NO:628 | +++ |
| VEGF | CK37281 | YNLYGWT (SEQ ID NO:9) | BBIt-A13I-S25K-M27A-L29R-S31A-A40H-F50R-V52L (RL8) | BBIt-AV of SEQ ID NO:630 | +++ |
| VEGF | CK37281 | YNLYGWT (SEQ ID NO:9) | BBdb | BBdb-AV SEQ ID NO:452 | +++ |
| VEGF | CK37281 | YNLYGWT (SEQ ID NO:9) | BBtc | BBtc-AV SEQ ID NO:454 | +++ |
| VEGF | Veg K | KYYLYWW (SEQ ID NO:458) | BBIt | BBIt-AV of SEQ ID NO:640 | +++ |
| VEGF | Veg T | TLWKSYW (SEQ ID NO:459) | BBIt | BBIt-AV of SEQ ID NO:641 | +++ |
| VEGF | Veg KD | KYDLYWW (SEQ ID NO:460) | BBIt | BBIt-AV of SEQ ID NO:642 | +++ |
| VEGF | Veg KD | KYDLYWW (SEQ ID NO:460) | BBIt-A13I-S25K-L29P-V52K | BBIt-AV of SEQ ID NO:643 | +++ |
| VEGF | V1 | SKHSQIT (SEQ ID NO:468) | BBIt-A13I-S25R-M27A-L29P-S31A-F50K-V52T | BBIt-AV-V1 of SEQ ID NO:491 | ++ |
| VEGF | V2 | KTNPSGS (SEQ ID NO:469) | BBIt-A13I-S25R-M27A-L29P-S31A-F50K-V52T | BBIt-AV-V2 of SEQ ID NO:632 | +++ |
| VEGF | V3 | RPTGHSL (SEQ ID NO:470) | BBIt-A13I-S25R-M27A-L29P-S31A-F50K-V52T | BBIt-AV-V3 of SEQ ID NO:633 | ++ |
| VEGF | V4 | KHSAKAE (SEQ ID NO:471) | BBIt-A13I-S25R-M27A-L29P-S31A-F50K-V52T | BBIt-AV-V4 of SEQ ID NO:634 | +++ |
| VEGF | V5 | KPSSASS (SEQ ID NO:472) | BBIt-A13I-S25R-M27A-L29P-S31A-I40A-F50K-V52T | BBIt-AV-V5 of SEQ ID NO:635 | ++ |
| VEGF | V6 | PVTKRVH (SEQ ID NO:473) | BBIt-A13I-S25R-M27A-L29P-S31A-F50K-V52T | BBIt-AV-V6 of SEQ ID NO:636 | +++ |
| VEGF | Wild-type negative control for VEGF binding | ALSYPAQ (SEQ ID NO:675) | BBI | sBBI SEQ ID NO:13 | - |
| FGF-5 | MM007 | RTQPYPL (SEQ ID NO:670) | BBIt-A13I-L29P-F50T-V52A | MM007-Q-BBIt-FGF-5 of SEQ ID NO:432 | ++ |
| FGF-5 | FGFps2 | TWIDSTP (SEQ ID NO:671) | BBIt-A13I-L29P-F50T-V52A | FGFps2-Q-BBIt-FGF-5 of SEQ ID NO:434 | ++ |
| FGF-5 | Wild-type negative control for FGF-5 binding | ALSYPAQ (SEQ ID NO:675) | BBI | sBBI SEQ ID NO:13 | - |
| TGFβ1 | PEN3 | PENINVLP (SEQ ID NO:672) | BBIt-A40L-F50N | PEN3-Q-BBIt-TGF-A40L-F50N (SEQ ID NO:677) | ++ |
| TGFβ1 | PEN3 | PENINVLP (SEQ ID NO:672) | BBIt-A13I-L29P-F50T-V52A | PEN3-Q-BBIt-TGF (SEQ ID NO:443) | ++ |
| TGFβ1 | MM021 W | KHNVDWL (SEQ ID NO:673) | BBIt-A13I-L29P-F50T-V52A | MM021W-Q-BBIt-TGF (SEQ ID NO:445) | ++ |
| TGFβ1 | WTQ | WTQHIHN (SEQ ID NO:674) | BBIt-A13I-L29P-F50T-V52A | WTQ-BBIt-Q-TGF (SEQ ID NO:447) | ++ |
| TGFβ1 | FGFps2 Negative control for TGFβ binding | TWIDSTP (SEQ ID NO:671) | BBIt-A13I-L29P-F50T-V52A | FGFps2-Q-BBIt-FGF-5 of SEQ ID NO:434 | - |
| TNFα | T1 | RYWQDIP (SEQ ID NO:474) | BBIt-A13I-S25R-M27A-L29P-S31A-F50K-V52T | BBIt-TNF-T1 SEQ ID NO:637 | ++ |
| TNFα | T2 | APEPILA (SEQ ID NO:475) | BBIt-A13I-S25R-M27A-L29P-S31A-F50K-V52T | BBIt-TNF-T2 SEQ ID NO:638 | + |
| TNFα | T3 | DMIMVSI (SEQ ID NO:476) | BBIt-A13I-S25R-M27A-L29P-S31A-F50K-V52T | BBIt-TNF-T3 SEQ ID NO:639 | + |
| TNFα | V1 Negative control for TNFα binding | SKHSQIT (SEQ ID NO:468) | BBIt-A13I-S25R-M27A-L29P-S31A- F50K-V52T | BBIt-AV of SEQ ID NO:491 | - |

| | | | | | |
|---|---|---|---|---|---|
| ^{•}Binding peptide used to replace the chymotrypsin loop of BBIt scaffold. ^{▲}Amino acid sequence of the binding peptide that replaces amino acids 42-48 of the BBIt of SEQ ID NO:187. *(+++) indicates an IC50 value of about 2 - 10 µM; (++) indicates an IC50 value of about 10 - 50 µM; (+) indicates an IC50 value of about 50 - 250 µM; and (-) indicates an IC50 value greater than about 250 µM | | | | | |

These data show that modified variant BBPIs in which the chymotrypsin loop is replaced by different VEGF binding peptides, and which further comprise at least one amino acid substitution in the backbone of the scaffold, specifically bind their target protein i.e.VEGF. Similarly, the modified variant BBPIs in which the chymotrypsin loop is replaced by other variant peptides e.g. FGF-5, TGFβ and TNFα, also specifically bind their corresponding target proteins *i.e.* FGF, TGF and TNF. In addition, other BBPI scaffolds *i.e.* BBtc, BBsb3 and BBdb in which the chymotrypsin loop has been replaced by a variant peptide *e.g.* VEGF variant peptide, are also capable of specifically binding to the VEGF target protein.

In conclusion, these data show that modified variant BBPI scaffolds comprising at least one amino acid substitution and carrying different variant peptides in place of the chymotrypsin loop retain the ability to bind the target protein that is bound by the free (ungrafted) binding peptide. Thus, the data indicate that different binding peptide sequences that were previously shown to bind their corresponding target protein can be used to replace the chymotrypsin inhibitory loop of a modified BBI protein and be expected to bind the cognate target protein.

### EXAMPLE 16

### Identification of peptide T-cell epitopes in wt BBI

The i-mune® assay was performed for the identification of peptide T-cell epitopes in wild type BBI using naïve human T-cells, as described in WO9953038A2 and Stickler et al, 2000 (J. Immunotherapy, 23(6), 654-660, [2000]). Peptides for use in the assay were prepared based on the wild-type BBI amino acid sequence DDESSKPCCDQCACTKSNPPQCRCSDMRLNSCHSACKSCICALSYPAQCFCVDIT DFCYEPCKPSEDDKEN (SEQ ID NO:13). From the full length amino acid sequence of BBI, 15mer peptides were synthetically prepared by Mimotopes US West (San Diego, CA). Each 15mer peptide sequence was designed to overlap with the previous and subsequent 15mer, except for three residues. The 20 peptides used to screen for T-cell epitopes, corresponding to wild type BBI sequence, were:

| | |
|---|---|
| P1 | DDESSKPCCDQCACT (P1; SEQ ID NO:649) |
| P2 | SSKPCCDQCACTKSN (P2; SEQ ID NO:650) |
| P3 | PCCDQCACTKSNPPQ (P3; SEQ ID NO:651) |
| P4 | DQCACTKSNPPQCRC (P4; SEQ ID NO:652) |
| P5 | ACTKSNPPQCRCSDM (P5; SEQ ID NO:653) |
| P6 | KSNPPQCRCSDMRLN (P6; SEQ ID NO:654) |
| P7 | PPQCRCSDMRLNSCH (P7; SEQ ID NO:655) |
| P8 | CRCSDMRLNSCHSAC (P8; SEQ ID NO:656) |
| P9 | SDMRLNSCHSACKSC (P9; SEQ ID NO:657) |
| P10 | RLNSCHSACKSCICA (P10; SEQ ID NO:658) |
| P11 | SCHSACKSCICALSY (P11; SEQ ID NO:659) |
| P12 | SACKSCICALSYPAQ (P12; SEQ ID NO:660) |
| P13 | KSCICALSYPAQCFC (P13; SEQ ID NO:661) |
| P14 | ICALSYPAQCFCVDI (P14; SEQ ID NO:662) |
| P15 | LSYPAQCFCVDITDF (P15; SEQ ID NO:663) |
| P16 | PAQCFCVDITDFCYE (P16; SEQ ID NO:664) |
| P17 | CFCVDITDFCYEPCK (P17; SEQ ID NO:665) |
| P18 | VDITDFCYEPCKPSE (P18; SEQ ID NO:666) |
| P19 | TDFCYEPCKPSEDDK (P19; SEQ ID NO:667) |
| P20 | FCYEPCKPSEDDKEN (20; SEQ ID NO:668) |

Briefly, human CD4+ T cells were co-cultured with dendritic cells and peptide for the intact peptide set, using the peptides shown in Figure Y. Cytokine responses were averaged within each experiment as described in Strickler et al, 2000 and a response to a peptide was tabulated as positive if the stimulation index (SI) was greater than 2.95 (Figure 18A). SI values for each donor were compiled for each peptide set and the percent of donors responsive to each peptide is shown in Figure 18B. The percent background response, 2.89%, was determined as the average percent responders over all the peptides in the set. The standard deviation for this dataset was 2.21%. Major epitopes are defined as having a percentage response that is 3-fold or more than the background. Based on the data shown in Figures 18A and 18B, none of the peptide responses were significant based on the statistical method for an unexposed donor population with a low background response rate.

Therefore, these data indicate that the wild type BBI molecule may be considered to have low immunogenicity potential in humans.

### EXAMPLE 17

### Hair Growth Inhibition

In this Example, experiments to determine the ability of the compositions of the present invention to inhibit the growth of hair are described. In particular, these experiments are conducted in order to assess the ability of the compositions of the present invention to decrease hair growth after depilation by shaving or use of depilatory creams or waxing.

A lotion for inhibiting hair growth and containing a modified variant BBPI in which the chymotrypsin loop of the parent BBPI is replaced with a VEGF-binding peptide is prepared according to the following formulation (A):

| **Ingredient (INCI name)** | **% in weight** |
|---|---|
| Water | 85.97 |
| Cetearyl alcohol | 8.50 |
| Ceteareth-20 | 2.70 |
| PPG-15 Stearyl Ether | 0.60 |
| Prunus Amygdalus Dulcis Oil (1) | 0.10 |
| Paraffinum Liquidum | 0.10 |
| Allantoin | 0.20 |
| Propylene glycol | 0.13 |
| CI77891 Titanium dioxide | 0.17 |
| Tetra sodium EDTA | 0.10, |
| VEGF-BBPI | 0.10 |
| Sodium Chloride | 0.091 |
| Citric acid | 0.11 |
| Sodium Hydroxyde | 0.034 |
| Phenoxyethanol | 1.0 |
| Robertet Artlande G10029876 (2) | 0.10 |

The composition includes at least one type of VEGF-BBPI chosen from SEQ ID NOS: 601, 602, 627-631, 491, 632-636. In some embodiments, the composition comprises at least two, at least three, at least four or at least five different types of VEGF-BBPIs chosen from SEQ ID NOS:601, 602, 627-631, 491, 632-636.

The formulation comprising the VEGF-BBPI is manufactured as follows:
1) Blending the fatty alcohol emulsifier and oil gelling agent together into a molten phase at a temperature of 60, preferably 70°C or more,
2) emulsifying the molten phase into an aqueous phase, the temperature of the aqueous phase prior to emulsification being 50°C, preferably 60°C, more preferably 70°C or more, whereby an emulsion is formed,
3) cooling the emulsion to a temperature of 35°C or less,
4) dispersing the perfume, preservative, citric acid solution buffer solution in the emulsion.
5) adding in the same manner the solution of VEGF-BBPI, finishing with the buffer solution over a period of approximately 5min.
6) Agitating the mixture for a further 10 minutes

A control formulation (B) is prepared according to the method described for the preparation of formulation A but excluding the VEGF-BBPI.

### Facial Hair Experiments

In these experiments, a group (*e.g*., 5) male subjects with Fitzpatrick Skin Classification II are tested. Individuals are requested to use no topical facial treatment prior to beginning the experiments. On day 1, facial hair growth is visually evaluated and photographed. Following this evaluation and photography, the composition(s) to be tested, as well as a vehicle control are applied at the desired concentration(s). Beginning on day 2, the individuals apply the composition(s) immediately after shaving. No other pre- or post-shave treatment is used for the duration of the experiments. In most cases, the experiment continues for a time period of 30 to 45 days. Facial hair growth is visually evaluated and photographed every third day during the experiments. The number of hairs, as well as the hair shaft length and width are measured using computerized image analysis. In preferred embodiments, there is a decrease in the number of hairs, hair thickness and/or hair length due to the application of the test compound(s).

### Leg Hair Experiments

In these experiments, a group (*e.g*., 5) female subjects with Fitzpatrick Skin Classification II are tested. Individuals are requested to use no topical leg treatment prior to beginning the experiments. On day 1, areas on both legs of each individual are marked and the hair growth is visually evaluated and photographed. Following this evaluation and photography, the composition(s) to be tested are applied at the desired concentration(s). Following this evaluation and photography, the composition(s) to be tested (*i.e.,* test compounds containing a desired concentration of VEGF-BBP), as well as a vehicle control, are applied at the desired concentration(s). In some methods, each individual is provided with two tubes, one of which contains the VEGF-BBPI and the other containing the vehicle control. These tubes are marked "left" and "right." Each day during the experiments, the subject applies the compositions in the two tubes the respective legs. After 7 days of application, the individuals are visually evaluated and photographs are taken. Both legs are then shaved or exposed to a depilatory and the test individuals continue to apply the compositions as before. Hair growth is then evaluated visually and by photographing appropriate areas on the legs every 2 days. After 10 days, the legs are again shaved and the test subjects continue to apply the compositions as before. In some methods, the experiments are conducted for 3 cycles and the hair growth is visually evaluated and photographs were taken. The experiments are then continued for an additional 8 days. In preferred embodiments, there is a decrease in the number of hairs, hair thickness and/or hair length due to the application of the test compound(s) in the marked area(s).

Beginning on day 2, the individuals apply the composition(s) immediately after shaving. No other pre- or post-shave treatment is used for the duration of the experiments. In most cases, the experiment continues for a time period of 30 to 45 days. Facial hair growth is visually evaluated and photographed every third day during the experiments. The number of hairs, as well as the hair shaft length and width are measured using computerized image analysis. In preferred embodiments, there is a decrease in the number of hairs, hair thickness and/or hair length due to the application of the test compound(s).

## Claims

1. An isolated modified Bowman Birk Protease Inhibitor (BBPI) comprising the sequence: 
DDESSKPCCDQCACTKSNPPQCRCSDMRLNSCHSACKSCACYNLYGWTCFCVDI
TDFCYEPCKPSEDDKEN (SEQ ID NO: 187)
 wherein residues 1 to 41 and 49 to 66 of SEQ ID NO: 187 represent a backbone and residues 42 to 48 of SEQ ID NO: 187 represent a binding peptide;
wherein said modified BBPI further comprises at least one substitution at a position chosen from positions equivalent to 1, 4, 5, 11, 13, 18, 25, 27, 29, 31, 38, 40, 50, 52, 55, and 65 of SEQ ID NO:187;
wherein, if a substitution is present at position 40, that substitution is chosen from 40H, 40K, 40Q, 40R, and 40Y;
wherein said modified BBPI has increased trypsin inhibitory activity and/or production yield compared to the unmodified BBPI lacking said at least one substitution;
and wherein the binding peptide is optionally replaced by an alternative binding peptide which is a VEGF-binding peptide, FGF-5-binding peptide, TGFβ-binding peptide or TNFα-binding peptide.

2. The isolated modified BBPI of Claim 1, wherein said binding peptide is:
a VEGF binding peptide chosen from KYYLYWW (SEQ ID NO:458), TLWKSYW (SEQ ID NO:459), DLYWW (SEQ ID NO:460),SKHSQIT (SEQ ID NO:468) KTNPSGS (SEQ ID NO:469) RPTGHSL (SEQ ID NO:470), KHSAKAE (SEQ ID NO:471) KPSSASS (SEQ ID NO:472), PVTKRVH (SEQ ID NO:473), TLHWWVT (SEQ ID NO:492), PYKASFY (SEQ ID NO:493), PLRTSHT (SEQ ID NO:494), EATPROT (SEQ ID NO:495), NPLHTLS (SEQ ID NO:496), KHERIWS (SEQ ID NO:497), ATNPPPM (SEQ ID NO:498), STTSPNM (SEQ ID NO:499), ADRSFRY (SEQ ID NO:500), PKADSKQ (SEQ ID NO:501), PNQSHLH (SEQ ID NO:502), SGSETWM (SEQ ID NO:503), ALSAPYS (SEQ ID NO:504), KMPTSKV (SEQ ID NO:505), ITPKRPY (SEQ ID NO:506), KWIVSET (SEQ ID NO:507), PNANAPS (SEQ ID NO:508), NVQSLPL (SEQ ID NO:509), TLWPTFW (SEQ ID NO:510), NLWPHFW (SEQ ID NO:511), SLWPAFW (SEQ ID NO:512), SLWPHFW (SEQ ID NO:513), APWNSHI (SEQ ID NO:514), APWNLHI (SEQ ID NO:515), LPSWHLR (SEQ ID NO:516), PTILEWY (SEQ ID NO:517), TLYPQFW (SEQ ID NO:518), HLAPSAV (SEQ ID NO:519), KYYLSWW (SEQ ID NO:520), WYTLYKW (SEQ ID NO:521), TYRLYWW (SEQ ID NO:522), RYSLYYW (SEQ ID NO:523), YYLYYWK (SEQ ID NO:524), NYQLYGW (SEQ ID NO:525), TKWPSYW (SEQ ID NO:226), TLWKSYW (SEQ ID NO:527), PLWPSYW (SEQ ID NO:528), RLWPSYW (SEQ ID NO:529), TLWPKYW (SEQ ID NO:530), KYDLYWW (SEQ ID NO;531), RYDLYWW (SEQ ID NO:532), DYRLYWW (SEQ ID NO:533), DYKLYWW (SEQ ID NO:534), EYKLYWW (SEQ ID NO:535), and RYPLYWW (SEQ ID NO:536);
an FGF-5 binding peptide chosen from TNIDSTP(SEQ ID NO:544), HLQTTET (SEQ ID NO:545), SLNNLTV (SEQ ID NO:546), TNIDSTP (SEQ ID NO:547), TNIDSTP (SEQ ID NO:548), LRILANK (SEQ ID NO:549), LLTPTLN (SEQ ID NO:550), ALPTHSN (SEQ ID NO:551), TNIDSTP (SEQ ID NO:552), LCRRFEN (SEQ ID NO:553), TNIDSTP (SEQ ID NO:554), TNIDSTP (SEQ ID NO:555), HLQTTET (SEQ ID NO:556), PLGLCPP (SEQ ID NO:557), GYFIPSI (SEQ ID NO:558), TKIDSTP (SEQ ID NO:559), HLQTTET (SEQ ID NO:560), WNIDSTP (SEQ ID NO:561), TWIDWTP (SEQ ID NO:562), RTQPYPL (SEQ ID NO:670) and TWIDSTP (SEQ ID NO:671);
a TGFβ binding peptide chosen from QSACIVYYVGRKPKVECASSD (SEQ ID NO:566), QSACILYYIGKTPKIECASSD (SEQ ID NO:567), QSACILYYVGRTPKVECASSD (SEQ ID NO:568), KHNVRLL (SEQ ID NO:570), NDTPSYF (SEQ ID NO:571), AKLYAGS (SEQ ID NO:572), RGPAHSL (SEQ ID NO:573), NSLAERR (SEQ ID NO:574), HPLASPH (SEQ ID NO:575), QPWNKLK (SEQ ID NO:576), PTKPAQQ (SEQ ID NO:578), PSLNRPQ (SEQ ID NO:579), HHARQEW (SEQ ID NO:580), RHHTPGP (SEQ ID NO:581), ASAINPH (SEQ ID NO:582), PENINVLP (SEQ ID NO;672) and KHNVDWL (SEQ ID NO:673); or
a TNFα binding peptide chosen from RYWQDIP (T1; SEQ ID NO:474), APEPILA (T2; SEQ ID NO:475), DMIMVSI (T3; SEQ ID NO:476), WTPKPTQ (SEQ ID NO:583), ATFPNQS (SEQ ID NO:584), ASTVGGL (SEQ ID NO:585), TMLPYRP (SEQ ID NO:586), AWHSPSV (SEQ ID NO:587), TQSFSS (SEQ ID NO:588), THKNTLR (SEQ ID NO:589), GQTHFHV (SEQ ID NO:590), LPILTQT (SEQ ID NO:591), SILPVSH (SEQ ID NO:592), SQPIPI (SEQ ID NO:593), and QPLRKLP (SEQ ID NO:594).

3. The isolated modified BBPI of Claim 1, wherein:
said substituted amino acid at position 1 is chosen from A and C;
said at least one substitution at position 4 is V;
said at least one substitution at position 5 is chosen from P and A;
said at least one substitution at position 11 is 11 G;
said at least one substitution at position 13 is chosen from 13Y, 13I, 13F, 13M, 13L, 13V, 13K, and 13R;
said at least one substitution at position 18 is chosen from 181 and 18V and 18L;
said at least one substitution at position 25 is chosen from 25K, 25N, 25W, 25I, 25A and 25R;
said at least one substitution at position 27 is chosen from 27H, 27K, 27V, 27A, and 27Q;
said at least one substitution at position 29 is chosen from 29R, 29K, and 29P;
said at least one substitution at position 31 is chosen from 31 Q, 31 H, 31 E, 31 A, 31 R, 31W, 31K and 31T;
said at least one substitution at position 38 is chosen from 38N, 38K, and 38R;
said at least one substitution at position 50 is chosen from 50R, 50Q, 50K, 50T, 50V, 50M, and 50S;
said at least one substitution at position 52 is chosen from 52K, 52T, 52R, 52Q, 52L, 52H, 52A, 52M, 52S and 52E;
said at least one substitution at position 55 is 55M; or
said at least one substitution at position 65 is chosen from 65E, 65Q, and 65D.

4. The isolated modified BBPI of Claim 1, further comprising an insert of SEQ ID NO: 389.

5. The isolated modified variant BBPI of Claim 1, wherein said modified variant BBPI is expressed as a fusion protein comprising the catalytic domain chosen from cellulase, cutinase and disulfide isomerase, e.g. wherein said modified variant BBPI is expressed as the fusion protein of SEQ ID NO:195.

6. The isolated modified BBPI of Claim 1, wherein said modified variant BBPI comprises:
(i) a combination of two amino acid substitutions at amino acid positions equivalent to positions 50 and 52 of SEQ ID NO:187, e.g. wherein said modified variant BBPI has the amino acid sequence of SEQ ID NO:595;
(ii) a combination of three amino acid substitutions chosen from amino acid substitutions at positions equivalent to positions 25, 29, 40, 50 and 52 of SEQ ID NO:187, e.g. wherein said combination of three amino acids is chosen from combinations 25L-50T-52A, 29P-50T-52A, 40K-50T-52A, e.g. wherein said modified variant BBPI has an amino acid sequence chosen from SEQ ID NOS:603, 607 and 609;
(iii) a combination of four amino acid substitutions chosen from amino acid substitutions at positions equivalent to positions 13, 29, 50 and 52 of SEQ ID NO:187, e.g. wherein said combination of four amino acids is chosen from combinations 13I-25L-50T-52A, 13I-29P-50T-52A, 13I-A0K-50T-52A, 25L-29P-50T-52A, 25L-40K-50T-52A, and 29P-40K-50T-52A, e.g. wherein said modified variant BBPI has an amino acid sequence chosen from SEQ ID NOS:596, 600, 602, 604, 606 and 608;
(iv) a combination of five amino acid substitutions chosen from amino acid substitutions at positions equivalent to positions 13, 25, 29, 40, 50, and 52 of SEQ ID NO:187, e.g. wherein said combination of five amino acids is chosen from combinations 13I-25L-29P-50T-52A, 13I-25L-40K-50T-52A, A13I-29P-40K-50T-52A, 25L-29P-40K-50T-52A, 13I-29P-40K-50K-52A, and 13I-29P-40K-50T-52T, e.g. wherein said modified variant BBPI has an amino acid sequence chosen from SEQ ID NOS:432, 434, 443, 445, 446, 597, 599. 601, 605, 615, 620, 624, and 625;
(v) a combination of six amino acid substitutions chosen from amino acid substitutions at positions equivalent to positions 1, 4, 5, 11, 13, 25, 29, 40, 50 and 52 of SEQ ID NO:187, e.g. wherein said combination of six amino acids is chosen from combinations 13I-25L-29P-40K-50T-52A, 1C-13I-29P-40K-50T-52A, 4V-13I-29P-40K-50T-52A, 5P-13I-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A, and 13I-29P-40K-50T-52A-65E, e.g. wherein said modified variant BBPI has an amino acid sequence chosen from SEQ ID NOS:598, 611, 612, 613, 614, 616, 619, 621, 622, 623, and 626;
(vi) a combination of seven amino acid substitutions chosen from amino acid substitutions at positions equivalent to positions 13, 25, 29, 31, 40, 50 and 52 of SEQ ID NO:187, e.g. wherein said combination of seven amino acids is chosen from combinations 13L-25R-29P-31A-40K-50T-52A, 13L-25R-29P-31R-40K-50T-52A and 13I-25R-27A-29P-31A-50K-52T, e.g. wherein said modified variant BBPI has an amino acid sequence chosen from SEQ ID NOS:491, 617, 618, and 632-639; or
(vii) a combination of eight amino acid substitutions chosen from amino acid substitutions at positions equivalent to positions 13, 25, 27, 29, 31, 40, 50 and 52 of SEQ ID NO:187, e.g. wherein said combination of eight amino acids is chosen from combinations 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L and 13I-25K-27Q-29P-31E-40H-50R-52Q, e.g. wherein said modified variant BBPI has an amino acid sequence chosen from SEQ ID NOS:627-631.

7. An isolated modified BBPI according to claim 1, wherein the binding peptide binds to FGF-5, TGFβ or TNFα, e.g. wherein said variant peptide is an FGF5 binding peptide chosen from SEQ ID NOS: 670 and 671, a TGFβ binding peptide chosen from SEQ ID NOS: 672 and 673, or a TNFα binding peptide chosen from SEQ ID NOS:474, 475, and 476, e.g. wherein said modified Bowman Birk Inhibitor is chosen from SEQ ID NO:432, 434, 443, 445, 447, 637, 638, and 639.

8. An isolated polynucleotide encoding a modified BBPI or a fusion protein according to any one of the preceding claims.

9. An expression vector comprising a polynucleotide according to claim 8.

10. A host cell comprising the expression vector of claim 9, e.g. wherein said host cell is a *Bacillus* species host cell.

11. A method of producing a modified BBPI or fusion protein according to any one of claims 1 to 7, comprising culturing a host cell according to claim 10 under suitable culture conditions to allow expression of said modified BBPI or fusion protein, optionally recovering said modified variant BBPI, and further optionally activating said modified variant BBPI.

12. A personal care composition comprising a modified BBPI according to any one of claims 1 to 8.

13. The personal care composition of claim 12, wherein said personal care composition is:
a skin care composition selected from the group consisting of skin creams, lotions, sprays, emulsions, colloidal suspensions, foams, aerosols, liquids, gels, sera, and solids;
a skin care composition selected from moisturizing body washes, body washes, antimicrobial cleansers, skin protective creams, body lotions, facial creams,
moisturizing creams, facial cleansing emulsions, facial gels, facial sera, surfactant-based facial cleansers, facial exfoliating gels, anti-acne treatments, facial toners, exfoliating creams, facial masks, after shave balms, pre-shave balms, tanning compositions, sunscreens, dipilatories, hair growth inhibitors and radioprotectives;
a cosmetic composition chosen from pressed powder formulations, and foundations; a skin care composition for treating an angiogenic skin disorder;
a skin care composition for improving the appearance and/or condition of skin in a subject suffering from a skin disorder chosen from psoriasis, scleroderma, venous ulcers, acne, rosacea, warts, eczema, hemangiomas and lymphangiogenesis; or a hair care composition.

14. The modified BBPI of any one of claims 1 to 7 wherein the binding peptide is a VEGF-binding peptide or a TGFβ-binding peptide, for use in a method of improving the appearance and/or condition of skin in a subject suffering from a skin disorder.

15. The modified BPPI for use according to claim 14 wherein the binding peptide is a VEGF-binding peptide and the skin disorder is selected from psoriasis, scleroderma, venous ulcers, acne, rosacea, warts, eczema, hemangiomas and lymphangiogenesis.

16. The modified BPPI for use according to claim 14 wherein the binding peptide is a TGFβ-binding peptide and the skin disorder is selected from psoriasis, scleroderma and skin cancer.

17. The modified BBPI of any one of claims 1 to 7 wherein the binding peptide is a TNFα-binding peptide, for use in a method of improving the condition of skin in a subject suffering from a dermatological inflammatory disorder, e.g. psoriasis, dermatitis, eczema, acne, rosacea and hives.

18. A method of inhibiting hair growth in a subject comprising applying a composition comprising a modified BBPI of any one of claims 1 to 7 wherein the binding peptide is a VEGF-binding peptide to an area where inhibition of hair growth is desired.

19. The modified BBPI of any one of claims 1 to 7 wherein the binding peptide is a FGF5-binding peptide or a TGFβ-binding peptide, for use in a method of promoting hair growth in a subject suffering from a condition that involves hair loss, e.g. a condition selected from inflammatory alopecia, pseudopelade, scleroderma, tick bites, lichen planus, psoriasis, lupus, seborrheic dermatitis, loose hair syndrome, hemochromatosis, androgenic alopecia, alopecia areata and cancer.

20. A method of promoting hair growth in a subject comprising applying a composition comprising a modified BBPI of any one of claims 1 to 7 wherein the binding peptide is a FGF-5-binding peptide, a TGFβ-binding peptide, or a TNFα-binding peptide to the subject in an area where hair growth is desired.

## Patentansprüche

1. Isolierter modifizierter Bowman-Birk-Protease-Inhibitor (BBPI), umfassend die Sequenz: 
DDESSKPCCDQCACTKSNPPQCRCSDMRLNSCHSACKSCACYNLYGWTC
FCVDITDFCYEPCKPSEDDKEN (SEQ ID NO: 187)
 wobei die Reste 1 bis 41 und 49 bis 66 von SEQ ID NO: 187 ein Grundgerüst darstellen und die Reste 42 bis 48 von SEQ ID NO: 187 ein bindendes Peptid darstellen;
wobei der modifizierte BBPI weiterhin mindestens eine Substitution an einer Position, ausgewählt aus den Positionen, äquivalent zu 1, 4, 5, 11, 13, 18, 25, 27, 29, 31, 38, 40, 50, 52, 55 und 65 von SEQ ID NO:187, umfasst;
wobei, wenn eine Substitution an Position 40 vorhanden ist, diese Substitution aus 40H, 40K, 40Q, 40R und 40Y ausgewählt ist;
wobei der modifizierte BBPI erhöhte Trypsin hemmende Aktivität und/oder Erzeugungsausbeute, verglichen mit dem unmodifizierten BBPI, dem die mindestens eine Substitution fehlt, hat;
und wobei das bindende Peptid wahlweise durch ein alternatives bindendes Peptid ersetzt ist, welches ein VEGF-bindendes Peptid, FGF-5-bindendes Peptid, TGFβ-bindendes Peptid oder TNFα-bindendes Peptid ist.

2. Isolierter modifizierter BBPI nach Anspruch 1, wobei das bindende Peptid ist: ein VEGF-bindendes Peptid, ausgewählt aus KYYLYWW (SEQ ID NO:458), TLWKSYW (SEQ ID NO:459), DLYWW (SEQ ID NO:460), SKHSQIT (SEQ ID NO:468), KTNPSGS (SEQ ID NO:469), RPTGHSL (SEQ ID NO:470), KHSAKAE (SEQ ID NO:471), KPSSASS (SEQ ID NO:472), PVTKRVH (SEQ ID NO:473), TLHWWVT (SEQ ID NO:492), PYKASFY (SEQ ID NO:493), PLRTSHT (SEQ ID NO:494), EATPROT (SEQ ID NO:495), NPLHTLS (SEQ ID NO:496), KHERIWS (SEQ ID NO:497), ATNPPPM (SEQ ID NO:498), STTSPNM (SEQ ID NO:499), ADRSFRY (SEQ ID NO:500), PKADSKQ (SEQ ID NO:501), PNQSHLH (SEQ ID NO:502), SGSETWM (SEQ ID NO:503), ALSAPYS (SEQ ID NO:504), KMPTSKV (SEQ ID NO:505), ITPKRPY (SEQ ID NO:506), KWIVSET (SEQ ID NO:507), PNANAPS (SEQ ID NO:508), NVQSLPL (SEQ ID NO:509), TLWPTFW (SEQ ID NO:510), NLWPHFW (SEQ ID NO:511), SLWPAFW (SEQ ID NO:512), SLWPHFW (SEQ ID NO:513), APWNSHI (SEQ ID NO:514), APWNLHI (SEQ ID NO:515), LPSWHLR (SEQ ID NO:516), PTILEWY (SEQ ID NO:517), TLYPQFW (SEQ ID NO:518), HLAPSAV (SEQ ID NO:519), KYYLSWW (SEQ ID NO:520), WYTLYKW (SEQ ID NO:521), TYRLYWW (SEQ ID NO:522), RYSLYYW (SEQ ID NO:523), YYLYYWK (SEQ ID NO:524), NYQLYGW (SEQ ID NO:525), TKWPSYW (SEQ ID NO:226), TLWKSYW (SEQ ID NO:527), PLWPSYW (SEQ ID NO:528), RLWPSYW (SEQ ID NO:529), TLWPKYW (SEQ ID NO:530), KYDLYWW (SEQ ID NO:531), RYDLYWW (SEQ ID NO:532), DYRLYWW (SEQ ID NO:533), DYKLYWW (SEQ ID NO:534), EYKLYWW (SEQ ID NO:535) und RYPLYWW (SEQ ID NO:536);
ein FGF-5-bindendes Peptid, ausgewählt aus TNIDSTP (SEQ ID NO:544), HLQTTET (SEQ ID NO:545), SLNNLTV (SEQ ID NO:546), TNIDSTP (SEQ ID NO:547), TNIDSTP (SEQ ID NO:548), LRILANK (SEQ ID NO:549), LLTPTLN (SEQ ID NO:550), ALPTHSN (SEQ ID NO:551), TNIDSTP (SEQ ID NO:552), LCRRFEN (SEQ ID NO:553), TNIDSTP (SEQ ID NO:554), TNIDSTP (SEQ ID NO:555), HLQTTET (SEQ ID NO:556), PLGLCPP (SEQ ID NO:557), GYFIPSI (SEQ ID NO:558), TKIDSTP (SEQ ID NO:559), HLQTTET (SEQ ID NO:560), WNIDSTP (SEQ ID NO:561), TWIDWTP (SEQ ID NO:562), RTQPYPL (SEQ ID NO:670) und TWIDSTP (SEQ ID NO:671);
ein TGFβ-bindendes Peptid, ausgewählt aus QSACIVYYVGRKPKVECASSD (SEQ ID NO:566), QSACILYYIGKTPKIECASSD (SEQ ID NO:567), QSACILYYVGRTPKVECASSD (SEQ ID NO:568), KHNVRLL (SEQ ID NO:570), NDTPSYF (SEQ ID NO:571), AKLYAGS (SEQ ID NO:572), RGPAHSL (SEQ ID NO:573), NSLAERR (SEQ ID NO:574), HPLASPH (SEQ ID NO:575), QPWNKLK (SEQ ID NO:576), PTKPAQQ (SEQ ID NO:578), PSLNRPQ (SEQ ID NO:579), HHARQEW (SEQ ID NO:580), RHHTPGP (SEQ ID NO:581), ASAINPH (SEQ ID NO:582), PENINVLP (SEQ ID NO:672) und KHNVDWL (SEQ ID NO:673);
oder
ein TNFα-bindendes Peptid, ausgewählt aus RYWQDIP (T1; SEQ ID NO:474), APEPILA (T2; SEQ ID NO:475), DMIMVSI (T3; SEQ ID NO:476), WTPKPTQ (SEQ ID 25 NO:583), ATFPNQS (SEQ ID NO:584), ASTVGGL (SEQ ID NO:585), TMLPYRP (SEQ ID NO:586), AWHSPSV (SEQ ID NO:587), TQSFSS (SEQ ID NO:588), THKNTLR (SEQ ID NO:589), GQTHFHV (SEQ ID NO:590), LPILTQT (SEQ ID NO:591), SILPVSH (SEQ ID NO:592), SQPIPI (SEQ ID NO:593) und QPLRKLP (SEQ ID NO:594).

3. Isolierter modifizierter BBPI nach Anspruch 1, wobei:
die substituierte Aminosäure an Position 1 aus A und C ausgewählt ist;
die mindestens eine Substitution an Position 4 V ist;
die mindestens eine Substitution an Position 5 aus P und A ausgewählt ist;
die mindestens eine Substitution an Position 11 11G ist;
die mindestens eine Substitution an Position 13 aus 13Y, 131, 13F, 13M, 13L, 13V, 13K und 13R ausgewählt ist;
die mindestens eine Substitution an Position 18 aus 181 und 18V und 18L ausgewählt ist;
die mindestens eine Substitution an Position 25 aus 25K, 25N, 25W, 251, 25A und 25R ausgewählt ist;
die mindestens eine Substitution an Position 27 aus 27H, 27K, 27V, 27A und 27Q ausgewählt ist;
die mindestens eine Substitution an Position 29 aus 29R, 29K und 29P ausgewählt ist;
die mindestens eine Substitution an Position 31 aus 31Q, 31H, 31E, 31A, 31R, 31 W, 31K und 31T ausgewählt ist;
die mindestens eine Substitution an Position 38 aus 38N, 38K und 38R ausgewählt ist;
die mindestens eine Substitution an Position 50 aus 50R, 50Q, 50K, 50T, 50V, 50M und 50S ausgewählt ist;
die mindestens eine Substitution an Position 52 aus 52K, 52T, 52R, 52Q, 52L, 52H, 52A, 52M, 52S und 52E ausgewählt ist;
die mindestens eine Substitution an Position 55 55M ist; oder
die mindestens eine Substitution an Position 65 aus 65E, 65Q und 65D ausgewählt ist.

4. Isolierter modifizierter BBPI nach Anspruch 1, weiterhin umfassend ein Insert von SEQ ID NO: 389.

5. Isolierter modifizierter varianter BBPI nach Anspruch 1, wobei der modifizierte variante BBPI als ein Fusionsprotein, umfassend die katalytische Domäne, ausgewählt aus Cellulase, Cutinase und Disulfid-Isomerase, exprimiert wird, z.B. wobei der modifizierte variante BBPI als das Fusionsprotein von SEQ ID NO:195 exprimiert wird.

6. Isolierter modifizierter BBPI nach Anspruch 1, wobei der modifizierte variante BBPI umfasst:
(i) eine Kombination von zwei Aminosäuresubstitutionen an den Aminosäurepositionen, äquivalent zu den Positionen 50 und 52 von SEQ ID NO: 187, z.B. wobei der modifizierte variante BBPI die Aminosäuresequenz von SEQ ID NO:595 hat;
(ii) eine Kombination von drei Aminosäuresubstitutionen, ausgewählt aus den Aminosäuresubstitutionen an Positionen, äquivalent zu den Positionen 25, 29, 40, 50 und 52 von SEQ ID NO: 187, z.B. wobei die Kombination von drei Aminosäuren aus den Kombinationen 25L-50T-52A, 29P-50T-52A, 40K-50T-52A ausgewählt ist, z.B. wobei der modifizierte variante BBPI eine Aminosäuresequenz, ausgewählt aus den SEQ ID NOS:603, 607 und 609, hat;
(iii) eine Kombination von vier Aminosäuresubstitutionen, ausgewählt aus den Aminosäuresubstitutionen an Positionen, äquivalent zu den Positionen 13, 29, 50 und 52 von SEQ ID NO:187, z.B. wobei die Kombination von vier Aminosäuren aus den Kombinationen 13I-25L-50T-52A, 13I-29P-50T-52A, 13I-A0K-50T-52A, 25L-29P-50T-52A, 25L-40K-50T-52A und 29P-40K-50T-52A ausgewählt ist, z.B. wobei der modifizierte variante BBPI eine Aminosäuresequenz, ausgewählt aus den SEQ ID NOS:596, 600, 602, 604, 606 und 608, hat;
(iv) eine Kombination von fünf Aminosäuresubstitutionen, ausgewählt aus den Aminosäuresubstitutionen an Positionen, äquivalent zu den Positionen 13, 25, 29, 40, 50 und 52 von SEQ ID NO: 187, z.B. wobei die Kombination von fünf Aminosäuren aus den Kombinationen 13I-25L-29P-50T-52A, 13I-25L-40K-50T-52A, A13I-29P-40K-50T-52A, 25L-29P-40K-50T-52A, 13I-29P-40K-50K-52A und 13I-29P-40K-50T-52T ausgewählt ist, z.B. wobei der modifizierte variante BBPI eine Aminosäuresequenz, ausgewählt aus den SEQ ID NOS:432, 434, 443, 445, 446, 597, 599, 601, 605, 615, 620, 624 und 625 hat;
(v) eine Kombination von sechs Aminosäuresubstitutionen, ausgewählt aus Aminosäuresubstitutionen an Positionen, äquivalent zu den Positionen 1, 4, 5, 11, 13, 25, 29, 40, 50 und 52 von SEQ ID NO:187, z.B. wobei die Kombination von sechs Aminosäuren aus den Kombinationen 13I-25L-29P-40K-50T-52A, 1C-13I-29P-40K-50T-52A, 4V-13I-29P-40K-50T-52A, 5P-13I-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A und 13I-29P-40K-50T-52A-65E ausgewählt ist, z.B. wobei der modifizierte variante BBPI eine Aminosäuresequenz, ausgewählt aus den SEQ ID NOS:598, 611, 612, 613, 614, 616, 619, 621, 622, 623 und 626, hat;
(vi) eine Kombination von sieben Aminosäuresubstitutionen, ausgewählt aus Aminosäuresubstitutionen an Positionen, äquivalent zu den Positionen 13, 25, 29, 31, 40, 50 und 52 von SEQ ID NO:187, z.B. wobei die Kombination von sieben Aminosäuren aus den Kombinationen 13L-25R-29P-31A-40K-50T-52A, 13L-25R-29P-31R-40K-50T-52A und 13I-25R-27A-29P-31A-50K-52T ausgewählt ist, z.B. wobei der modifizierte variante BBPI eine Aminosäuresequenz, ausgewählt aus den SEQ ID NOS:491, 617, 618 und 632-639, hat; oder
(vii) eine Kombination von acht Aminosäuresubstitutionen, ausgewählt aus Aminosäuresubstitutionen an Positionen, äquivalent zu den Positionen 13, 25, 27, 29, 31, 40, 50 und 52 von SEQ ID NO:187, z.B. wobei die Kombination von acht Aminosäuren aus den Kombinationen 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L und 13I-25K-27Q-29P-31E-40H-50R-52Q ausgewählt ist, z.B. wobei der modifizierte variante BBPI eine Aminosäuresequenz, ausgewählt aus den SEQ ID NOS:627-631, hat.

7. Isolierter modifizierter BBPI gemäß Anspruch 1, wobei das bindende Peptid an FGF-5, TGFβ oder TNFα bindet, z.B. wobei das variante Peptid ein FGF-5-bindendes Peptid, ausgewählt aus den SEQ ID NOS: 670 und 671, ein TGFβ-bindendes Peptid, ausgewählt aus den SEQ ID NOS: 672 und 673, oder ein TNFα-bindendes Peptid, ausgewählt aus den SEQ ID NOS:474, 475 und 476, ist, z.B. wobei der modifizierte Bowman-Birk-Inhibitor aus SEQ ID NO:432, 434, 443, 445, 447, 637, 638 und 639 ausgewählt ist.

8. Isoliertes Polynucleotid, codierend einen modifizierten BBPI oder ein Fusionsprotein gemäß einem der vorhergehenden Ansprüche.

9. Expressionsvektor, umfassend ein Polynucleotid gemäß Anspruch 8.

10. Wirtszelle, umfassend den Expressionsvektor nach Anspruch 9, z.B. wobei die Wirtszelle eine Wirtszelle der Spezies *Bacillus* ist.

11. Verfahren zum Erzeugen eines modifizierten BBPI oder Fusionsproteins gemäß einem der Ansprüche 1 bis 7, umfassend Kultivieren einer Wirtszelle gemäß Anspruch 10 unter geeigneten Kulturbedingungen, um die Expression des modifizierten BBPI oder Fusionsproteins, wahlweise Gewinnen des modifizierten varianten BBPI und weiterhin wahlweise Aktivieren des modifizierten varianten BBPI zu erlauben.

12. Persönliche Pflegezusammensetzung, umfassend einen modifizierten BBPI gemäß einem der Ansprüche 1 bis 8.

13. Persönliche Pflegezusammensetzung nach Anspruch 12, wobei die persönliche Pflegezusammensetzung ist:
eine Hautpflegezusammensetzung, ausgewählt aus der Gruppe, bestehend aus Hautcremes, Lotionen, Sprays, Emulsionen, kolloidalen Suspensionen, Schäumen, Aerosolen, Liquida, Gelen, Seren und Feststoffen;
eine Hautpflegezusammensetzung, ausgewählt aus feuchtigkeitsspendenden Körperwaschgelen, Körperwaschgelen, antimikrobiellen Reinigungsmitteln, Hautschutzcremes, Körperlotionen, Gesichtscremes, Feuchtigkeitscremes, Gesichtsreinigungsemulsionen, Gesichtsgelen, Gesichtsseren, auf grenzflächenaktiven Mitteln basierenden Gesichtsreinigungsmitteln, Gesichtspeeling-Gelen, anti-Akne-Behandlungen, Gesichtstonern, Peelingcremes, Gesichtsmasken, Aftershavebalsamen, Preshavebalsamen, Bräunungszusammensetzungen, Sonnenschutzmitteln, Haarentfernungsmitteln, Haarwachstumshemmstoffen und Strahlenschutzmitteln;
eine kosmetische Zusammensetzung, ausgewählt aus gepressten Pulverformulierungen und Grundierungen;
eine Hautpflegezusammensetzung zum Behandeln einer angiogenen Hauterkrankung;
eine Hautpflegezusammensetzung zum Verbessern des Aussehens und/oder Zustands der Haut bei einem Patienten, der an einer Hauterkrankung, ausgewählt aus Psoriasis, Sklerodermie, venösen Geschwüren, Akne, Rosacea, Warzen, Ekzem, Hämangiomen und Lymphangiogenese, leidet; oder
eine Haarpflegezusammensetzung.

14. Modifizierter BBPI nach einem der Ansprüche 1 bis 7, wobei das bindende Peptid ein VEGF-bindendes Peptid oder ein TGFβ**-**bindendes Peptid ist, zur Verwendung in einem Verfahren zum Verbessern des Aussehens und/oder Zustands von Haut bei einem Patienten, der an einer Hauterkrankung leidet.

15. Modifizierter BBPI zur Verwendung gemäß Anspruch 14, wobei das bindende Peptid ein VEGF-bindendes Peptid ist und die Hauterkrankung, aus Psoriasis, Sklerodermie, venösen Geschwüren, Akne, Rosacea, Warzen, Ekzem, Hämangiomen und Lymphangiogenese ausgewählt ist.

16. Modifizierter BBPI zur Verwendung gemäß Anspruch 14, wobei das bindende Peptid ein TGFβ-bindendes Peptid ist und die Hauterkrankung aus Psoriasis, Sklerodermie und Hautkrebs ausgewählt ist.

17. Modifizierter BBPI nach einem der Ansprüche 1 bis 7, wobei das bindende Peptid ein TNFα-bindendes Peptid ist, zur Verwendung in einem Verfahren zum Verbessern des Zustands der Haut bei einem Patienten, der an einer dermatologischen entzündlichen Erkrankung, z.B. Psoriasis, Dermatitis, Ekzem, Akne, Rosacea und Nesselausschlägen, leidet.

18. Verfahren zum Hemmen des Haarwachstums bei einem Patienten, umfassend Aufbringen einer Zusammensetzung, umfassend einen modifizierten BBPI nach einem der Ansprüche 1 bis 7, wobei das bindende Peptid ein VEGF-bindendes Peptid ist, auf eine Fläche, wo Hemmung des Haarwachstums gewünscht ist.

19. Modifizierter BBPI nach einem der Ansprüche 1 bis 7, wobei das bindende Peptid ein FGF-5-bindendes Peptid oder ein TGFβ-bindendes Peptid ist, zur Verwendung in einem Verfahren zum Fördern des Haarwachstums bei einem Patienten, der an einem Zustand leidet, der Haarverlust beinhaltet, z.B. einem Zustand, ausgewählt aus entzündlicher Alopezie, Pseudopelade, Sklerodermie, Zeckenbissen, Lichen planus, Psoriasis, Lupus, seborrhoischer Dermatitis, Syndrom der losen Haare, Hämochromatose, androgener Alopezie, Alopecia areata und Krebs.

20. Verfahren zum Fördern des Haarwachstums bei einem Patienten, umfassend Aufbringen einer Zusammensetzung, umfassend einen modifizierten BBPI nach einem der Ansprüche 1 bis 7, wobei das bindende Peptid ein FGF-5-bindendes Peptid, ein TGFβ-bindendes Peptid oder ein TNFα-bindendes Peptid ist, auf den Patienten in einer Fläche, wo Haarwachstum gewünscht ist.

## Revendications

1. Inhibiteur isolé modifié de la protéase de type Bowman-Birk (BBPI), comprenant la séquence: 
DDESSKPCCDQCACTKSNPPQCRCSDMRLNSCHSACKSCACYNLYGWTC
FCVDITDFCYEPCKPSEDDKEN (SEQ ID NO: 187)
 où les résidus 1 à 41 et 49 à 66 de la SEQ ID NO: 187 représentent un squelette et les résidus 42 à 48 de la SEQ ID NO: 187 représentent un peptide de liaison;
où ledit BBPI modifié comprend en outre au moins une substitution à une position choisie parmi des positions équivalentes à 1, 4, 5, 11, 13, 18, 25, 27, 29, 31, 38, 40, 50, 52, 55 et 65 de la SEQ ID NO: 187;
où, si une substitution est présente à la position 40, cette substitution est choisie parmi 40H, 40K, 40Q, 40R et 40Y;
où ledit BBPI modifié a une activité inhibitrice de trypsine et/ou un rendement de production augmentés comparé au BBPI non modifié dépourvu de ladite au moins une substitution;
et où le peptide de liaison est optionnellement remplacé par un peptide de liaison alternatif qui est un peptide de liaison au VEGF, un peptide de liaison au FGF-5, un peptide de liaison au TGFβ ou un peptide de liaison au TNFα.

2. BBPI isolé modifié selon la revendication 1, dans lequel ledit peptide de liaison est un peptide de liaison au VEGF choisi parmi KYYLYWW (SEQ ID NO: 458), TLWKSYW (SEQ ID NO: 459), DLYWW (SEQ ID NO: 460), SKHSQIT (SEQ ID NO: 468), KTNPSGS (SEQ ID NO: 469), RPTGHSL (SEQ ID NO: 470), KHSAKAE (SEQ ID NO: 471), KPSSASS (SEQ ID NO: 472), PVTKRVH (SEQ ID NO: 473), TLHWWVT (SEQ ID NO: 492), PYKASFY (SEQ ID NO: 493), PLRTSHT (SEQ ID NO: 494), EATPROT (SEQ ID NO: 495), NPLHTLS (SEQ ID NO: 496), KHERIWS (SEQ ID NO: 497), ATNPPPM (SEQ ID NO: 498), STTSPNM (SEQ ID NO: 499), ADRSFRY (SEQ ID NO: 500), PKADSKQ (SEQ ID NO: 501), PNQSHLH (SEQ ID NO: 502), SGSETWM (SEQ ID NO: 503), ALSAPYS (SEQ ID NO: 504), KMPTSKV (SEQ ID NO: 505), ITPKRPY (SEQ ID NO: 506), KWIVSET (SEQ ID NO: 507), PNANAPS (SEQ ID NO: 508), NVQSLPL (SEQ ID NO: 509), TLWPTFW (SEQ ID NO: 510), NLWPHFW (SEQ ID NO: 511), SLWPAFW (SEQ ID NO: 512), SLWPHFW (SEQ ID NO: 513), APWNSHI (SEQ ID NO: 514), APWNLHI (SEQ ID NO: 515), LPSWHLR (SEQ ID NO: 516), PTILEWY (SEQ ID NO: 517), TLYPQFW (SEQ ID NO: 518), HLAPSAV (SEQ ID NO: 519), KYYLSWW (SEQ ID NO: 520), WYTLYKW (SEQ ID NO: 521), TYRLYWW (SEQ ID NO: 522), RYSLYYW (SEQ ID NO: 523), YYLYYWK (SEQ ID NO: 524), NYQLYGW (SEQ ID NO: 525), TKWPSYW (SEQ ID NO: 226), TLWKSYW (SEQ ID NO: 527), PLWPSYW (SEQ ID NO: 528), RLWPSYW (SEQ ID NO: 529), TLWPKYW (SEQ ID NO: 530), KYDLYWW (SEQ ID NO: 531), RYDLYWW (SEQ ID NO: 532), DYRLYWW (SEQ ID NO: 533), DYKLYWW (SEQ ID NO: 534), EYKLYWW (SEQ ID NO: 535) et RYPLYWW (SEQ ID NO: 536);
un peptide de liaison au FGF-5 choisi parmi TNIDSTP (SEQ ID NO: 544), HLQTTET (SEQ ID NO: 545), SLNNLTV (SEQ ID NO: 546), TNIDSTP (SEQ ID NO: 547), TNIDSTP (SEQ ID NO: 548), LRILANK (SEQ ID NO: 549), LLTPTLN (SEQ ID NO: 550), ALPTHSN (SEQ ID NO: 551), TNIDSTP (SEQ ID NO: 552), LCRRFEN (SEQ ID NO: 553), TNIDSTP (SEQ ID NO: 554), TNIDSTP (SEQ ID NO: 555), HLQTTET (SEQ ID NO: 556), PLGLCPP (SEQ ID NO: 557), GYFIPSI (SEQ ID NO: 558), TKIDSTP (SEQ ID NO: 559), HLQTTET (SEQ ID NO: 560), WNIDSTP (SEQ ID NO: 561), TWIDWTP (SEQ ID NO: 562), RTQPYPL (SEQ ID NO: 670) et TWIDSTP (SEQ ID NO: 671);
un peptide de liaison au TGFβ choisi parmi QSACIVYYVGRKPKVECASSD (SEQ ID NO: 566), QSACILYYIGKTPKIECASSD (SEQ ID NO: 567), QSACILYYVGRTPKVECASSD (SEQ ID NO: 568), KHNVRLL (SEQ ID NO: 570), NDTPSYF (SEQ ID NO: 571), AKLYAGS (SEQ ID NO: 572), RGPAHSL (SEQ ID NO: 573), NSLAERR (SEQ ID NO: 574), HPLASPH (SEQ ID NO: 575), QPWNKLK (SEQ ID NO: 576) PTKPAQQ (SEQ ID NO: 578), PSLNRPQ (SEQ ID NO: 579), HHARQEW (SEQ ID NO: 580), RHHTPGP (SEQ ID NO: 581), ASAINPH (SEQ ID NO: 582), PENINVLP (SEQ ID NO: 672) et KHNVDWL (SEQ ID NO: 673);
ou bien
un peptide de liaison au TNFα choisi parmi RYWQDIP (T1; SEQ ID NO: 474), APEPILA (T2; SEQ ID NO: 475), DMIMVSI (T3; SEQ ID NO: 476), WTPKPTQ (SEQ ID NO: 583), ATFPNQS (SEQ ID NO: 584), ASTVGGL (SEQ ID NO: 585), TMLPYRP (SEQ ID NO: 586), AWHSPSV (SEQ ID NO: 587), TQSFSS (SEQ ID NO: 588), THKNTLR (SEQ ID NO: 589), GQTHFHV (SEQ ID NO: 590), LPILTQT (SEQ ID NO: 591), SILPVSH (SEQ ID NO: 592), SQPIPI (SEQ ID NO: 593) et QPLRKLP (SEQ ID NO: 594).

3. BBPI isolé modifié selon la revendication 1, dans lequel:
ledit acide aminé substitué à la position 1 est choisi parmi A et C;
ladite au moins une substitution à la position 4 est V;
ladite au moins une substitution à la position 5 est choisie parmi P et A;
ladite au moins une substitution à la position 11 est 11G;
ladite au moins une substitution à la position 13 est choisie parmi 13Y, 13I, 13F, 13M, 13L, 13V, 13K et 13R;
ladite au moins une substitution à la position 18 est choisie parmi 181 et 18V et 18L;
ladite au moins une substitution à la position 25 est choisie parmi 25K, 25N, 25W, 251, 25A et 25R;
ladite au moins une substitution à la position 27 est choisie parmi 27H, 27K, 27V, 27A et 27Q;
ladite au moins une substitution à la position 29 est choisie parmi 29R, 29K et 29P;
ladite au moins une substitution à la position 31 est choisie parmi 31Q, 31H, 31E, 31A, 31R, 31W, 31K et 31T;
ladite au moins une substitution à la position 38 est choisie parmi 38N, 38K et 38R;
ladite au moins une substitution à la position 50 est choisie parmi 50R, 50Q, 50K, 50T, 50V, 50M et 50S;
ladite au moins une substitution à la position 52 est choisie parmi 52K, 52T, 52R, 52Q, 52L, 52H, 52A, 52M, 52S et 52E;
ladite au moins une substitution à la position 55 est 55M; ou bien
ladite au moins une substitution à la position 65 est choisie parmi 65E, 65Q et 65D.

4. BBPI isolé modifié selon la revendication 1, comprenant en outre un insert de la SEQ ID NO: 389.

5. BBPI isolé modifié variant selon la revendication 1 , où ledit BBPI modifié variant est exprimé comme une protéine de fusion comprenant le domaine catalytique choisi parmi cellulase, cutinase et disulfure isomérase, par ex. où ledit BBPI modifié variant est exprimé comme la protéine de fusion de la SEQ ID NO: 195.

6. BBPI isolé modifié selon la revendication 1, où ledit BBPI modifié variant comprend:
(i) une combinaison de deux substitutions d'acides aminés à des positions d'acides aminés équivalentes aux positions 50 et 52 de la SEQ ID NO: 187, par ex. où ledit BBPI modifié variant a la séquence d'acides aminés de la SEQ ID NO: 595;
(ii) une combinaison de trois substitutions d'acides aminés choisies parmi des substitutions d'acides aminés à des positions équivalentes aux positions 25, 29, 40, 50 et 52 de la SEQ ID NO: 187, par ex. où ladite combinaison de trois acides aminés est choisie parmi les combinaisons 25L-50T-52A, 29P-50T-52A, 40K-50T-52A, par ex. où ledit BBPI modifié variant a une séquence d'acides aminés choisie parmi les SEQ ID NO: 603, 607 et 609;
(iii) une combinaison de quatre substitutions d'acides aminés choisies parmi des substitutions d'acides aminés à des positions équivalentes aux positions 13, 29, 50 et 52 de la SEQ ID NO: 187, par ex. où ladite combinaison de quatre acides aminés est choisie parmi les combinaisons 13I-25L-50T-52A, 13I-29P-50T-52A, 13I-A0K-50T-52A, 25L-29P-50T-52A, 25L-40K-50T-52A et 29P-40K-50T-52A, par ex. où ledit BBPI modifié variant a une séquence d'acides aminés choisie parmi les SEQ ID NO: 596, 600, 602, 604, 606 et 608;
(iv) une combinaison de cinq substitutions d'acides aminés choisies parmi des substitutions d'acides aminés à des positions équivalentes aux positions 13, 25, 29, 40, 50 et 52 de la SEQ ID NO: 187, par ex. où ladite combinaison de cinq acides aminés est choisie parmi les combinaisons 13I-25L-29P-50T-52A, 13I-25L-40K-50T-52A, A13I-29P-40K-50T-52A, 25L-29P-40K-50T-52A, 13I-29P-40K-50K-52A et 13I-29P-40K-50T-52T, par ex. où ledit BBPI modifié variant a une séquence d'acides aminés choisie parmi les SEQ ID NO: 432, 434, 443, 445, 446, 597, 599, 601, 605, 615, 620, 624 et 625;
(v) une combinaison de six substitutions d'acides aminés choisies parmi des substitutions d'acides aminés à des positions équivalentes aux positions 1, 4, 5, 11, 13, 25, 29, 40, 50 et 52 de la SEQ ID NO: 187, par ex. où ladite combinaison de six acides aminés est choisie parmi les combinaisons 13I-25L-29P-40K-50T-52A, 1C-13I-29P-40K-50T-52A, 4V-13I-29P-40K-50T-52A, 5P-131-29P-40K-50T-52A, 11G-13I-29P-40K-50T-52A, 13I-25R-29P-40K-50T-52A, 13I-27R-29P-40K-50T-52A, 13I-29P-31A-40K-50T-52A, 13I-29P-31R-40K-50T-52A, 13I-29P-38N-40K-50T-52A et 13I-29P-40K-50T-52A-65E, par ex. où ledit BBPI modifié variant a une séquence d'acides aminés choisie parmi les SEQ ID NO: 598, 611, 612, 613, 614, 616, 619, 621, 622, 623 et 626;
(vi) une combinaison de sept substitutions d'acides aminés choisies parmi des substitutions d'acides aminés à des positions équivalentes aux positions 13, 25, 29, 31, 40, 50 et 52 de la SEQ ID NO: 187, par ex. où ladite combinaison de sept acides aminés est choisie parmi les combinaisons 13L-25R-29P-31A-40K-50T-52A, 13L-25R-29P-31R-40K-50T-52A et 13I-25R-27A-29P-31A-50K-52T, par ex. où ledit BBPI modifié variant a une séquence d'acides aminés choisie parmi les SEQ ID NO: 491, 617, 618 et 632-639;
ou bien
(vii) une combinaison de huit substitutions d'acides aminés choisies parmi des substitutions d'acides aminés à des positions équivalentes aux positions 13, 25, 27, 29, 31, 40, 50 et 52 de la SEQ ID NO: 187, par ex. où ladite combinaison de huit acides aminés est choisie parmi les combinaisons 13I-25R-27A-29P-31A-40H-50K-52T, 13I-25K-27A-29R-31E-40K-50Q-52Q, 13I-25K-27R-29E-31A-40H-50R-52K, 13I-25K-27A-29R-31A-40H-50R-52L et 13I-25K-27Q-29P-31E-40H-50R-52Q, par ex. où ledit BBPI modifié variant a une séquence d'acides aminés choisie parmi les SEQ ID NO: 627-631.

7. BBPI isolé modifié selon la revendication 1, dans lequel le peptide de liaison se lie au FGF-5, TGFβ ou TNFα, par ex. où ledit peptide variant est un peptide de liaison au FGF-5 choisi parmi les SEQ ID NO: 670 et 671, un peptide de liaison au TGFβ choisi parmi les SEQ ID NO: 672 et 673 ou un peptide de liaison au TNFα choisi parmi les SEQ ID NO: 474, 475 et 476, par ex. où ledit inhibiteur de type Bowman-Birk modifié est choisi parmi les SEQ ID NO: 432, 434, 443, 445, 447, 637, 638 et 639.

8. Polynucléotide isolé codant un BBPI modifié ou une protéine de fusion selon l'une quelconque des revendications précédentes.

9. Vecteur d'expression comprenant un polynucléotide selon la revendication 8.

10. Cellule hôte comprenant le vecteur d'expression selon la revendication 9, par ex. où ladite cellule hôte est une cellule hôte de l'espèce *Bacillus.*

11. Procédé de production d'un BBPI modifié ou d'une protéine de fusion selon l'une quelconque des revendications 1 à 7, comprenant cultiver une cellule hôte selon la revendication 10 dans des conditions de culture appropriées pour permettre l'expression dudit BBPI modifié ou de ladite protéine de fusion, récupérer optionnellement ledit BBPI modifié variant et optionnellement en outre activer ledit BBPI modifié variant.

12. Composition de soins personnels comprenant un BBPI modifié selon l'une quelconque des revendications 1 à 8.

13. Composition de soins personnels selon la revendication 12, où ladite composition de soins personnels est:
une composition de soin pour la peau sélectionnée parmi le groupe consistant en crèmes, lotions, vaporisations, émulsions, suspensions colloïdales, mousses, aérosols, liquides, gels, sérums et solides pour la peau;
une composition de soin pour la peau sélectionnée parmi des produits hydratants pour le lavage du corps, produits pour le lavage du corps, nettoyants antimicrobiens, crèmes protectrices pour la peau, lotions pour le corps, crèmes pour le visage, crèmes hydratantes, émulsions nettoyantes pour le visage, gels pour le visage, sérums pour le visage, nettoyants pour le visage à base de tensioactif, gels exfoliants pour le visage, traitements anti-acné, lotions toniques pour le visage, crèmes exfoliantes, masques pour le visage, lotions après rasage, lotions avant rasage, compositions bronzantes, écrans solaires, épilatoires, inhibiteurs de croissance pileuse et radioprotecteurs;
une composition cosmétique choisie parmi des formulations de poudre compacte et des fonds de teint;
une composition de soin pour la peau pour le traitement d'un trouble angiogénique de la peau;
une composition de soin pour la peau destinée à améliorer l'aspect et/ou la condition de la peau chez un sujet souffrant d'un trouble de la peau choisi parmi le psoriasis, scléroderme, ulcères veineux, acné, rosacée, verrues, eczéma, hémangiomes et lymphangiogenèse; ou
une composition pour le soin des cheveux.

14. BBPI modifié selon l'une quelconque des revendications 1 à 7, où le peptide de liaison est un peptide de liaison au VEGF ou un peptide de liaison au TGFβ, à utiliser dans un procédé destiné à améliorer l'aspect et/ou la condition de la peau chez un sujet souffrant d'un trouble de la peau.

15. BBPI modifié à utiliser selon la revendication 14, où le peptide de liaison est un peptide de liaison au VEGF et le trouble de la peau est sélectionné parmi le psoriasis, scléroderme, ulcères veineux, acné, rosacée, verrues, eczéma, hémangiomes et lymphangiogenèse.

16. BBPI modifié à utiliser selon la revendication 14, où le peptide de liaison est un peptide de liaison au TGFβ et le trouble de la peau est sélectionné parmi le psoriasis, le scléroderme et le cancer de la peau.

17. BBPI modifié selon l'une quelconque des revendications 1 à 7, où le peptide de liaison est un peptide de liaison au TNFα, à utiliser dans un procédé destiné à améliorer la condition de la peau chez un sujet souffrant d'un trouble dermatologique inflammatoire, par ex. psoriasis, dermite, eczéma, acné, rosacée et urticaire.

18. Procédé d'inhibition de croissance pileuse chez un sujet comprenant appliquer une composition, comprenant un BBPI modifié selon l'une quelconque des revendications 1 à 7 où le peptide de liaison est un peptide de liaison au VEGF, à une zone où l'inhibition de croissance pileuse est désirée.

19. BBPI modifié selon l'une quelconque des revendications 1 à 7, où le peptide de liaison est un peptide de liaison au FGF-5 ou un peptide de liaison au TGFβ, à utiliser dans un procédé destiné à favoriser la croissance pileuse chez un sujet souffrant d'une condition qui implique une perte de cheveux, par ex. une condition sélectionnée parmi une alopécie inflammatoire, pseudopelade, scléroderme, morsures de tiques, lichen plan, psoriasis, lupus, dermite séborrhéique, syndrome des cheveux anagènes caducs, hémochromatose, alopécie androgénique, pelade et cancer.

20. Procédé destiné à favoriser la croissance pileuse chez un sujet comprenant appliquer une composition, comprenant un BBPI modifié selon l'une quelconque des revendications 1 à 7 où le peptide de liaison est un peptide de liaison au FGF-5, un peptide de liaison au TGFβ ou un peptide de liaison au TNFα, au sujet dans une zone où la croissance pileuse est désirée.
